(19) Europäisches Patentamt
European Patent Office
Office européen des brevets

(11) EP 4 137 496 A1

(12) EUROPEAN PATENT APPLICATION
published in accordance with Art. 153(4) EPC

(43) Date of publication:
**22.02.2023 Bulletin 2023/08**

(21) Application number: **21804453.5**

(22) Date of filing: **12.05.2021**

(51) International Patent Classification (IPC):
***C07D 513/10*** (2006.01) ***A61K 31/425*** (2006.01)
***A61P 25/24*** (2006.01) ***A61P 25/22*** (2006.01)
***A61P 25/28*** (2006.01) ***A61P 25/00*** (2006.01)

(52) Cooperative Patent Classification (CPC):
**A61K 31/425; A61P 25/00; A61P 25/22; A61P 25/24; A61P 25/28; C07D 513/10**

(86) International application number:
**PCT/CN2021/093264**

(87) International publication number:
**WO 2021/228123 (18.11.2021 Gazette 2021/46)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **12.05.2020 CN 202010397265**

(71) Applicant: **Beijing Greatway Pharmaceutical Technology Co., Ltd.**
**Beijing 100070 (CN)**

(72) Inventors:
- **GU, Wei**
  **Beijing 100070 (CN)**
- **DONG, Zhaoji**
  **Beijing 100070 (CN)**
- **WANG, Ruiluan**
  **Beijing 100070 (CN)**

(74) Representative: **E. Blum & Co. AG**
**Patent- und Markenanwälte VSP**
**Vorderberg 11**
**8044 Zürich (CH)**

# (54) COMPOUND FOR ADJUSTING ACTIVITY OF NMDA RECEPTOR, AND PHARMACEUTICAL COMPOSITION AND USE THEREOF

(57) Provided are a compound for adjusting the activity of an NMDA receptor, and a pharmaceutical composition and the use thereof. In particular, provided are a compound as represented by formula I, a pharmaceutically acceptable salt or ester, a stereoisomer, or a solvate thereof. The compound has the effect of enhancing the activity of the NMDA receptor, and can be used to prepare drugs for preventing/treating depression, anxiety disorder, tension, learning and cognitive defects, neuropathic pain and other diseases,

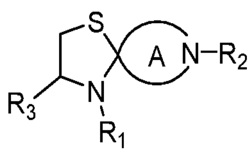

.

EP 4 137 496 A1

## Description

### Technical Field

The invention belongs to the field of pharmaceutical and chemical industry, which relates to a compound for adjusting the activity of an NMDA receptor, and a pharmaceutical composition and the use thereof. Specifically, the present invention relates to the compound capable of enhancing the activity of the NMDA receptor and use of the compound in preparation of medicaments for preventing/treating depression, anxiety, tension, learning and cognitive deficits, neuropathic pain and other diseases.

### Background Art

N-methyl-D-aspartic acid (NMDA) receptor is an ionotropic receptor for an excitatory neurotransmitter glutamate and is involved in many complex physiological and pathological mechanisms. The NMDA receptor can mediate $Ca^{2+}$ influx, enhance synaptic plasticity, and participate in learning-memory and nervous system development; when excitatory amino acid of the body is increased sharply, a large influx of $Ca^{2+}$ can be induced by stimulating the NMDA receptors, resulting in cell death. The imbalance of NMDA receptor activity regulation has been considered to be an important reason of central nervous system diseases such as neurodegenerative diseases, depression, epilepsy and ischemic brain injury.

The NMDA receptor has an important role during excitatory neurotoxic process. The excitatory neurotoxic process is characterized in that the excessive release of extracellular glutamate results in excessive activation of NMDA receptor, causes influx of $Ca^{2+}$ and $Na^+$ and $K^+$ efflux, causes activation of intracellular signaling pathway, intracellular $Ca^{2+}$ overload, and cell dysfunction, induces apoptosis or initiates cell death signal transduction pathway, and ultimately leads to neuronal death, resulting in neurodegenerative diseases, depression and other diseases, while an NMDA receptor antagonist can reduce the excitatory neurotoxic effect of cells by inhibiting the activity of NMDA receptor. In addition, by mediating $Ca^{2+}$ influx, NMDA receptor can also enhance synaptic plasticity and neuronal excitability, induce long term promotion (LTP), and promote neural cell development, and NMDA receptor plays an important role in the treatment of cognitive dysfunction caused by the diseases such as neurodegenerative diseases, depression and schizophrenia. Therefore, reasonable regulation of the activity of NMDA receptor plays an important role in the treatment of neurological diseases.

Studies show that the NMDA receptor antagonist has rapid antidepressant effects, and an NMDA receptor agonist can also regulate the sensitivity of postsynaptic AMPA by increasing the release of the neurotransmitter mediated by NMDA receptor to generate the antidepressant effects. An NMDA receptor partial agonist can exert dual (antagonist/agonist) effects on NMDA receptor through allosteric sites.

In the field of antidepressant research, an NMDA receptor modulator which is designed and synthesized, such as the NMDA partial agonist/antagonist, is of great significance for the treatment of depression by exerting partial agonistic and/or antagonistic effects on NMDA receptors.

### Contents of Invention

After intensive research and creative labor, inventors of the invention obtain a compound represented by formula I. The inventors are surprised to find that the compound of formula I or a pharmaceutically acceptable salt, a solvate thereof, or a mixture thereof can be the agonist of NMDA receptor, especially the partial agonist, and has a high affinity with NMDA receptor; while reflected in animal models, the compound can rapidly, effectively and persistently prevent and treat depression and anxiety states of the model animals.

<u>Compound of the present invention</u>

One aspect of the present invention relates to a compound represented by formula I, a pharmaceutically acceptable salt or ester, stereoisomer or solvate thereof,

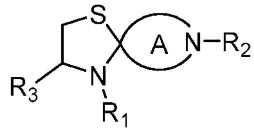

I

wherein,

Ring A is3- to 8-membered aliphatic heterocycle;
$R_1$ is selected from H, C1-C6 alkyl, aryl-C1-C4 alkyl, C2-C6 acyl, -CONRR' and natural amino acid fragments;
$R_2$ is selected from H, C1-C6 alkyl, C1-C6 alkoxycarbonyl, C2-C6 acyl, -CONRR' and natural amino acid fragments;
$R_3$ is selected from H, cyano, 3- to 8-membered nitrogen-containing aliphatic heterocycle- C1-C4 acyl, C1-C6 alkoxycarbonyl and -CONHR$_4$;
R and R' are each independently selected from H and C1-C6 alkyl;
$R_4$ is selected from H, C1-C6 alkyl, natural amino acid fragments and carboxylic acid derivatives of the said natural amino acid fragments;
optionally, the C1-C6 alkyl, aryl-C1-C4 alkyl, C2-C6 acyl, 3- to 8-membered nitrogen-containing aliphatic heterocycle-C1-C4 acyl, C1-C6 alkoxycarbonyl, -CONRR', -CONHR$_4$ and natural amino acid fragments are each independently substituted by one or more substituents selected from: halogen, amino, hydroxyl, cyano, carboxyl, nitro, C1-C6 alkyl, and C1-C6 alkoxy;
and, $R_1$, $R_2$ and $R_3$ satisfy:

(1) $R_1$, $R_2$ and $R_3$ are not H at the same time;
(2) when $R_1$ and $R_3$ are H, $R_2$ is not Boc;
(3) when the formula I is

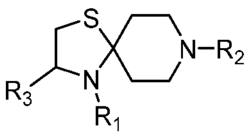
,

$R_1$ is

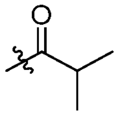
,

and $R_3$ is H; $R_2$ is not H,

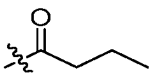, 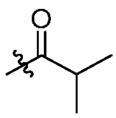, 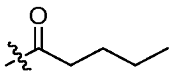, 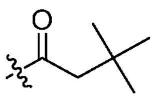, 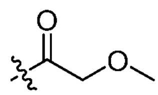, 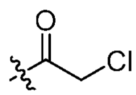, 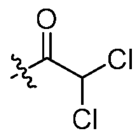,

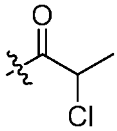, 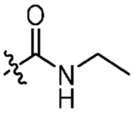, 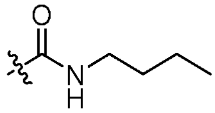, 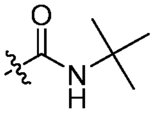 or 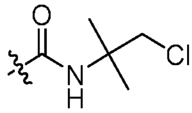;

(4) when the formula I is

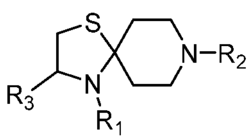
,

$R_1$ is

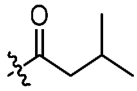,

and $R_3$ is H; $R_2$ is not C2-C6 acyl or -CONRR';
(5) when the formula I is

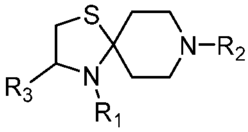,

$R_1$ and $R_3$ are H; $R_2$ is not C1-C4 alkyl, acetyl,

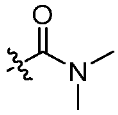 or 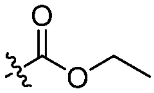;

(6) the compound of the formula I is not

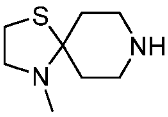, 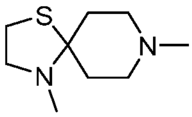, 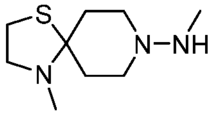,

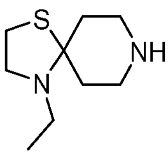, 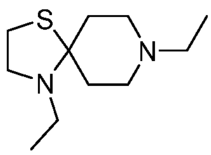, 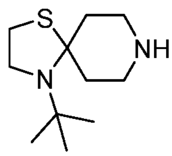, 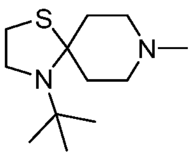, 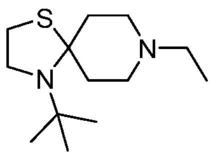,

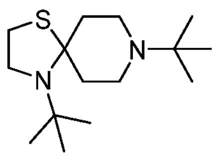, 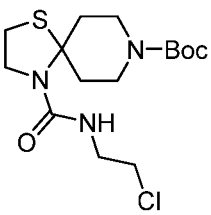, 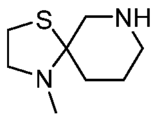, 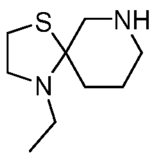, 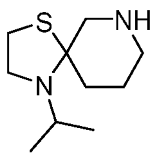 or 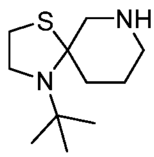.

In some embodiments, Ring A is selected from 4- to 7-membered aliphatic heterocycle, and preferably 4- to 6-membered aliphatic heterocycle.

In some embodiments, Ring A is selected from 4- to 7-membered nitrogen-containing aliphatic heterocycle, and preferably 4- to 6-membered nitrogen-containing aliphatic heterocycle.

In some embodiments, Ring A is selected from

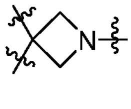, 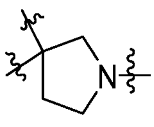, 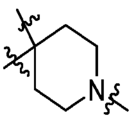,

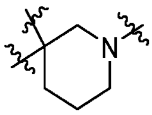, 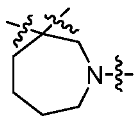 and 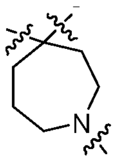.

In some embodiments, Ring A is selected from

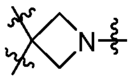, 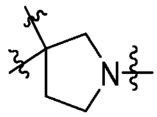, 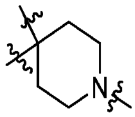

and

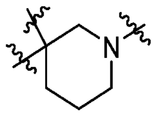.

In some embodiments, $R_1$ is selected from H, C1-C6 alkyl, phenyl-C1-C4 alkyl, C2-C6 acyl and natural amino acid fragments.

In some embodiments, $R_1$ is selected from H, C1-C6 alkyl and C2-C6 acyl.

In some embodiments, $R_1$ is H.

In some embodiments, $R_1$ is selected from carboxyl residues of natural amino acids.

In some embodiments, $R_1$ is selected from H, acetyl, methyl,

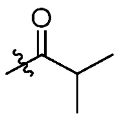, 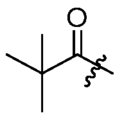,

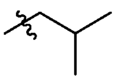, 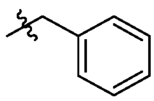, 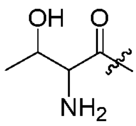, 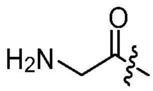, 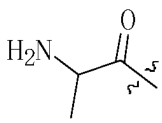, 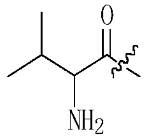, 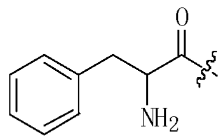,

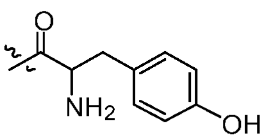, 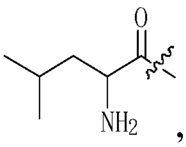, 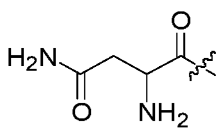 and 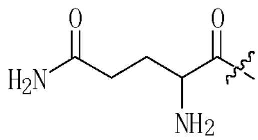.

In some embodiments, $R_1$ is selected from H, acetyl, methyl,

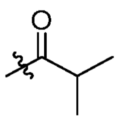, 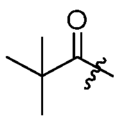,

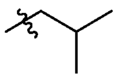, 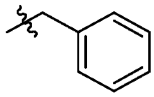, 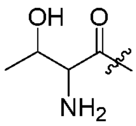, 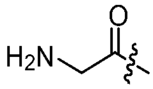, 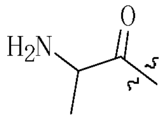, 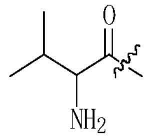, 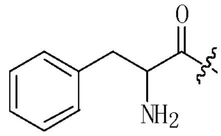,

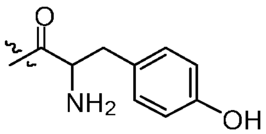, 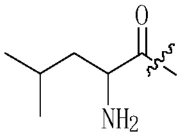 and 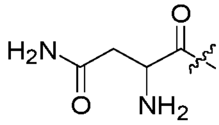.

In some embodiments, $R_1$ is selected from H, acetyl, methyl,

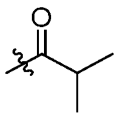, 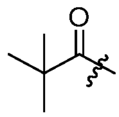,

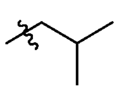, 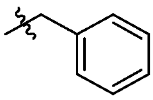, 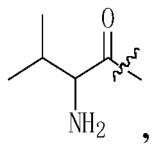, 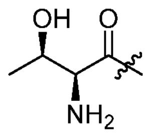 and 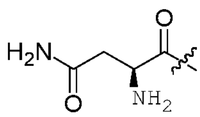.

In some embodiments, $R_2$ is selected from H, C1-C6 alkyl, C1-C6 alkoxycarbonyl, C2-C6 acyl, and natural amino acid fragments; optionally, the C1-C6 alkyl, phenyl-C1-C4 alkyl, C2-C6 acyl and natural amino acid fragments are each independently substituted by one or more substituents selected from: halogen, amino, hydroxyl, cyano, carboxyl, nitro, C1-C6 alkyl and C1-C6 alkoxy.

In some embodiments, $R_2$ is selected from H, C1-C4 alkoxycarbonyl, C2-C6 acyl and carboxyl residues of the natural amino acids.

In some embodiments, $R_2$ is selected from H, C1-C6 alkyl, C2-C6 acyl and carboxyl residues of natural amino acids.

In some embodiments, $R_2$ is selected from H, C1-C6 alkyl and C2-C6 acyl.

In some embodiments, $R_2$ is selected from carboxyl residues of natural amino acids.

In some embodiments, $R_2$ is selected from H, methyl, ethyl, propyl, butyl, tert-butoxycarbonyl, acetyl,

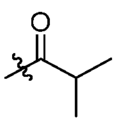, 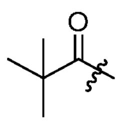, 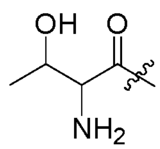, 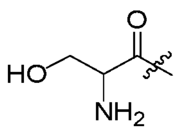, 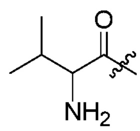,

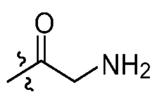, 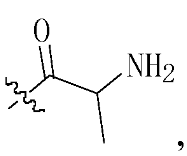, 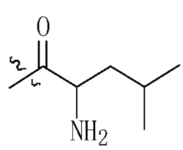, 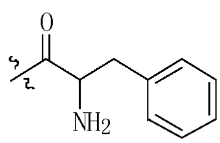, 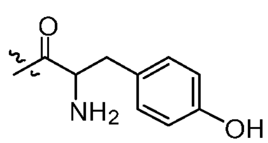, 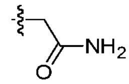,

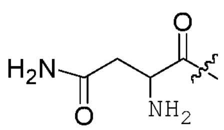 and 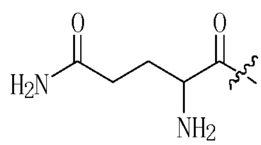.

In some embodiments, $R_2$ is selected from H, methyl, ethyl, tert-butoxycarbonyl, acetyl,

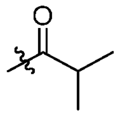, 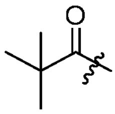, 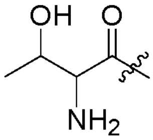, 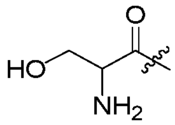, 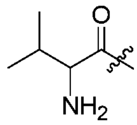, 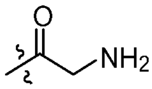, 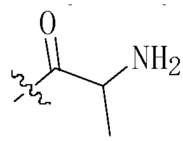,

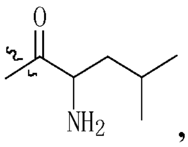, 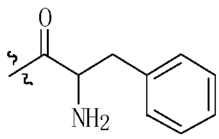, 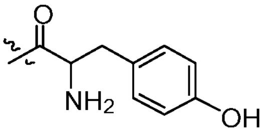, 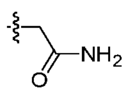 and 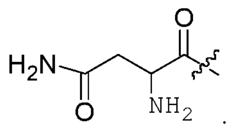.

In some embodiments, $R_2$ is selected from H, methyl, tert-butoxycarbonyl, acetyl,

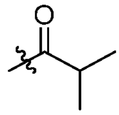, 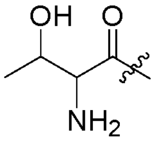, 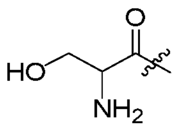 and 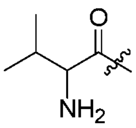.

In some embodiments, $R_3$ is selected from H, cyano, 5- to 7-membered (e.g. 6-membered) nitrogen-containing aliphatic heterocycle-C1-C3 acyl (e.g. formyl), C1-C4 alkoxycarbonyl and -CONHR$_4$; $R_4$ is selected from H, natural amino acid fragments, amide derivatives of the said natural amino acid fragments and cyano derivatives of the said natural amino acid fragments.

In some embodiments, $R_3$ is selected from H, cyano, 5- to 7-membered nitrogen-containing aliphatic heterocycle-C1-C3 acyl, C1-C4 alkoxycarbonyl and -CONHR$_4$; $R_4$ is selected from H, natural amino acid fragments and amide derivatives of the said natural amino acid fragments.

In some embodiments, $R_3$ is selected from H, cyano, 5- to 7-membered nitrogen-containing aliphatic heterocycle-C1-C3 acyl, C1-C4 alkoxycarbonyl and -CONHR$_4$; $R_4$ is selected from H and natural amino acid fragments.

In some embodiments, $R_3$ is selected from H, cyano, 5- to 7-membered nitrogen-containing aliphatic heterocycle- C1-C3 acyl, C1-C4 alkoxycarbonyl and -CONHR$_4$; $R_4$ is selected from H and amide derivatives of natural amino acid fragments.

In some embodiments, $R_3$ is H.

In some embodiments, $R_3$ is -CONHR$_4$; $R_4$ is selected from amino residues of natural amino acids, and amide derivatives of the said amino residue of natural amino acids.

In some embodiments, $R_3$ is selected from H, cyano,

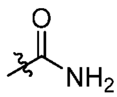, 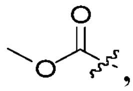, 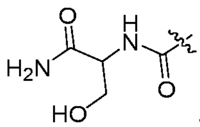,

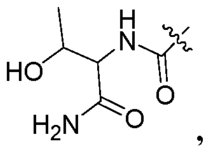, 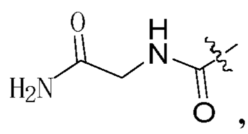, 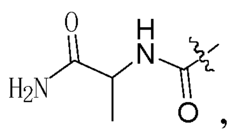, 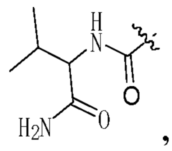, 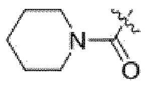, 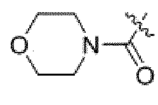,

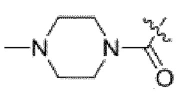, 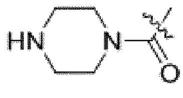 and 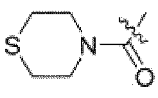.

In some embodiments, $R_3$ is selected from H, cyano,

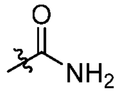, 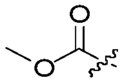, 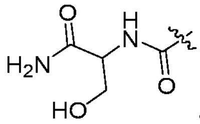,

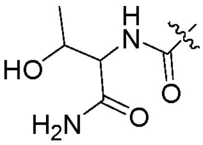, 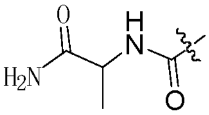, 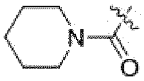, 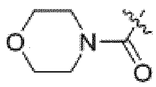, 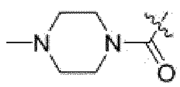, 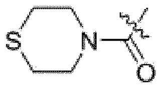

and

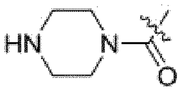.

In some embodiments, $R_3$ is selected from H, cyano, C1-C4 alkoxycarbonyl and - $CONHR_4$; $R_4$ is selected from H, natural amino acid fragments, amide derivatives of the said natural amino acid fragments, and cyano derivatives of the said natural amino acid fragments.

In some embodiments, $R_3$ is selected from H, cyano, C1-C4 alkoxycarbonyl and - $CONHR_4$; $R_4$ is selected from H, natural amino acid fragments and amide derivatives of the said natural amino acid fragments.

In some embodiments, $R_3$ is selected from H, cyano, C1-C4 alkoxycarbonyl and - $CONHR_4$; $R_4$ is selected from H and natural amino acid fragments.

In some embodiments, $R_3$ is selected from H, cyano, C1-C4 alkoxycarbonyl and - $CONHR_4$; $R_4$ is selected from H and amide derivatives of natural amino acid fragments.

In some embodiments, Ring A is selected from

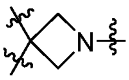, 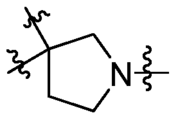, 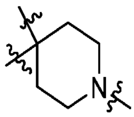,

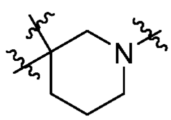, 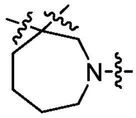, 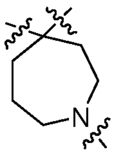;

$R_1$ is independently selected from -H, C1-C6 alkyl and C2-C6 acyl;
$R_2$ is selected from H, Boc, C2-C6 acyl and natural amino acid fragments;
$R_3$ is H.

In some embodiments, Ring A is selected from

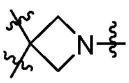, 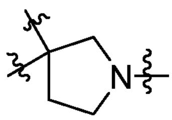, 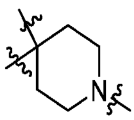,

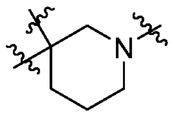, 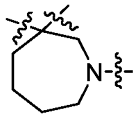, 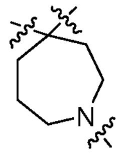;

$R_1$ is selected from natural amino acid fragments;
$R_2$ is selected from H, C1-C6 alkyl, C2-C6 acyl and natural amino acid fragments;
$R_3$ is H.

In some embodiments, Ring A is selected from

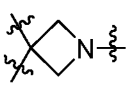, 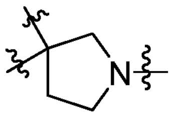, 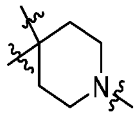,

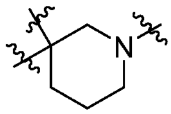, 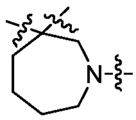, 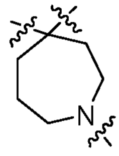;

$R_1$ is H;
$R_2$ is selected from natural amino acid fragments;
$R_3$ is selected from methyl ester, ethyl ester, propyl ester, isopropyl ester, formamide, cyano and $CONHR_4$;
$R_4$ is selected from natural amino acid fragments and carboxylic acid derivatives of the said natural amino acid fragments.

In some embodiments, Ring A is selected from

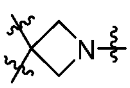, 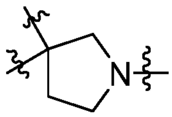, 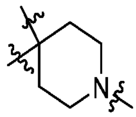,

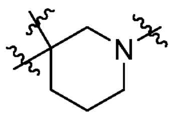, 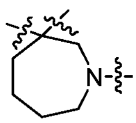, 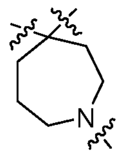;

$R_1$ is H;
$R_2$ is selected from H, C1-C6 alkyl, C2-C6 acyl;
$R_3$ is selected from $CONHR_4$;
$R_4$ is selected from natural amino acid fragments and carboxylic acid derivatives of the said natural amino acid fragments.

In some embodiments, the said natural amino acid fragments are selected from the carboxyl residues of the following amino acids: Thr, Ser, Val, Gly, Ala, Ile, Phe, Gln and Tyr.
In some embodiments, the said natural amino acid fragments are selected from the amino residues of the following amino acids: Thr, Ser, Val, Gly, Ala, Ile, Phe, Gln and Tyr.
In some embodiments, natural amino acid fragments are selected from

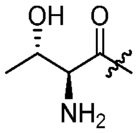,

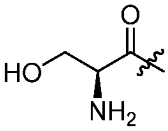, 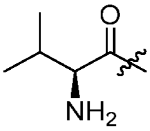, 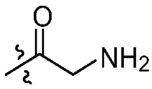, 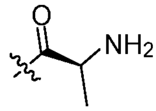, 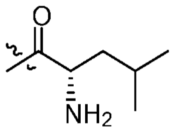, 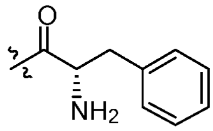,

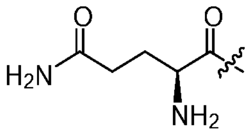 and 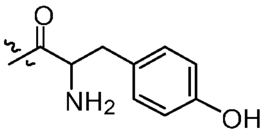.

In some embodiments, natural amino acid fragments are selected form

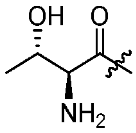,

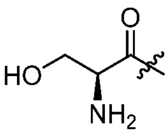, 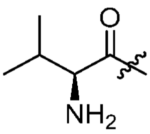, 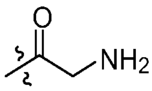, 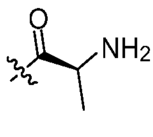, 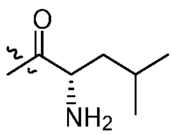, 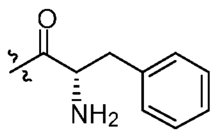 and

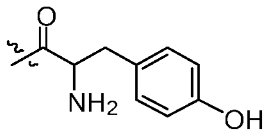.

In some embodiments, natural amino acid fragments are selected from

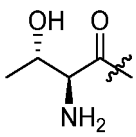,

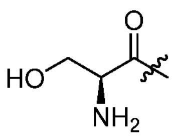 and 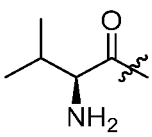.

In some embodiments, natural amino acid fragments are selected from

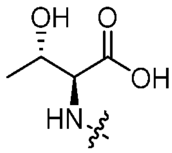,

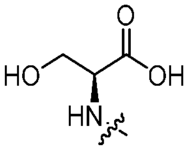, 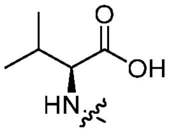, 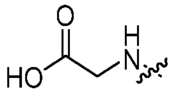, 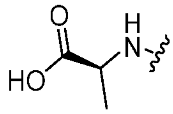, 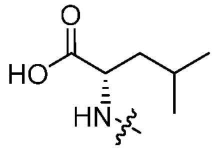,

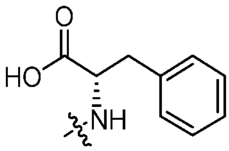, 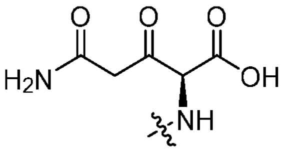 and 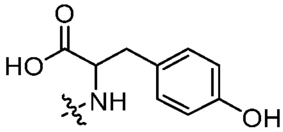.

In the embodiments of the present invention, the carboxylic acid derivative of the natural amino acid fragments is formed by replacing a part of carboxyl group in the natural amino acid fragment with other atomic group and hydrolyzed to obtain the carboxylic acid. Amide derivatives, cyano derivatives and carboxylate derivatives of the said natural amino acid fragments are included. In some embodiments, the carboxylic acid derivatives of the natural amino acid fragments include, but are not limited to, amide derivatives, cyano derivatives and carboxylate derivatives of the following amino acid fragments: Thr, Ser, Val, Gly, Ala, Ile, Phe, Gln and Tyr. In some embodiments, the carboxylic acid derivative of the natural amino acid fragments is, for example:

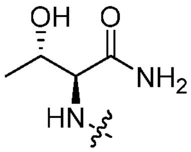, 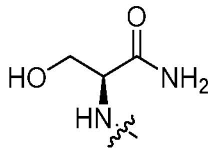,

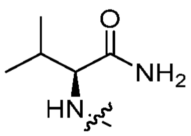, 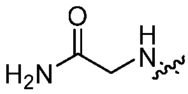, 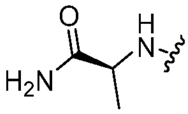, 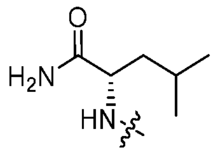, 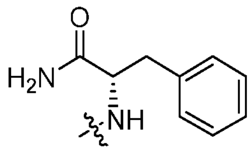,

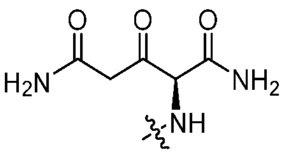, 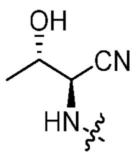, 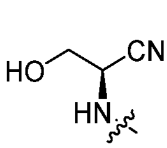, 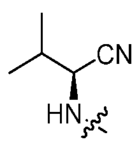, 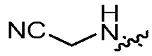, 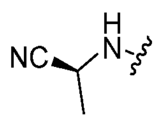,

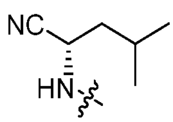, 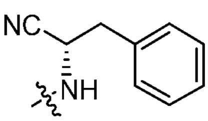 or 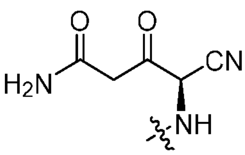.

In some embodiments, Ring A is 3- to 8-membered aliphatic heterocycle;

$R_1$ is selected from H, C1-C6 alkyl, aryl-C1-C4 alkyl, C2-C6 acyl, -CONRR' and natural amino acid fragments;
$R_2$ is selected from H, C1-C6 alkyl, C1-C6 alkoxycarbonyl, C2-C6 acyl, -CONRR' and natural amino acid fragments;
$R_3$ is selected from H, cyano, C1-C6 alkoxycarbonyl and $-CONHR_4$;
R and R' are each independently selected from H and C1-C6 alkyl;
$R_4$ is selected from H, C1-C6 alkyl, natural amino acid fragments and carboxylic acid derivatives of the said natural amino acid fragments;
optionally, the C1-C6 alkyl, aryl-C1-C4 alkyl, C2-C6 acyl, C1-C6 alkoxycarbonyl, - CONRR', $-CONHR_4$ and natural amino acid fragments are each independently substituted by one or more substituents selected from: halogen, amino, hydroxyl, cyano, carboxyl, nitro, C1-C6 alkyl and C1-C6 alkoxy.

In some embodiments, $R_1$, $R_2$ and $R_3$ satisfy:

(1) $R_1$, $R_2$ and $R_3$ are not H at the same time;

(2) when $R_1$ and $R_3$ are H, $R_2$ is not Boc;

(3) when the formula I is

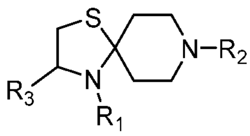,

$R_1$ is

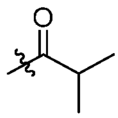,

and $R_3$ is H; $R_2$ is not H,

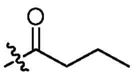, 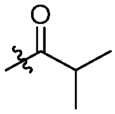, 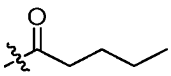, 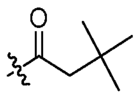, 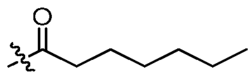, 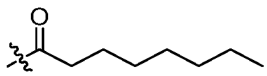,

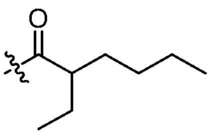, 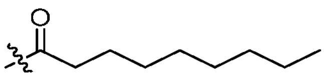, 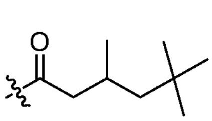, 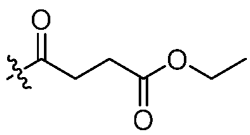, 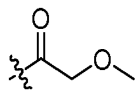,

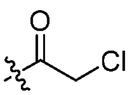, 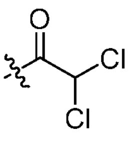, 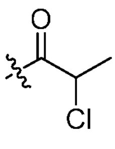, 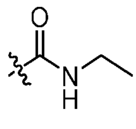, 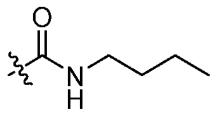, 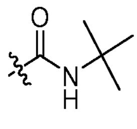 or 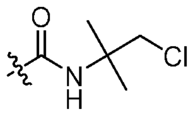;

(4) when the formula I is

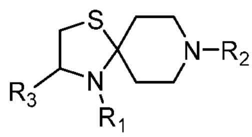,

$R_1$ is

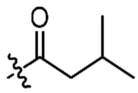,

and $R_3$ is H; $R_2$ is not C2-C6 acyl or -CONRR';

(5) when the formula I is

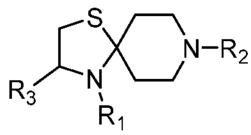,

$R_1$ and $R_3$ are H; $R_2$ is not C1-C4 alkyl, acetyl,

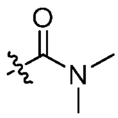 or 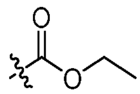;

(6) the compound of the formula I is not

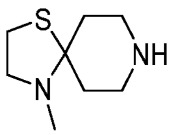, 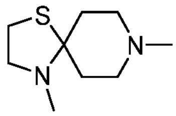, 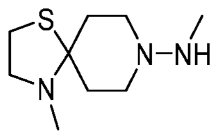,

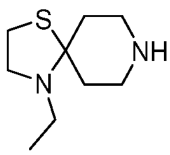, 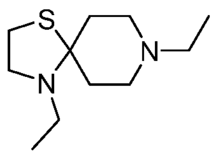, 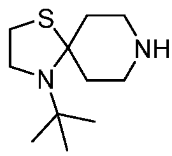, 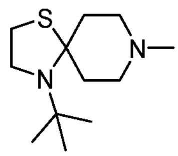, 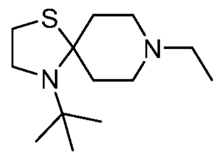,

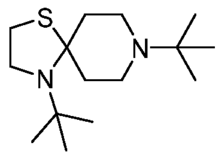 or 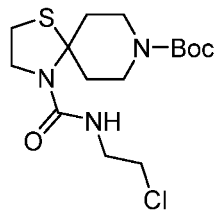.

In some embodiments, $R_3$ is selected from H, cyano, C1-C4 alkoxycarbonyl and - $CONHR_4$; $R_4$ is selected from H, natural amino acid fragments, amide derivatives of the said natural amino acid fragments, and cyano derivatives of the said natural amino acid fragments.

In some embodiments, the present invention provides the following compounds or salts thereof.

Table 1 compounds of examples or salts thereof

| Number | Structural formula | Number | Structural formula |
|---|---|---|---|
| A-001 | 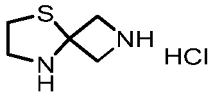 | A-002 | 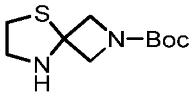 |
| A-003 | 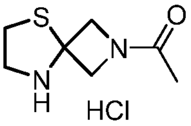 | A-004 | 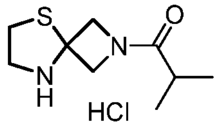 |
| A-005 | 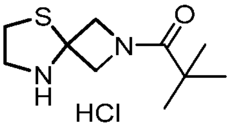 | A-006 | 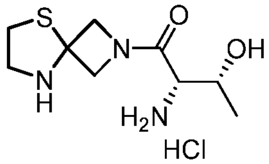 |
| A-007 | 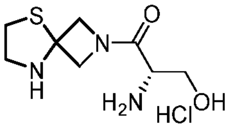 | A-007-1 | 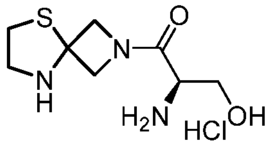 |
| A-008 | 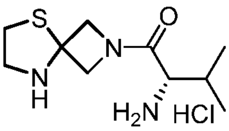 | A-008-1 | 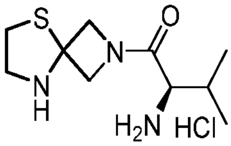 |
| A-009 | 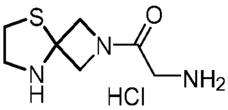 | A-010 | 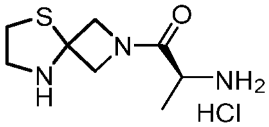 |
| A-011 | 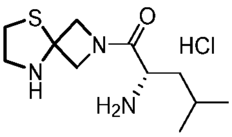 | A-012 | 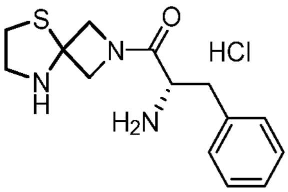 |
| A-013 | 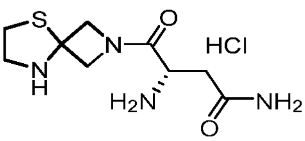 | A-014 | 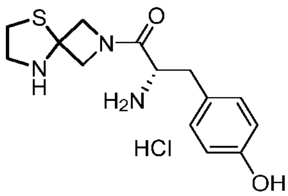 |
| A-015 | 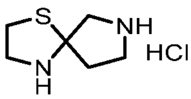 | A-016 | 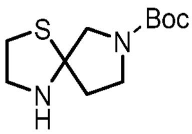 |

(continued)

| Number | Structural formula | Number | Structural formula |
|---|---|---|---|
| A-017 | 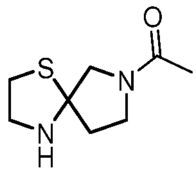 | A-018 | 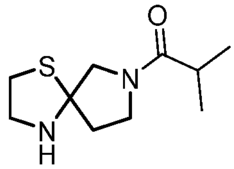 |
| A-019 | 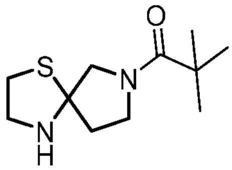 | A-020 | 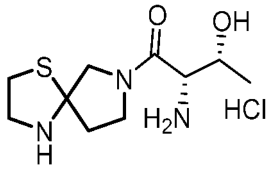 |
| A-021 | 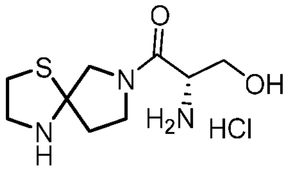 | A-022 | 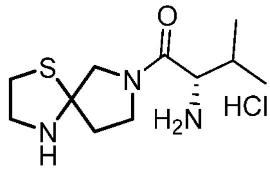 |
| A-023 | 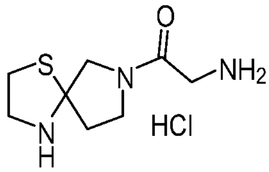 | A-024 | 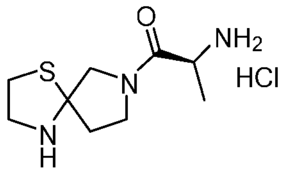 |
| A-025 | 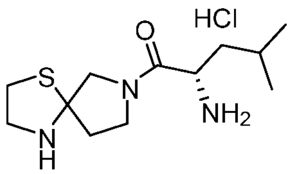 | A-026 | 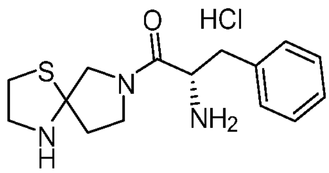 |
| A-027 | 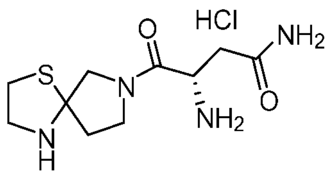 | A-028 | 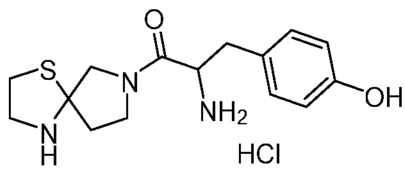 |
| A-029 | 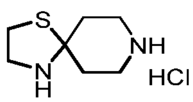 | A-030 | 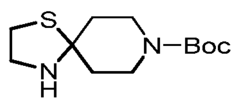 |
| A-031 | 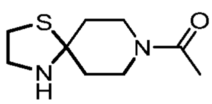 | A-032 | 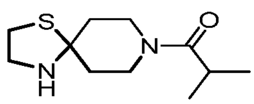 |
| A-033 | 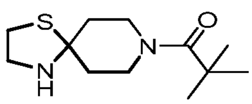 | A-034 | 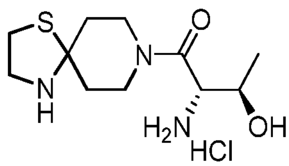 |

(continued)

| Number | Structural formula | Number | Structural formula |
|---|---|---|---|
| **A-035** | 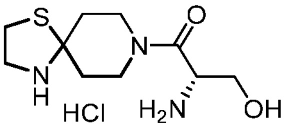 | **A-036** | 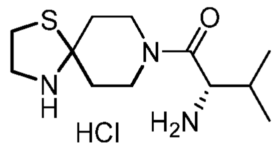 |
| **A-037** | 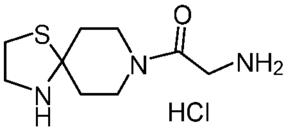 | **A-038** | 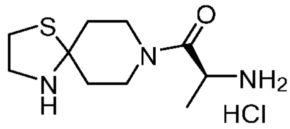 |
| **A-039** | 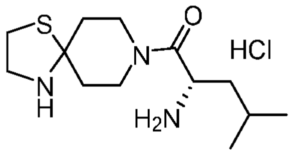 | **A-040** | 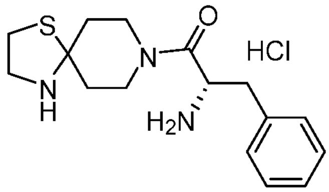 |
| **A-041** | 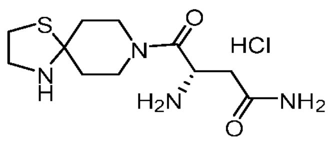 | **A-042** | 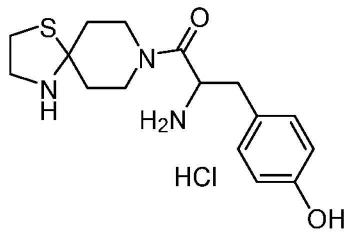 |
| **A-043** | 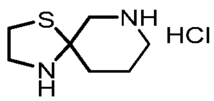 | **A-044** | 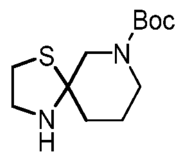 |
| **A-045** | 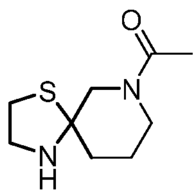 | **A-046** | 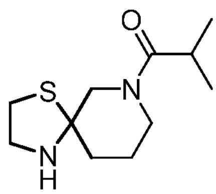 |
| **A-047** | 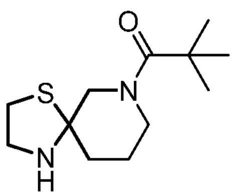 | **A-048** | 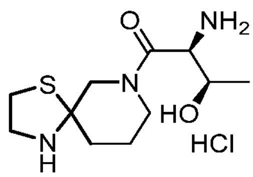 |
| **A-049** | 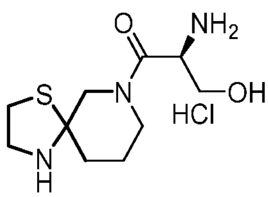 | **A-050** | 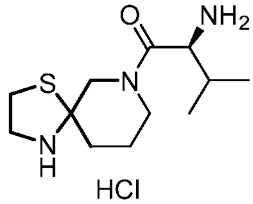 |

(continued)

| Number | Structural formula | Number | Structural formula |
|---|---|---|---|
| **A-051** | 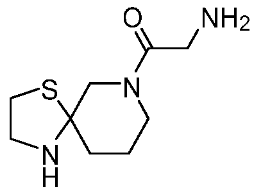 | **A-052** | 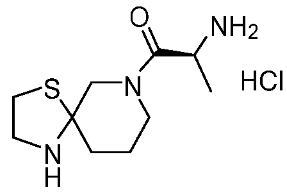 |
| **A-053** | 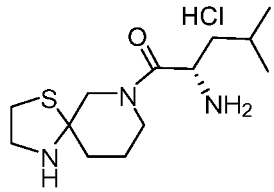 | **A-054** | 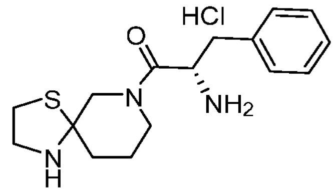 |
| **A-055** | 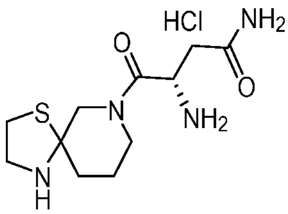 | **A-056** | 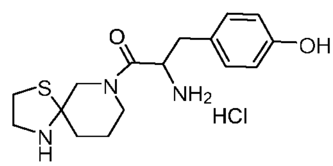 |
| **A-057** | 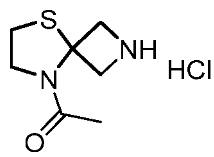 | **A-058** | 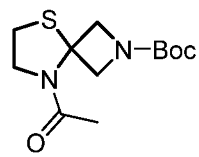 |
| **A-059** | 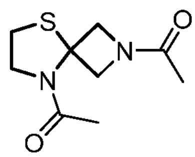 | **A-060** | 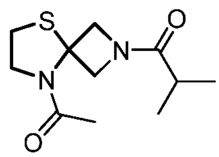 |
| **A-061** | 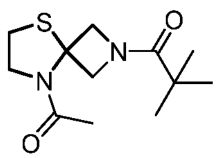 | **A-062** | 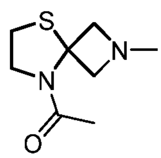 |
| **A-063** | 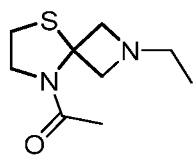 | **A-064** | 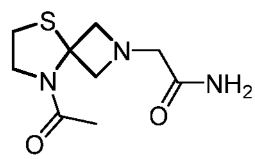 |
| **A-065** | 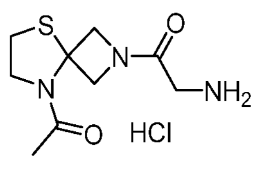 | **A-066** | 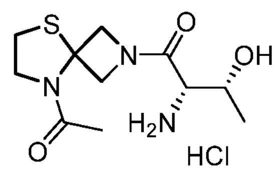 |

(continued)

| Number | Structural formula | Number | Structural formula |
|---|---|---|---|
| **A-067** | 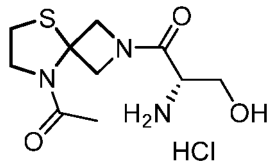 | **A-068** | 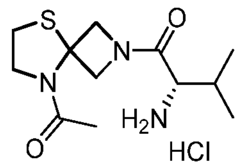 |
| **A-069** | 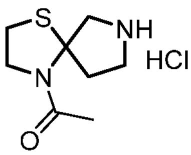 | **A-070** | 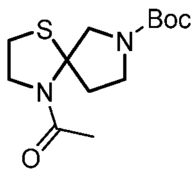 |
| **A-071** | 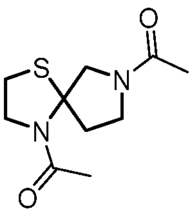 | **A-072** | 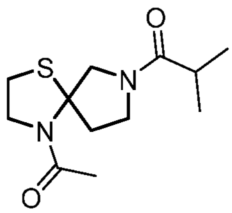 |
| **A-073** | 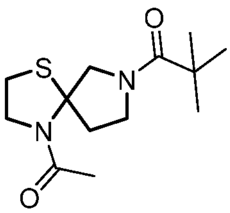 | **A-074** | 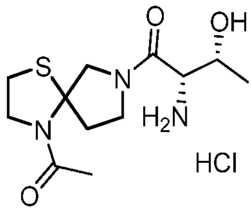 |
| **A-075** | 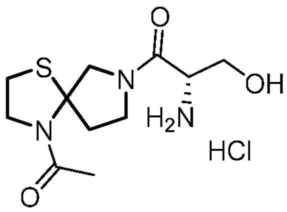 | **A-076** | 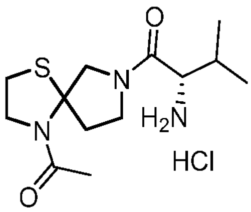 |
| **A-077** | 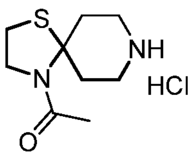 | **A-078** | 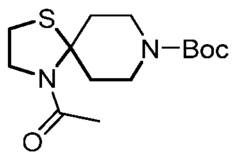 |
| **A-079** | 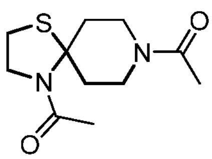 | **A-080** | 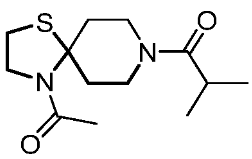 |
| **A-081** | 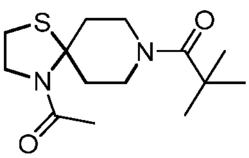 | **A-082** | 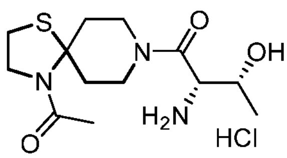 |

(continued)

| Number | Structural formula | Number | Structural formula |
|---|---|---|---|
| **A-083** | 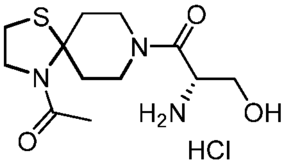 HCl | **A-084** | 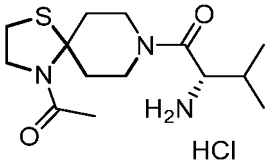 HCl |
| **A-085** | 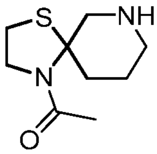 HCl | **A-086** | 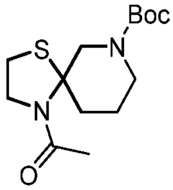 |
| **A-087** | 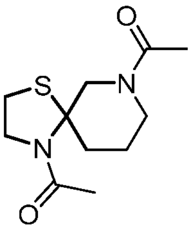 | **A-088** | 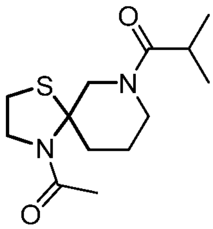 |
| **A-089** | 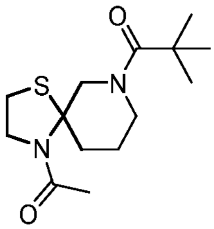 | **A-090** | 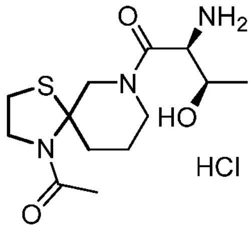 HCl |
| **A-091** | 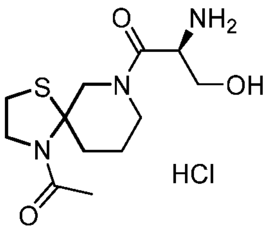 HCl | **A-092** | 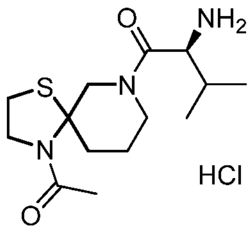 HCl |
| **A-093** | 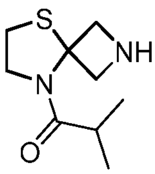 HCl | **A-094** | 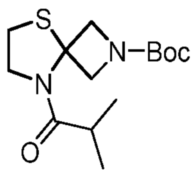 |
| **A-095** | 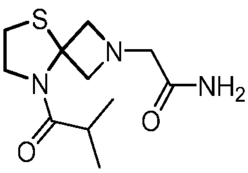 | **A-096** | 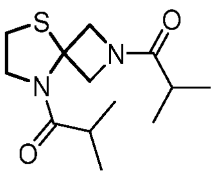 |

(continued)

| Number | Structural formula | Number | Structural formula |
|---|---|---|---|
| **A-097** | 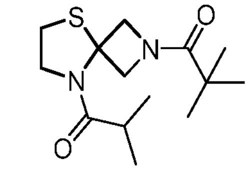 | **A-098** | 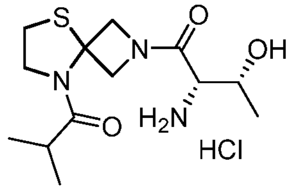 |
| **A-099** | 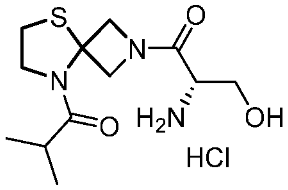 | **A-100** | 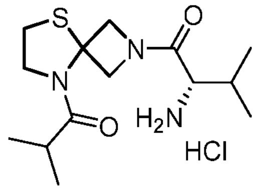 |
| **A-101** | 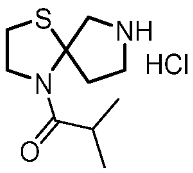 | **A-102** | 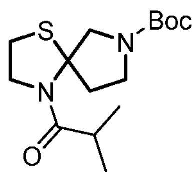 |
| **A-103** | 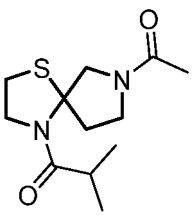 | **A-104** | 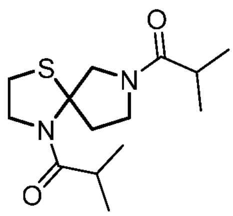 |
| **A-105** | 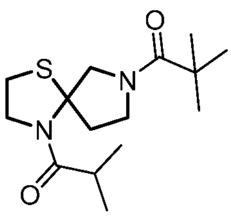 | **A-106** | 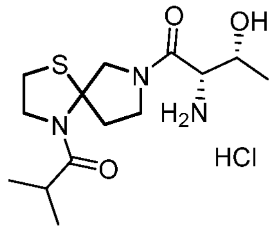 |
| **A-107** | 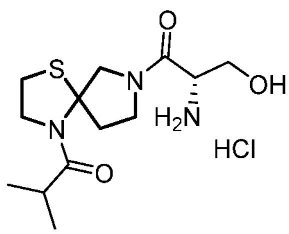 | **A-108** | 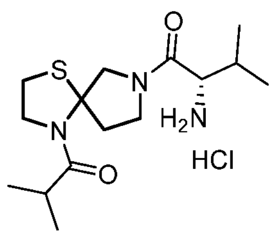 |
| **A-109** | 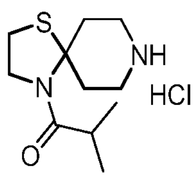 | **A-110** | 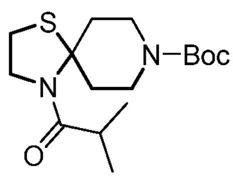 |

(continued)

| Number | Structural formula | Number | Structural formula |
|---|---|---|---|
| **A-111** | 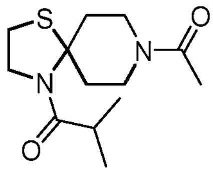 | **A-112** | 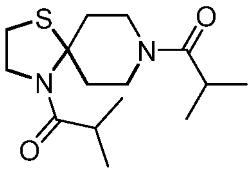 |
| **A-113** | 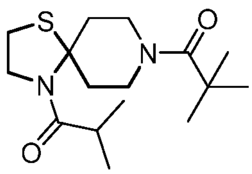 | **A-114** | 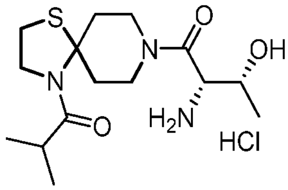 |
| **A-115** | 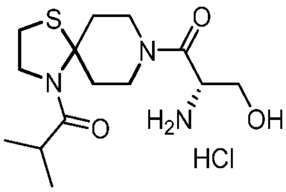 | **A-116** | 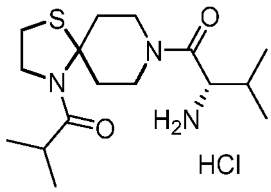 |
| **A-117** | 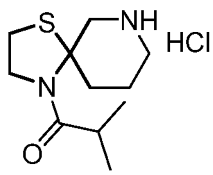 | **A-118** | 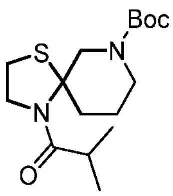 |
| **A-119** | 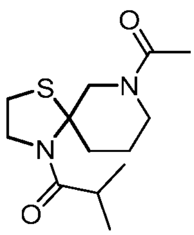 | **A-120** | 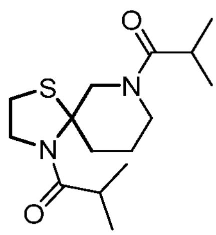 |
| **A-121** | 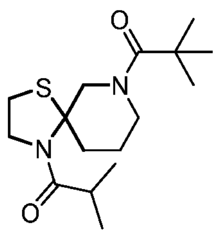 | **A-122** | 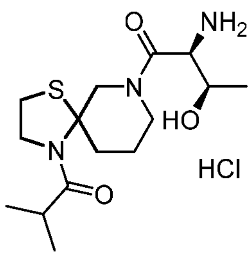 |
| **A-123** | 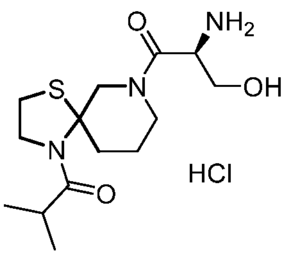 | **A-124** | 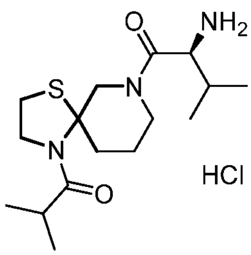 |

(continued)

| Number | Structural formula | Number | Structural formula |
|---|---|---|---|
| A-125 | 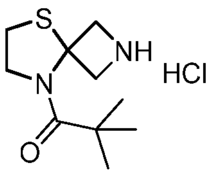 | A-126 | 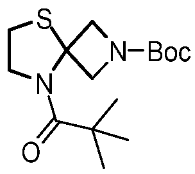 |
| A-127 | 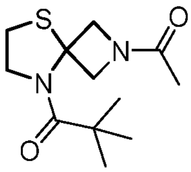 | A-128 | 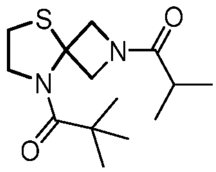 |
| A-129 | 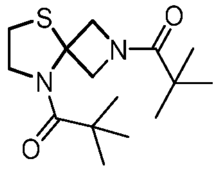 | A-130 | 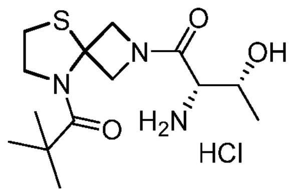 |
| A-131 | 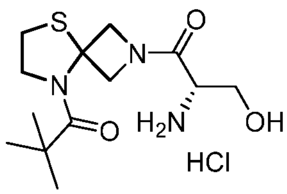 | A-132 | 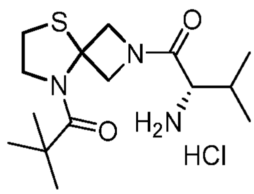 |
| A-133 | 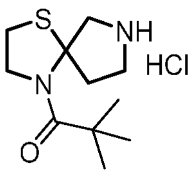 | A-134 | 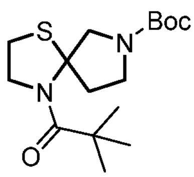 |
| A-135 | 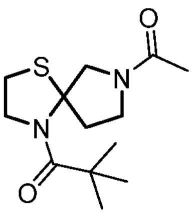 | A-136 | 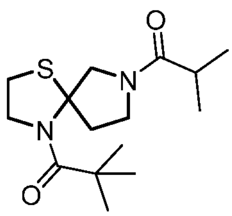 |
| A-137 | 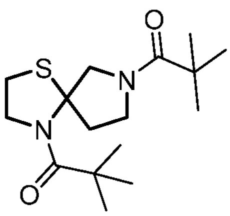 | A-138 | 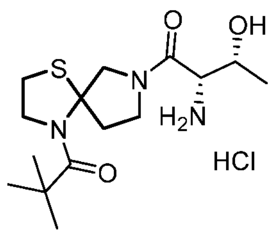 |

(continued)

| Number | Structural formula | Number | Structural formula |
|---|---|---|---|
| **A-139** | 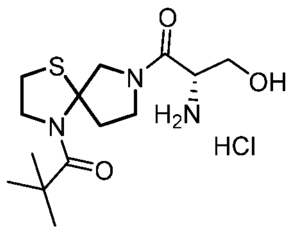 | **A-140** | 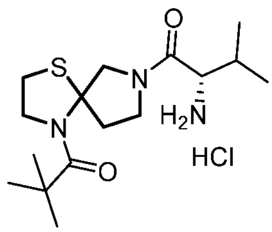 |
| **A-141** | 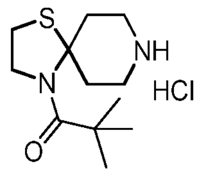 | **A-142** | 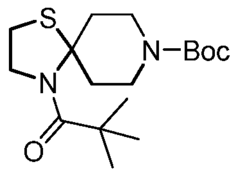 |
| **A-143** | 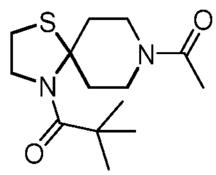 | **A-144** | 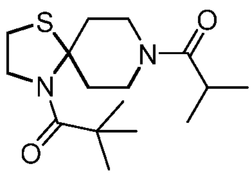 |
| **A-145** | 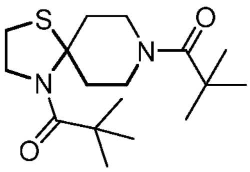 | **A-146** | 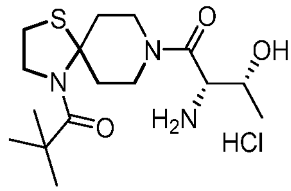 |
| **A-147** | 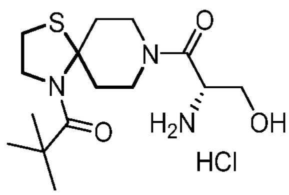 | **A-148** | 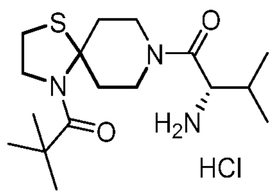 |
| **A-149** | 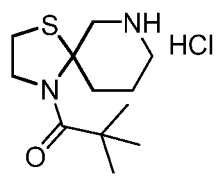 | **A-150** | 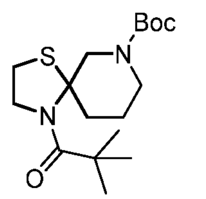 |
| **A-151** | 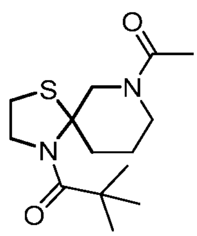 | **A-152** | 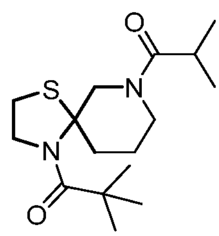 |

(continued)

| Number | Structural formula | Number | Structural formula |
|---|---|---|---|
| **A-153** | 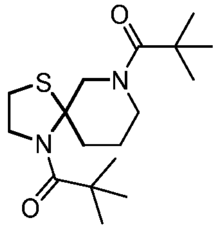 | **A-154** | 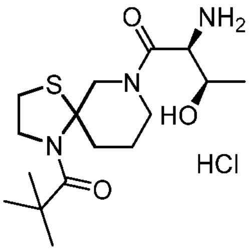 |
| **A-155** | 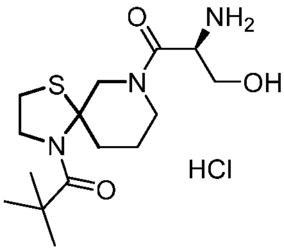 | **A-156** | 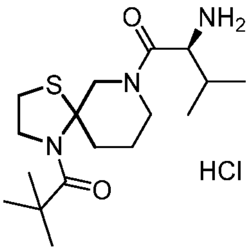 |
| **A-157** | 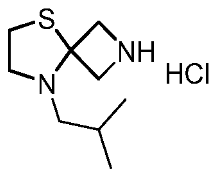 | **A-158** | 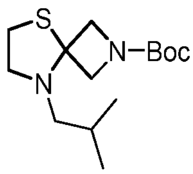 |
| **A-159** | 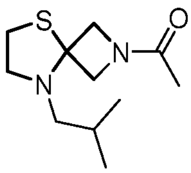 | **A-160** | 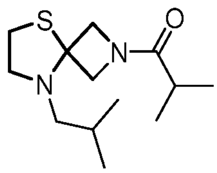 |
| **A-161** | 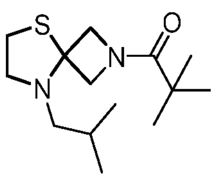 | **A-162** | 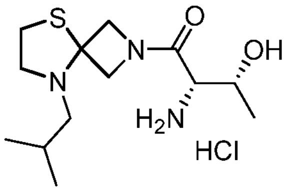 |
| **A-163** | 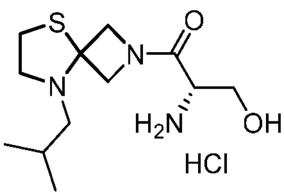 | **A-164** | 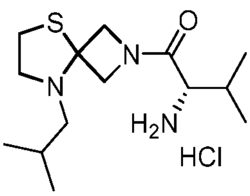 |
| **A-165** | 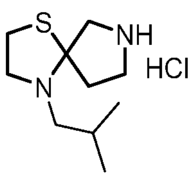 | **A-166** | 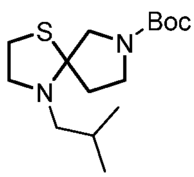 |

(continued)

| Number | Structural formula | Number | Structural formula |
|---|---|---|---|
| **A-167** | 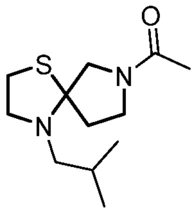 | **A-168** | 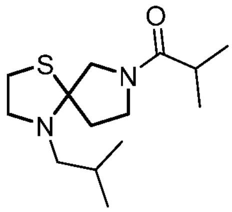 |
| **A-169** | 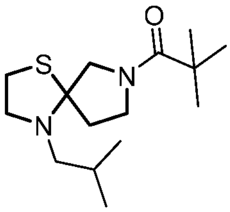 | **A-170** | 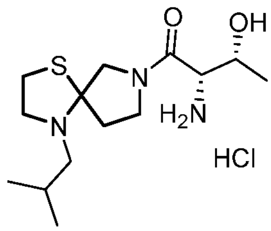 |
| **A-171** | 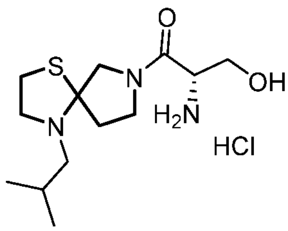 | **A-172** | 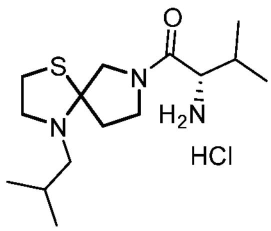 |
| **A-173** | 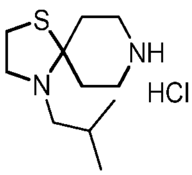 | **A-174** | 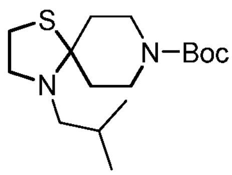 |
| **A-175** | 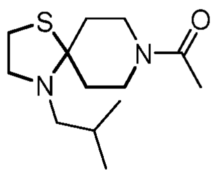 | **A-176** | 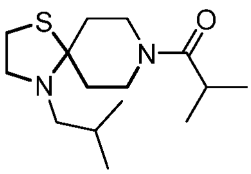 |
| **A-177** | 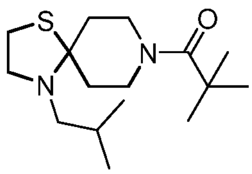 | **A-178** | 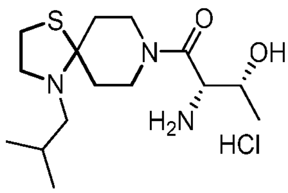 |
| **A-179** | 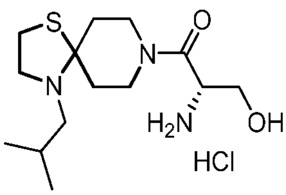 | **A-180** | 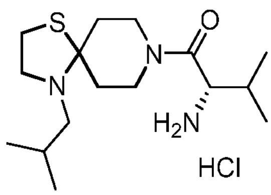 |

(continued)

| Number | Structural formula | Number | Structural formula |
|---|---|---|---|
| **A-181** | 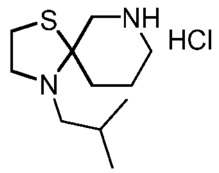 | **A-182** | 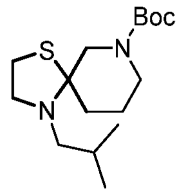 |
| **A-183** | 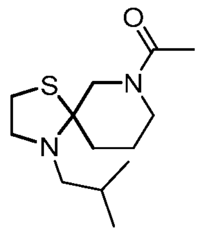 | **A-184** | 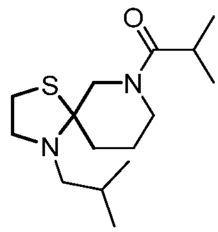 |
| **A-185** | 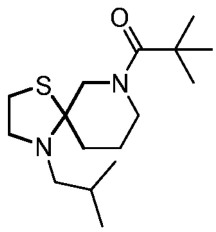 | **A-186** | 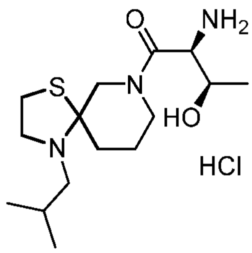 |
| **A-187** | 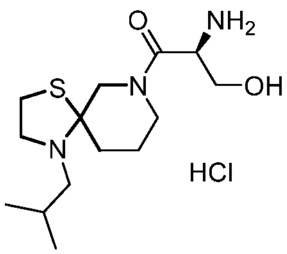 | **A-188** | 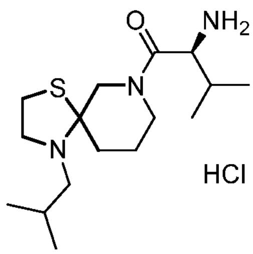 |
| **A-189** | 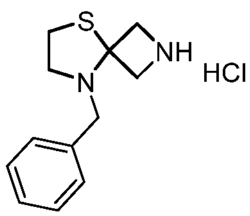 | **A-190** | 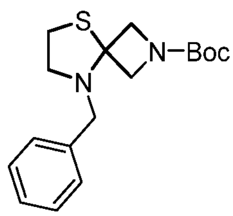 |
| **A-191** | 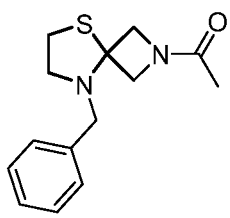 | **A-192** | 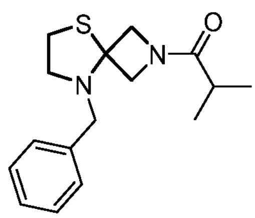 |

(continued)

| Number | Structural formula | Number | Structural formula |
|---|---|---|---|
| **A-193** | 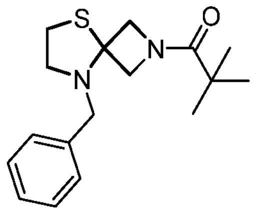 | **A-194** | 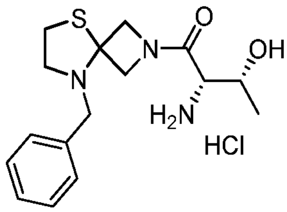 |
| **A-195** | 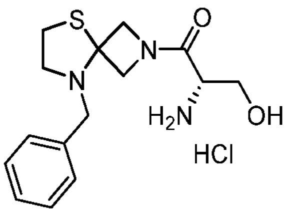 | **A-196** | 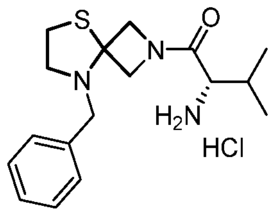 |
| **A-197** | 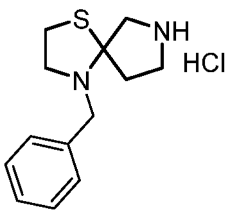 | **A-198** | 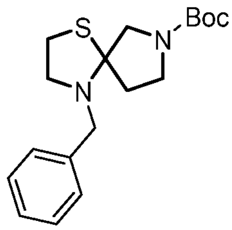 |
| **A-199** | 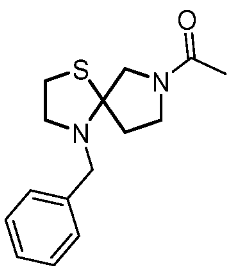 | **A-200** | 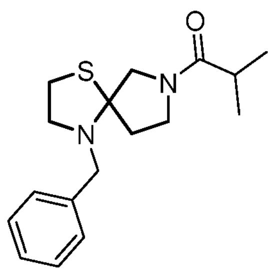 |
| **A-201** | 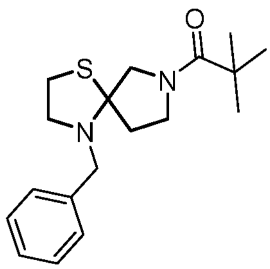 | **A-202** | 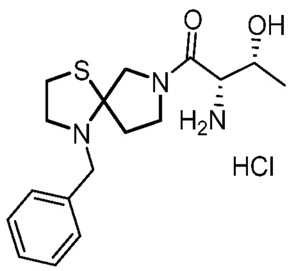 |
| **A-203** | 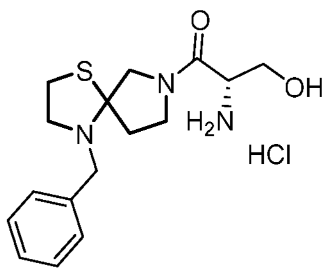 | **A-204** | 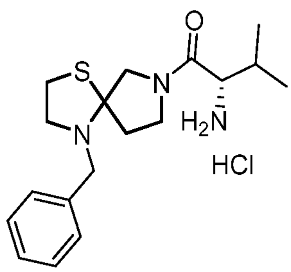 |

(continued)

| Number | Structural formula | Number | Structural formula |
|---|---|---|---|
| A-205 | 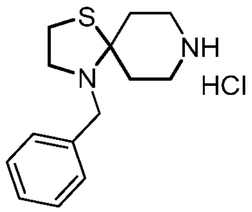 | A-206 | 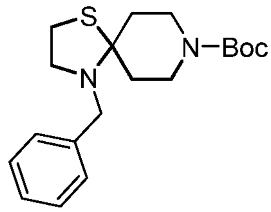 |
| A-207 | 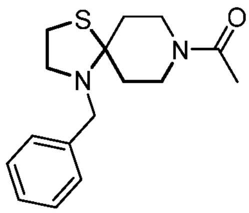 | A-208 | 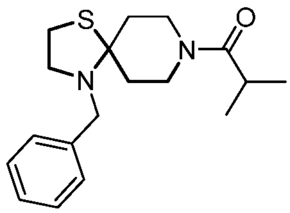 |
| A-209 | 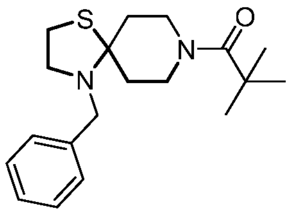 | A-210 | 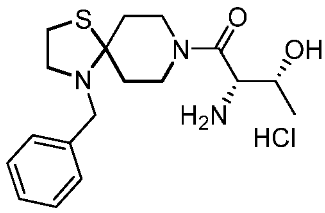 |
| A-211 | 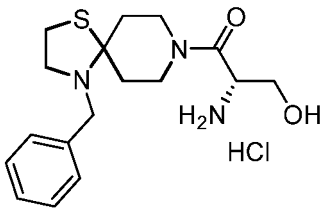 | A-212 | 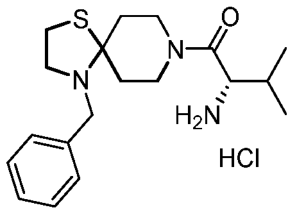 |
| A-213 | 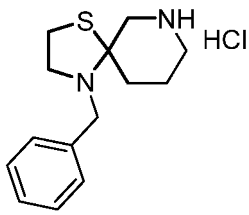 | A-214 | 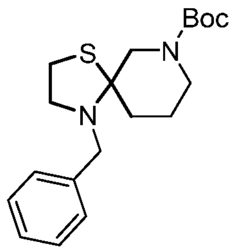 |
| A-215 | 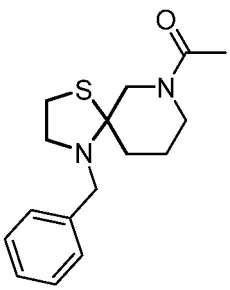 | A-216 | 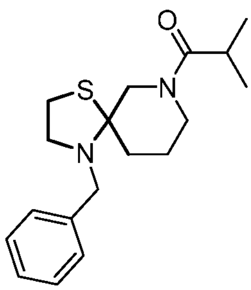 |

(continued)

| Number | Structural formula | Number | Structural formula |
|---|---|---|---|
| **A-217** | 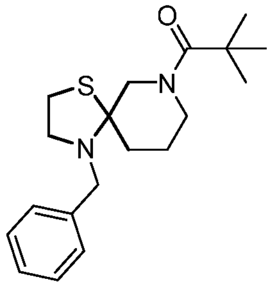 | **A-218** | 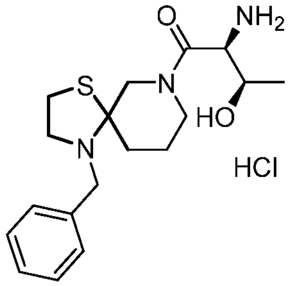 |
| **A-219** | 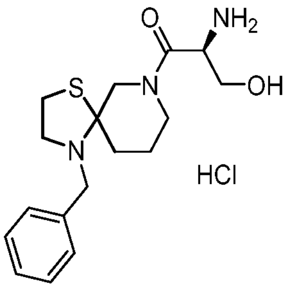 | **A-220** | 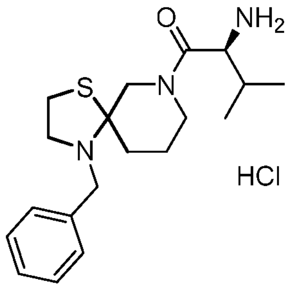 |
| **A-221** | 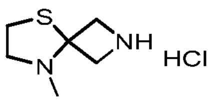 | **A-222** | 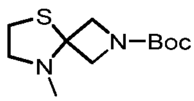 |
| **A-223** | 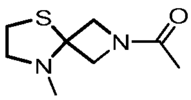 | **A-224** | 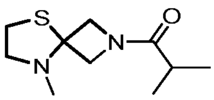 |
| **A-225** | 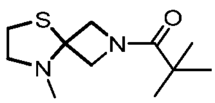 | **A-226** | 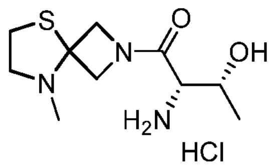 |
| **A-227** | 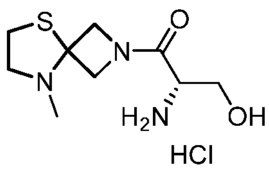 | **A-228** | 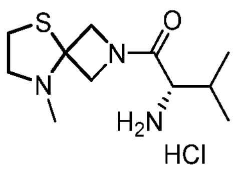 |
| **A-229** | 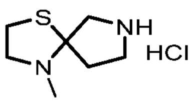 | **A-230** | 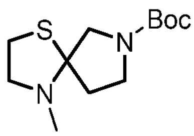 |
| **A-231** | 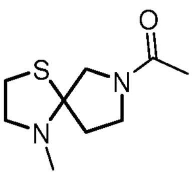 | **A-232** | 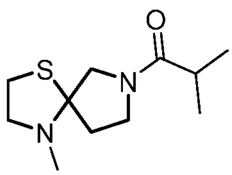 |

(continued)

| Number | Structural formula | Number | Structural formula |
|---|---|---|---|
| A-233 | 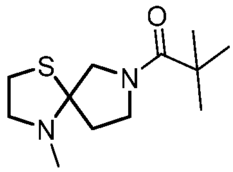 | A-234 | 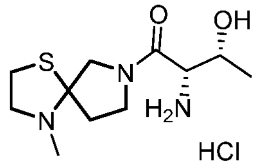 |
| A-235 | 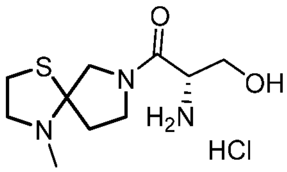 | A-236 | 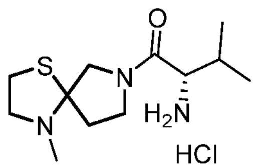 |
| A-237 | 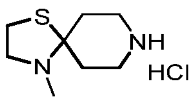 | A-238 | 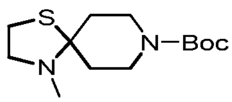 |
| A-239 | 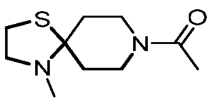 | A-240 | 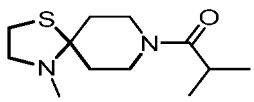 |
| A-241 | 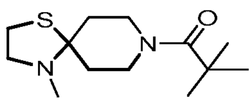 | A-242 | 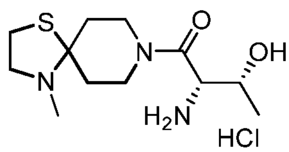 |
| A-243 | 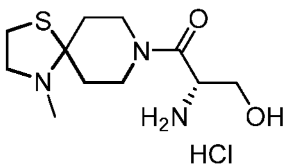 | A-244 | 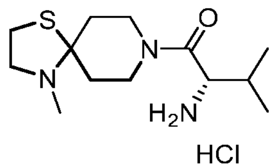 |
| A-245 | 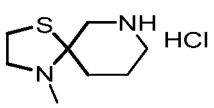 | A-246 | 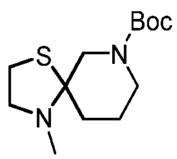 |
| A-247 | 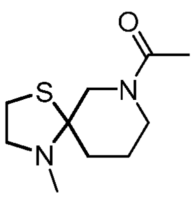 | A-248 | 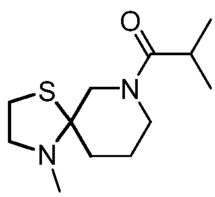 |
| A-249 | 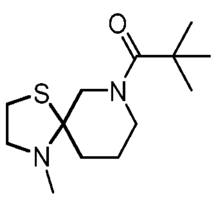 | A-250 | 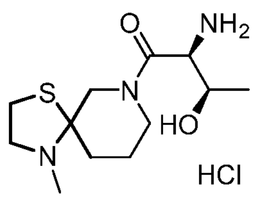 |

(continued)

| Number | Structural formula | Number | Structural formula |
|---|---|---|---|
| **A-251** | 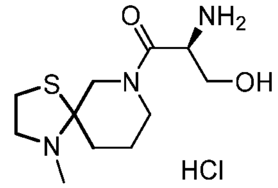 | **A-252** | 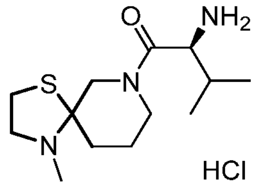 |
| **A-253** | 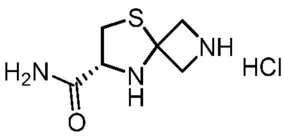 | **A-254** | 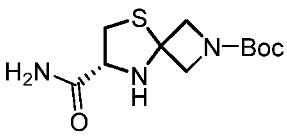 |
| **A-255** | 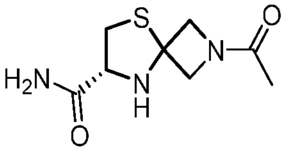 | **A-256** | 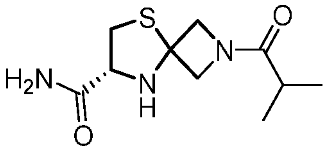 |
| **A-257** | 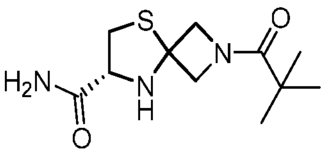 | **A-258** | 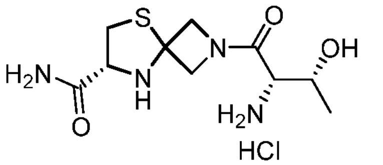 |
| **A-259** | 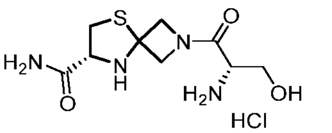 | **A-260** | 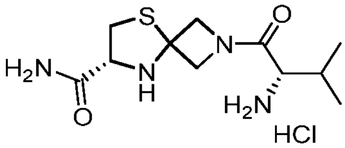 |
| **A-261** | 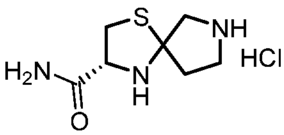 | **A-262** | 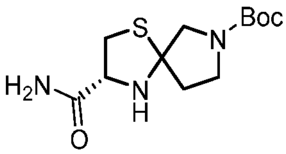 |
| **A-263** | 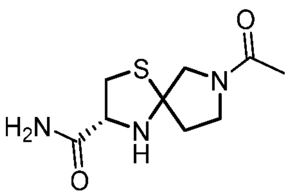 | **A-264** | 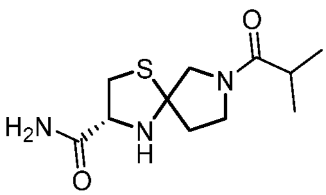 |
| **A-265** | 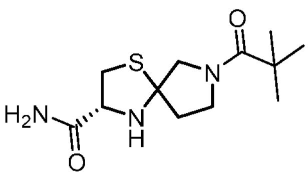 | **A-266** | 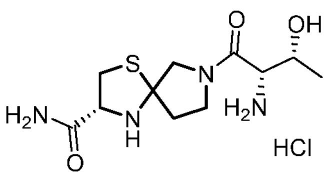 |
| **A-267** | 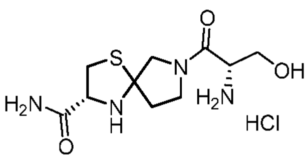 | **A-268** | 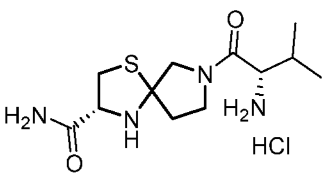 |

(continued)

| Number | Structural formula | Number | Structural formula |
|---|---|---|---|
| **A-269** | 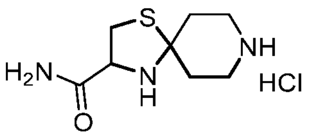 | **A-270** | 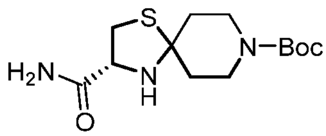 |
| **A-271** | 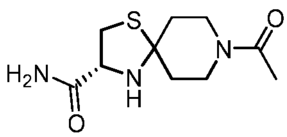 | **A-272** | 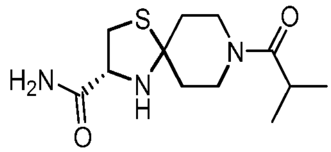 |
| **A-273** | 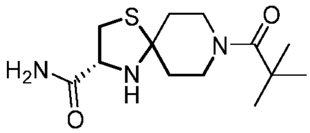 | **A-274** | 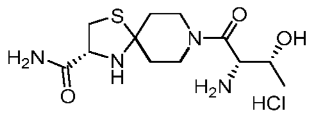 |
| **A-275** | 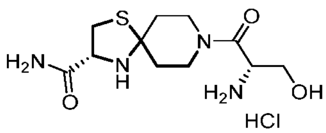 | **A-276** | 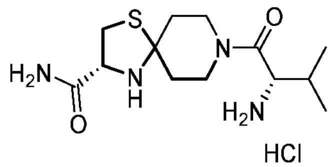 |
| **A-277** | 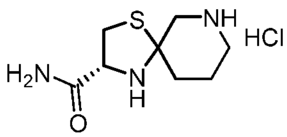 | **A-278** | 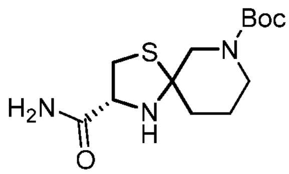 |
| **A-279** | 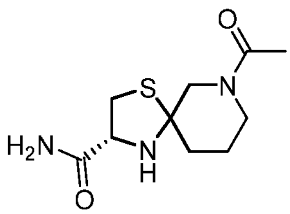 | **A-280** | 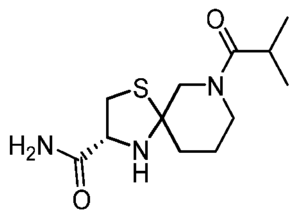 |
| **A-281** | 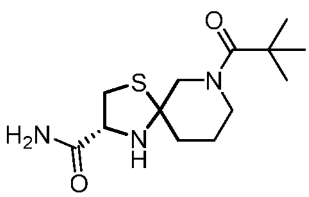 | **A-282** | 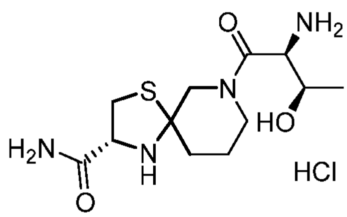 |
| **A-283** | 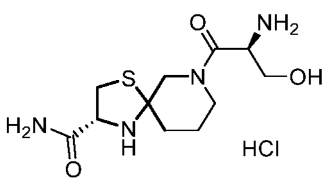 | **A-284** | 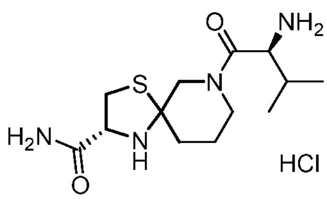 |

(continued)

| Number | Structural formula | Number | Structural formula |
|---|---|---|---|
| **A-285** | 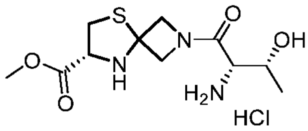 | **A-286** | 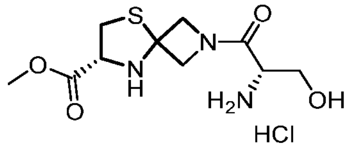 |
| **A-287** | 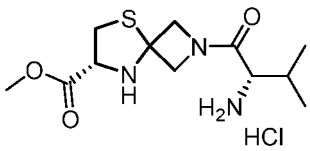 | **A-288** | 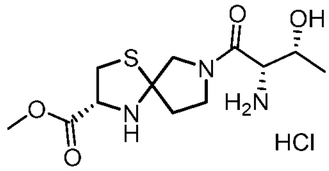 |
| **A-289** | 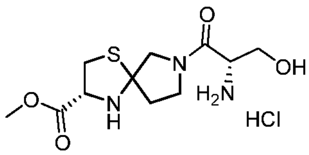 | **A-290** | 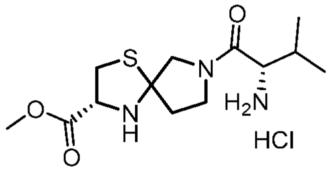 |
| **A-291** | 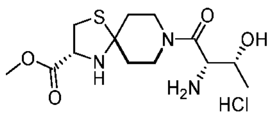 | **A-292** | 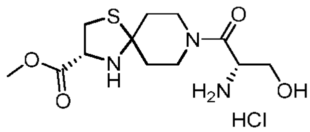 |
| **A-293** | 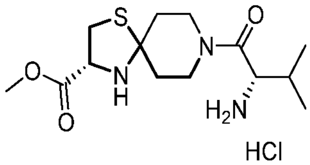 | **A-294** | 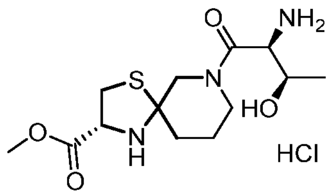 |
| **A-295** | 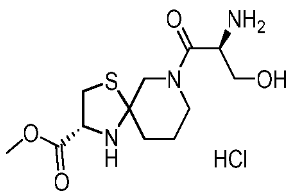 | **A-296** | 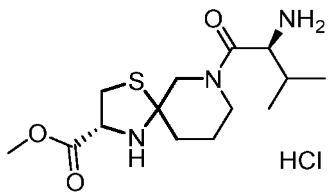 |
| **A-297** | 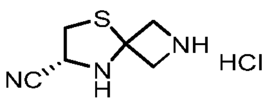 | **A-298** | 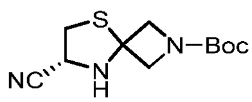 |
| **A-299** | 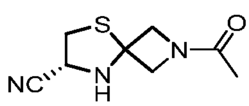 | **A-300** | 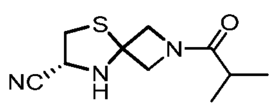 |
| **A-301** | 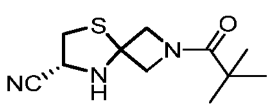 | **A-302** | 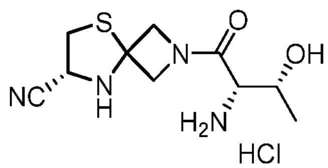 |

(continued)

| Number | Structural formula | Number | Structural formula |
|---|---|---|---|
| A-303 | 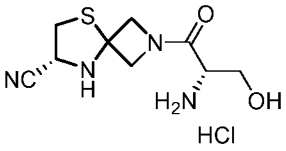 | A-304 | 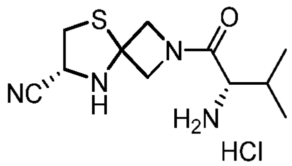 |
| A-305 | 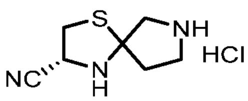 | A-306 | 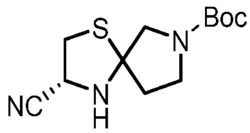 |
| A-307 | 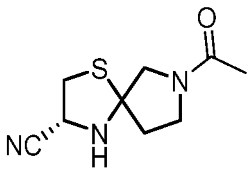 | A-308 | 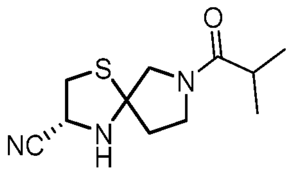 |
| A-309 | 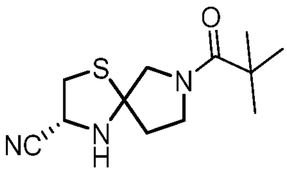 | A-310 | 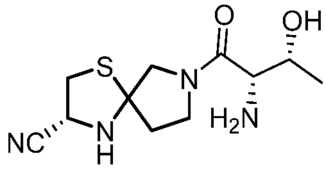 |
| A-311 | 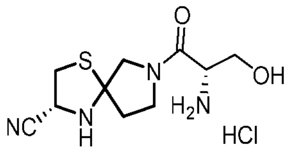 | A-312 | 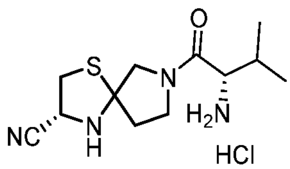 |
| A-313 | 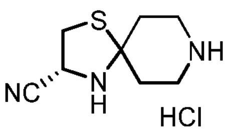 | A-314 | 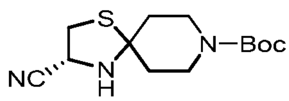 |
| A-315 | 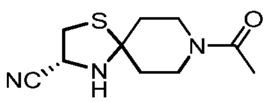 | A-316 | 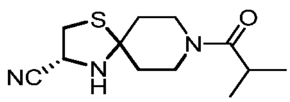 |
| A-317 | 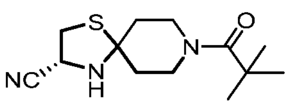 | A-318 | 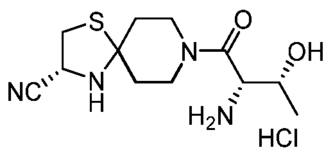 |
| A-319 | 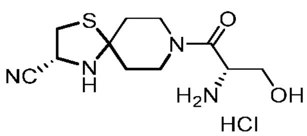 | A-320 | 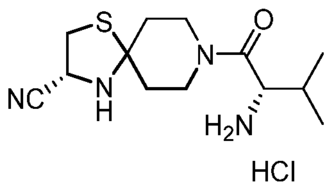 |

(continued)

| Number | Structural formula | Number | Structural formula |
|---|---|---|---|
| **A-321** | 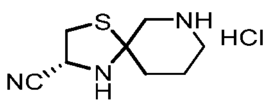 | **A-322** | 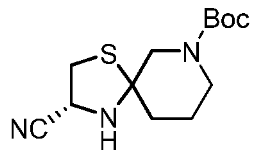 |
| **A-323** | 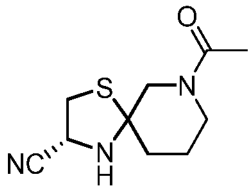 | **A-324** | 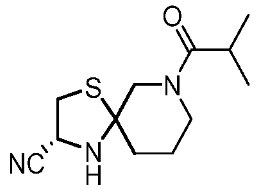 |
| **A-325** | 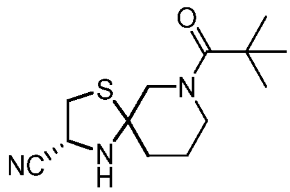 | **A-326** | 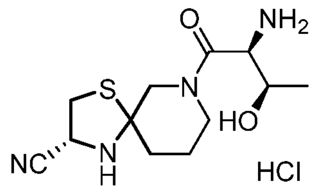 |
| **A-327** | 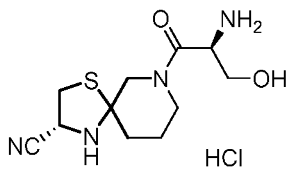 | **A-328** | 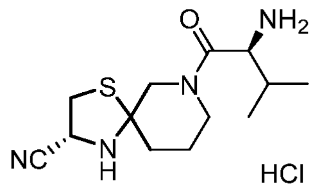 |
| **A-329** | 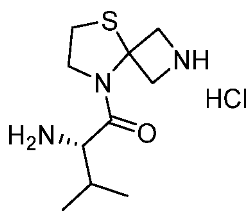 | **A-330** | 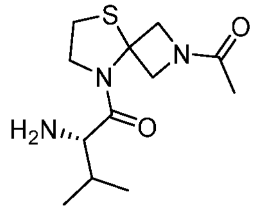 |
| **A-331** | 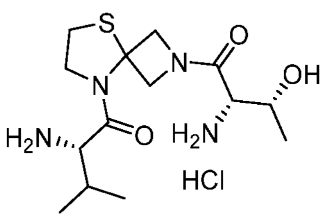 | **A-332** | 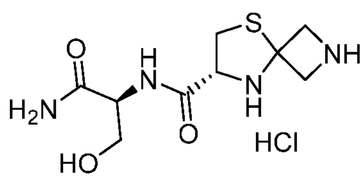 |
| **A-333** | 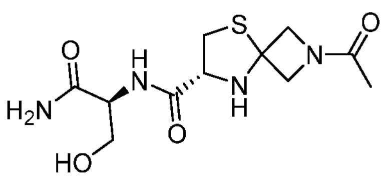 | **A-334** | 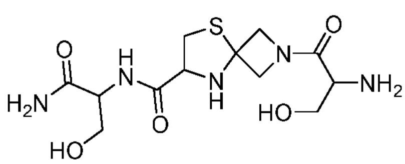 |

(continued)

| Number | Structural formula | Number | Structural formula |
|---|---|---|---|
| **A-335** | 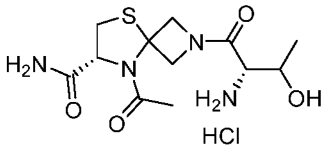 | **A-336** | 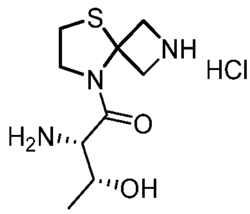 |
| **A-337** | 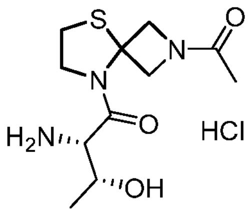 | **A-338** | 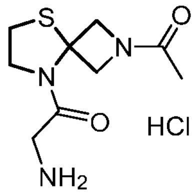 |
| **A-339** | 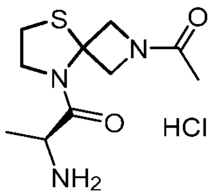 | **A-340** | 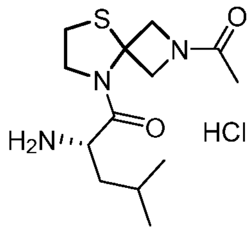 |
| **A-341** | 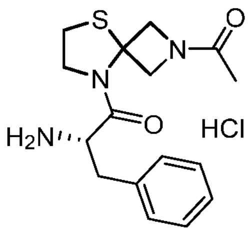 | **A-342** | 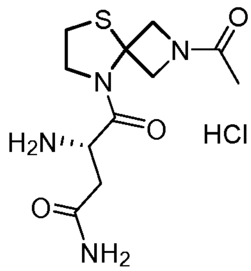 |
| **A-343** | 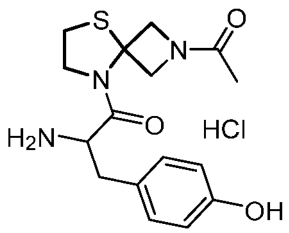 | **A-344** | 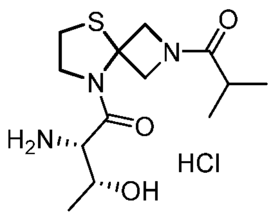 |
| **A-345** | 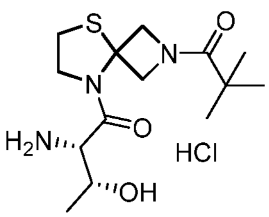 | **A-346** | 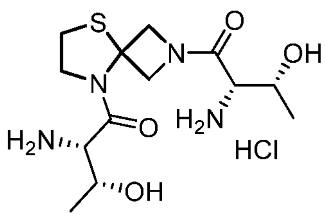 |

(continued)

| Number | Structural formula | Number | Structural formula |
|---|---|---|---|
| A-347 | 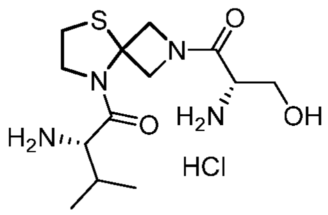 | A-348 | 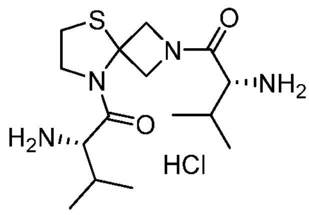 |
| A-349 | 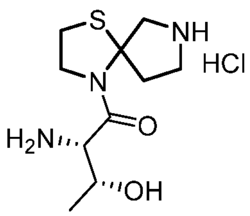 | A-350 | 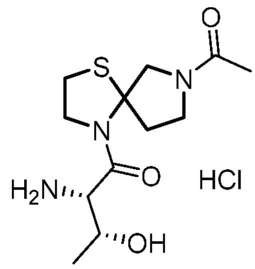 |
| A-351 | 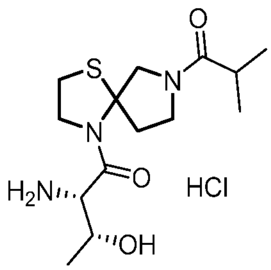 | A-352 | 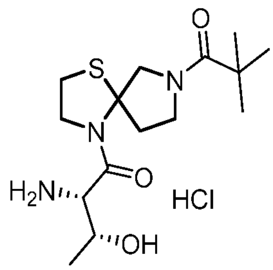 |
| A-353 | 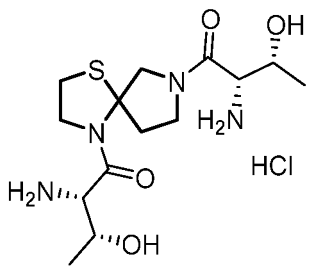 | A-354 | 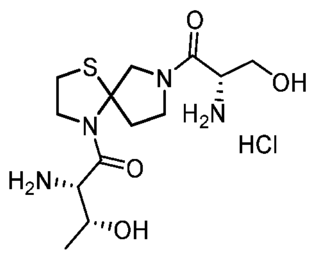 |
| A-355 | 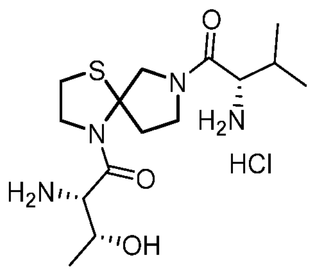 | A-356 | 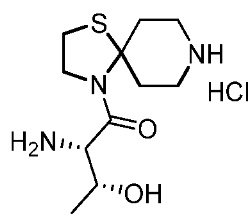 |
| A-357 | 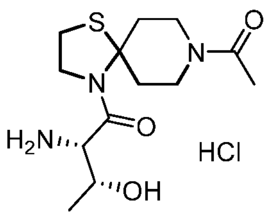 | A-358 | 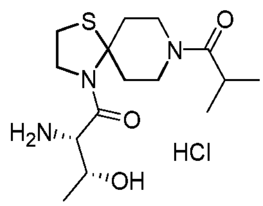 |

(continued)

| Number | Structural formula | Number | Structural formula |
| --- | --- | --- | --- |
| **A-359** | 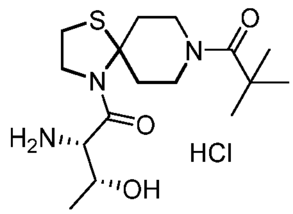 | **A-360** | 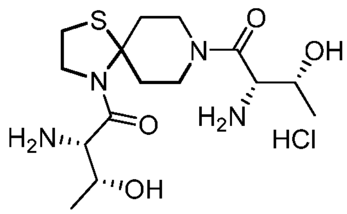 |
| **A-361** | 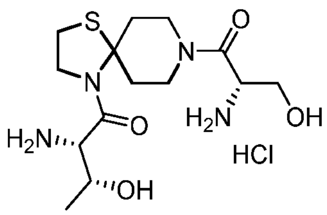 | **A-362** | 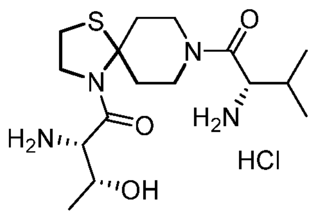 |
| **A-363** | 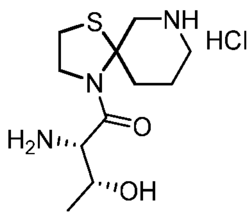 | **A-364** | 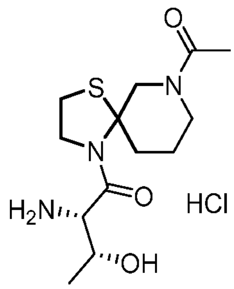 |
| **A-365** | 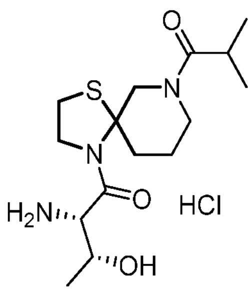 | **A-366** | 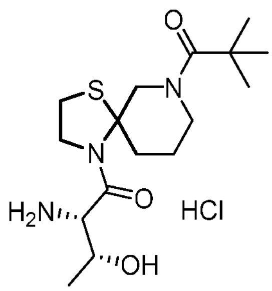 |
| **A-367** | 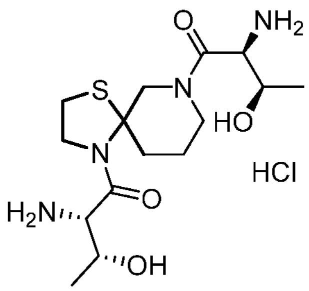 | **A-368** | 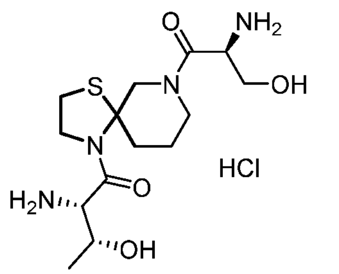 |
| **A-369** | 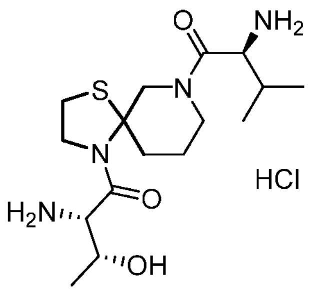 | **A-370** | 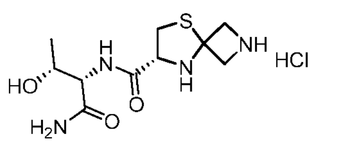 |

(continued)

| Number | Structural formula | Number | Structural formula |
|---|---|---|---|
| **A-371** | 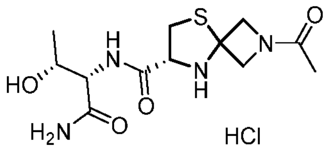 | **A-372** | 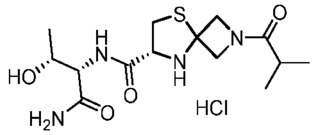 |
| **A-373** | 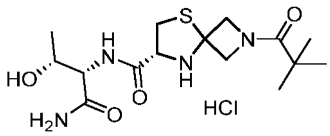 | **A-374** | 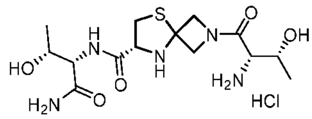 |
| **A-375** | 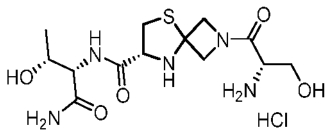 | **A-376** | 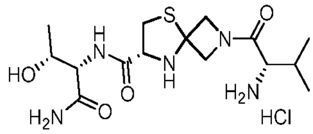 |
| **A-377** | 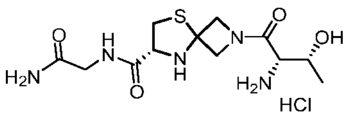 | **A-378** | 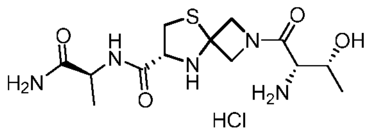 |
| **A-379** | 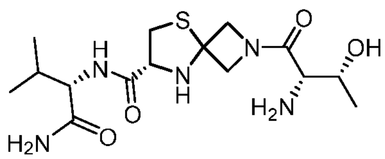 | **A-380** | 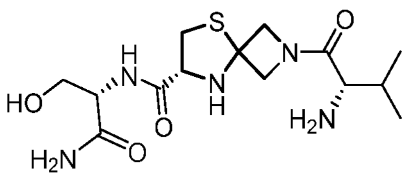 |
| **A-381** | 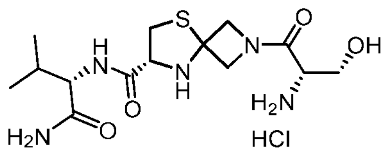 | **A-382** | 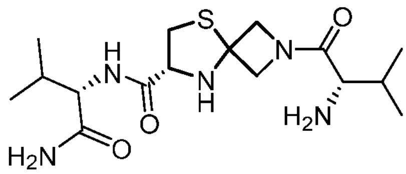 |
| **A-383** | 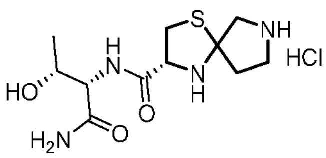 | **A-384** | 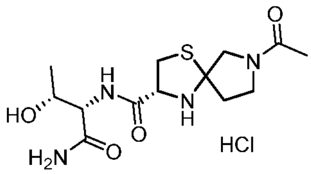 |
| **A-385** | 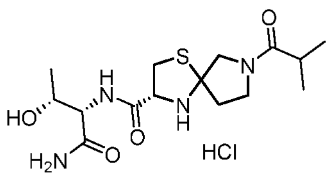 | **A-386** | 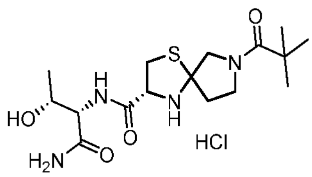 |
| **A-387** | 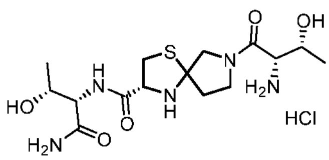 | **A-388** | 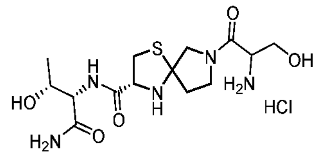 |

(continued)

| Number | Structural formula | Number | Structural formula |
|---|---|---|---|
| **A-389** | 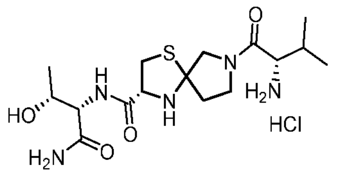 | **A-390** | 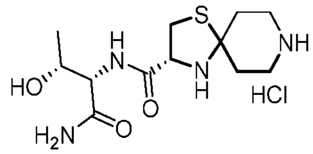 |
| **A-391** | 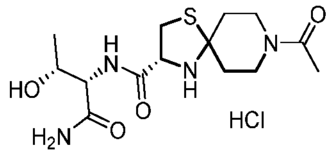 | **A-392** | 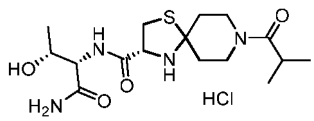 |
| **A-393** | 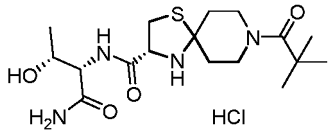 | **A-394** | 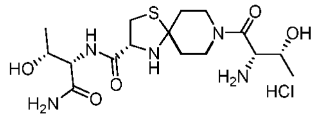 |
| **A-395** | 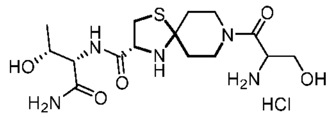 | **A-396** | 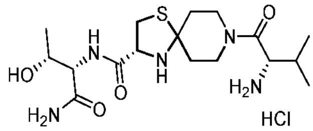 |
| **A-397** | 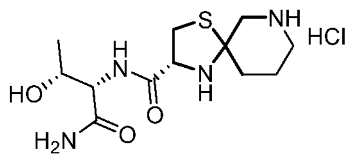 | **A-398** | 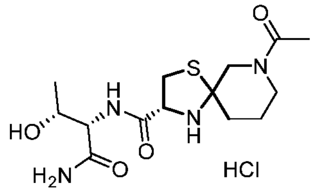 |
| **A-399** | 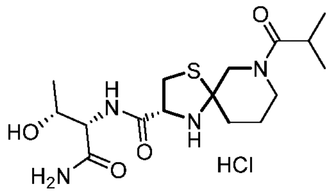 | **A-400** | 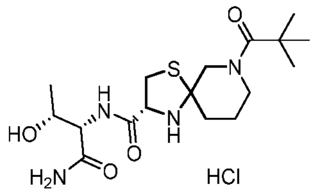 |
| **A-401** | 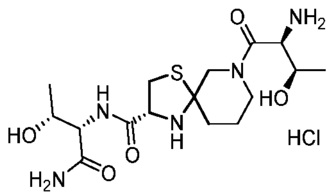 | **A-402** | 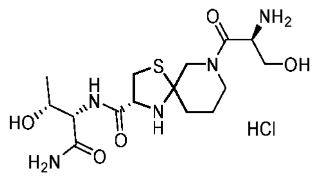 |
| **A-403** | 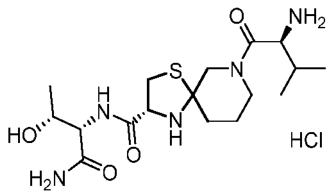 | **A-404** | 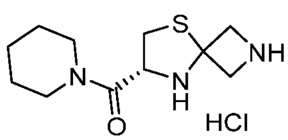 |

(continued)

| Number | Structural formula | Number | Structural formula |
|---|---|---|---|
| **A-405** | 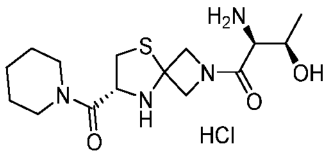 | **A-406** | 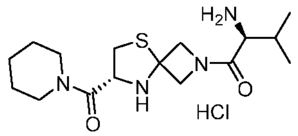 |
| **A-407** | 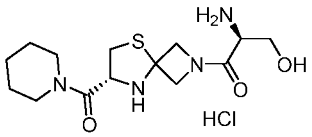 | **A-408** | 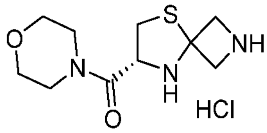 |
| **A-409** | 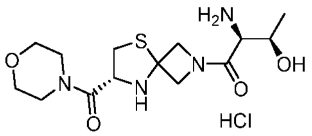 | **A-410** | 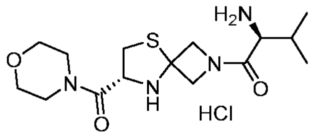 |
| **A-411** | 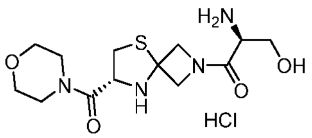 | **A-412** | 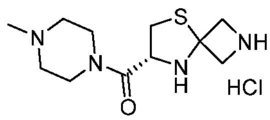 |
| **A-413** | 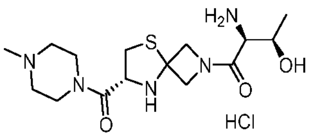 | **A-414** | 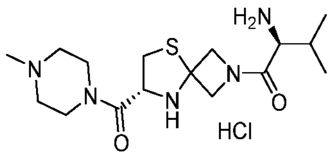 |
| **A-415** | 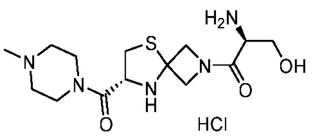 | **A-416** | 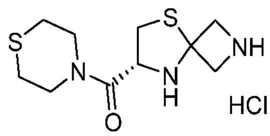 |
| **A-417** | 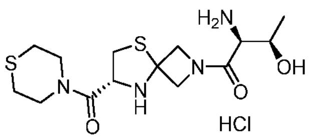 | **A-418** | 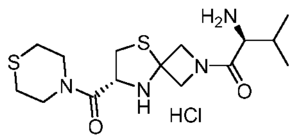 |
| **A-419** | 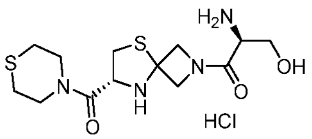 | **A-420** | 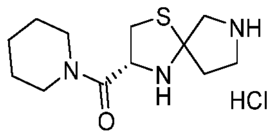 |
| **A-421** | 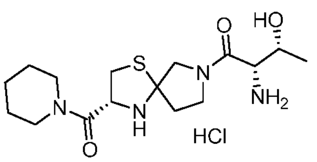 | **A-422** | 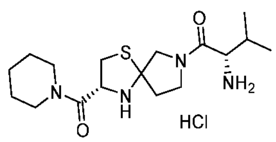 |
| **A-423** | 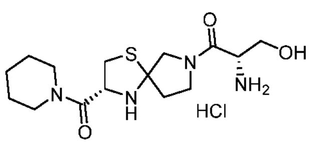 | **A-424** | 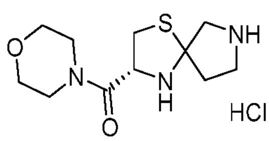 |

(continued)

| Number | Structural formula | Number | Structural formula |
|---|---|---|---|
| **A-425** | 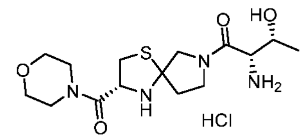 | **A-426** | 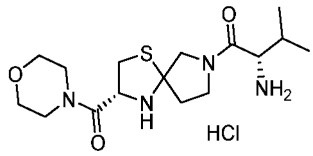 |
| **A-427** | 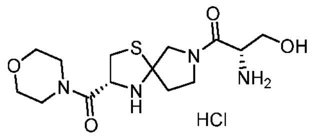 | **A-428** | 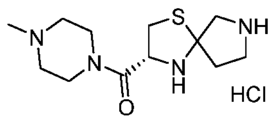 |
| **A-429** | 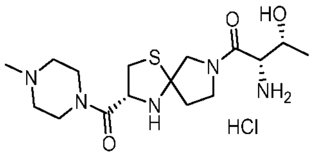 | **A-430** | 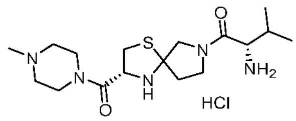 |
| **A-431** | 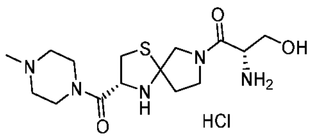 | **A-432** | 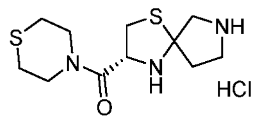 |
| **A-433** | 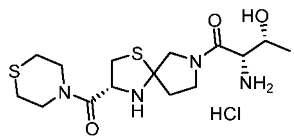 | **A-434** | 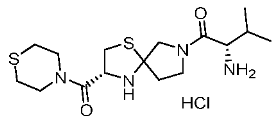 |
| **A-435** | 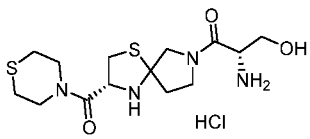 | **A-436** | 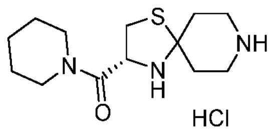 |
| **A-437** | 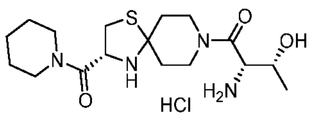 | **A-438** | 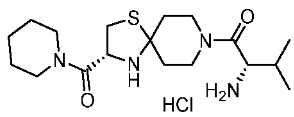 |
| **A-439** | 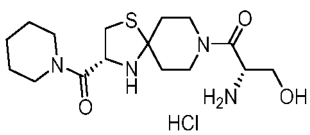 | **A-440** | 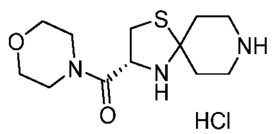 |
| **A-441** | 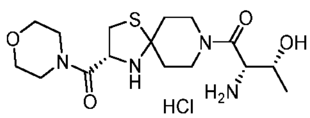 | **A-442** | 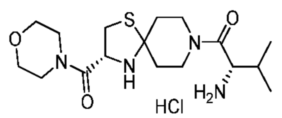 |
| **A-443** | 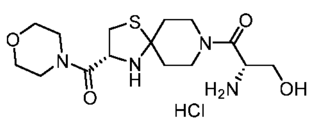 | **A-444** | 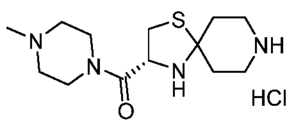 |

(continued)

| Number | Structural formula | Number | Structural formula |
|---|---|---|---|
| **A-445** | 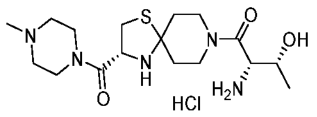 | **A-446** | 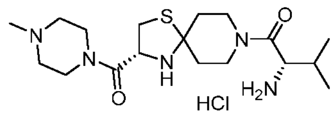 |
| **A-447** | 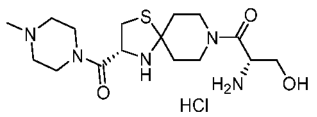 | **A-448** | 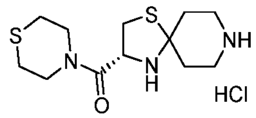 |
| **A-449** | 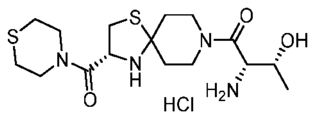 | **A-450** | 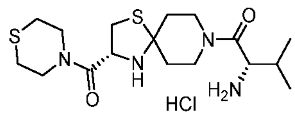 |
| **A-451** | 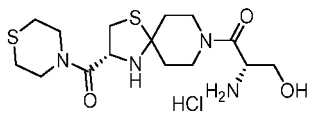 | **A-452** | 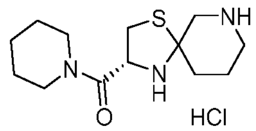 |
| **A-453** | 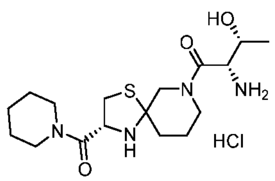 | **A-454** | 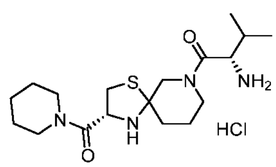 |
| **A-455** | 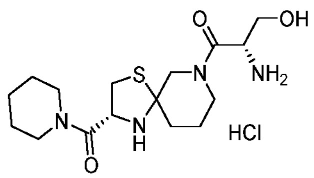 | **A-456** | 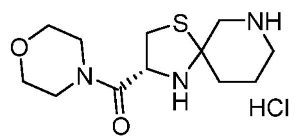 |
| **A-457** | 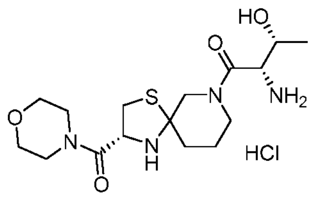 | **A-458** | 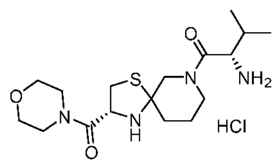 |
| **A-459** | 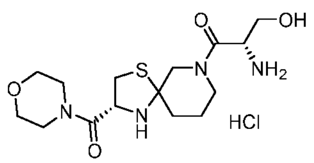 | **A-460** | 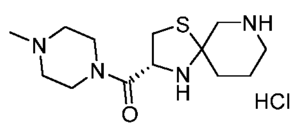 |
| **A-461** | 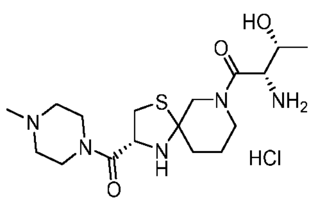 | **A-462** | 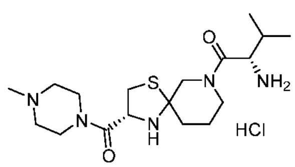 |

(continued)

| Number | Structural formula | Number | Structural formula |
|---|---|---|---|
| **A-463** | 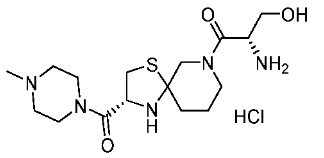 | **A-464** | 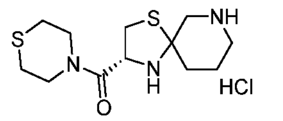 |
| **A-465** | 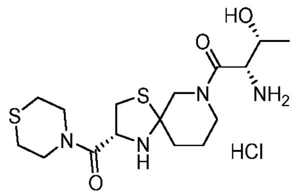 | **A-466** | 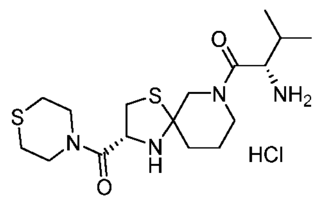 |
| **A-467** | 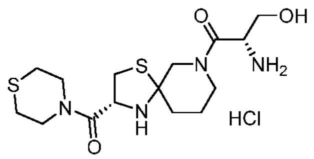 | **A-468** | 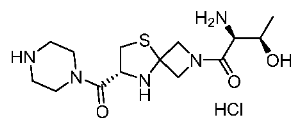 |
| **A-469** | 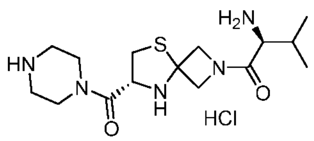 | **A-470** | 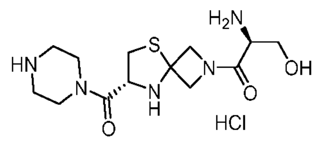 |
| **A-471** | 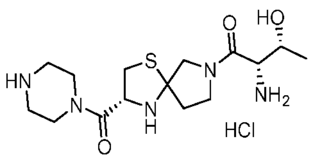 | **A-472** | 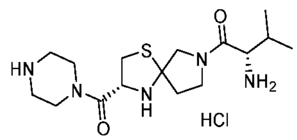 |
| **A-473** | 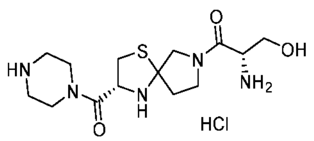 | **A-474** | 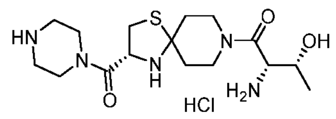 |
| **A-475** | 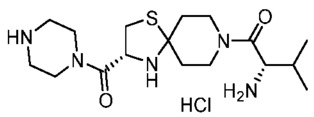 | **A-476** | 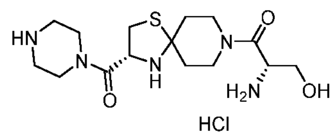 |
| **A-477** | 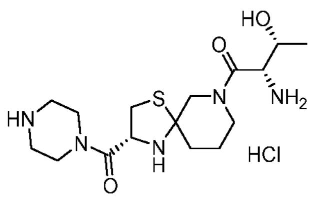 | **A-478** | 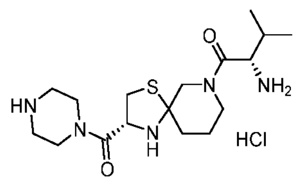 |
| **A-479** | 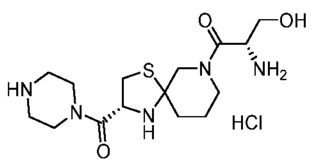 | | |

Preparation of compound

The compound of the invention can be prepared by various methods known in the field.

In some embodiments, when $R_1$, $R_2$ and $R_3$ are H in the formula I, the compound of the formula I can be prepared by the following steps:

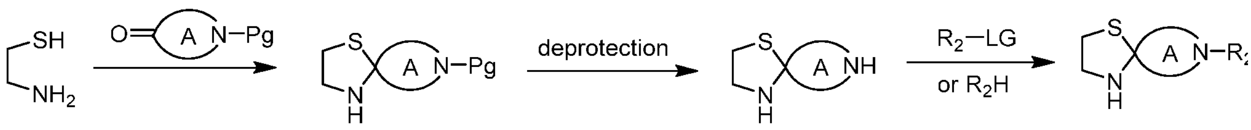


I-1 I-2 I-3

In some embodiments, the following series of the compounds of the formula I can be further obtained from the compound of the formula I-1:

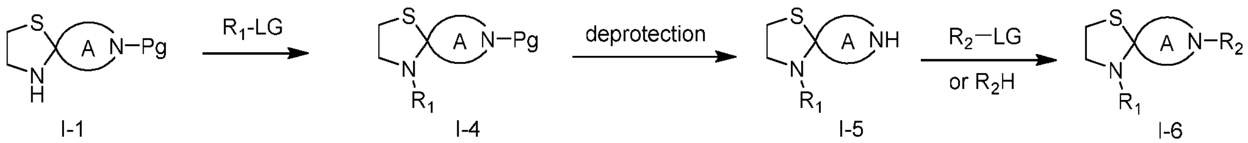


I-1 I-4 I-5 I-6

In some embodiments, the following series of the compounds of the formula I can be obtained from L-Csteine Methyl Ester Hydrochloride as a starting material:

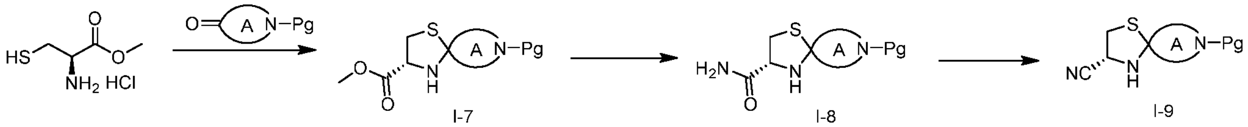

I-7 I-8 I-9

In some embodiments, the compounds of the formulas I-7, I-8, and I-9 can be respectively further deprotected, and reacted with $R_2$-LG or $R_3$H to obtain the corresponding compound of the formula I.

In the above scheme, LG represents a leaving group of a nucleophilic substitution reaction, e.g., halogen, etc.; Pg represents an amino protecting group, e.g., Boc, Fmoc, etc.

In some embodiments, the sequence of the above experimental steps can be adjusted according to a target product structure.

Treatment and Pharmaceutical composition

In another respect, the invention provides a pharmaceutical composition, comprising a compound of the invention, a pharmaceutically acceptable salt or ester, stereoisomer or solvate thereof; optionally, the pharmaceutical composition further comprises one or more pharmaceutically acceptable excipients.

In some embodiments, the pharmaceutical composition of the invention comprises the compound of the invention, and its pharmaceutically acceptable excipients.

In some embodiments, the pharmaceutical composition of the present invention contains 0.1 to 90 weight% of the compound of the formula I and/or its physiologically acceptable salt.

The compound in the pharmaceutical composition of the present invention, a pharmaceutically acceptable salt or ester, stereoisomer or solvate thereof can be used for a subject for:

(a) Regulation (e.g., up- or down-regulation) of activity of an NMDA receptor;
(b) Enhancement of activity of an NMDA receptor;
(c) Prevention of depression;
(d) Treatment of depression;
(e) Prevention of anxiety;
(f) Treatment of anxiety;
(g) Prevention of stroke;
(h) Treatment of stroke;

(i) Prevention of Huntington's disease;
(j) Treatment of Huntington's disease;
(k) Prevention of Alzheimer's disease;
(l) Treatment of Alzheimer's disease;
(m) Prevention of neuralgia;
(n) Treatment of neuralgia;
(o) Prevention of schizophrenia;
(p) Treatment of schizophrenia;
(q) Any combination of (a) - (p).

In another respect, the present invention provides use of the compound, a pharmaceutically acceptable salt or ester, stereoisomer or solvate thereof in preparation of medicaments for:

(a) Regulation (e.g., up- or down-regulation) of activity of an NMDA receptor;
(b) Enhancement of activity of an NMDA receptor;
(c) Prevention of depression;
(d) Treatment of depression;
(e) Prevention of anxiety;
(f) Treatment of anxiety;
(g) Prevention of stroke;
(h) Treatment of stroke;
(i) Prevention of Huntington's disease;
(j) Treatment of Huntington's disease;
(k) Prevention of Alzheimer's disease;
(l) Treatment of Alzheimer's disease;
(m) Prevention of neuralgia;
(n) Treatment of neuralgia;
(o) Prevention of schizophrenia;
(p) Treatment of schizophrenia;
(q) Any combination of (a) - (p).

In another respect, the present invention provides a method for preventing and/or treating depression, anxiety, stroke, Huntington's disease, Alzheimer's disease, neuralgia or schizophrenia in subjects, the method includes administering an effective amount of the compound of the invention, pharmaceutically acceptable salt or ester, stereoisomer or solvate thereof or the pharmaceutical composition of the invention to the subjects in need.

In another respect, the present invention provides a method for regulating (e.g., up- or down-regulating) activity of NMDA receptor (e.g., a human NMDA receptor) in vivo or in vitro, including providing a subject, a mammalian cell or an NMDA receptor with an effective amount of the compound of the formula I of the invention, pharmaceutically acceptable salt or ester, stereoisomer or solvate thereof.

In another respect, the present invention provides the compound of the formula I, pharmaceutically acceptable salt or ester, stereoisomer or solvate thereof for use in treatment and/or prevention of depression, anxiety, stroke, Huntington's disease, Alzheimer's disease, neuralgia or schizophrenia.

In another respect, the present invention provides the compound of the formula I, pharmaceutically acceptable salt or ester, stereoisomer or solvate thereof for use in in-vivo or in-vitro regulation (e.g., up- or down-regulation) of the activity of an NMDA receptor (e.g., human NMDA receptor).

The compound of the present invention, a pharmaceutically acceptable salt or ester, stereoisomer or solvate thereof, or the pharmaceutical composition of the present invention can be prepared to any dosage form known in the medical field, such as tablets, pills, suspensions, emulsions, solutions, gels, capsules, powders, granules, elixir, lozenges, suppositories, injections (including injections, sterile powders for injection, and concentrated solutions for injection), inhalants, sprays, etc. A preferred dosage form depends on an intended administration mode and therapeutic use. The pharmaceutical composition of the invention should be sterile and stable under production and storage conditions. One preferred dosage form is an injection. The injection can be a sterile injectable solution. For example, the sterile injectable solution can be prepared by the following steps: doping a required dose of the compound of the present invention, a pharmaceutically acceptable salt or ester, stereoisomer or solvate therof, in a suitable solvent, and optionally, doping other desired constituents (including, but not limited to, a pH modifying agent, a surfactant, an ionic strength enhancing agent, an isotonic agent, a preservative, a diluent, or any combination thereof) at the same time, and then the solution is subjected to filtration and sterilization. In addition, the sterile injectable solution can be prepared as a sterile lyophilized powder (e.g., by vacuum drying or freeze-drying) to facilitate storage and use. The sterile lyophilized powder can be

dispersed in a suitable carrier before use, such as sterile pyrogen-free water.

In addition, the compound of the present invention, a pharmaceutically acceptable salt or ester, stereoisomer or solvate thereof may exist in the pharmaceutical composition in a unit dose form for application.

The compound of the present invention, a pharmaceutically acceptable salt or ester, stereoisomer or solvate thereof, and the pharmaceutical composition of the present invention can be applied by any suitable method known in the field including, but not limited to, oral administration, oral, sublingual, ocular, topical, parenteral, rectal, intrathecal, intracytoplasmic reticulum, inguinal, intravesical, topical (e.g., powder, ointments, or drops), or nasal routes. However, for many therapeutic uses, the preferred administration route/mode is parenteral administration (e.g., intravenous injection, subcutaneous injection, intraperitoneal injection, intramuscular injection). The technician should understand that an administration route and/or mode can be changed according to an intended purpose. In a preferred embodiment, the compound of the present invention, a pharmaceutically acceptable salt or ester, stereoisomers or solvate thereof, and a pharmaceutical composition are administered by intravenous infusion or injection.

The pharmaceutical composition of the present invention may include the compound of the invention, a pharmaceutically acceptable salt or ester, stereoisomers, or solvate thereof with a "therapeutically effective amount" or a "prophylactically effective amount". The term "prophylactically effective amount" refers to the amount sufficient to prevent, stop or delay the occurrence of diseases. The term "therapeutically effective amount" refers to the amount sufficient to treat or at least partially prevent a disease and its complications of a patient who already has the disease. The therapeutically effective amount of the compound, a pharmaceutically acceptable salt or ester, stereoisomer or solvate thereof may vary according to the following factors: severity of disease to be treated, an overall state of patient's own immune system, patient's general conditions such as age, weight and sex, an administration mode of medicine, and other concurrent treatment measures.

In the present invention, a dosage regimen can be adjusted to obtain the best target response (e.g., therapeutic or preventive response). For example, single administration can be applied, multiple administration over a period of time also can be applied, or a dose can be reduced or increased proportionally with the urgency of the treatment.

A typical non-limit range for prophylactically or therapeutically effective amount of the compound of the present invention, a pharmaceutically acceptable salt or ester, stereoisomer, or solvate thereof is 0.02 to 100 mg/kg, e.g., 0.1 to 100 mg/kg, 0.1 to 50 mg/kg, or 1 to 50 mg/kg. It should be noted that the dose can be changed according to the type and severity of symptoms of the disease to be treated. In addition, it is understood by technicians in the field that, for any particular patient, a particular dosage regimen should be adjusted over time in accordance with the patient's needs and the professional evaluation of a physician; and the dosage ranges given herein are for illustrative purposes only and do not limit the use or scope of the pharmaceutical composition of the present invention.

In the present invention, the subject can be a mammal such as a human.

In the present invention, unless otherwise stated, scientific and technical terms used herein have the meanings generally understood by the technicians in the field. Moreover, cell culture, biochemistry, nucleic acid chemistry, immunology laboratory and other operation steps used in this invention are all routine steps widely used in the corresponding fields. In the meantime, for a better understanding of the present invention, definitions and explanations of the relevant terms are provided below.

As used herein, the term "natural amino acids" mainly includes the following 20 kinds of common amino acids: alanine (Ala), arginine (Arg), aspartic acid (Asn), asparagine (Asp), cysteine (Cys), glutamine (Gln), glutamic acid (Glu), glycine (Gly), histidine (His), leucine (Leu), isoleucine (Ile), lysine (Lys), methionine (Met), phenylalanine (Phe), proline (Pro), serine (Ser), threonine (Thr), tryptophan (Trp), tyrosine (Tyr) and valine (Val). The amino acid molecules are known to contain two functional groups, an amino group and a carboxyl group. The "amino acid fragment" described in this invention refers to a remaining part of amino acid molecules after removing a hydrogen from the amino group or deleting a hydroxyl group from the carboxyl group. Therefore, the "carboxyl residue" refers to the structure remaining after one hydroxyl group is deleted from the carboxyl group in the amino acid molecule.

As used herein, the term "alkyl" is defined as a linear or branched saturated aliphatic hydrocarbon group. In some embodiments, the alkyl contains 1 to 6 (e.g., 1, 2, 3, 4, 5, or 6) carbon atoms. For example, as used herein, the term "C1-C6 alkyl" refers to straight or branched alkyl groups of 1 to 6 carbon atoms (e.g. methyl, ethyl, n-propyl, isopropyl, n-butyl, isobutyl, sec-butyl, tert-butyl, n-amyl, isoamyl, neo-amyl or n-hexyl, etc.).

As used herein, the term "C1- C6 alkoxy" refers to the C1-C6 alkyl-O-group, wherein the C1 - C6 alkyl is described previously. Unrestricted examples of suitable C1 - C6 alkoxy include methoxy, ethoxy, and isopropoxy groups, and the like.

As used herein, the term "C2-C6 acyl" refers to a univalent group containing 2 to 6 (e.g. 2, 3, 4, 5, or 6) carbon atoms with a general formula R-C (O) -, wherein R can be an alkyl, enyl, etc. Unrestricted examples of suitable C2-C6 acyl include acetyl, propionyl, isopropionyl, n-butyryl, sec-butyryl, t-butyryl, n-pentanoyl, isovaleryl, and tervaloyl.

As used herein, the term "C1-C6 alkoxycarbonyl" refers to the C1-C6 alkyl-OC(O)-group, wherein the C1-C6 alkyl is described previously. Unrestricted examples of the suitable C1-C6 alkoxycarbonyl include methoxycarbonyl, ethoxycarbonyl, propoxycarbonyl, tert-butoxycarbonyl, etc.

As used herein, the term "aryl" refers to a monocyclic or condensed ring group with aromaticity. For example, phenyl, naphthyl, etc.

As used herein, the term "aliphatic heterocycle" refers to a cyclic aliphatic hydrocarbon that contains at least one ring heteroatom selected from N, O, and S. In some embodiments, the term "3- to 8-membered aliphatic heterocycle" refers to the above aliphatic heterocycle containing 3 to 8 ring members (e.g., 3, 4, 5, 6, 7, or 8), e.g., 3- to 8-membered N-containing aliphatic heterocycle, 4- to 6-membered N-containing aliphatic heterocycle. Specific examples include, but are not limited to:

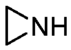 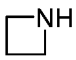 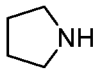 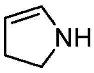 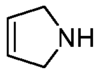 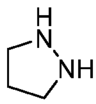 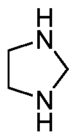 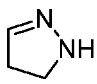

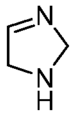 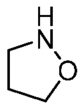 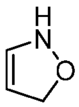 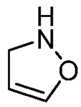 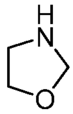 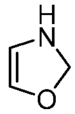 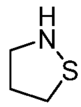 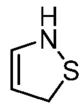

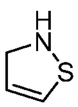 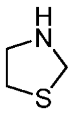 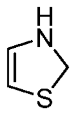 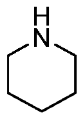 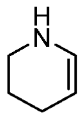 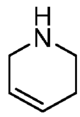 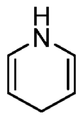 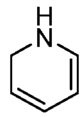

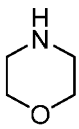 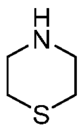 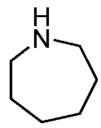 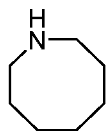 etc.

As used herein, the term "nitrogen-containing aliphatic heterocycle" refers to the aliphatic heterocycle containing at least one N atom, wherein the aliphatic heterocycle is defined as previously; the term of 3- to 8-membered nitrogen-containing aliphatic heterocycle refers to the nitrogen-containing aliphatic heterocycle described above that contains 3 to 8 ring members (e.g., 3, 4, 5, 6, 7, or 8).

As used herein, the term "3- to 8-membered nitrogen-containing aliphatic heterocycle-C1-C4 acyl" refers to a monovalent group of the 3- to 8-membered nitrogen-containing aliphatic heterocycle-R-C (O) -structure, wherein, the 3- to 8-membered nitrogen-containing aliphatic heterocycle is described previously, and R does not exist or is alkyl, enyl, etc.

Examples include, but are not limited to:

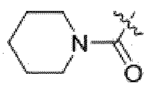, 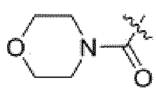, 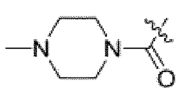,

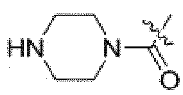 and 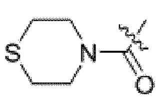.

As used herein, the terms "substitution" and "substituted" refer to selective substitution of one or more (e.g., one, two, three, or four) hydrogens connected to a specified atom by an indicated group, provided that the normal valence of the specified atom in the current situation is not exceeded and that the substitution results in a stable compound. The combination of substituents and/or variables are only allowed if such combination forms a stable compound.

If substituents are described as "independently selected from" a set of groups, each substituent is selected independently of the other. Therefore, each substituent may be the same or different from another (other) substituent.

As used herein, the term "one or more" means 1 or more under reasonable conditions, e.g. 2, 3, 4, 5 or 10.

Unless otherwise indicated, as used herein, a connection point of the substituent may come from any suitable

position of the substituent.

As used herein, the term "stereoisomer" refers to an isomer that has a same atom connection order but has different spatial arrangement modes of atoms, e.g., enantiomers, geometric isomers, etc. The term "enantiomer" indicates an isomer formed due to at least one asymmetric center. In a compound with one or more (e.g., one, two, three, or four) asymmetric centers, enantiomers and diastereomers can be generated. When the molecule contains double bonds, such as C = C, C = N or N = N double bonds, the two atoms connected by the double bonds cannot rotate freely and can also exist in the form of geometric isomers (cis/trans).

As used herein, a solid line (), a solid wedge line (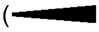) or a dashed wedge line (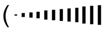) can be used to delineate the chemical bonds of the compound of the present invention. The solid line is used to describe bonding to asymmetric carbon atoms, which indicates that all possible stereoisomers formed by the side carbon atoms are included. The solid or dashed wedge line is used to describe bonding to the asymmetric carbon atoms, which indicates the existence of the indicated stereoisomers.

The compound of the present invention may exist in the form of a solvate (preferably a hydrate), wherein the compound of the present invention contains a polar solvent as a structural element of crystal lattice of the compound, in particular water, methanol or ethanol. The amount of the polar solvent, especially water, can be existed in stoichiometric or non-stoichiometric ratios. It should be understood that, although any solvate of the compound of the formula I used in the treatment of the disease or condition of this application may provide different properties (including pharmacokinetic properties), once the solvate is absorbed into a subject, the compound of the formula I is obtained, such that the use of the compound of the formula I covers the use of any solvate of the compound of the formula I respectively.

It should also be understood that some of the compounds of the present invention may exist in free-form for therapeutic use or, where applicable, in the form of their pharmaceutically acceptable derivatives. In the present invention, the pharmaceutically acceptable derivative includes, but is not limited to, the pharmaceutically acceptable salt or ester that are capable of providing, directly or indirectly, the compound of the present invention or metabolites or residues thereof after the pharmaceutically acceptable derivative is administered to a patient in need of it.

The pharmaceutically acceptable salts of the compound include an acid addition salt and an alkali addition salt.

An overview of suitable salts can be seen in Stahl and Wermuth's "Handbook of Pharmaceutical Salts: Properties, Selection, and Use" (Wiley-VCH, 2002), or S. M. Berge et al., "Pharmaceutical Salts", J. Pharm. Sci. 1977, 66, 1-19. A method for preparing the pharmaceutically acceptable salts of the compound of the present invention is known to technicians in the field.

The suitable acid addition salt is formed from acids that form the pharmaceutically acceptable salts. The suitable base addition salt is formed from bases that form pharmaceutically acceptable salts. In some embodiments, suitable inorganic acids are hydrochloric acid, hydrobromic acid, hydroiodic acid, sulfuric acid, phosphoric acid, or nitric acid, etc.; organic acids are formic acid, acetic acid, acetoacetic acid, pyruvic acid, trifluoroacetic acid, propionic acid, butyric acid, caproic acid, enanthic acid, undecanoic acid, lauric acid, benzoic acid, salicylic acid, 2- (4-hydroxybenzoyl) -benzoic acid, camphoric acid, cinnamic acid, cyclopentanepropionic acid, glucaric acid, 3-hydroxy-2-naphthoic acid, nicotinic acid, pamoic acid, pectinic acid, 3-phenylpropionic acid, picric acid, pivalic acid, 2-hydroxyethanesulfonic acid, ecanoic acid, aminosulfonic acid, trifluoromethanesulphonic acid, dodecyl sulfuric acid, ethanesulfonic acid, benzenesulfonic acid, p-toluenesulfonic acid, methanesulfonic acid, 2-naphthalic acid, naphthalene disulfonic acid, camphoric acid, citric acid, tartaric acid, stearic acid, lactic acid, oxalic acid, propandioic acid, succinic acid, malic acid, adipic acid, alginic acid, maleic acid, fumaric acid, D-glucosylic acid, mandelic acid, ascorbic acid, glucoheptonic acid, glycerophosphoric acid, aspartic acid, sulfosalicylic acid, etc. For example, HCl (or hydrochloric acid), HBr (or a hydrobromic acid solution), methanesulfonic acid, sulfuric acid, tartaric acid, or fumaric acid can be used to form pharmaceutically acceptable salts with the compound represented by the formula I.

As used herein, the term "pharmaceutically acceptable ester" means an ester derived from the compound of the present invention, which includes a physiologically hydrolyzable ester (the ester can be hydrolyzed under physiological conditions to release the compound of the present invention in form of free acid or alcohol). The compound of the invention can also be an ester.

As used herein, the term "metabolic stability" refers to the ability of a compound to enter and stably exist in the body as a prototype drug and not be metabolized to other structural forms.

As used herein, the term "subject" can refer to a patient or other animals that receive a composition of the present invention to treat, prevent, mitigate, and/or alleviate a disease or condition described in the present invention, and the animals particularly refers to mammals such as humans, dogs, monkeys, cattle, horses, etc.

As used herein, the term "excipients" refers to an excipient or a medium used to administer the compound, including, but not limited to, diluents, disintegrants, precipitation inhibitors, surfactants, flow aids, adhesives, lubricants, coating materials, etc. The examples of excipients include but are not limited to aluminum monostearate, aluminum stearate, carboxymethylcellulose, sodium carboxymethylcellulose, crospovidone, glyceryl isostearate, glyceryl monostearate, hydroxyethyl cellulose, hydroxymethylcellulose, octacosyl hydroxystearate, hydroxypropyl cellulose, lactose, a

lactose monohydrate, magnesium stearate, mannitol, microcrystalline cellulose, etc.

Specific Models for Carrying Out the Present Invention

The embodiments of the present invention will be completely described below in conjunction with examples, but it is understood by the technicians in the field that the following examples are intended only to illustrate the invention and are not considered to limit the scope of the invention. If no specific condition is indicated in the examples, the general condition or the condition recommended by a manufacturer shall be followed. The reagents or apparatuses used without indication of the manufacturer are conventional products that can be obtained by market purchase.

Abbreviation Table

| Abbreviation | Term |
|---|---|
| NMDA | N-methyl-D-aspartic acid |
| DCM | dichloromethane |
| DMF | N,N-DiMethylforMaMide |
| MeOH | Methanol |
| EtOAc | ethyl acetate |
| PE | Petroleum ether |
| TEA | Triethylamine |
| TFAA | Trifluoroacetic anhydride |
| tBu | tert-butyl |
| HOBt | N-Hydroxybenzotrizole |
| EDCI | N-(3-Dimethylaminopyl)-N'-Ethylcarbodiimede Hydrochloride |
| Thr | Threonine |
| Ser | Serine |
| Val | Valine |
| Boc | Tert-butoxycarbonyl |
| Fmoc | Fluorene methoxycarbonyl |
| TBTU | 2-(1H-Benzotriazole-1-yl)-1,1,3,3-tetramethyluronium tetrafluoroborate |
| TLC | thin layer chromatography |

Exemplary Synthetic Scheme

Examples of synthesis of A-001 and A-002

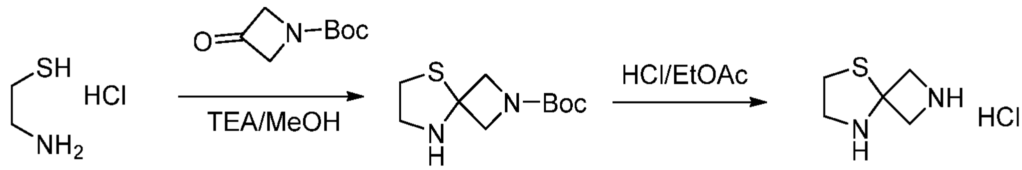


A-002

A-001

At -15°C, 1.14g (10 mmol, 1.0eq) of a raw material mercaptoethylamine hydrochloride was dissolved in 100ml of methanol, 1.75ml (15 mmol, 1.5eq) of triethylamine was added and sufficiently dissociated, 1.8g (10 mmol, 1.0eq) of 1-Boc-3-azetidinone was added, the materials were continuously reacted at a low temperature for 15 min and then transferred to room temperature; after the completion of the materials reaction was monitored by TLC, vacuum concentration was performed, a concentrate was dissolved with 100ml of ethyl acetate, washed with a small amount of water for many times (15ml×4), dried with anhydrous sodium sulfate, filtered, and then concentrated. A crude product was purified with a silica gel column (PE: EtOAc = 2:1) to obtain a total of 2.25 g of a white solid A-002 with a yield of 92.6%. $^1$H-NMR (400MHz, DMSO-$d_6$), δppm: 1.37(s, 9H,-$CH_3$),3.12(t,2H, J=8.2Hz), 3.34 (t,2H,J=6.1Hz), 4.1-4.20(d,2H,J=9.2Hz), 4.59-4.62(d,2H,J=9.5Hz),9.3 (s,1H). M+1:231.11.

1 g (6 mmol) of an intermediate A-002 was dissolved in 10 mL of anhydrous ethyl acetate, and then 10 mL of 4 mol/L HCl/EtOAc was added and stirred overnight at room temperature, and a white solid was generated in the system; after the completion of the materials reaction was monitored by TLC, the reaction was terminated. Filtration and washing with anhydrous diethyl ether were performed, and then a total of 0.54 g of a white solid A-001 was obtained with a yield of 76%. $^1$H-NMR (400MHz, DMSO-$d_6$), δppm: 3.10(t,2H, J=6.8Hz), 3.37(t,2H, J=6.4Hz), 4.1-4.15(d,2H, J=11.2Hz),4.55-4.6(d,2H,J=10.5Hz), 9.5(s,1H), 9.8(s,1H). M+1:131.06.

According to the preparation method of A-002, the corresponding products A-016, A-030 and A-044 of five-membered ring or six-membered ring can be prepared. The methods were the same as that of A-002, except that the corresponding cycloketone was replaced; similarly, the corresponding products A-015, A-029 and A-043 of five-membered ring or six-membered ring were prepared according to the preparation method of A-001.

A-016: the raw material 1-Boc-3-azetidinone in the synthesis step of A-002 was replaced with N-Boc-3-pyrrolidone, and the rest operations were the same as those in the synthesis of A-002. M+1: 245.12.

A-030: the raw material 1-Boc-3-azetidinone in the synthesis step of A-002 was replaced with N-Boc-4-piperidone and the rest operations were the same as those in the synthesis of A-002. M+1: 259.14.

A-044: the raw material 1-Boc-3-azetidinone in the synthesis step of A-002 was replaced with N-Boc-3-piperidone, and the rest operations were the same as those in the synthesis of A-002. M+1: 259.14.

A-015: the intermediate A-002 in the synthesis step of A-001 was replaced with A-016, and the rest operations were the same as those in the synthesis of A-001. M+1: 145.07.

A-029: the intermediate A-002 in the synthesis step of A-001 was replaced with A-030, and the rest operations were the same as those in the synthesis of A-001. M+1: 159.09.

A-043: the intermediate A-002 in the synthesis step of A-001 was replaced with A-044, and the rest operations were the same as those in the synthesis of A-001. M+1: 159.09.

Example of A-003

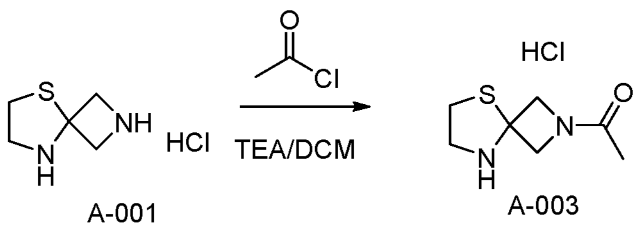

A-001 A-003

A total of 0.4 g (2.4 mmol) of the compound A-001 was dissolved in 20 ml of anhydrous DCM, and 665 μL (4.8 mmol) of TEA was added for dissociation; 155 μL (2.2 mmol) of acetyl chloride was added to 5 ml of DCM, and it was slowly dripped to the above reaction system with a dropper funnel under ice bath conditions, and the materials were continuously reacted at low temperature for half an hour and then moved to room temperature; the reaction was performed for 4 h at room temperature; when the completion of the materials reaction was monitored by TLC, the reaction was terminated. A small amount of water was used for washing (5 ml × 3), an organic phase was taken, dried, filtered, concentrated, and separated through a silica gel column to obtain a brown oily substance, after stirring with hydrochloric acid to generate salt, a solid was generated, and a total of 0.31 g of a product A-003 was obtained with a yield of 61.9%. $^1$H-NMR (400MHz, DMSO-$d_6$), δppm: 2.03 (s,3H),3.15 (t, 2H, J=6.8Hz), 3.29 (t,2H, J=7.4Hz), 4.11-4.21 (d,2H, J=7.1Hz), 4.51-4.63 (d,2H,J=8.1Hz), 9.53 (s,1H). M+1:173.07.

Examples of preparation of A-004 and A-005:

A-004: acetyl chloride in the synthesis step of A-003 was replaced with isobutyryl chloride, and the rest operations were the same as those in the synthesis of A-003. M+1: 201.10.

A-005: acetyl chloride in the synthesis step of A-003 was replaced with pivaloyl chloride, and the rest operations were the same as those in the synthesis of A-003. M+1: 215.11.

Examples of preparation of A-017 to A-019:

A-017: A-001 in the synthesis step of A-003 was replaced with A-015, and the rest operations were the same as those in the synthesis of A-003. M+1: 187.08.

A-018: A-001 in the synthesis step of A-003 was replaced with A-015 and acetyl chloride was replaced with isobutyryl chloride, and the rest operations were the same as those in the synthesis of A-003. M+1: 215.11.

A-019: A-001 in the synthesis step of A-003 was replaced with A-015 and acetyl chloride was replaced with pivaloyl chloride, and the rest operations were the same as those in the synthesis of A-003. M+1: 229.13.

Examples of preparation of A-031 to A-033:

A-031: A-001 in the synthesis step of A-003 was replaced with A-029, and the rest operations were the same as those in the synthesis of A-003. M+1: 201.10.

A-032: A-001 in the synthesis step of A-003 was replaced with A-029 and acetyl chloride was replaced with isobutyryl chloride, and the rest operations were the same as those in the synthesis of A-003. M+1: 229.13.

A-033: A-001 in the synthesis step of A-003 was replaced with A-029 and acetyl chloride was replaced with pivaloyl chloride, and the rest operations were the same as those in the synthesis of A-003. M+1: 243.15.

Examples of preparation of A-045 to A-047:

A-045: A-001 in the synthesis step of A-003 was replaced with A-043, and the rest operations were the same as those in the synthesis of A-003. M+1: 201.10.

A-046: A-001 in the synthesis step of A-003 was replaced with A-043 and acetyl chloride was replaced with isobutyryl chloride, and the rest operations were the same as those in the synthesis of A-003. M+1: 229.13.

A-047: A-001 in the synthesis step of A-003 was replaced with A-043 and acetyl chloride was replaced with pivaloyl chloride, and the rest operations were the same as those in the synthesis of A-003. M+1: 243.15.

Example of A-006

The example illustrates a synthesis method of compound A-006.

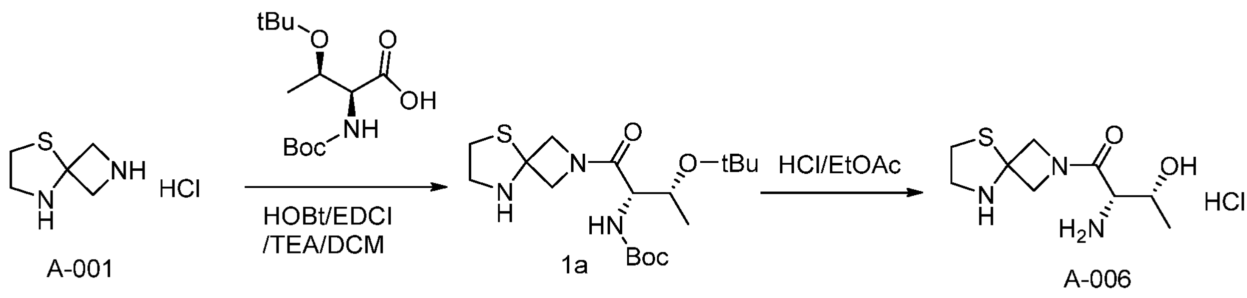

A-001 1a A-006

A total of 0.4 g (2.4 mmol) of the intermediate A-001 was dissolved in 20 ml of anhydrous DCM, 665 μL (4.8 mmol) of TEA was added for dissociation, and after stirring for 5 min, 0.63 g (2.3 mmol) of Boc-tBu-Thr, 0.49 g (3.6 mmol) of HOBt, and 0.62 g (3.6 mmol) of EDCI were added and the raw materials were reacted overnight at room temperature. When the completion of the raw materials reaction was monitored by TLC, the reaction was terminated. A saturated citric acid aqueous solution was used for washing (10 mL×2), and an organic phase was taken, dried with anhydrous sodium sulfate, filtered, concentrated, and separated through a silica gel column to obtain a total of 0.61 g of a colorless oily substance of intermediate 1a with a yield of 68.8%. $^1$H-NMR (400MHz, DMSO-$d_6$), δppm: 1.14-1.20(m,12H), 1.37 (s,9H), 3.24 (t,2H, J=7.2Hz), 3.38-3.65 (m,2H), 3.72-3.95 (m,3H), 4.10-4.30 (m,1H), 4.65-4.94 (m,3H), 7.34 (s,1H), 8.23 (s,1H). M+1: 388.22.

A total of 0.5 g (1.3 mmol) of the intermediate 1a was dissolved in 5 ml of anhydrous ethyl acetate, and 10 mL

of 4 mol/L HCl/EtOAc was added and the raw materials were reacted overnight at room temperature, and a white solid was generated in the system; when the completion of the materials reaction was monitored by TLC, the reaction was terminated. The product was filtered under reduced pressure and washed with anhydrous diethyl ether to obtain a total of 0.25 g of a white solid A-006 with a yield of 73%. $^{1}$H-NMR (400MHz, DMSO-$d_6$), δppm: 1.21(m,3H),3.21(t,2H, J=6.4Hz), 3.36-3.65 (m,2H), 3.7-3.97 (m,3H), 4.10-4.30 (m,1H), 4.60-4.71 (m,2H), 4.86-5.01(m,1H), 5.65 (s,1H), 8.23-8.28 (d,2H, J=21Hz). M+1: 232.10.

Examples of preparation of A-007 to A-014:

A-007:

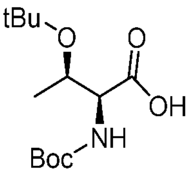

in the synthesis step of A-006 was replaced with

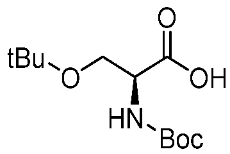

,

and the rest operations were the same as those in A-006 synthesis. M+1: 218.09.

A-007-1:

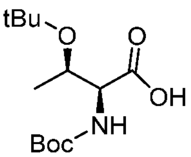

in the synthesis step of A-006 was replaced with

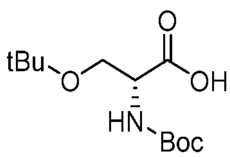

,

and the rest operations were the same as those in A-006 synthesis. M+1: 218.09.

A-008:

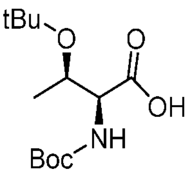

in the synthesis step of A-006 was replaced with

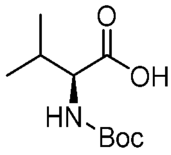

,

and the rest operations were the same as those in A-006 synthesis. M+1: 230.12.

A-008-1:

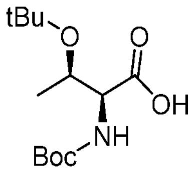

in the synthesis step of A-006 was replaced with

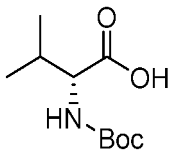

,

and the rest operations were the same as those in A-006 synthesis. M+1: 230.12.

A-009:

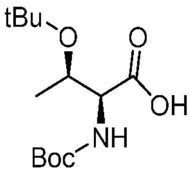

in the synthesis step of A-006 was replaced with

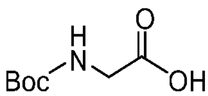

,

and the rest operations were the same as those in A-006 synthesis. M+1: 188.08.

A-010:

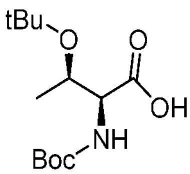

in the synthesis step of A-006 was replaced with

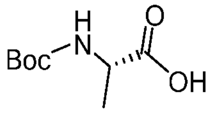,

and the rest operations were the same as those in A-006 synthesis. M+1: 202.09.

A-011:

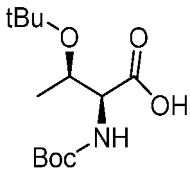

in the synthesis step of A-006 was replaced with

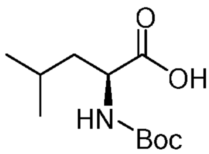,

and the rest operations were the same as those in A-006 synthesis. M+1: 244.14.

A-012:

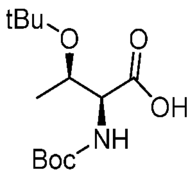

in the synthesis step of A-006 was replaced with

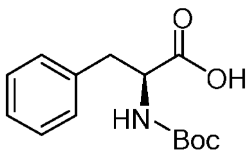,

and the rest operations were the same as those in A-006 synthesis. M+1: 278.12.

A-013:

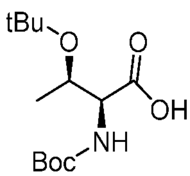

in the synthesis step of A-006 was replaced with

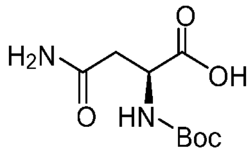

,

and the rest operations were the same as those in A-006 synthesis. M+1: 245.10.

A-014:

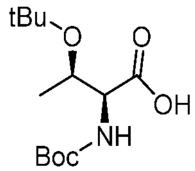

in the synthesis step of A-006 was replaced with

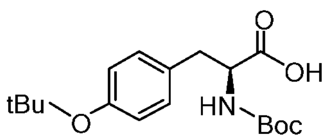

,

and the rest operations were the same as those in A-006 synthesis. M+1: 294.12.

Examples of preparation of A-020 to A-028:

A-020: A-001 in the synthesis step of A-006 was replaced with A-015, and the rest operations were the same as those in A-006 synthesis. M+1: 246.12.

A-021: A-001 in the synthesis step of A-006 was replaced with A-015,

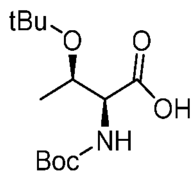

was replaced with

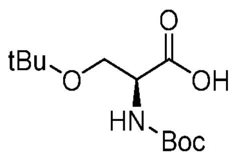

,

and the rest operations were the same as those in A-006 synthesis. M+1: 232.10.

A-022: A-001 in the synthesis step of A-006 was replaced with A-015,

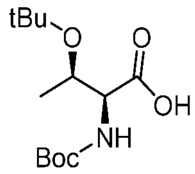

was replaced with

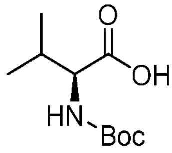,

and the rest operations were the same as those in A-006 synthesis. M+1: 244.14.

A-023: A-001 in the synthesis step of A-006 was replaced with A-015,

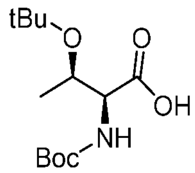

was replaced with

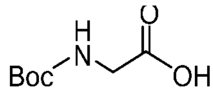

and the rest operations were the same as those in A-006 synthesis. M+1: 202.09.

A-024: A-001 in the synthesis step of A-006 was replaced with A-015,

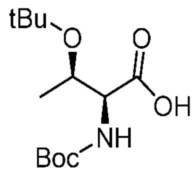

was replaced with

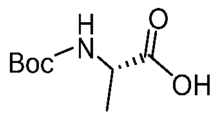

and the rest operations were the same as those in A-006 synthesis. M+1: 216.11.

A-025: A-001 in the synthesis step of A-006 was replaced with A-015,

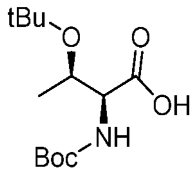

was replaced with

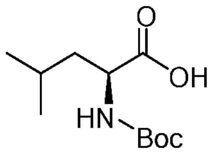

and the rest operations were the same as those in A-006 synthesis. M+1: 258.16.

A-026: A-001 in the synthesis step of A-006 was replaced with A-015,

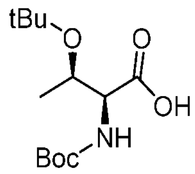

was replaced with

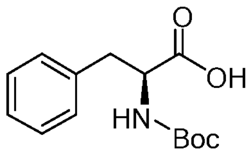

and the rest operations were the same as those in A-006 synthesis. M+1: 292.14.

A-027: A-001 in the synthesis step of A-006 was replaced with A-015,

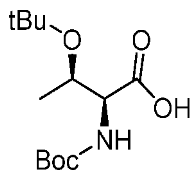

was replaced with

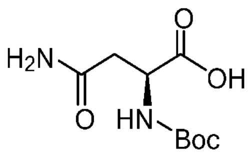

and the rest operations were the same as those in A-006 synthesis. M+1: 259.12.

A-028: A-001 in the synthesis step of A-006 was replaced with A-015,

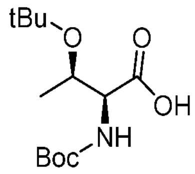

was replaced with

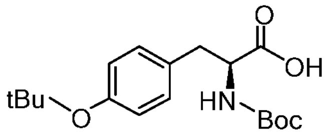

and the rest operations were the same as those in A-006 synthesis. M+1: 308.14.

Examples of preparation of A-034 to A-042:

A-034: A-001 in the synthesis step of A-006 was replaced with A-029, and the rest operations were the same as those in A-006 synthesis. M+1: 260.14.

A-035: A-001 in the synthesis step of A-006 was replaced with A-029,

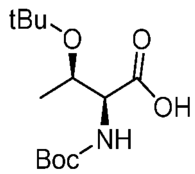

was replaced with

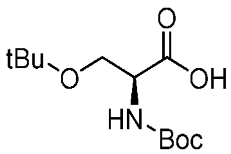,

and the rest operations were the same as those in A-006 synthesis. M+1: 246.12.

A-036: A-001 in the synthesis step of A-006 was replaced with A-029,

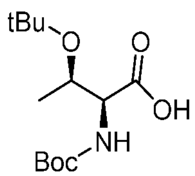

was replaced with

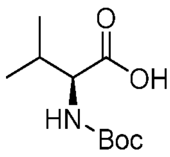,

and the rest operations were the same as those in A-006 synthesis. M+1: 258.16.

A-037: A-001 in the synthesis step of A-006 was replaced with A-029,

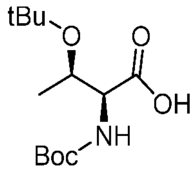

was replaced with

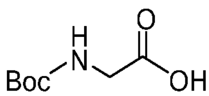

and the rest operations were the same as those in A-006 synthesis. M+1: 216.11.

A-038: A-001 in the synthesis step of A-006 was replaced with A-029,

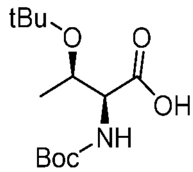

was replaced with

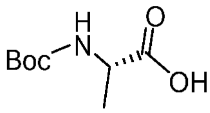

and the rest operations were the same as those in A-006 synthesis. M+1: 230.12.

A-039: A-001 in the synthesis step of A-006 was replaced with A-029,

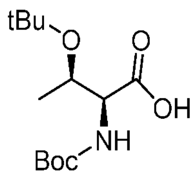

was replaced with

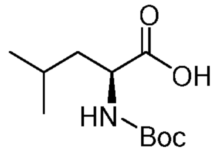

and the rest operations were the same as those in A-006 synthesis. M+1: 272.17.

A-040: A-001 in the synthesis step of A-006 was replaced with A-029,

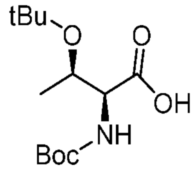

was replaced with

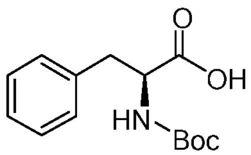

and the rest operations were the same as those in A-006 synthesis. M+1: 306.16.

A-041: A-001 in the synthesis step of A-006 was replaced with A-029,

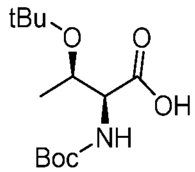

was replaced with

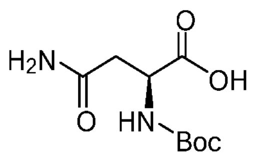

and the rest operations were the same as those in A-006 synthesis. M+1: 273.13.

A-042: A-001 in the synthesis step of A-006 was replaced with A-029,

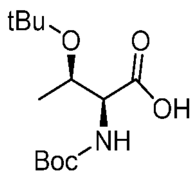

was replaced with

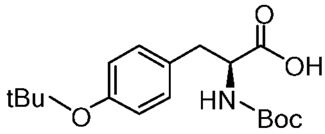

and the rest operations were the same as those in A-006 synthesis. M+1: 322.15.

Examples of preparation of A-048 to A-056:

A-048: A-001 in the synthesis step of A-006 was replaced with A-043, and the rest operations were the same as those in A-006 synthesis. M+1: 260.14.

A-049: A-001 in the synthesis step of A-006 was replaced with A-043,

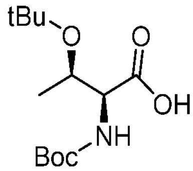

was replaced with

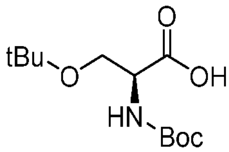,

and the rest operations were the same as those in A-006 synthesis. M+1: 246.12.

A-050: A-001 in the synthesis step of A-006 was replaced with A-043,

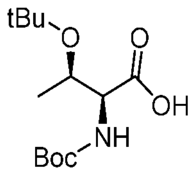

was replaced with

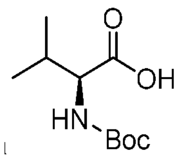,

and the rest operations were the same as those in A-006 synthesis. M+1: 258.16.

A-051: A-001 in the synthesis step of A-006 was replaced with A-043,

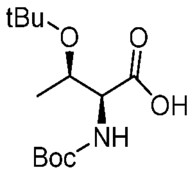

was replaced with

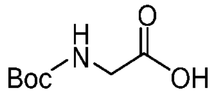,

and the rest operations were the same as those in A-006 synthesis. M+1: 216.11.

A-052: A-001 in the synthesis step of A-006 was replaced with A-043,

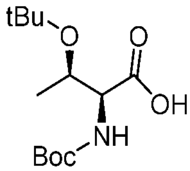

was replaced with

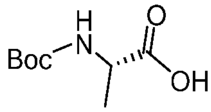,

and the rest operations were the same as those in A-006 synthesis. M+1: 230.12.

A-053: A-001 in the synthesis step of A-006 was replaced with A-043,

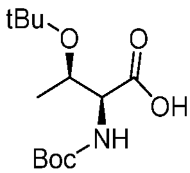

was replaced with

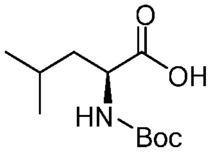,

and the rest operations were the same as those in A-006 synthesis. M+1: 272.17.

A-054: A-001 in the synthesis step of A-006 was replaced with A-043,

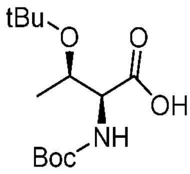

was replaced with

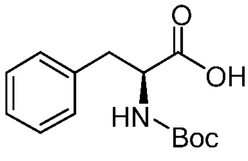,

and the rest operations were the same as those in A-006 synthesis. M+1: 306.16.

A-055: A-001 in the synthesis step of A-006 was replaced with A-043,

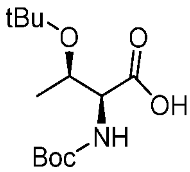

was replaced with

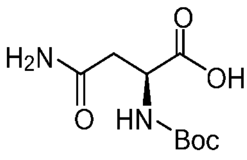,

and the rest operations were the same as those in A-006 synthesis. M+1: 273.13.

A-056: A-001 in the synthesis step of A-006 was replaced with A-043,

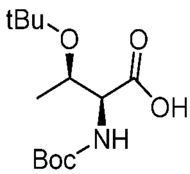

was replaced with

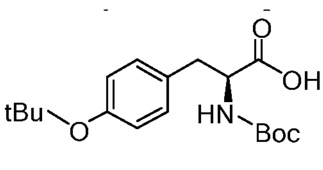,

and the rest operations were the same as those in A-006 synthesis. M+1: 322.15.

Example of A-059

The example illustrates a synthesis method of compound A-059.

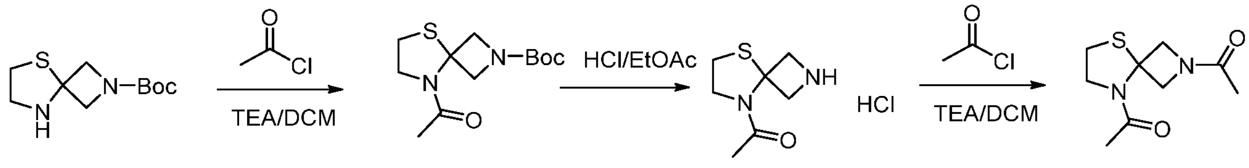


A-002 A-058 A-057 A-059

1. Synthesis of an intermediate A-058

A total of 0.6 g (2.6 mmol) of the compound A-002 was dissolved in 20 ml of anhydrous DCM, and 1.4 mL (10 mmol) of TEA was added; 350 μL (5 mmol) of acetyl chloride was added to 5 mL of DCM, and it was slowly dripped to the above reaction system with a dropper funnel under ice bath conditions, and the materials were reacted at low temperature for half an hour and then moved to room temperature, the reaction was continually performed for 4 h at room temperature; when the completion of the materials reaction was monitored by TLC, the reaction was terminated. After washing with water (10 mL×3), an organic phase was taken, dried, filtered, concentrated, and separated through a silica gel column to obtain a total of 0.57 g of a brown oily substance A-058 with a yield of 81.4%. $^1$H-NMR (400MHz, DMSO-$d_6$), δppm: 1.38 (s,9H), 2.05 (s,3H), 3.14 (t,2H, J=7.8Hz), 3.35 (t,2H,J=5.8Hz), 4.13-4.22 (d,2H,J=8.7Hz), 4.60-4.63 (d,2H, J=10.2Hz). M+1: 273.12.

2. Synthesis of an intermediate A-057

A-057 was obtained by deprotecting A-058 and was synthesized in the same method as that of A-001. $^1$H-NMR (400MHz, DMSO-$d_6$), δppm: 2.06 (s,3H), 3.17 (t,2H, *J*=6.3Hz), 3.34 (t,2H,J=5.8Hz), 4.09-4.17 (d,2H,J=6.9Hz), 4.49-4.61 (d,2H,J=7.9Hz), 9.21 (s,1H). M+1:173.07.

3. Synthesis of the end product A-059

A synthesis method of the end product A-059 was the same as that of the A-058. $^1$H-NMR (400MHz, DMSO-$d_6$), δppm: 1.73 (s,3H), 2.06 (s,3H), 3.01 (t,2H,J=6Hz), 3.74-3.82 (m,3H), 4.05-4.07 (d,1H,*J*=10Hz), 4.64-4.66 (d,1H,*J*=9.6Hz), 4.90-4.92 (d,1H,*J*=8.8Hz). M+1: 215.08.

According to the synthesis method of A-058, the following products were prepared:

Examples of preparation of A-070, A-078, A-086:

A-070: A-002 was replaced with A-016, and the rest operations were the same as those in A-058 synthesis. M+1: 287.14.
A-078: A-002 was replaced with A-030, and the rest operations were the same as those in A-058 synthesis. M+1: 301.15.
A-086: A-002 was replaced with A-044, and the rest operations were the same as those in A-058 synthesis. M+1: 301.15.

Examples of preparation of A-094, A-102, A-110, A-118:

A-094: acetyl chloride was replaced with isobutyryl chloride, and the rest operations were the same as those in A-058 synthesis. M+1: 301.15.
A-102: A-002 was replaced with A-016, acetyl chloride was replaced with isobutyryl chloride, and the rest operations were the same as those in A-058 synthesis. M+1: 315.17.
A-110: A-002 was replaced with A-030, acetyl chloride was replaced with isobutyryl chloride, and the rest operations were the same as those in A-058 synthesis. M+1: 329.18.

A-118: A-002 was replaced with A-044, acetyl chloride was replaced with isobutyryl chloride, and the rest operations were the same as those in A-058 synthesis. M+1: 329.18.

Examples of preparation of A-126, A-134, A-142, A-150:

A-126: acetyl chloride was replaced with pivaloyl chloride, and the rest operations were the same as those in A-058 synthesis. M+1: 315.17.
A-134: A-002 was replaced with A-016, acetyl chloride was replaced with pivaloyl chloride, and the rest operations were the same as those in A-058 synthesis. M+1: 329.18.
A-142: A-002 was replaced with A-030, acetyl chloride was replaced with pivaloyl chloride, and the rest operations were the same as those in A-058 synthesis. M+1: 343.20.
A-150: A-002 was replaced with A-044, acetyl chloride was replaced with pivaloyl chloride, and the rest operations were the same as those in A-058 synthesis. M+1: 343.20.

Examples of preparation of A-158, A-166, A-174, A-182:

A-158: The solvent was replaced with DMF, acetyl chloride was replaced with isobutyl iodide, TEA was replaced with potassium carbonate, and the rest operations were the same as those in A-058 synthesis. M+1: 287.17.
A-166: A-002 was replaced with A-016, the solvent was replaced with DMF, acetyl chloride was replaced with isobutyl iodide, TEA was replaced with potassium carbonate, and the rest operations were the same as those in A-058 synthesis. M+1: 301.19.
A-174: A-002 was replaced with A-030, the solvent was replaced with DMF, acetyl chloride was replaced with isobutyl iodide, TEA was replaced with potassium carbonate, and the rest operations were the same as those in A-058 synthesis. M+1: 315.20.
A-182: A-002 was replaced with A-044, the solvent was replaced with DMF, acetyl chloride was replaced with isobutyl iodide, TEA was replaced with potassium carbonate, and the rest operations were the same as those in A-058 synthesis. M+1: 315.20.

Examples of preparation of A-190, A-198, A-206, A-214:

A-190: isobutyl iodide was replaced with benzyl bromide, and the rest operations were the same as those in A-158 synthesis. M+1: 321.16.
A-198: isobutyl iodide was replaced with benzyl bromide, and the rest operations were the same as those in A-158 synthesis. M+1: 335.17.
A-206: isobutyl iodide was replaced with benzyl bromide, and the rest operations were the same as those in A-158 synthesis. M+1: 349.19.
A-214: isobutyl iodide was replaced with benzyl bromide, and the rest operations were the same as those in A-158 synthesis. M+1: 349.19.

Examples of preparation of A-222, A-230, A-238, A-246:

A-222: the solvent was replaced with DMF, acetyl chloride was replaced with iodomethane, TEA was replaced with potassium carbonate, and the rest operations were the same as those in A-058 synthesis. M+1: 245.12.
A-230: A-002 was replaced with A-016, the solvent was replaced with DMF, acetyl chloride was replaced with iodomethane, TEA was replaced with potassium carbonate, and the rest operations were the same as those in A-058 synthesis. M+1: 259.14.
A-238: A-002 was replaced with A-030, the solvent was replaced with DMF, acetyl chloride was replaced with iodomethane, TEA was replaced with potassium carbonate, and the rest operations were the same as those in A-058 synthesis. M+1: 273.16.
A-246: A-002 was replaced with A-044, the solvent was replaced with DMF, acetyl chloride was replaced with

iodomethane, TEA was replaced with potassium carbonate, and the rest operations were the same as those in A-058 synthesis. M+1: 273.16.

According to the synthesis method of A-057, the following products were prepared:

Examples of preparation of A-069, A-077 and A-085:

A-069: A-058 was replaced with A-070, and the rest operations were the same as those in A-057 synthesis. M+1: 187.08.
A-077: A-058 was replaced with A-078, and the rest operations were the same as those in A-057 synthesis. M+1: 201.10.
A-085: A-058 was replaced with A-086, and the rest operations were the same as those in A-057 synthesis. M+1: 201.10.

Examples of preparation of A-093, A-101, A-109 and A-117:

A-093: A-058 was replaced with A-094, and the rest operations were the same as those in A-057 synthesis. M+1: 201.10.
A-101: A-058 was replaced with A-102, and the rest operations were the same as those in A-057 synthesis. M+1: 215.11.
A-109: A-058 was replaced with A-110, and the rest operations were the same as those in A-057 synthesis. M+1: 229.13.
A-117: A-058 was replaced with A-118, and the rest operations were the same as those in A-057 synthesis. M+1: 229.13.

Examples of preparation of A-125, A-133, A-141 and A-149:

A-125: A-058 was replaced with A-126, and the rest operations were the same as those in A-057 synthesis. M+1: 215.11.
A-133: A-058 was replaced with A-134, and the rest operations were the same as those in A-057 synthesis. M+1: 229.13.
A-141: A-058 was replaced with A-142, and the rest operations were the same as those in A-057 synthesis. M+1: 243.15.
A-149: A-058 was replaced with A-150, and the rest operations were the same as those in A-057 synthesis. M+1: 243.15.

Examples of preparation of A-157, A-165, A-173 and A-181:

A-157: A-058 was replaced with A-158, and the rest operations were the same as those in A-057 synthesis. M+1: 187.12.
A-165: A-058 was replaced with A-166, and the rest operations were the same as those in A-057 synthesis. M+1: 201.13.
A-173: A-058 was replaced with A-174, and the rest operations were the same as those in A-057 synthesis. M+1: 215.15.
A-181: A-058 was replaced with A-182, and the rest operations were the same as those in A-057 synthesis. M+1: 215.15.

Examples of preparation of A-189, A-197, A-205 and A-213:

A-189: A-058 was replaced with A-190, and the rest operations were the same as those in A-057 synthesis. M+1: 221.10.
A-197: A-058 was replaced with A-198, and the rest operations were the same as those in A-057 synthesis. M+1: 235.12.
A-205: A-058 was replaced with A-206, and the rest operations were the same as those in A-057 synthesis. M+1: 249.13.
A-213: A-058 was replaced with A-214, and the rest operations were the same as those in A-057 synthesis. M+1: 249.13.

Examples of preparation of A-221, A-229, A-237 and A-245:

A-221: A-058 was replaced with A-222, and the rest operations were the same as those in A-057 synthesis. M+1: 145.07.
A-229: A-058 was replaced with A-230, and the rest operations were the same as those in A-057 synthesis. M+1: 159.09.
A-237: A-058 was replaced with A-238, and the rest operations were the same as those in A-057 synthesis. M+1: 173.10.
A-245: A-058 was replaced with A-246, and the rest operations were the same as those in A-057 synthesis. M+1: 173.10.

According to the synthesis method of A-059, the following products were prepared:

Examples of preparation of A-060, A-061:

A-060: A-057 was used as a raw material, acetyl chloride was replaced with isobutyryl chloride, and the rest operations were the same as those in A-059 synthesis. M+1: 243.11.
A-061: A-057 was used as a raw material, acetyl chloride was replaced with pivaloyl chloride, and the rest operations were the same as those in A-059 synthesis. M+1: 257.12.

Examples of preparation of A-062 to A-064:

A-062: A-057 was used as a raw material, the solvent was replaced with DMF, acetyl chloride was replaced with iodomethane, TEA was replaced with potassium carbonate, and the rest operations were the same as those in A-059 synthesis. M+1: 187.08.
A-063: A-057 was used as a raw material, the solvent was replaced with DMF, acetyl chloride was replaced with iodoethane, TEA was replaced with potassium carbonate, and the rest operations were the same as those in A-062 synthesis. M+1: 201.10.
A-064: A-057 was used as a raw material, the solvent was replaced with DMF, acetyl chloride was replaced with

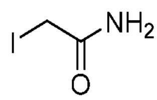,

TEA was replaced with potassium carbonate, and the rest operations were the same as those in A-062 synthesis. M+1: 230.09.

Examples of preparation of A-071 to A-073:

A-071: A-069 was used as a raw material, and the rest operations were the same as those in A-059. M+1: 229.09.

A-072: A-069 was used as a raw material, acetyl chloride was replaced with isobutyryl chloride, and the rest operations were the same as those in A-059 synthesis. M+1: 257.12.
A-073: A-069 was used as a raw material, acetyl chloride was replaced with pivaloyl chloride, and the rest operations were the same as those in A-059 synthesis. M+1: 271.14.

Examples of preparation of A-079 to A-081:

A-079: A-077 was used as a raw material, and the rest operations were the same as those in A-059 synthesis. M+1: 243.11.
A-080: A-077 was used as a raw material, acetyl chloride was replaced with isobutyryl chloride, and the rest operations were the same as those in A-059 synthesis. M+1: 271.14.
A-081: A-077 was used as a raw material, acetyl chloride was replaced with pivaloyl chloride, and the rest operations were the same as those in A-059 synthesis. M+1: 285.16.

Examples of preparation of A-087 to A-089:

A-087: A-085 was used as a raw material, and the rest operations were the same as those in A-059 synthesis. M+1: 243.11.
A-088: A-085 was used as a raw material, acetyl chloride was replaced with isobutyryl chloride, and the rest operations were the same as those in A-059 synthesis. M+1: 271.14.
A-089: A-085 was used as a raw material, acetyl chloride was replaced with pivaloyl chloride, and the rest operations were the same as those in A-059 synthesis. M+1: 285.16.

Examples of preparation of A-095 to A-097:

A-095: A-093 was used as a raw material, acetyl chloride was replaced with

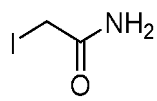,

and the rest operations were the same as those in A-059 synthesis. M+1: 258.12.
A-096: A-093 was used as a raw material, acetyl chloride was replaced with isobutyryl chloride, and the rest operations were the same as those in A-059 synthesis. M+1: 271.14.
A-097: A-093 was used as a raw material, acetyl chloride was replaced with pivaloyl chloride, and the rest operations were the same as those in A-059 synthesis. M+1: 285.16.

Examples of preparation of A-103 to A-105:

A-103: A-101 was used as a raw material, and the rest operations were the same as those in A-059 synthesis. M+1: 257.12.
A-104: A-101 was used as a raw material, acetyl chloride was replaced with isobutyryl chloride, and the rest operations were the same as those in A-059 synthesis. M+1: 285.16.
A-105: A-101 was used as a raw material, acetyl chloride was replaced with pivaloyl chloride, and the rest operations were the same as those in A-059 synthesis. M+1: 299.17.

Examples of preparation of A-111 to A-113:

A-111: A-109 was used as a raw material, and the rest operations were the same as those in A-059 synthesis;
A-112: A-109 was used as a raw material, acetyl chloride was replaced with isobutyryl chloride, and the rest operations were the same as those in A-059 synthesis. M+1: 299.17.
A-113: A-109 was used as a raw material, acetyl chloride was replaced with pivaloyl chloride, and the rest operations were the same as those in A-059 synthesis. M+1: 313.19.

Examples of preparation of A-119 to A-121:

A-119: A-117 was used as a raw material, and the rest operations were the same as those in A-059 synthesis. M+1: 271.14.
A-120: A-117 was used as a raw material, acetyl chloride was replaced with isobutyryl chloride, and the rest operations were the same as those in A-059 synthesis. M+1: 299.17.
A-121: A-117 was used as a raw material, acetyl chloride was replaced with pivaloyl chloride, and the rest operations were the same as those in A-059 synthesis. M+1: 313.19.

Examples of preparation of A-127 to A-129:

A-127: A-125 was used as a raw material, and the rest operations were the same as those in A-059. M+1: 257.12.
A-128: A-125 was used as a raw material, acetyl chloride was replaced with isobutyryl chloride, and the rest operations were the same as those in A-059 synthesis. M+1: 285.16.
A-129: A-125 was used as a raw material, acetyl chloride was replaced with pivaloyl chloride, and the rest operations were the same as those in A-059 synthesis. M+1: 299.17.

Examples of preparation of A-135 to A-137:

A-135: A-133 was used as a raw material, and the rest operations were the same as those in A-059 synthesis. M+1: 271.14.
A-136: A-133 was used as a raw material, acetyl chloride was replaced with isobutyryl chloride, and the rest operations were the same as those in A-059 synthesis. M+1: 299.17.
A-137: A-133 was used as a raw material, acetyl chloride was replaced with pivaloyl chloride, and the rest operations were the same as those in A-059 synthesis. M+1: 313.19.

Examples of preparation of A-143 to A-145:

A-143: A-141 was used as a raw material, and the rest operations were the same as those in A-059 synthesis. M+1: 285.16.
A-144: A-141 was used as a raw material, acetyl chloride was replaced with isobutyryl chloride, and the rest operations were the same as those in A-059 synthesis. M+1: 313.19.
A-145: A-141 was used as a raw material, acetyl chloride was replaced with pivaloyl chloride, and the rest operations were the same as those in A-059 synthesis. M+1: 327.20.

Examples of preparation of A-151 to A-153:

A-151: A-149 was used as a raw material, and the rest operations were the same as those in A-059 synthesis. M+1: 285.16.
A-152: A-149 was used as a raw material, acetyl chloride was replaced with isobutyryl chloride, and the rest operations were the same as those in A-059 synthesis. M+1: 313.19.
A-153: A-149 was used as a raw material, acetyl chloride was replaced with pivaloyl chloride, and the rest operations

were the same as those in A-059 synthesis. M+1: 327.20.

Examples of preparation of A-159 to A-161:

A-159: A-157 was used as a raw material, and the rest operations were the same as those in A-059 synthesis. M+1: 229.13.
A-160: A-157 was used as a raw material, acetyl chloride was replaced with isobutyryl chloride, and the rest operations were the same as those in A-059 synthesis. M+1: 257.16.
A-161: A-157 was used as a raw material, acetyl chloride was replaced with pivaloyl chloride, and the rest operations were the same as those in A-059 synthesis. M+1: 271.18.

Examples of preparation of A-167 to A-169:

A-167: A-165 was used as a raw material, and the rest operations were the same as those in A-059 synthesis. M+1: 243.15.
A-168: A-165 was used as a raw material, acetyl chloride was replaced with isobutyryl chloride, and the rest operations were the same as those in A-059 synthesis. M+1: 271.18.
A-169: A-165 was used as a raw material, acetyl chloride was replaced with pivaloyl chloride, and the rest operations were the same as those in A-059 synthesis. M+1: 285.19.

Examples of preparation of A-175 to A-177:

A-175: A-173 was used as a raw material, and the rest operations were the same as those in A-059 synthesis. M+1: 257.16.
A-176: A-173 was used as a raw material, acetyl chloride was replaced with isobutyryl chloride, and the rest operations were the same as those in A-059 synthesis. M+1: 285.19.
A-177: A-173 was used as a raw material, acetyl chloride was replaced with pivaloyl chloride, and the rest operations were the same as those in A-059 synthesis. M+1: 299.21.

Examples of preparation of A-183 to A-185:

A-183: A-181 was used as a raw material, and the rest operations were the same as those in A-059 synthesis. M+1: 257.16.
A-184: A-181 was used as a raw material, acetyl chloride was replaced with isobutyryl chloride, and the rest operations were the same as those in A-059 synthesis. M+1: 285.19.
A-185: A-181 was used as a raw material, acetyl chloride was replaced with pivaloyl chloride, and the rest operations were the same as those in A-059 synthesis. M+1: 299.21.

Examples of preparation of A-191 to A-193:

A-191: A-189 was used as a raw material, and the rest operations were the same as those in A-059 synthesis. M+1: 263.11.
A-192: A-189 was used as a raw material, acetyl chloride was replaced with isobutyryl chloride, and the rest operations were the same as those in A-059 synthesis. M+1: 291.15.
A-193: A-189 was used as a raw material, acetyl chloride was replaced with pivaloyl chloride, and the rest operations were the same as those in A-059 synthesis. M+1: 305.16.

Examples of preparation of A-199 to A-201:

A-199: A-197 was used as a raw material, and the rest operations were the same as those in A-059 synthesis. M+1: 277.13.
A-200: A-197 was used as a raw material, acetyl chloride was replaced with isobutyryl chloride, and the rest operations were the same as those in A-059 synthesis. M+1: 305.16.
A-201: A-197 was used as a raw material, acetyl chloride was replaced with pivaloyl chloride, and the rest operations were the same as those in A-059 synthesis. M+1: 319.18.

Examples of preparation of A-207 to A-209:

A-207: A-205 was used as a raw material, and the rest operations were the same as those in A-059 synthesis. M+1: 291.15.
A-208: A-205 was used as a raw material, acetyl chloride was replaced with isobutyryl chloride, and the rest operations were the same as those in A-059 synthesis. M+1: 319.18.
A-209: A-205 was used as a raw material, acetyl chloride was replaced with pivaloyl chloride, and the rest operations were the same as those in A-059 synthesis. M+1: 333.19.

Examples of preparation of A-215 to A-217:

A-215: A-213 was used as a raw material, and the rest operations were the same as those in A-059 synthesis. M+1: 291.15.
A-216: A-213 was used as a raw material, acetyl chloride was replaced with isobutyryl chloride, and the rest operations were the same as those in A-059 synthesis. M+1: 319.18.
A-217: A-213 was used as a raw material, acetyl chloride was replaced with pivaloyl chloride, and the rest operations were the same as those in A-059 synthesis. M+1: 333.19.

Examples of preparation of A-223 to A-225:

A-223: A-221 was used as a raw material, and the rest operations were the same as those in A-059 synthesis. M+1: 187.08.
A-224: A-221 was used as a raw material, acetyl chloride was replaced with isobutyryl chloride, and the rest operations were the same as those in A-059 synthesis. M+1: 215.11.
A-225: A-221 was used as a raw material, acetyl chloride was replaced with pivaloyl chloride, and the rest operations were the same as those in A-059 synthesis. M+1: 229.13.

Examples of preparation of A-231 to A-233:

A-231: A-229 was used as a raw material, and the rest operations were the same as those in A-059 synthesis. M+1: 201.10.
A-232: A-229 was used as a raw material, acetyl chloride was replaced with isobutyryl chloride, and the rest operations were the same as those in A-059 synthesis. M+1: 229.13.
A-233: A-229 was used as a raw material, acetyl chloride was replaced with pivaloyl chloride and the rest operations were the same as those in A-059 synthesis. M+1: 243.15.

Examples of preparation of A-239 to A-241:

A-239: A-237 was used as a raw material, and the rest operations were the same as those in A-059 synthesis. M+1: 215.11.
A-240: A-237 was used as a raw material, acetyl chloride was replaced with isobutyryl chloride, and the rest operations were the same as those in A-059 synthesis. M+1: 243.15.
A-241: A-237 was used as a raw material, acetyl chloride was replaced with pivaloyl chloride and the rest operations were the same as those in A-059 synthesis. M+1: 257.16.

Examples of preparation of A-247 to A-249:

A-247: A-245 was used as a raw material, and the rest operations were the same as those in A-059. M+1: 215.11.
A-248: A-245 was used as a raw material, acetyl chloride was replaced with isobutyryl chloride, and the rest operations were the same as those in A-059 synthesis. M+1: 243.15.
A-249: A-245 was used as a raw material, acetyl chloride was replaced with pivaloyl chloride, and the rest operations were the same as those in A-059 synthesis. M+1: 257.16.

Example of A-066

The example illustrates a synthesis method of compound A-066.

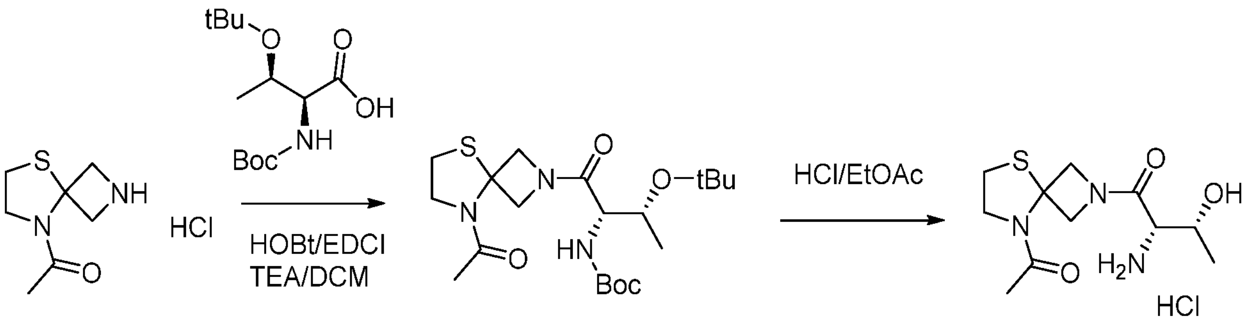


A-057 2a A-066

1. Synthesis method of an intermediate 2a

Under ice bath conditions, a total of 0.3 g (1.74 mmol) of the compound A-057 was dissolved in 20 ml of anhydrous DCM, and 490 μL (3.5 mmol) of TEA was added and stirred for dissociation for 5 min, 0.49 g (1.74 mmol) of Boc-tBu-Thr, 0.35 g (2.6 mmol) of HOBt and 0.5 g (2.6 mmol) of EDCI were added, the raw materials were gradually returned to room temperature, the reaction was performed for 6 h at room temperature; when the completion of the raw materials reaction was monitored by TLC, the reaction was terminated. A saturated citric acid aqueous solution was used for washing (10 mL×3), and an organic phase was taken, dried with anhydrous sodium sulfate, filtered, concentrated, and separated through a silica gel column to obtain a total of 0.59 g of a colorless oily substance of an intermediate 2a with a yield of 78%. $^1$H-NMR (400MHz, DMSO-$d_6$), δppm: 1.13-1.22 (m,12H), 1.37 (s,9H), 2.34 (s,3H), 3.25 (t,2H, J=5.9Hz), 3.37-3.98 (m,4H), 4.09-4.28 (m,1H), 4.58-4.89 (m,3H), 7.89 (s,1H). M+1: 430.23.

2. Synthesis method of the end product A-066

A total of 0.5 g (1.17 mmol) of the intermediate 2a was dissolved in 5 mL of anhydrous ethyl acetate, 10 mL of 4 mol/L HCl/EtOAc was added, and the materials were reacted overnight at room temperature, and a white solid was generated in the system; when the completion of the materials reaction was monitored by TLC, the reaction was terminated. The product was filtered under reduced pressure and washed with anhydrous diethyl ether to obtain a total of 0.26 g of a white solid A-066 with a yield of 72.6%. $^1$H-NMR (400MHz, DMSO-$d_6$), δppm: 1.17-1.22(m,3H), 2.36(s,3H), 3.23(t,2H, J=6.7Hz), 3.35-3.95(m,4H), 4.12-4.32(m,1H), 4.58-4.92(m,3H), 5.65 (s,1H), 8.21-8.29 (d,2H,J=19Hz). M+1: 274.11.

Examples of preparation of A-065, A-067 and A-068:

A-065: the raw material

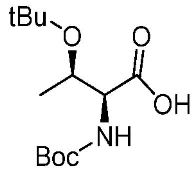

was replaced with

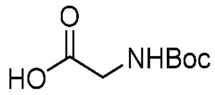

and the rest operations were the same as those in A-066 synthesis. M+1: 230.09.

A-067: the raw material

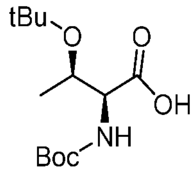

was replaced with

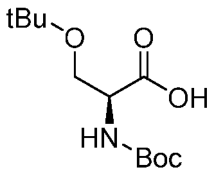,

and the rest operations were the same as those in A-066 synthesis. M+1: 260.10.

A-068: the raw material

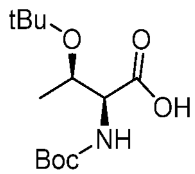

was replaced with

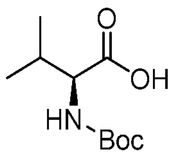,

and the rest operations were the same as those in A-066 synthesis. M+1: 272.14.

Examples of preparation of A-074 to A-076:

A-074: A-057 was replaced with A-069, and the rest operations were the same as those in A-066 synthesis. M+1:288.13.

A-075: A-057 was replaced with A-069, the raw material

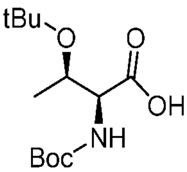

was replaced with

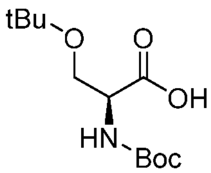,

and the rest operations were the same as those in A-066. M+1: 274.11.

A-076: A-057 was replaced with A-069, the raw material

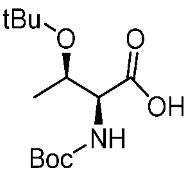

was replaced with

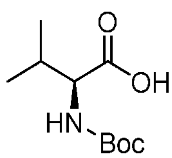,

and the rest operations were the same as those in A-066. M+1: 286.15.

Examples of preparation of A-082 to A-084:

A-082: A-057 was replaced with A-077, and the rest operations were the same as those in A-066 synthesis.

A-083: A-057 was replaced with A-077, the raw material

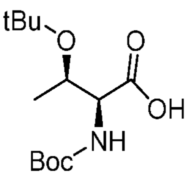

was replaced with

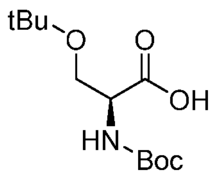

,

and the rest operations were the same as those in A-066 synthesis. M+1: 288.13.

A-084: A-057 was replaced with A-077, the raw material

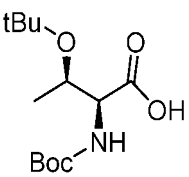

was replaced with

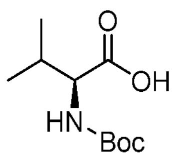

,

and the rest operations were the same as those in A-066 synthesis. M+1: 300.17.

Examples of preparation of A-090 to A-092:

A-090: A-057 was replaced with A-085, and the rest operations were the same as those in A-066 synthesis. M+1: 302.15.

A-091: A-057 was replaced with A-085, the raw material

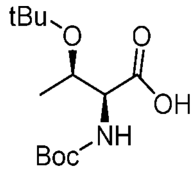

was replaced with

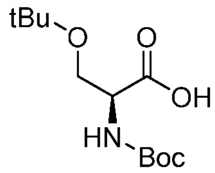,

and the rest operations were the same as those in A-066 synthesis. M+1: 288.13.

A-092: A-057 was replaced with A-085, the raw material

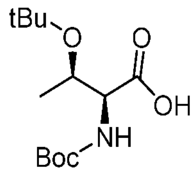

was replaced with

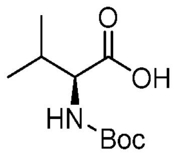,

and the rest operations were the same as those in A-066 synthesis. M+1: 300.17.

Examples of preparation of A-098 to A-100:

A-098: A-057 was replaced with A-093, and the rest operations were the same as those in A-066 synthesis. M+1: 302.15.

A-099: A-057 was replaced with A-093, the raw material

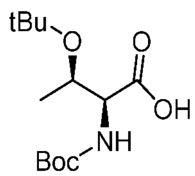

was replaced with

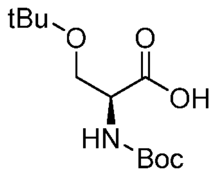

, and the rest operations were the same as those in A-066. M+1: 288.13.

A-100: A-057 was replaced with A-093, the raw material

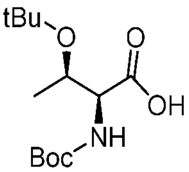

was replaced with

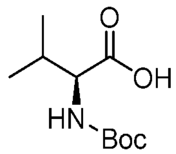

, and the rest operations were the same as those in A-066 synthesis. M+1: 300.17.

Examples of preparation of A-106 to A-108:

A-106: A-057 was replaced with A-101, and the rest operations were the same as those in A-066 synthesis. M+1: 316.16.

A-107: A-057 was replaced with A-101, the raw material

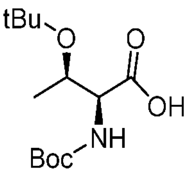

was replaced with

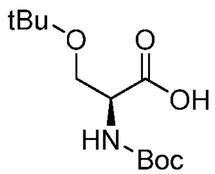

, and the rest operations were the same as those in A-066 synthesis. M+1: 302.15.

A-108: A-057 was replaced with A-101, the raw material

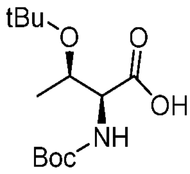

was replaced with

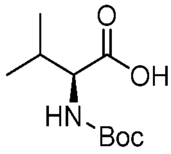,

and the rest operations were the same as those in A-066 synthesis. M+1: 314.18.

Examples of preparation of A-114 to A-116:

A-114: A-057 was replaced with A-109, and the rest operations were the same as those in A-066 synthesis. M+1: 330.18.

A-115: A-057 was replaced with A-109, the raw material

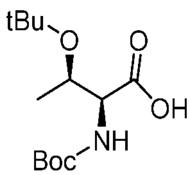

was replaced with

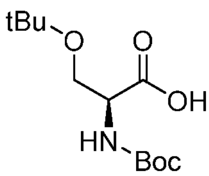,

and the rest operations were the same as those in A-066 synthesis. M+1: 316.16.

A-116: A-057 was replaced with A-109, the raw material

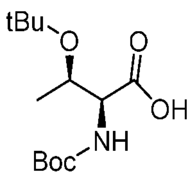

was replaced with

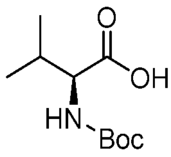,

and the rest operations were the same as those in A-066 synthesis. M+1: 328.20.

Examples of preparation of A-122 to A-124:

A-122: A-057 was replaced with A-117, and the rest operations were the same as those in A-066 synthesis. M+1: 330.18.

A-123: A-057 was replaced with A-117, the raw material

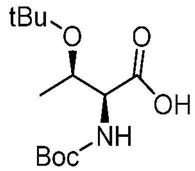

was replaced with

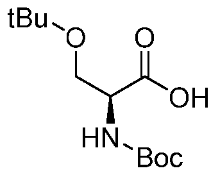,

and the rest operations were the same as those in A-066 synthesis. M+1: 316.16.

A-124: A-057 was replaced with A-117, the raw material

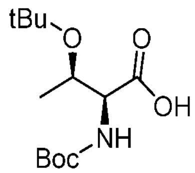

was replaced with

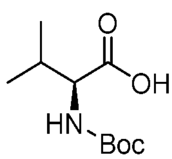,

and the rest operations were the same as those in A-066 synthesis. M+1: 328.20.

Examples of preparation of A-130 to A-132:

A-130: A-057 was replaced with A-125, and the rest operations were the same as those in A-066 synthesis. M+1: 316.16.

A-131: A-057 was replaced with A-125, the raw material

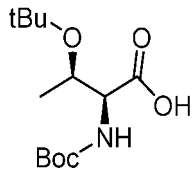

was replaced with

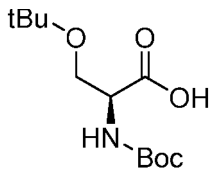

,

and the rest operations were the same as those in A-066 synthesis. M+1: 302.15.

A-132: A-057 was replaced with A-125, the raw material

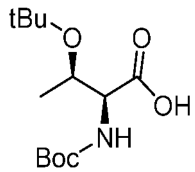

was replaced with

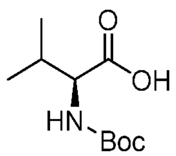

,

and the rest operations were the same as those in A-066 synthesis. M+1: 314.18.

Examples of preparation of A-138 to A-140:

A-138: A-057 was replaced with A-133, and the rest operations were the same as those in A-066 synthesis. M+1: 330.18.

A-139: A-057 was replaced with A-133, the raw material

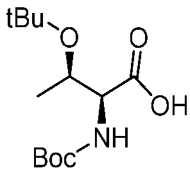

was replaced with

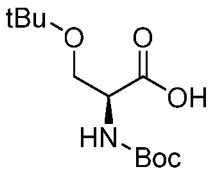,

and the rest operations were the same as those in A-066 synthesis. M+1: 316.16.

A-140: A-057 was replaced with A-133, the raw material

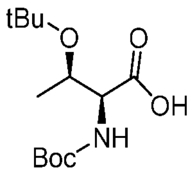

was replaced with

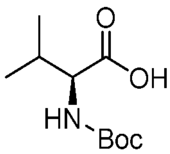,

and the rest operations were the same as those in A-066 synthesis. M+1: 328.20.

Examples of preparation of A-146 to A-148:

A-146: A-057 was replaced with A-141, and the rest operations were the same as those in A-066 synthesis. M+1: 344.19.

A-147: A-057 was replaced with A-141, the raw material

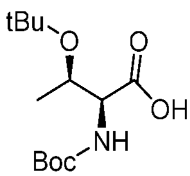

was replaced with

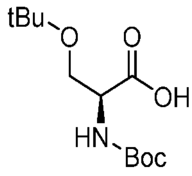,

and the rest operations were the same as those in A-066 synthesis. M+1: 330.18.

A-148: A-057 was replaced with A-141, the raw material

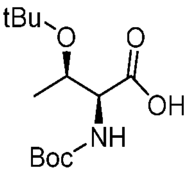

was replaced with

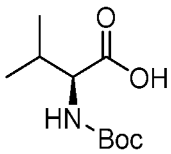,

, and the rest operations were the same as those in A-066 synthesis. M+1: 342.21.

Examples of preparation of A-154 to A-156:

A-154: A-057 was replaced with A-149, and the rest operations were the same as those in A-066 synthesis. M+1: 344.19.

A-155: A-057 was replaced with A-149, the raw material

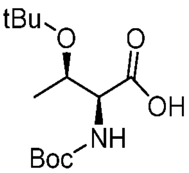

was replaced with

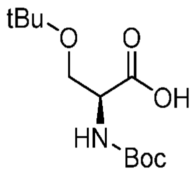,

and the rest operations were the same as those in A-066 synthesis. M+1: 330.18.

A-156: A-057 was replaced with A-149, the raw material

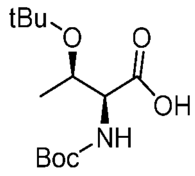

was replaced with

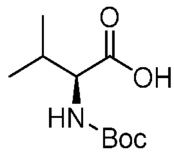
,

and the rest operations were the same as those in A-066 synthesis. M+1: 342.21.

Examples of preparation of A-162 to A-164:

A-162: A-057 was replaced with A-157, and the rest operations were the same as those in A-066 synthesis. M+1: 288.17.

A-163: A-057 was replaced with A-157, the raw material

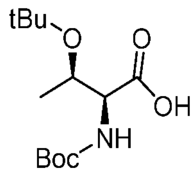

was replaced with

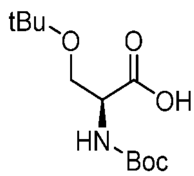
,

and the rest operations were the same as those in A-066 synthesis. M+1: 274.15.

A-164: A-057 was replaced with A-157, the raw material

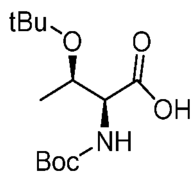

was replaced with

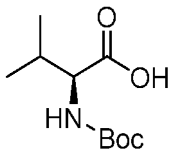,

and the rest operations were the same as those in A-066 synthesis. M+1: 286.19.

Examples of preparation of A-170 to A-172:

A-170: A-057 was replaced with A-165, and the rest operations were the same as those in A-066 synthesis. M+1: 302.18.

A-171: A-057 was replaced with A-165, the raw material

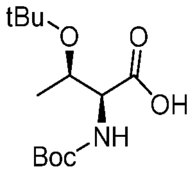

was replaced with

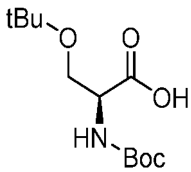,

and the rest operations were the same as those in A-066 synthesis. M+1: 288.17.

A-172: A-057 was replaced with A-165, the raw material

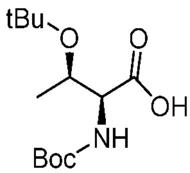

was replaced with

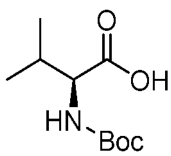,

and the rest operations were the same as those in A-066 synthesis. M+1: 300.20.

Examples of preparation of A-178 to A-180:

A-178: A-057 was replaced with A-173, and the rest operations were the same as those in A-066 synthesis. M+1: 316.20.

A-179: A-057 was replaced with A-173, the raw material

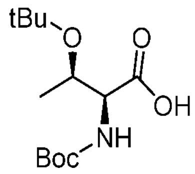

was replaced with

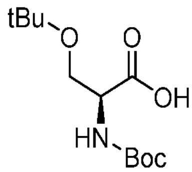

,

and the rest operations were the same as those in A-066 synthesis. M+1: 302.18.

A-180: A-057 was replaced with A-173, the raw material

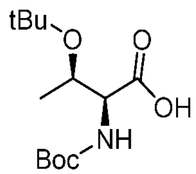

was replaced with

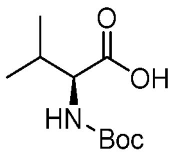

,

and the rest operations were the same as those in A-066 synthesis. M+1: 314.22.

Examples of preparation of A-186 to A-188:

A-186: A-057 was replaced with A-181, and the rest operations were the same as those in A-066 synthesis. M+1: 315.20.

A-187: A-057 was replaced with A-181, the raw material

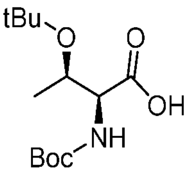

was replaced with

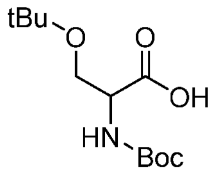,

and the rest operations were the same as those in A-066 synthesis. M+1: 302.18.

A-188: A-057 was replaced with A-181, the raw material

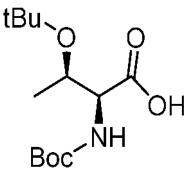

was replaced with

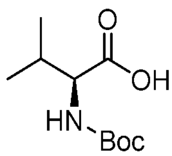,

and the rest operations were the same as those in A-066 synthesis. M+1: 314.22.

Examples of preparation of A-194 to A-196:

A-194: A-057 was replaced with A-189, and the rest operations were the same as those in A-066 synthesis. M+1: 322.15.

A-195: A-057 was replaced with A-189, the raw material

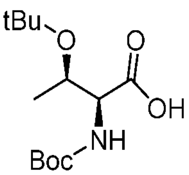

was replaced with

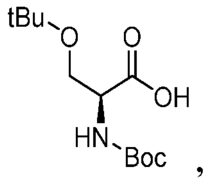

and the rest operations were the same as those in A-066 synthesis. M+1: 308.14.

A-196: A-057 was replaced with A-189, the raw material

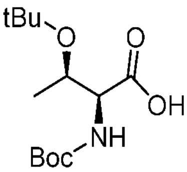

was replaced with

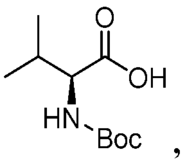

and the rest operations were the same as those in A-066 synthesis. M+1: 320.17.

Examples of preparation of A-202 to A-204:

A-202: A-057 was replaced with A-197, and the rest operations were the same as those in A-066 synthesis. M+1: 336.17.

A-203: A-057 was replaced with A-197, the raw material

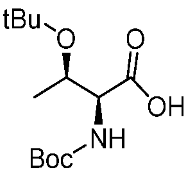

was replaced with

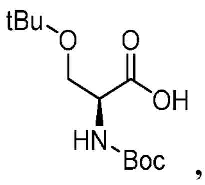

and the rest operations were the same as those in A-066 synthesis. M+1: 322.15.

A-204: A-057 was replaced with A-197, the raw material

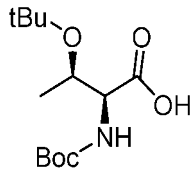

was replaced with

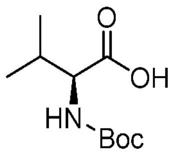

,

and the rest operations were the same as those in A-066 synthesis. M+1: 334.19.

Examples of preparation of A-210 to A-212:

A-210: A-057 was replaced with A-205, and the rest operations were the same as those in A-066 synthesis. M+1: 350.18.

A-211: A-057 was replaced with A-205, the raw material

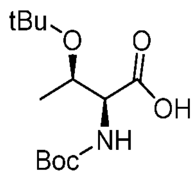

was replaced with

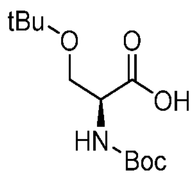

,

and the rest operations were the same as those in A-066 synthesis. M+1: 336.17.

A-212: A-057 was replaced with A-205, the raw material

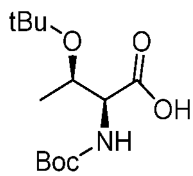

was replaced with

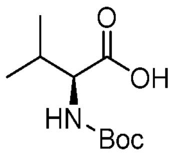

,

and the rest operations were the same as those in A-066 synthesis. M+1: 348.20.

Examples of preparation of A-218 to A-220:

A-218: A-057 was replaced with A-213, and the rest operations were the same as those in A-066 synthesis. M+1: 350.18.

A-219: A-057 was replaced with A-213, the raw material

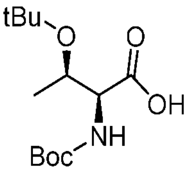

was replaced with

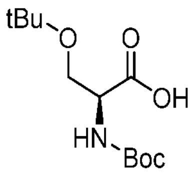

,

and the rest operations were the same as those in A-066 synthesis. M+1: 336.17.

A-220: A-057 was replaced with A-213, the raw material

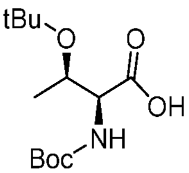

was replaced with

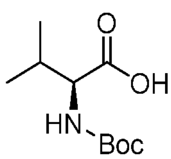

,

and the rest operations were the same as those in A-066 synthesis. M+1: 348.20.

Examples of preparation of A-226 to A-228:

A-226: A-057 was replaced with A-221, and the rest operations were the same as those in A-066 synthesis. M+1: 246.12.

A-227: A-057 was replaced with A-221, the raw material

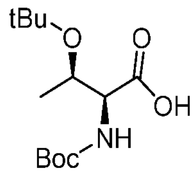

was replaced with

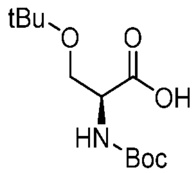

,

and the rest operations were the same as those in A-066 synthesis. M+1: 232.10.

A-228: A-057 was replaced with A-221, the raw material

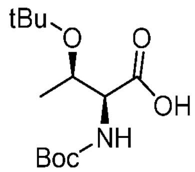

was replaced with

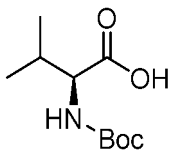

,

and the rest operations were the same as those in A-066 synthesis. M+1: 244.14.

Examples of preparation of A-234 to A-236:

A-234: A-057 was replaced with A-229, and the rest operations were the same as those in A-066 synthesis. M+1: 260.14.

A-235: A-057 was replaced with A-229, the raw material

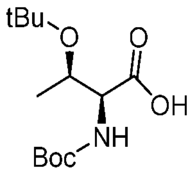

was replaced with

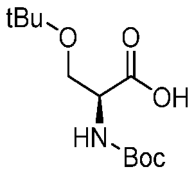,

and the rest operations were the same as those in A-066 synthesis. M+1: 246.12.

A-236: A-057 was replaced with A-229, the raw material

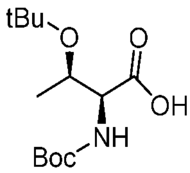

was replaced with

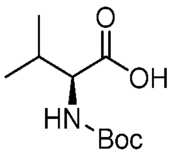,

and the rest operations were the same as those in A-066 synthesis. M+1: 258.16.

Examples of preparation of A-242 to A-244:

A-242: A-057 was replaced with A-237, and the rest operations were the same as those in A-066 synthesis. M+1: 274.15.

A-243: A-057 was replaced with A-237, the raw material

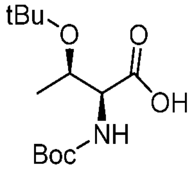

was replaced with

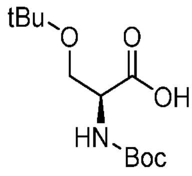,

and the rest operations were the same as those in A-066 synthesis. M+1: 260.14.

A-244: A-057 was replaced with A-237, the raw material

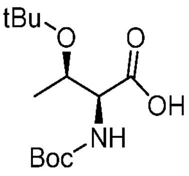

was replaced with

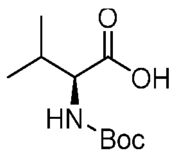,

and the rest operations were the same as those in A-066 synthesis. M+1: 272.17.

Examples of preparation of A-250 to A-252:

A-250: A-057 was replaced with A-245, and the rest operations were the same as those in A-066 synthesis. M+1: 274.15.

A-251: A-057 was replaced with A-245, the raw material

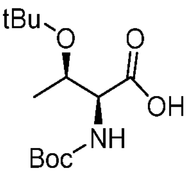

was replaced with

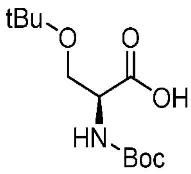,

and the rest operations were the same as those in A-066 synthesis. M+1: 260.14.

A-252: A-057 was replaced with A-245, the raw material

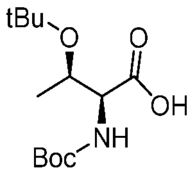

was replaced with

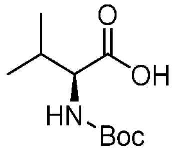,

and the rest operations were the same as those in A-066 synthesis. M+1: 272.17.

Example A-253

The example illustrates a synthesis method of compound A-253.

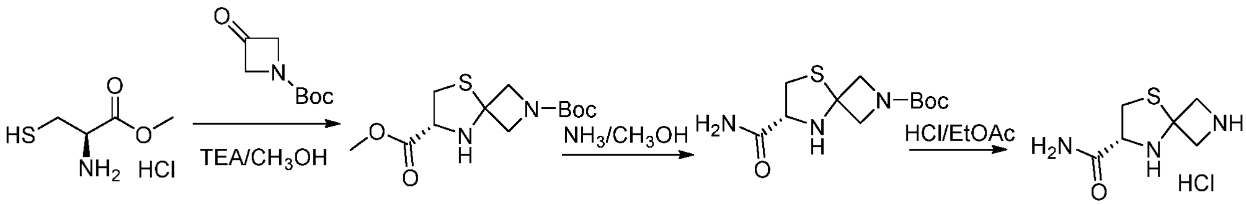


3a A-254 A-253

1. Synthesis method of an intermediate 3a

Under -15°C, 1.8 g (10 mmol) of a raw material L-cysteine methyl ester hydrochloride was dissolved in 100 mL of methanol, fully stirred to be dissolved, 1.75 mL (15 mmol) of TEA was added, after the raw material was fully dissociated, 1.8 g (10 mmol, 1.0 eq) of a raw material 1-Boc-3-azetidinone was added, then continued to be subjected to a low-temperature reaction for 15 min, and transferred to room temperature. After reaction at room temperature for 4 h, when the completion of the materials reaction was monitored by TLC, a reaction solution was subjected to rotary evaporation, a concentrate was dissolved with 100 mL of ethyl acetate, washed with a small amount of water for multiple times (15ml×4), dried with anhydrous sodium sulfate, filtered, and then subjected to rotary evaporation. A crude product was purified with a silica column (PE: EtOAc = 2:1) to obtain a total of 2.4 g of a yellow transparent oily intermediate 3a with a yield of 83%. $^1$H-NMR (400MHz, DMSO-$d_6$), δppm: 1.37(s,9H), 3.06-3.55(m,6H), 3.91-4.19 (m,4H), 8.69(s,1H). M+1: 289.11.

2. Synthesis method of an intermediate A-254

A total of 0.8 g (2.8 mmol) of the intermediate 3a was dissolved in 5 mL of anhydrous methanol, 5 mL of 7 mol/L $NH_3/CH_3OH$ solution was added, stirred overnight at room temperature; when the completion of the materials reaction was monitored by TLC, the reaction was terminated. After vacuum concentration, a white solid was obtained, the traits were improved by stirring with petroleum ether, and the material was filtered to obtain 0.68 g of a white solid, namelyA-254, with a yield of 90.7%. $^1$H-NMR (400MHz, DMSO-$d_6$), δppm: 1.37(s,9H), 3.02-3.27(m,2H), 3.36-3.45(m,1H), 3.94-4.23 (m,4H), 8.14 (s,2H), 8.78(s,1H). M+1: 274.11.

3. Synthesis method of product A-253

0.4 g of A-254 was dissolved in 3 mL of anhydrous ethyl acetate, a total of 5 mL of 4 mol/L HCl/EtOAc was added, and stirred at room temperature for 4 h; when the completion of the materials reaction was monitored by TLC,

a white solid was obtained in the system, then filtered and washed with the anhydrous ether to obtain 0.21 g of the end product A-253 with a yield of 70%. $^1$H-NMR (400MHz, DMSO-$d_6$), δppm: 3.04-3.28(m,2H), 3.37-3.42(m,1H), 3.91-4.22(m,4H), 8.15(s,2H), 8.54(s,1H). M+1: 174.06.

Similarly, in the synthesis of the intermediate 3a, the raw material 1-Boc-3-azetidinone was replaced with N-Boc-3-pyrrolidone, N-Boc-4-piperidone, and N-Boc-3-piperidone, and the other operations were the same as those of the synthesis of the intermediate 3a, and an intermediate 3b, an intermediate 3c, and an intermediate 3d were prepared respectively.

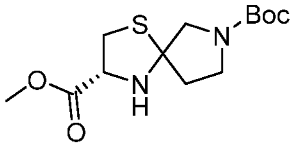

3b

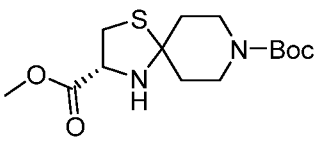

3c

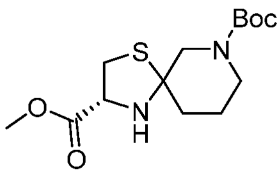

3d

Examples of preparation of A-262, A-270, A-278:

A-262: the intermediate 3a was replaced with the intermediate 3b, and the rest operations were the same as those in A-254 synthesis. M+1: 288.13.

A-270: the intermediate 3a was replaced with the intermediate 3c, and the rest operations were the same as those in A-254 synthesis. M+1: 302.15.

A-278: the intermediate 3a was replaced with the intermediate 3d, and the rest operations were the same as those in A-254 synthesis. M+1: 302.15.

Examples of preparation of A-261, A-269 and A-277:

A-261: A-254 was replaced with A-262, and the rest operations were the same as those in A-253 synthesis. M+1: 188.08.

A-269: A-254 was replaced with A-270, and the rest operations were the same as those in A-253 synthesis. M+1: 202.09.

A-277: A-254 was replaced with A-278, and the rest operations were the same as those in A-253 synthesis. M+1: 202.09.

Example A-255

The example illustrates a synthesis method of compound A-255.

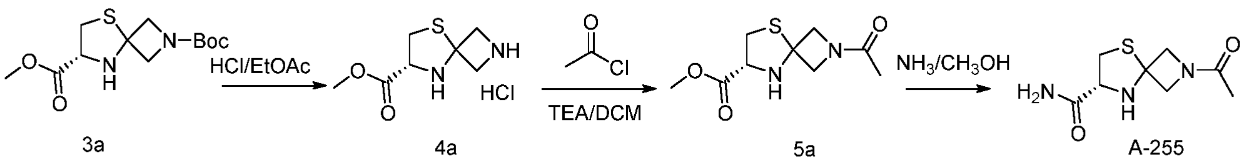


3a 4a 5a A-255

1. Synthesis method of an intermediate 4a

A total of 2.4 g (8.3 mmol) of the intermediate 3a was dissolved in 10 mL of anhydrous ethyl acetate, and 20 mL of 4 mol/L hydrochloric acid/ethyl acetate solution was added under ice bath, the raw materials were reacted overnight, an off-white solid was precipitated; when the completion of the raw materials reaction was monitored by TLC, 1.8 g of an intermediate 4a was obtained after filtration, washed with anhydrous ether, and dried. $^1$H-NMR (400MHz, DMSO-$d_6$), δppm: 3.05-3.55(m,6H), 3.91-4.19 (m,4H), 8.26(s,1H), 8.69(s,1H). M+1: 189.06.

2. Synthesis method of an intermediate 5a

1.6 g (7.15 mmol) of the intermediate 4a was dissolved in 20 mL DCM under ice bath, and after being added 4 mL (28 mmol) of TEA for dissociation, 1 mL (14 mmol) of acetyl chloride was slowly added dropwise, the raw materials were gradually returned to room temperature and reacted overnight; when the completion of the raw materials reaction was monitored by TLC, the reaction was terminated; a saturated citric acid aqueous solution was used for washing (10 mL×3), an organic phase was taken, dried with anhydrous sodium sulfate, filtered, concentrated, and separated through a silica gel column to obtain 1.53 g of a yellowish oily substance, namely an intermediate 5a, with a yield of 76.5%. $^1$H-NMR (400MHz, DMSO-$d_6$), δppm: 2.05(s,3H), 3.07-3.53(m,6H), 3.95-4.21 (m,4H), 8.48(s,1H). M+1: 231.07.

3. Synthesis method of a product A-255

The intermediate 5a was ammonolyzed, and the synthesis method of this step was the same as that of A-254. $^1$H-NMR (400MHz, DMSO-$d_6$), δppm: 2.04(s,3H), 3.02-3.25(m,2H), 3.38-3.47(m,1H), 3.97-4.22 (m,4H), 8.23(s,2H), 9.01(s,1H). M+1: 216.07.

In the synthesis of the intermediate 4a, the raw material intermediate 3a was replaced with the intermediate 3b, the intermediate 3c and the intermediate 3d respectively, and the other operations were the same as those in the synthesis of the intermediate 4a, and an intermediate 4b, an intermediate 4c and an intermediate 4d were prepared respectively;

similarly, in the synthesis process of the intermediate 5a, the raw material intermediate 4a was replaced with the intermediate 4b, the intermediate 4c and the intermediate 4d respectively, and the other operations were the same as those in the synthesis of the intermediate 5a, and an intermediate 5b, an intermediate 5c and an intermediate 5d were prepared respectively;

similarly, in the synthesis of the intermediate 5a, acetyl chloride was replaced with isobutyryl chloride and pivaloyl chloride respectively, and an intermediate 6a, an intermediate 6b, an intermediate 6c, and an intermediate 6d, and an intermediate 7a, an intermediate 7b, an intermediate 7c, and an intermediate 7d were prepared respectively.

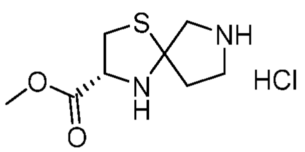

4b

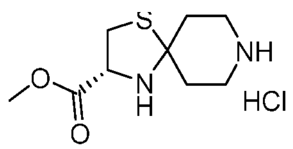

4c

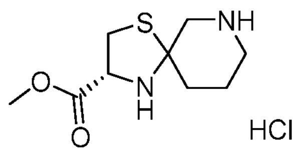

4d

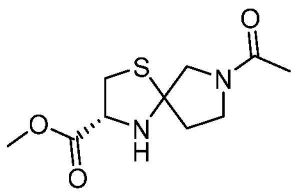

5b

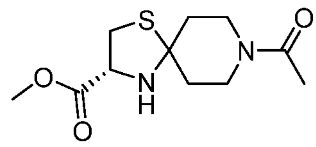

5c

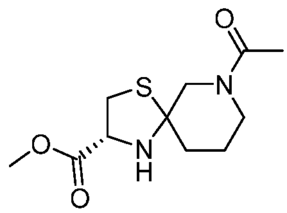

5d

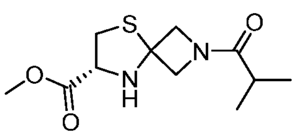

6a

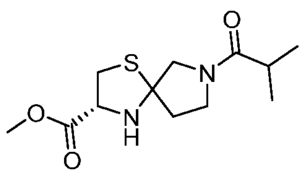

6b

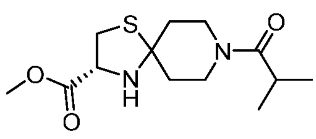

6c

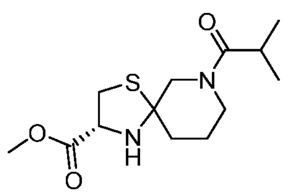

6d

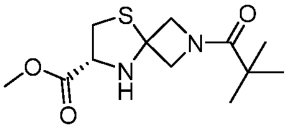

7a

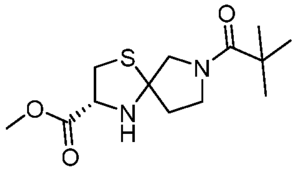

7b

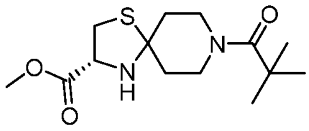

7c

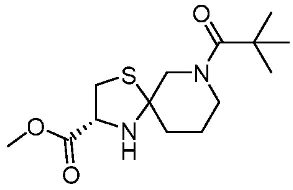

7d

Preparation of A-256 and A-257:

A-256: the intermediate 5a was replaced with the intermediate 6a, and other operations were the same as those in A-255 synthesis. M+1: 244.10.

A-257: the intermediate 5a was replaced with the intermediate 7a, and other operations were the same as those in A-255 synthesis. M+1: 258.12.

Preparation of A-263 and A-265:

A-263: the intermediate 5a was replaced with the intermediate 5b, and other operations were same as those in A-255 synthesis. M+1: 230.09.

A-264: the intermediate 5a was replaced with the intermediate 6b, and other operations were same as those in A-255 synthesis. M+1: 258.12.

A-265: the intermediate 5a was replaced with the intermediate 7b, and other operations were same as those in A-255 synthesis. M+1: 272.14.

Preparation of A-271 and A-273:

A-271: the intermediate 5a was replaced with the intermediate 5c, and other operations were same as those in A-255 synthesis. M+1: 244.10.

A-272: the intermediate 5a was replaced with the intermediate 6c, and other operations were same as those in A-255 synthesis. M+1: 272.14.

A-273: the intermediate 5a was replaced with the intermediate 7c, and other operations were same as those in A-255 synthesis. M+1: 286.15.

Preparation of A-279 and A-281:

A-279: the intermediate 5a was replaced with the intermediate 5d, and other operations were same as those in A-255 synthesis. M+1: 244.10.

A-280: the intermediate 5a was replaced with the intermediate 6d, and other operations were same as those in A-255 synthesis. M+1: 272.14.

A-281: the intermediate 5a was replaced with the intermediate 7d, and other operations were same as those in A-255 synthesis. M+1: 286.15.

Example of A-258

The example illustrates a synthesis method of compound A-258.

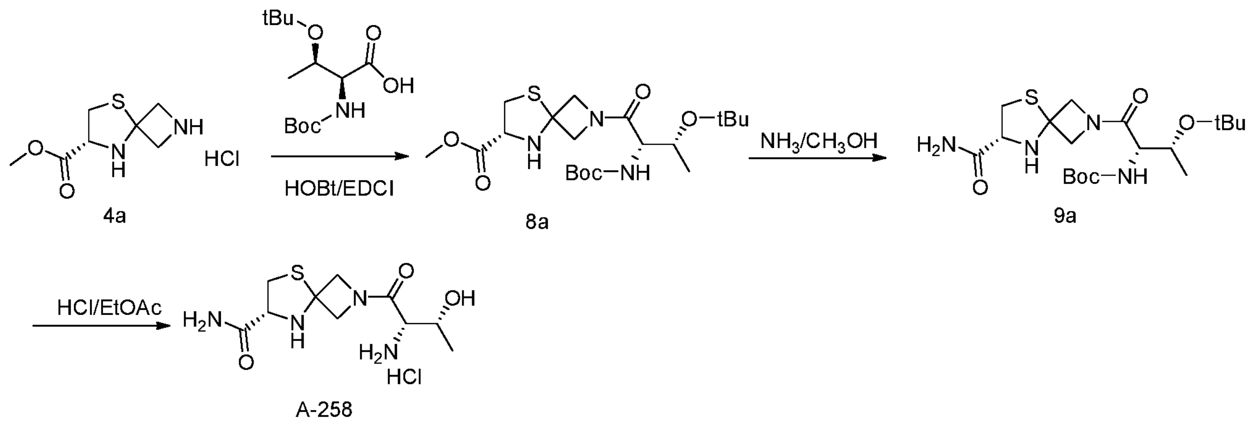


4a 8a 9a

A-258

1. Synthesis method of an intermediate 8a

A total of 1.8 g (8 mmol) of the intermediate 4a was dissolved in 50 mL of dichloromethane, 0.89 g (8.8 mmol) of triethylamine was added under ice bath, and the materials were fully dissociated, then 2.2 g (8 mmol) of Boc-tBu-Thr, 1.18 g (8.8 mmol) of HOBt and 1.68 g (8.8 mmol) of EDCI were added, and the raw materials were naturally returned to room temperature for a reaction; after the completion of the raw materials reaction was monitored by TLC, a citric acid aqueous solution, a saturated sodium bicarbonate solution and a saturated sodium chloride solution were used for washing in order, and the material was dried with anhydrous sodium sulfate, filtered and concentrated. A concentrate was purified with a silica gel column, wherein the ratio of petroleum ether to ethyl acetate was 1:1, and a total of 1.9 g of an intermediate 8a was obtained with a yield of 53%. $^1$H-NMR (400MHz, DMSO-$d_6$), δppm: 1.12-1.20(m,12H), 1.37(s,9H), 3.14-3.41(m,2H), 3.59-3.87(m,5H), 4.04-4.84(m,5H), 7.84(s,1H), 8.52(s,1H). M+1: 446.22.

2. Synthesis method of an intermediate 9a

A total of 1.5 g (3.37 mmol) of the intermediate 8a was dissolved in a small amount of methanol, and 10 mL of 7 mol/L $NH_3$/$CH_3OH$ solution was added under ice bath, and the raw materials were reacted overnight; after the completion of the raw materials reaction was monitored by TLC, a reaction solution was subjected to rotary evaporation to obtain a total of 1.45 g of an off-white solid intermediate 9a with a yield of 99.4%. $^1$H-NMR (400MHz, DMSO-$d_6$), δppm: 1.12-1.22(m,12H), 1.37(s,9H), 3.15-3.43(m,2H), 3.59-3.89(m,2H), 4.07-4.81(m,5H), 7.84(s,1H), 8.24(s,2H), 8.57(s,1H). M+1: 431.22

3. Synthesis method of a product A-258

A total of 1.3 g (3 mmol) of the intermediate 9a was dissolved in 10 mL of anhydrous ethyl acetate, and 10 mL of 4 mol/L HCl/EtOAc solution was added under ice bath, and the materials were gradually returned to room temperature and reacted for 6 h; when the completion of the raw materials reaction was monitored by TLC, a white solid was precipitated in the system, filtered, and washed with anhydrous diethyl ether to obtain a total of 0.81 g of the end product A-258 with a yield of 86.1%. $^1$H-NMR (400MHz, DMSO-$d_6$), δppm: 1.12-1.17 (m,3H), 3.05-3.17 (m,1H), 3.39-3.43 (m,1H), 3.72-3.92 (m,2H), 4.06-4.81 (m,5H), 5.65 (s,1H), 7.55 (s,1H), 7.93 (s,2H), 8.24 (s,2H). M+1: 275.11.

The intermediate 4a was respectively replaced with the intermediate 4b, the intermediate 4c and the intermediate 4d, and an intermediate 8b, an intermediate 8c and an intermediate 8d were respectively prepared; and an intermediate 9b, an intermediate 9c and an intermediate 9d were prepared accordingly.

The intermediate 4a was respectively replaced with the intermediate 4b, the intermediate 4c and the intermediate 4d, the raw material

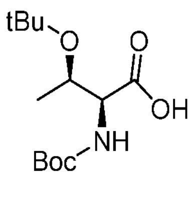

was replaced with

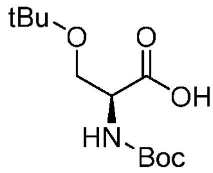

,

and an intermediate 10a, an intermediate 10b, an intermediate 10c and an intermediate 10d were prepared respectively; and an intermediate 11a, an intermediate 11b, an intermediate 11c and an intermediate 11d were prepared accordingly.

The intermediate 4a was respectively replaced with the intermediate 4b, the intermediate 4c and the intermediate 4d, the raw material

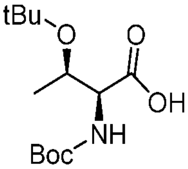

was replaced with

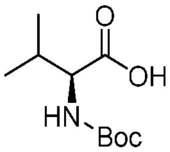

,

and an intermediate 12a, an intermediate 12b, an intermediate 12c and an intermediate 12d were prepared respectively; and an intermediate 13a, an intermediate 13b, an intermediate 13c and an intermediate 13d were prepared accordingly.

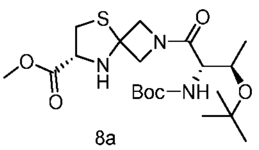

8a

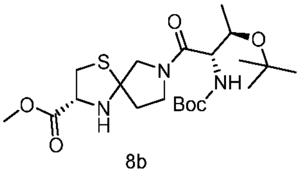

8b

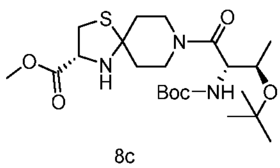

8c

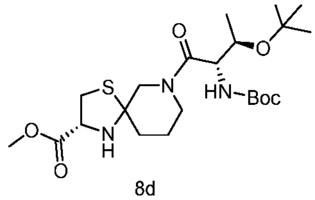

8d

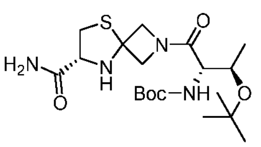

9a

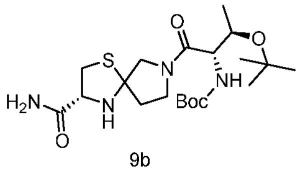

9b

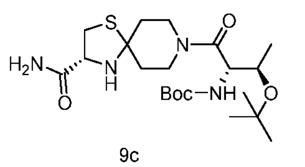

9c

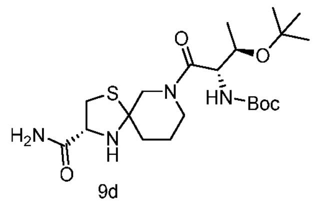

9d

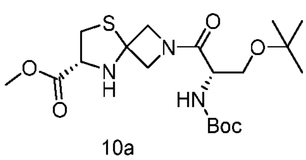

10a

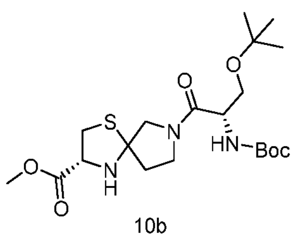

10b

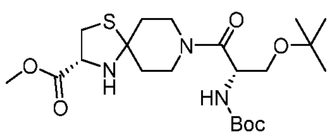

10c

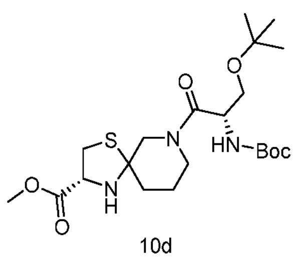

10d

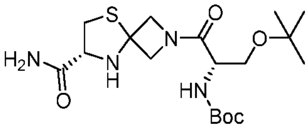
11a
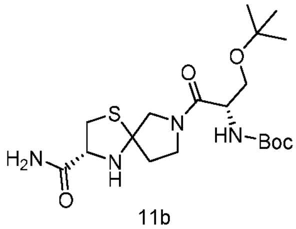
11b
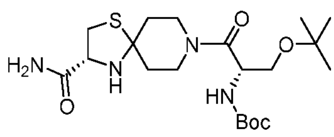
11c
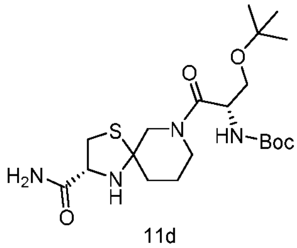
11d

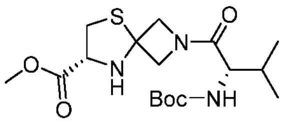
12a
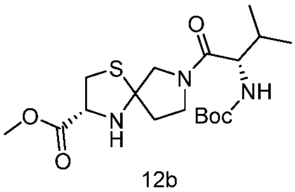
12b
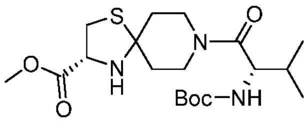
12c
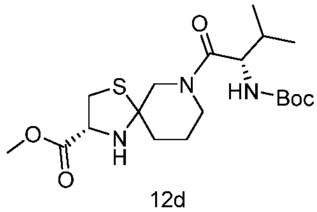
12d

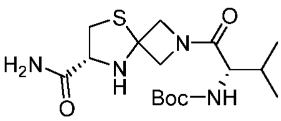
13a
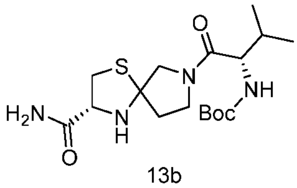
13b
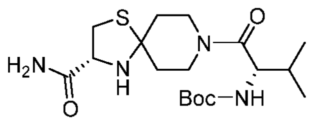
13c
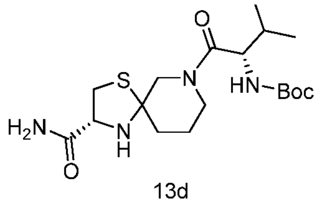
13d

Examples of preparation of A-259 to A-260:

A-259: the raw material

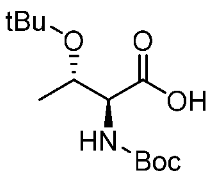

was replaced with

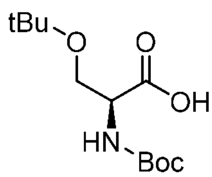
,

and the rest operations were the same as those in A-258 synthesis. M+1: 261.09.

A-260: the raw material

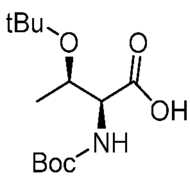

was replaced with

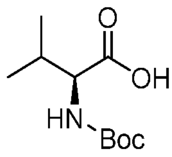

,

and the rest operations were the same as those in A-258 synthesis. M+1: 273.13.

Examples of preparation of A-266 to A-268:

A-266: the raw material intermediate 4a was replaced with the intermediate 4b, and the rest operations were the same as those in A-258 synthesis. M+1: 289.13.

A-267: the raw material intermediate 4a was replaced with the intermediate 4b, the raw material

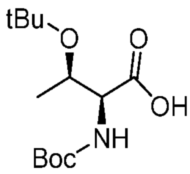

was replaced with

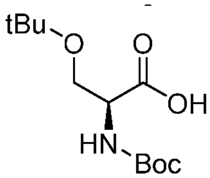

,

and the rest operations were the same as those in A-258 synthesis. M+1: 275.11.

A-268: the raw material intermediate 4a was replaced with the intermediate 4b, the raw material

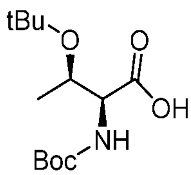

was replaced with

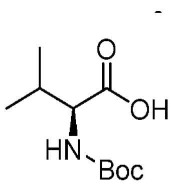

,

and the rest operations were the same as those in A-258 synthesis. M+1: 287.15.

Examples of preparation of A-274 to A-276:

A-274: the raw material intermediate 4a was replaced with the intermediate 4c, and the rest operations were the same as those in A-258 synthesis. M+1: 303.14.

A-275: the raw material intermediate 4a was replaced with the intermediate 4c, the raw material

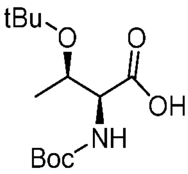

was replaced with

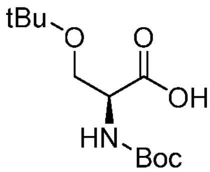

,

and the rest operations were the same as those in A-258 synthesis. M+1: 289.13.

A-276: the raw material intermediate 4a was replaced with the intermediate 4c, the raw material

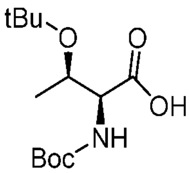

was replaced with

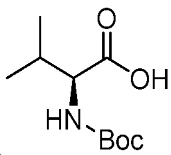

,

, and the rest operations were the same as those in A-258 synthesis. M+1: 301.16.

Examples of preparation of A-282 to A-284:

A-282: the raw material intermediate 4a was replaced with the intermediate 4d, and the rest operations were the same as those in A-258 synthesis. M+1: 303.14.

A-283: the raw material intermediate 4a was replaced with the intermediate 4d, the raw material

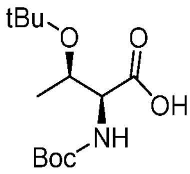

was replaced with

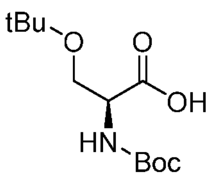

,

and the rest operations were the same as those in A-258 synthesis. M+1: 289.13.

A-284: the raw material intermediate 4a was replaced with the intermediate 4d, the raw material

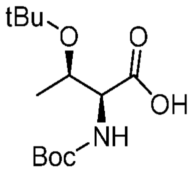

was replaced with

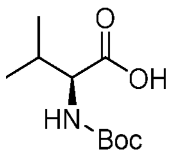

,

and the the rest operations were the same as those in A-258 synthesis. M+1: 301.16.

Example of A-285

The example illustrates a synthesis method of compound A-285.

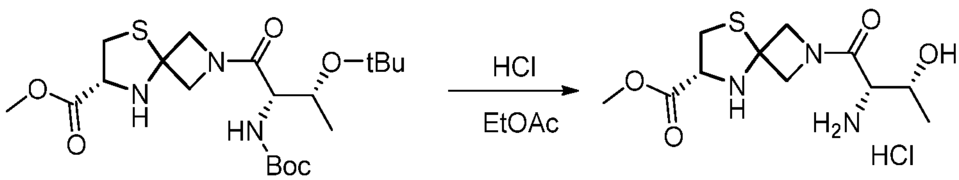


8a A-285

Under ice bath conditions, 0.8 g (1.74 mmol) of the intermediate 8a was dissolved in 10 mL of anhydrous ethyl acetate, and a total of 5 mL of 4 mol/L HCl/EtOAc was added, and the raw materials were gradually returned to room temperature and reacted for 4 hours; when the completion of the raw materials reaction was monitored by TLC, a white solid was generated in the system. The reaction was terminated, after vacuum filtration a filter cake was taken, continuously stirred for 10 min in 15 mL of anhydrous ethyl acetate, and then filtered to obtain a total of 0.51 g of white solid A-285 with a yield of 90.1%. $^1$H-NMR (400MHz, DMSO-$d_6$), δppm: 1.12-1.17(m,3H), 3.07-3.19(m,1H), 3.38-3.42(m,1H), 3.68-3.91(m,5H), 4.07-4.83(m,5H), 5.63(s,1H), 7.53 (s,1H), 7.88(s,2H). M+1: 290.11.

Examples of preparation of A-286 and A-287

A-286: the intermediate 8a was replaced with the intermediate 10a, and the rest operations were the same as those in A-285 synthesis. M+1: 276.09.
A-287: the intermediate 8a was replaced with the intermediate 12a, and the rest operations were the same as those in A-285 synthesis. M+1: 288.13.

Examples of preparation of A-288 and A-290

A-288: the intermediate 8a was replaced with the intermediate 8b, and the rest operations were the same as those in A-285 synthesis. M+1: 304.13.
A-289: the intermediate 8a was replaced with the intermediate 10b, and the rest operations were the same as those in A-285 synthesis. M+1: 290.11.
A-290: the intermediate 8a was replaced with the intermediate 12b, and the rest operations were the same as those in A-285 synthesis. M+1: 302.15.

Examples of preparation of A-291 and A-293

A-291: the intermediate 8a was replaced with the intermediate 8c, and the rest operations were the same as those in A-285 synthesis. M+1: 318.14.
A-292: the intermediate 8a was replaced with the intermediate 10c, and the rest operations were the same as those in A-285 synthesis. M+1: 304.13.
A-293: the intermediate 8a was replaced with the intermediate 12c, and the rest operations were the same as those in A-285 synthesis. M+1: 316.16.

Examples of preparation of A-294 and A-296

A-294: the intermediate 8a was replaced with the intermediate 8d, and the rest operations were the same as those in A-285 synthesis. M+1: 318.14.
A-295: the intermediate 8a was replaced with the intermediate 10d, and the rest operations were the same as those in A-285 synthesis. M+1: 304.13.
A-296: the intermediate 8a was replaced with the intermediate 12d, and the rest operations were the same as those in A-285 synthesis. M+1: 316.16.

Example of A-302

The example illustrates a synthesis method of compound A-302.

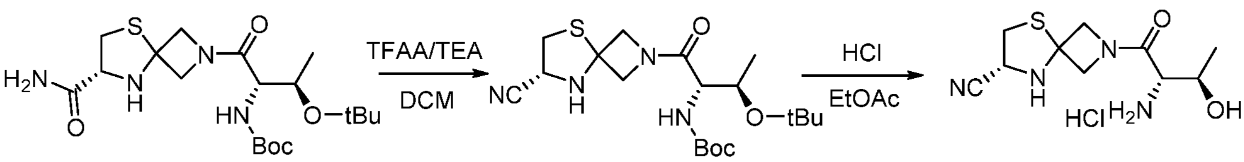


9a 14a A-302

1. Synthesis method of an intermediate 14a

0.7 g (1.6 mmol) of the intermediate 9a was dissolved in 10 mL of anhydrous DCM, 832 μL (6 mmol) of TEA was added, 0.7 g of trifluoroacetic anhydride (TFAA, 3.2 mmol) was added under ice bath conditions, and the materials

were gradually returned to room temperature, and stirred at the room temperature for 12 h; when the completion of the raw materials reaction was monitored by TLC, the reaction was terminated; an organic phase was washed with 10 mL of water and 10 mL of saturated aqueous sodium chloride respectively, dried with anhydrous sodium sulfate, filtrated, concentrated, and separated through a silica gel column (PE: EtOAc = 2:1) to obtain a total of 0.62 g of an end-product intermediate 14a with a yield of 92%. $^1$H-NMR (400MHz, DMSO-$d_6$), δppm: 1.13-1.21(m,12H), 1.37(s,9H), 3.03-3.14(m,1H), 3.39-3.42(m,1H), 3.69-3.86(m,2H), 4.03-4.75(m,5H), 7.81(s,1H), 8.34(s,1H). M+1: 413.21.

2. Preparation of A-302:

0.5 g of the intermediate 14a was dissolved in 10 mL of anhydrous ethyl acetate under ice bath conditions, fully stirred and dissolved, a total of 5 mL of 4 mol/L HCl/EtOAc was added, the raw material was gradually returned to room temperature and reacted at room temperature for 5 hours, and a yellow solid was generated in the system; when the completion of the raw materials reaction was monitored by TLC, the reaction was terminated. After filtration, a filter cake was taken and stirred with anhydrous ethyl acetate for 15 min and then filtered to obtain a total of 0.26 g of A-302 with a yield of 83%. $^1$H-NMR (400MHz, DMSO-$d_6$), δppm: 1.13-1.18(m,3H), 3.06-3.18(m,1H), 3.39-3.42(m,1H), 3.74-3.91(m,2H), 4.05-4.79(m,5H), 5.68 (s,1H), 8.29(s,2H), 8.52(s,1H). M+1: 257.10.

Examples of preparation of A-297 to A-301, A-303 and A-304:

A-297: the intermediate 9a was replaced with A-254, and the rest operations were the same as those of A-302. M+1: 156.05.

A-298: the intermediate 9a was replaced with A-254, and the rest operations were the same as those of the intermediate 14a. M+1: 256.10.

A-299: the intermediate 9a was replaced with A-255, and the rest operations were the same as those of the intermediate 14a. M+1: 198.06.

A-300: the intermediate 9a was replaced with A-256, and the rest operations were the same as those of the intermediate 14a. M+1: 226.09.

A-301: the intermediate 9a was replaced with A-257, and the rest operations were the same as those of the intermediate 14a. M+1: 240.11.

A-303: the intermediate 9a was replaced with the intermediate 11a, and the rest operations were the same as those of A-302. M+1: 243.08.

A-304: the intermediate 9a was replaced with the intermediate 13a, and the rest operations were the same as those of A-302. M+1: 255.12.

Examples of preparation of A-305 to A-312:

A-305: the intermediate 9a was replaced with A-262, and the rest operations were the same as those of A-302. M+1: 170.07.

A-306: the intermediate 9a was replaced with A-262, and the rest operations were the same as those of the intermediate 14a. M+1: 270.12.

A-307: the intermediate 9a was replaced with A-263, and the rest operations were the same as those of the intermediate 14a. M+1: 212.08.

A-308: the intermediate 9a was replaced with A-264, and the rest operations were the same as those of the intermediate 14a. M+1: 240.11.

A-309: the intermediate 9a was replaced with A-265, and the rest operations were the same as those of the intermediate 14a. M+1: 254.12.

A-310: the intermediate 9a was replaced with the intermediate 9b, and the rest operations were the same as those of A-302. M+1: 271.12.

A-311: the intermediate 9a was replaced with the intermediate 11b, and the rest operations were the same as those of A-302. M+1: 257.10.

A-312: the intermediate 9a was replaced with the intermediate 13b, and the rest operations were the same as those of A-302. M+1: 269.14.

Examples of preparation of A-313 to A-320:

A-313: the intermediate 9a was replaced with A-270, and the rest operations were the same as those of A-302. M+1: 184.08

A-314: the intermediate 9a was replaced with A-270, and the rest operations were the same as those of the intermediate 14a. M+1: 284.14.

A-315: the intermediate 9a was replaced with A-271, and the rest operations were the same as those of the intermediate 14a. M+1: 226.09.

A-316: the intermediate 9a was replaced with A-272, and the rest operations were the same as those of the intermediate 14a. M+1: 254.12.

A-317: the intermediate 9a was replaced with A-273, and the rest operations were the same as those of the intermediate 14a. M+1: 268.14.

A-318: the intermediate 9a was replaced with the intermediate 9c, and the rest operations were the same as those of A-302. M+1: 285.13.

A-319: the intermediate 9a was replaced with the intermediate 11c, and the rest operations were the same as those of A-302. M+1: 271.12.

A-320: the intermediate 9a was replaced with the intermediate 13c, and the rest operations were the same as those of A-302. M+1: 283.15.

Examples of preparation of A-321 to A-328:

A-321: the intermediate 9a was replaced with A-278, and the rest operations were the same as those of A-302. M+1: 184.08.

A-322: the intermediate 9a was replaced with A-278, and the rest operations were the same as those of the intermediate 14a. M+1: 284.14.

A-323: the intermediate 9a was replaced with A-279, and the rest operations were the same as those of the intermediate 14a. M+1: 226.09.

A-324: the intermediate 9a was replaced with A-280, and the rest operations were the same as those of the intermediate 14a. M+1: 254.12.

A-325: the intermediate 9a was replaced with A-281, and the rest operations were the same as those of the intermediate 14a. M+1: 268.14.

A-326: the intermediate 9a was replaced with the intermediate 9d, and the rest operations were the same as those of A-302. M+1: 285.13.

A-327: the intermediate 9a was replaced with the intermediate 11d, and the rest operations were the same as those of A-302. M+1: 271.12.

A-328: the intermediate 9a was replaced with the intermediate 13d, and the rest operations were the same as those of A-302. M+1: 283.15.

Example of synthesis of A-329

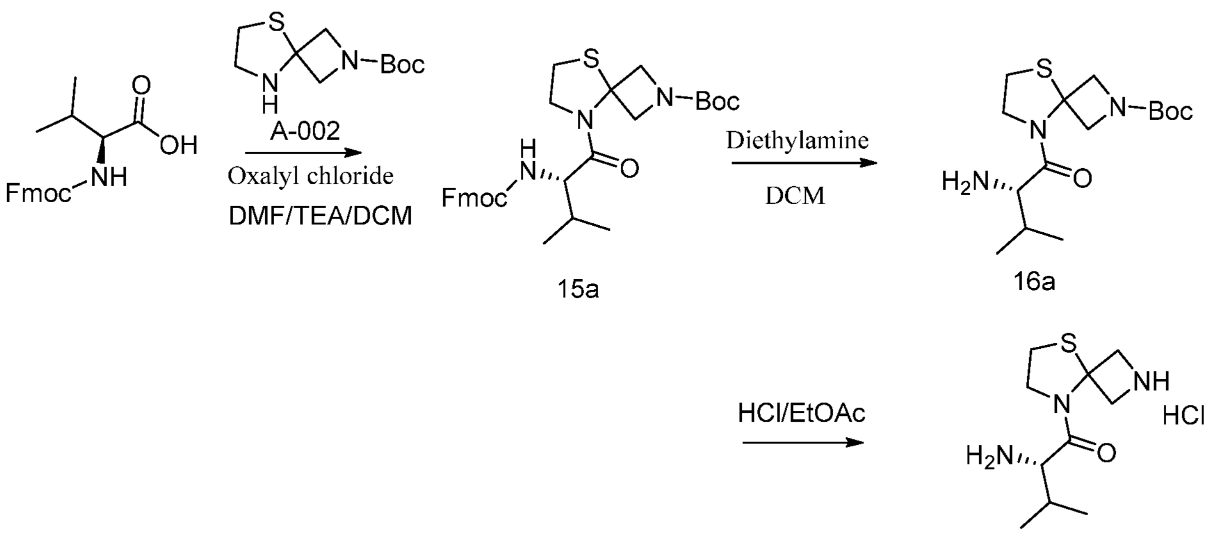


15a

16a

A-329

1. Synthesis of intermediate 15a

Under ice bath, 0.5 g (1.5 mmol) of Fmoc-Val was dissolved in 10 mL of anhydrous DCM, 2 drops of DMF were added for catalysis, then 230 μL (1.65 mmol) of TEA was added, 133 μL (1.58 mmol) of oxalyl chloride was added drop by drop, and the raw materials were returned to room temperature, and reacted overnight; when the completion of the raw materials reaction was monitored by TLC, 0.35 g (1.5 mmol) of compound A-002 was added to the reaction system, and the reaction system was continuously stirred at the room temperature for 4 h; when the completion of the raw materials reaction was monitored by TLC, the reaction was terminated; saturated citric acid was used for washing twice, saturated sodium chloride solution was used for washing once, and an organic phase was taken, dried with anhydrous sodium sulfate, filtered, concentrated, and separated through a silica gel column (PE: EtOAC= 3:1) to obtain a total of 0.69 g of colorless oily substance with a yield of 83.3%. $^1$H-NMR (400MHz, DMSO-$d_6$), δppm: 1.12-1.23(m,6H), 1.37(9H), 2.15-2.25(m,1H), 3.16-3.73(m,5H), 4.16-4.79(m,8H), 7.21-8.29(m,9H). M+1: 552.25.

2. Synthesis of intermediate 16a

0.48 g of the intermediate 15a was dissolved in 5 mL of anhydrous acetonitrile, 5 mL of diethylamine was added, and the raw materials were reacted at 50°C for 4 h; when the completion of the raw materials reaction was monitored by TLC, the reaction was terminated; the reaction product was subjected to rotary evaporation under reduced pressure, 10 ml of DCM was added, and the product was continuously subjected to rotary evaporation, then the above steps were repeated twice; and then 0.25 g of an oily substance with a yield of 87.2% was obtained by separating through a silica gel column. $^1$H-NMR (400MHz, DMSO-$d_6$), δppm: 1.12-1.20(m,6H), 1.37(s,9H), 2.14-2.27(m,1H), 3.13-3.77(m,5H), 4.13-4.57(m,4H), 8.23(s,2H). M+1: 330.18.

3.Synthesis of A-329

0.25 g of the intermediate 16 was dissolved in 4 mL of ethyl acetate, 3 mL of 4 mol/L HCl/EtOAc was added, and the raw materials were stirred at room temperature for 3 h; when the completion of the raw materials reaction was monitored by TLC, a white solid was generated in the system, filtered, and dried to obtain 0.16 g of the white solid with a yield of 79%. $^1$H-NMR (400MHz, DMSO-$d_6$), δppm: 1.12-1.19(m,6H), 2.14-2.25(m,1H), 3.13-3.75(m,5H), 4.16-4.61(m,4H), 8.29(s,2H), 9.01(s,1H). M+1: 230.12.

Example of synthesis of A-330

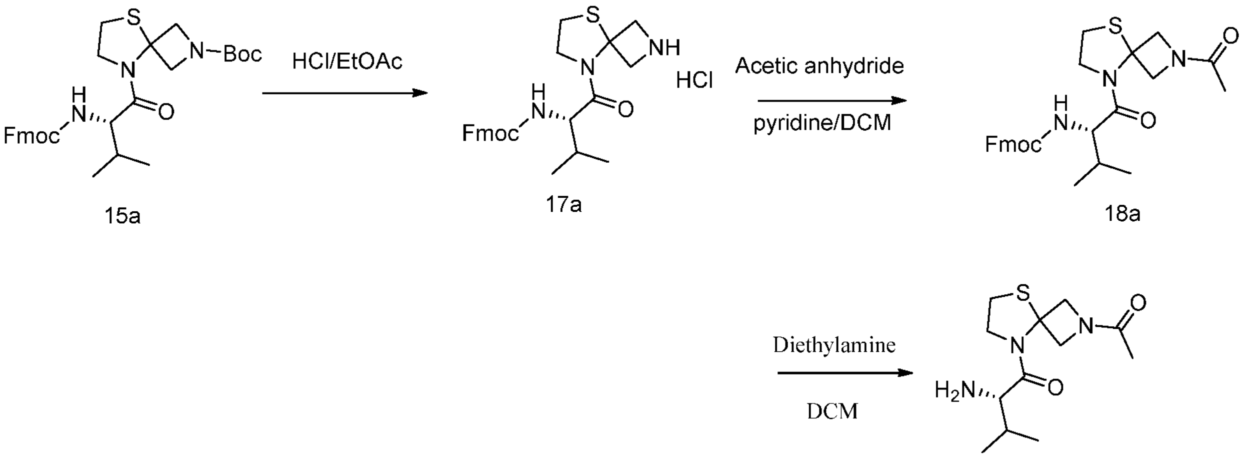


15a 17a 18a

A-330

Synthesis of intermediate 17a:

The synthesis method was similar to that of A-329, and a total of 0.65 g of a yellowish solid was obtained with a yield of 89.3%. $^1$H-NMR (400MHz, DMSO-$d_6$), δppm: 1.14-1.27(m,6H), 2.15-2.25(m,1H), 3.16-3.73(m,5H), 4.16-4.79(m,8H), 7.21-8.29(m,9H). M+1: 452.19.

Synthesis of intermediate 18a:

Under ice bath conditions, a total of 0.62 g of the intermediate 17 was dissolved in 10 mL of anhydrous DCM, 300 μL of TEA was added for dissociation, 185 μL of acetic anhydride was slowly added dropwise, and the raw materials were gradually returned to room temperature and continuously reacted for 6 h; when the completion of the raw materials reaction was monitored by TLC, the reaction was terminated; the mixture was washed with a saturated citric acid aqueous solution and then with a saturated sodium chloride solution, dried with anhydrous sodium sulfate, filtered, concentrated, and separated through a silica gel column to obtain 0.52 g of an oily substance with a yield of 83%. $^1$H-NMR (400MHz, DMSO-$d_6$), δppm: 1.13-1.27(m,6H), 2.05-2.27(m,4H), 3.15-3.70(m,5H), 4.12-4.75(m,8H), 7.21-8.28(m,9H). M+1: 494.2.

Example of synthesis of A-330:

The synthesis method was similar to that of the intermediate 16a, and a total of 0.38 g of an oily substance was obtained, HCl/EtOAc was added and the mixture was stirred, then a white solid was generated in the system, filtered, and dried to obtain 0.34 g of a product A-330. $^1$H-NMR (400MHz, DMSO-$d_6$), δppm: 1.12-1.18(m,6H), 2.05-2.24(m,4H), 3.11-3.78(m,5H), 4.12-4.65(m,4H), 8.60(s,2H). M+1: 272.14.

Example of synthesis of A-331

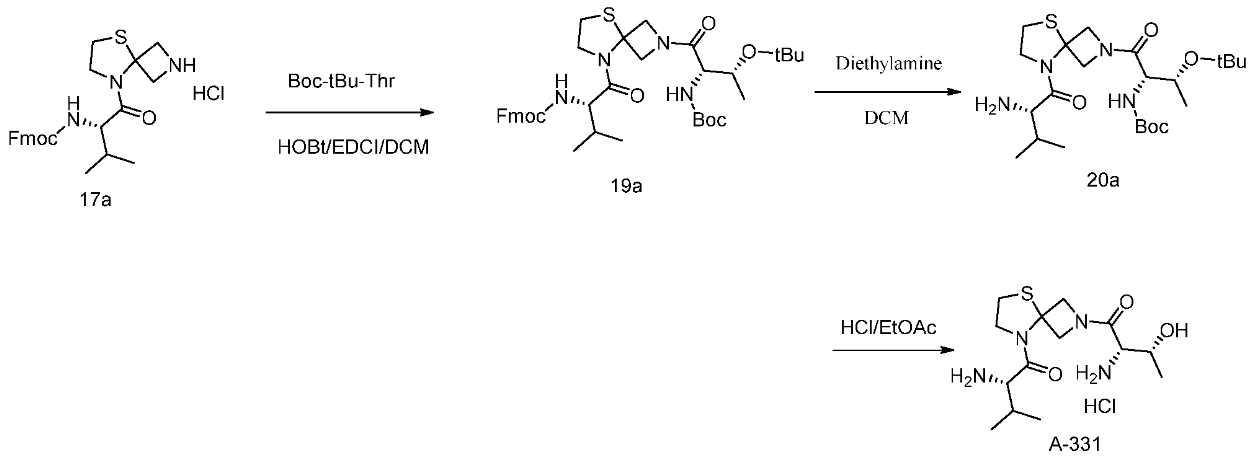


17a 19a 20a A-331

Synthesis of intermediate 19a:

The synthesis method was similar to that of the intermediate 8a, and a total of 1.52 g of a colorless oily substance was obtained with a yield of 81.3%. $^1$H-NMR (400MHz, DMSO-$d_6$), δppm: 1.03-1.37 (m,27H), 2.32-2.38(m,1H), 3.02-3.73(m,4H), 4.14-4.68(m,10H), 7.12-8.32(m,10H). M+1: 709.36.

Synthesis of intermediate 20a:

The synthesis method was similar to that of the intermediate 16a, and 0.84 g of an oily substance was obtained with a yield of 86.9%. $^1$H-NMR (400MHz, DMSO-$d_6$), δppm: 1.01-1.38 (m,27H), 2.32-2.38(m,1H), 3.05-3.77(m,4H), 4.12-4.71(m,7H), 7.76(s,1H), 8.35(s,2H). M+1: 487.29.

Example of synthesis of A-331:

The synthesis method was similar to that of A-329, and 0.49 g of a white solid was obtained with a yield of 87.5%. $^1$H-NMR (400MHz, DMSO-$d_6$), δppm: 1.07-1.22 (m,9H), 2.18-2.27 (m,1H), 3.07-3.78 (m,7H), 4.14-4.59 (m,4H), 5.72(s,1H), 8.21-8.32(m,4H). M+1: 331.17.

Example of synthesis of A-347 and A-348

A-347:

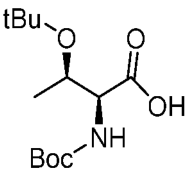

in the synthesis steps of A-331 was replaced with

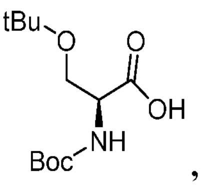
,

and the rest operation was the same as that in the A-331 synthesis. M+1: 317.16.

A-348:

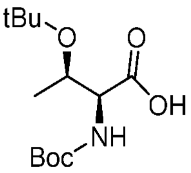

in the synthesis steps of A-331 was replaced with

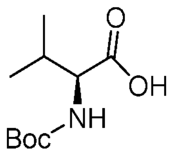

,

and the rest operations were the same as those in A-331 synthesis. M+1: 329.20.

Example of synthesis of A-332

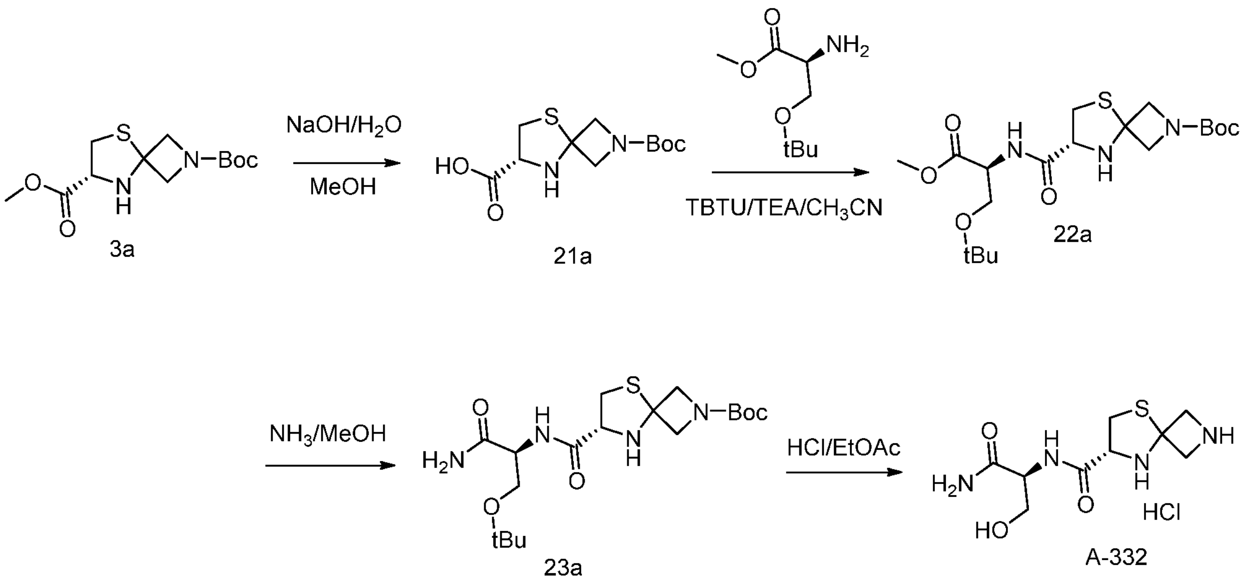


Synthesis of intermediate 21a:

5.2g (18 mmol) of the intermediate 3a was dissolved in 20 mL of methanol, 3.6g (90 mmol) of NaOH was taken and dissolved in 10 mL of water to obtain an aqueous NaOH solution, the aqueous NaOH solution was slowly added to the system of the intermediate 3a under ice bath conditions and the color of the reaction solution became darker, and the raw materials were gradually returned to room temperature and reacted for 3 hours; when the completion of the raw materials reaction was monitored by TLC, the reaction was terminated; the mixture was concentrated under reduced pressure to remove methanol in the system, pH value of the concentrate was adjusted to slightly acidic with dilute hydrochloric acid, a white solid was precipitated from the system, filtered, and dried by infrared lamp, and 4.3 g of the white solid was obtained with a yield of 86.9%. $^1$H-NMR (400MHz, DMSO-$d_6$), δppm: 1.37(s,9H), 3.06-3.56(m,3H), 3.94-4.23 (m,4H), 8.54(s,1H). M-1: 273.1.

Synthesis of intermediate 22a:

Under ice bath conditions, a total of 0.8 g (2.9 mmol) of the intermediate 21a was dissolved in 20 mL of acetonitrile, 0.8 mL (5.8 mmol) of TEA and 1.1 g (3.5 mmol) of TBTU were added, then 0.53 g (3.1 mmol) of O-tert-butyl-L-serine methyl ester hydrochloride was added, and the raw materials were gradually returned to the room temperature and reacted for 4 hours; when the completion of the raw materials reaction was monitored by TLC, the reaction was terminated. The mixture was washed with saturated citric acid and then washed with saturated sodium chloride, dried with anhydrous sodium sulfate, filtered, concentrated, and separated through a silica gel column to obtain 0.86 g of an oily substance with a yield of 76.1%. $^1$H-NMR (400MHz, DMSO-$d_6$), δppm: 1.13(s,9H), 1.42(s,9H), 2.67-4.38(m,13H), 7.43 (s,1H), 8.21(s,1H). M+1: 432.21.

Synthesis of intermediate 23a:

The synthesis method was similar to that of the intermediate 9a, and a total of 0.76 g of an oily substance was obtained with a yield of 93.5%. $^1$H-NMR (400MHz, DMSO-$d_6$), δppm: 1.13(s,9H), 1.42(s,9H), 2.67-4.38(m,10H), 7.06-7.21 (m,3H), 8.21(s,1H). M+1: 417.21.

Example of synthesis of A-332:

The synthesis method was similar to that of A-329, and a total of 0.53 g of a white solid was obtained with a yield of 81.3%. $^1$H-NMR (400MHz, DMSO-$d_6$), δppm: 2.67-4.54(m,10H), 4.94(s,1H), 7.21-8.11(m,5H). M+1: 261.10.

Example of synthesis of A-370

A-370: the synthesis method was similar to that of A-332,

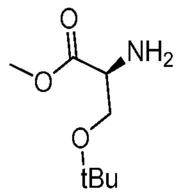

in A-332 synthesis was replaced with

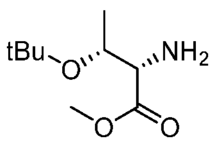

,

and the rest operations were the same as those of A-332. M+1: 275.11.

Examples of synthesis of A-383, A-390 and A-397

A-383: the intermediate 5a in A-332 synthesis was replaced with the intermediate 5b,

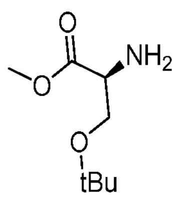

was replaced with

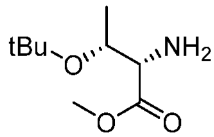

and the rest operations were the same as those in the A-332 synthesis. M+1: 289.13.

A-390: the intermediate 5a in A-332 synthesis was replaced with the intermediate 5c,

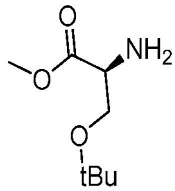

was replaced with

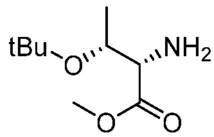

and the rest operations were the same as those in the A-332 synthesis. M+1: 303.14.

A-397: the intermediate 5a in A-332 synthesis was replaced with the intermediate 5d,

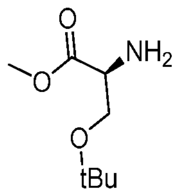

was replaced with

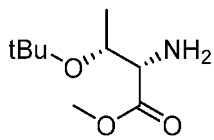

and the rest operations were the same as those in the A-332 synthesis. M+1: 303.14.

Example of synthesis of A-371

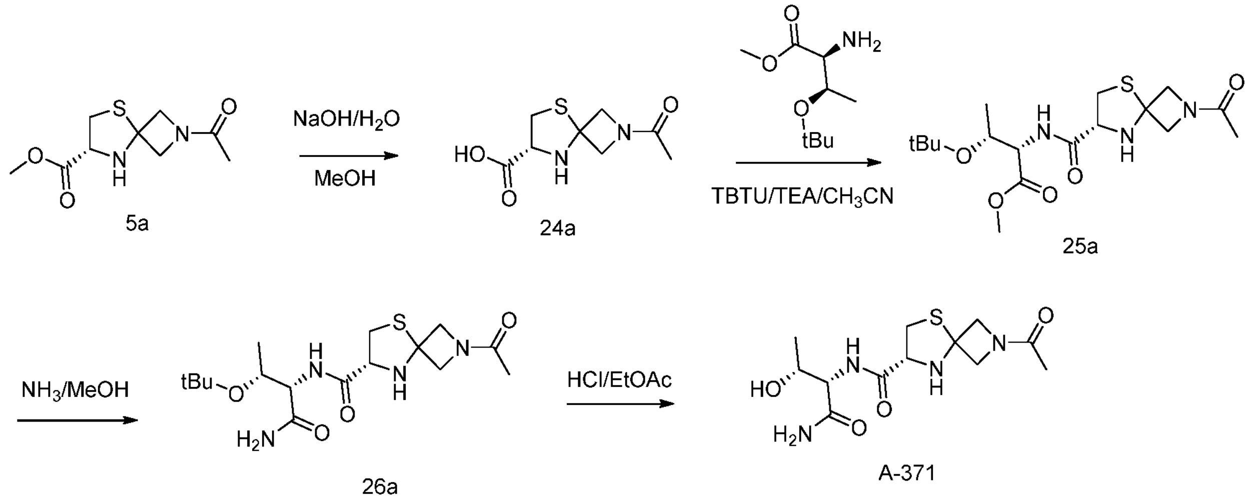


5a 24a 25a 26a A-371

Synthesis of intermediate 24a:

The synthesis method was similar to that of the intermediate 21a, and a total of 0.68 g of an oily substance was obtained with a yield of 76.7%. $^1$H-NMR (400MHz, DMSO-$d_6$), δppm: 2.10 (s,3H), 2.67-2.92 (m,2H), 3.67 (m,1H), 4.12-4.38 (m,4H), 7.06 (s,1H), 12.39 (s,1H). M+1: 217.06.

Synthesis of intermediate 25a:

The synthesis method was similar to that of the intermediate 22a, and a total of 0.93 g of an oily substance was obtained with a yield of 79.1%. $^1$H-NMR (400MHz, DMSO-$d_6$), δppm: 1.13 (s,12H), 2.10 (s,3H), 2.67-2.92 (m,2H), 3.67 (s,3H), 3.69-4.38 (m,7H), 7.06 (s,1H), 8.32 (s,1H). M+1: 374.17.

Synthesis of intermediate 26a:

The synthesis method was similar to that of the intermediate 23a, and a total of 0.67 g of a substance was obtained with a yield of 75.2%. $^1$H-NMR (400MHz, DMSO-$d_6$), δppm: 1.13 (s,12H), 2.10 (s,3H), 2.67-2.92 (m,2H), 3.33-4.38 (m,7H), 7.06 (s,1H), 7.21(s,2H), 8.32 (s,1H). M+1: 359.17.

Synthesis of A-371:

The synthesis method was similar to that of A-332, and a total of 0.48 g of a colorless oily substance was obtained, and then evacuated by an oil pump to obtain a white solid with a yield of 85.7%. $^1$H-NMR (400MHz, DMSO-$d_6$), δppm: 2.10(s,6H), 2.33-2.85(m,2H), 4.12-4.54(m,7H), 4.94(s,1H), 7.21(s,2H), 8.26(s,2H). M+1: 303.11.

Example of synthesis of A-333

A-333:

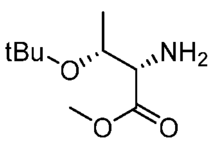

in the synthesis steps of A-371 was replaced with

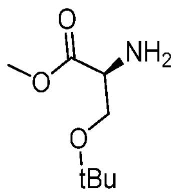

,

and the rest operations were the same as those of A-371. M+1: 317.12.

Examples of A-372 and A-373

A-372: 5a in the synthesis steps of A-371 was replaced with 6a, and the rest operations were the same as those of A-371. M+1: 345.16.

A-373: 5a in the synthesis steps of A-371 was replaced with 7a, and the rest operations were the same as those of A-371. M+1: 359.17.

Examples of synthesis of A-384, A-391 and A-398

A-384: the intermediate 5a in the synthesis steps of A-371 was replaced with the intermediate 5b, and the rest operations were the same as those of A-371. M+1: 331.14.

A-391: the intermediate 5a in the synthesis steps of A-371 was replaced with the intermediate 5c, and the rest operations were the same as those of A-371. M+1: 345.16.

A-398: the intermediate 5a in the synthesis steps of A-371 was replaced with the intermediate 5d, and the rest operations were the same as those of A-371. M+1: 345.16.

Examples of synthesis of A-385 and A-386

A-385: the intermediate 5a in the synthesis steps of A-371 was replaced with the intermediate 6b, and the rest operations were the same as those of A-371. M+1: 359.17.

A-386: the intermediate 5a in the synthesis steps of A-371 was replaced with the intermediate 7b, and the rest operations were the same as those of A-371. M+1: 373.19.

Examples of synthesis of A-392 and A-393

A-392: the intermediate 5a in the synthesis steps of A-371 was replaced with the intermediate 6c, and the rest operations were the same as those of A-371. M+1: 373.18.

A-393: the intermediate 5a in the synthesis steps of A-371 was replaced with the intermediate 7c, and the rest operations were the same as those of A-371. M+1: 387.2.

Examples of synthesis of A-399 and A-400

A-399: the intermediate 5a in the synthesis steps of A-371 was replaced with the intermediate 6d, and the rest operations were the same as those of A-371. M+1: 373.19.

A-400: the intermediate 5a in the synthesis steps of A-371 was replaced with the intermediate 7d, and the rest operations were the same as those of A-371. M+1: 387.21.

Example of synthesis of A-334

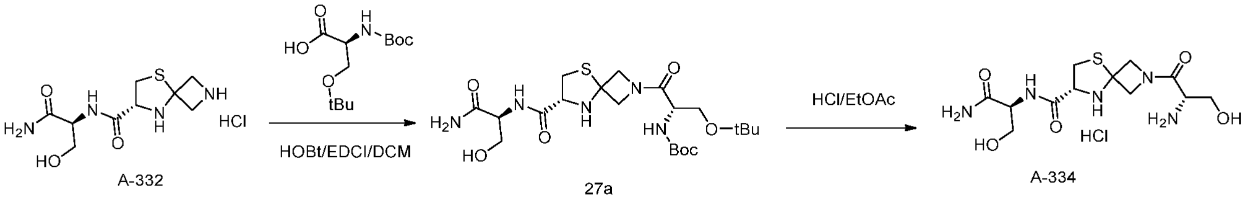


A-332 27a A-334

Synthesis of intermediate 27a:

The synthesis method was similar to that of the intermediate 19a, and a total of 0.56 g of a colorless oily substance was obtained with a yield of 75.4%. $^1$H-NMR (400MHz, DMSO-$d_6$), δppm: 1.19(s,9H), 1.42(s,9H), 2.32-2.67(m,2H), 3.33-4.54(m,11H), 4.94(s,1H), 7.06(s,1H), 7.21(s,2H), 7.31(s,1H), 8.06(s,1H). M+1: 504.24.

Synthesis of A-334:

The synthesis method was similar to that of A-371, and a total of 0.31 g of a white solid was obtained with a yield of 87.9%. $^1$H-NMR (400MHz, DMSO-$d_6$), δppm: 2.67-2.92(m,2H), 3.54-4.52(m,11H), 5.08(s,2H), 7.06-7.21(m,3H), 8.32(s,1H), 8.92(s,2H). M+1: 348.13.

Example of synthesis of A-335

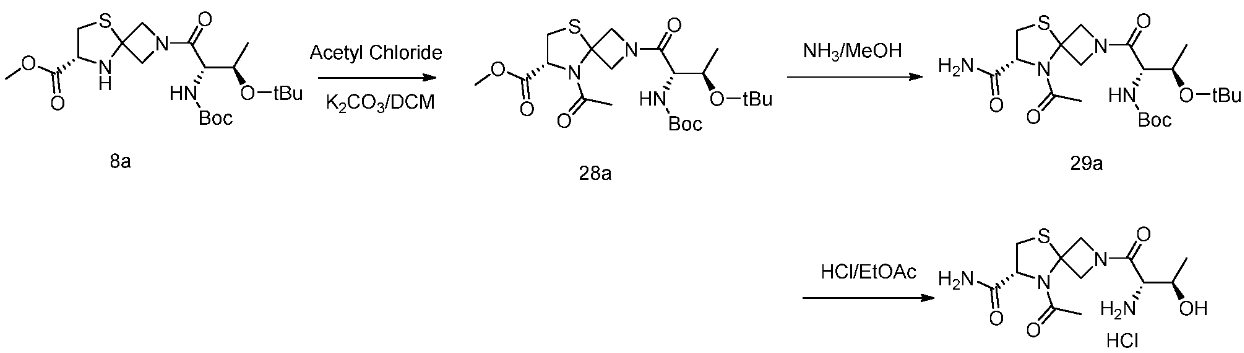


8a 28a 29a A-335

Synthesis of intermediate 28a:

Under ice bath conditions, a total of 0.7 g of the intermediate 8a was dissolved in 20 mL of DCM, 0.4 g of $K_2CO_3$ was added, 170 μL of acetyl chloride was dissolved in 5 mL of DCM to obtain a solution, and the solution was dripped to the reaction system and the raw materials were gradually returned to room temperature and reacted for 4 hours; when the completion of the raw materials reaction was monitored by TLC, the reaction was terminated; the mixture was washed with a saturated citric acid solution and then washed with a saturated sodium chloride solution, dried with anhydrous sodium sulfate, filtered, concentrated, and separated by a silica gel column to obtain a total of 0.53 g of a colorless oily substance intermediate 27a with a yield of 69%. $^1$H-NMR (400MHz, DMSO-$d_6$), δppm: 1.13-1.23(m,12H), 1.37(s,9H), 2.05(s,3H), 3.15-3.43(m,2H), 3.61-3.87(m,5H), 4.05-4.87(m,5H), 7.84(s,1H). M+1: 488.24.

Synthesis of intermediate 29a:

The synthesis method was similar to that of the intermediate 23a, and a total of 0.46 g of an oily substance was obtained with a yield of 92.6%. $^1$H-NMR (400MHz, DMSO-$d_6$), δppm: 1.13-1.25(m,12H), 1.37(s,9H), 2.04(s,3H), 3.16-3.44(m,2H), 3.62-3.87(m,2H), 4.06-4.85(m,5H), 7.84(s,1H), 8.35(s,2H). M+1: 473.24.

Synthesis of A-335:

The synthesis method was similar to that of A-329, and a total of 0.25 g of a white solid was obtained with a yield of 87.5%. $^1$H-NMR (400MHz, DMSO-$d_6$), δppm: 1.13-1.17(m,3H), 2.03(s,3H), 3.07-3.18(m,1H), 3.39-3.43(m,1H), 3.73-3.92(m,2H), 4.07-4.83(m,5H), 5.63 (s,1H), 7.57(s,1H), 7.95 (s,1H), 8.25(s,2H). M+1: 317.12.

Example of synthesis of A-336

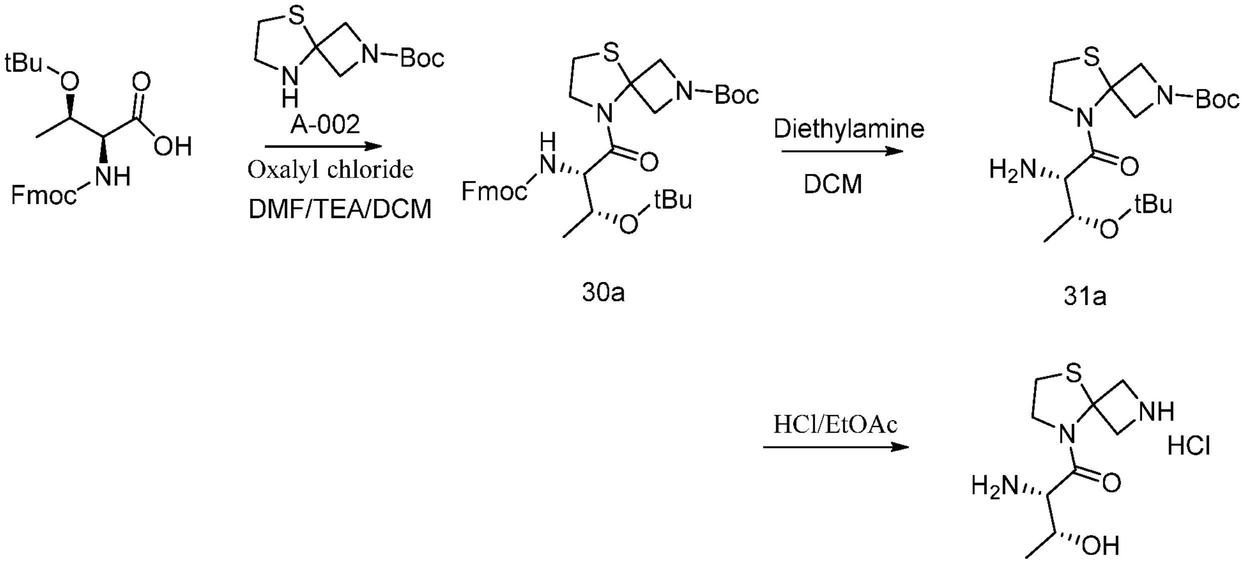

30a
31a
A-336

Synthesis of intermediate 30a:

The synthesis method was similar to that of the intermediate 15a, and a total of 0.89 g of a yellow powdered solid was obtained with a yield of 78.6%. $^1$H-NMR (400MHz, DMSO-$d_6$), δppm: 1.13-1.42(m,21H), 2.7-3.57(m,4H), 4.26-4.51(m,9H), 7.28-7.91(m,9H). M+1: 610.29.

Synthesis of intermediate 31a:

The synthesis method was similar to that of the intermediate 16a, and a total of 0.45 g of a yellow solid was obtained with a yield of 80.3%. $^1$H-NMR (400MHz, DMSO-$d_6$), δppm: 1.13-1.42(m,21H), 2.7-3.57(m,4H), 4.26-4.51(m,9H), 8.96 (s,1H). M+1: 388.22.

Synthesis of A-336:

The synthesis method was similar to that of A-329, and a total of 0.22 g of white powder was obtained with a yield of 70.9%. $^1$H-NMR (400MHz, DMSO-$d_6$), δppm: 1.16(d,3H), 2.70(t,2H), 3.47-4.21(m,8H), 5.37(s,1H), 8.96(s,3H). M+1: 232.11.

Examples of synthesis of A-349, A-356 and A-363

A-349: A-002 in the synthesis steps of A-336 was replaced with A-016, and the rest operations were the same as those in A-336 synthesis. M+1: 246.12.

A-356: A-002 in the synthesis steps of A-336 was replaced with A-030, and the rest operations were the same as those in A-336 synthesis. M+1: 260.14.

A-363: A-002 in the synthesis steps of A-336 was replaced with A-044, and the rest operations were the same as

those in A-336 synthesis. M+1: 260.11.

Example of synthesis of A-337

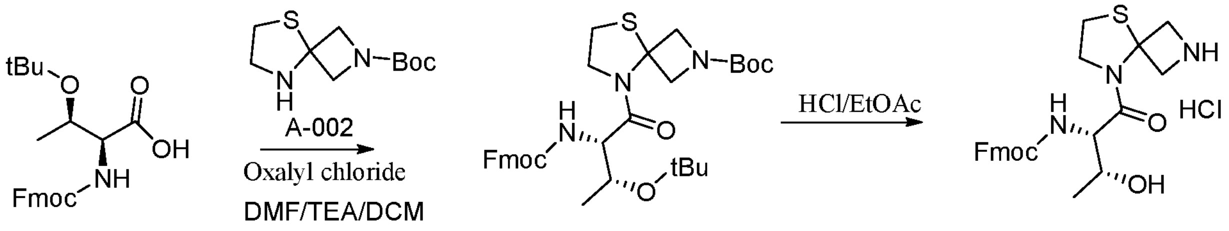


30a

32a

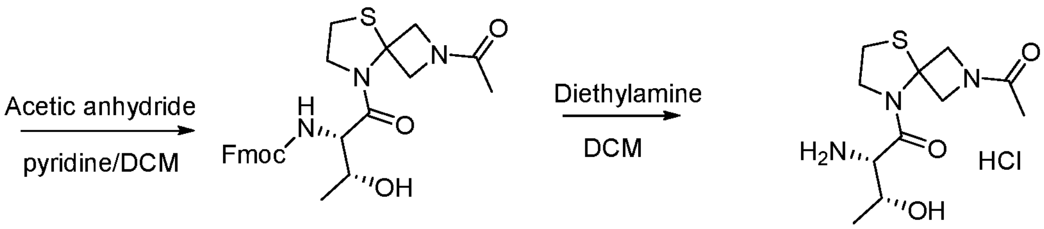


33a

A-337

Synthesis of intermediate 32a:

The synthesis method was similar to that of the intermediate 17a, and a total of 0.42 g of white powder was obtained with a yield of 102.4%. $^1$H-NMR (400MHz, DMSO-$d_6$), δppm: 1.43 (s,3H), 2.7-3.57(m,4H), 4.26-4.51(m,9H), 5.44(s,1H), 7.28-7.91(m,10H). M+1: 454.18.

Synthesis of intermediate 33a:

The synthesis method was similar to that of 18a, and a total of 0.33 g of a solid was obtained with a yield of 77.6%. $^1$H-NMR (400MHz, DMSO-d6), δppm: 1.43 (s,3H), 2.10(s,3H), 2.7-3.57(m,4H), 4.26-4.51(m,9H), 5.44(s,1H), 7.28-7.91(m,9H). M+1: 496.19.

Synthesis of A-337:

The synthesis method was similar to that of A-330, and a total of 0.14 g was obtained with a yield of 70%. $^1$H-NMR (400MHz, DMSO-$d_6$), δppm: 1.43(s,3H), 2.10(s,3H), 2.7-3.57(m,4H), 4.26-4.51(m,9H), 5.44(s,1H), 7.8(s,2H). M+1: 274.12.

In the synthesis process of the intermediate 30a, the intermediate A-002 was replaced with the intermediate A-016, the intermediate A-030 and the intermediate A-044 respectively, and the other operations were the same as those of the synthesis of the intermediate 30a, and an intermediate 30b, an intermediate 30c and an intermediate 30d were prepared respectively;

similarly, in the synthesis of the intermediate 32a, the intermediate 30a was replaced with the intermediate 30b, the intermediate 30c and the intermediate 30d respectively, and the other operations were the same as those of the synthesis of the intermediate 32a, and the intermediate 32b, intermediate 32c and intermediate 32d were prepared respectively.

similarly, in the synthesis process of the intermediate 33a, the intermediate 30a was replaced with the intermediate 30b, the intermediate 30c and the intermediate 30d respectively, and the other operations were the same as those in the synthesis of the intermediate 33a, and an intermediate 33b, an intermediate 33c and an intermediate 33d were prepared respectively;

similarly, in the synthesis of the intermediate 33a, acetyl chloride was replaced with isobutyryl chloride and pivaloyl chloride respectively, and an intermediate 34a, an intermediate 34b, an intermediate 34c, and an intermediate 34d,

and an intermediate 35a, an intermediate 35b, an intermediate 35c, and an intermediate 35d were prepared respectively.

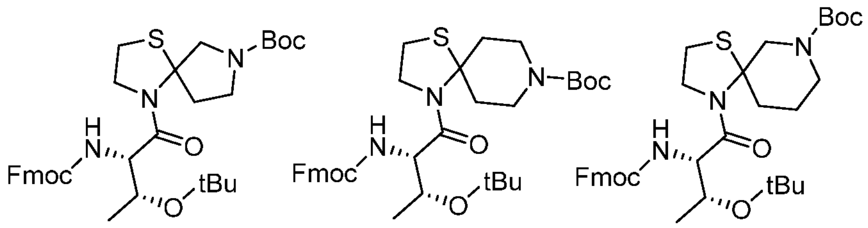

30b 30c 30d

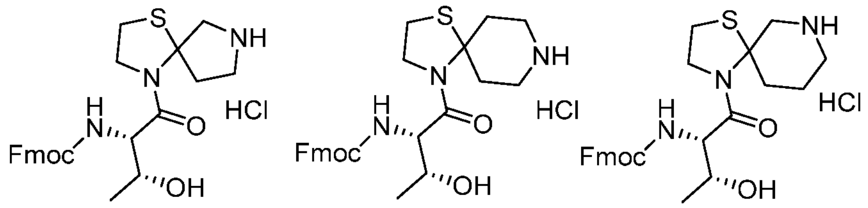

32b 32c 32d

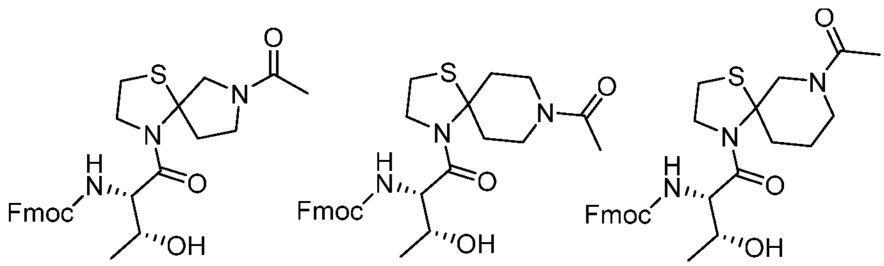

33b 33c 33d

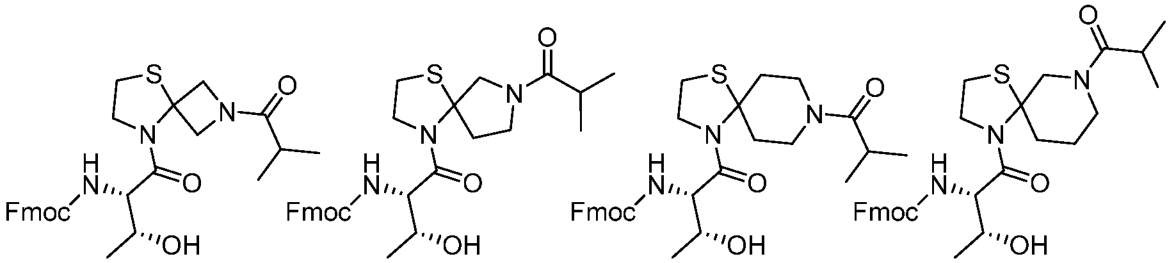

34a 34b 34c 34d

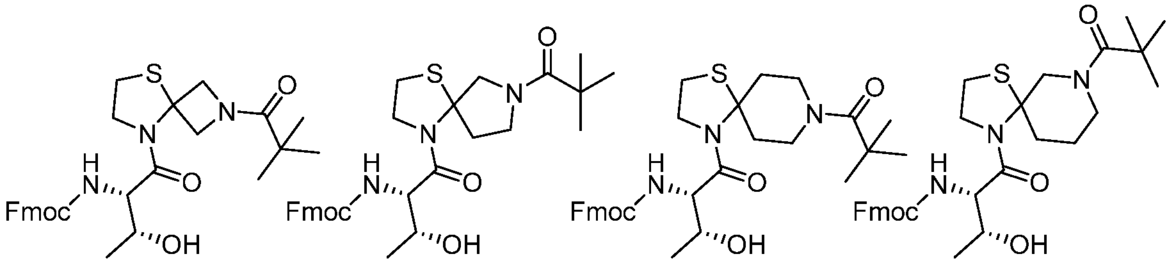

35a 35b 35c 35d

Examples of synthesis of A-338 to A-343:

A-338:

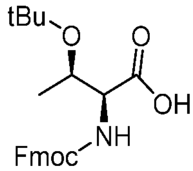

in A-337 synthesis was replaced with

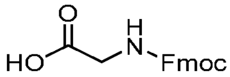,

and the rest operations were the same as those in A-337 synthesis. M+1: 230.09.

A-339:

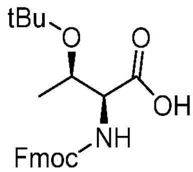

in A-337 synthesis was replaced with

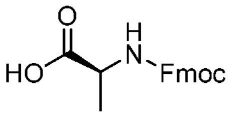,

and the rest operations were the same as those in A-337 synthesis. M+1: 244.11.

A-340:

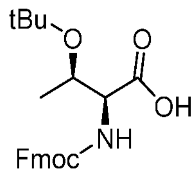

in A-337 synthesis was replaced with

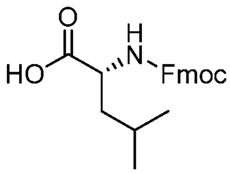,

and the rest operations were the same as those in A-337 synthesis. M+1: 286.15.

A-341:

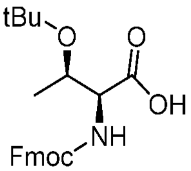

in A-337 synthesis was replaced with

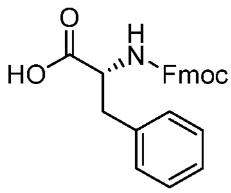

,

and the rest operations were the same as those in A-337 synthesis. M+1: 320.14.

A-342:

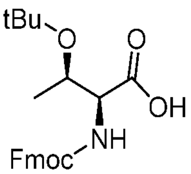

in A-337 synthesis was replaced with

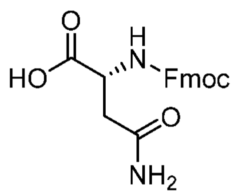

,

and the rest operations were the same as those of A-337. M+1: 287.11.

A-343:

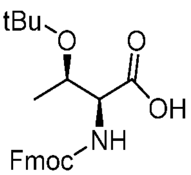

in A-337 synthesis was replaced with

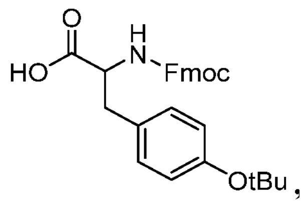

and the rest operations were the same as those in A-337 synthesis. M+1: 336.13.

Examples of synthesis of A-344 and A-345

A-344: 33a in A-337 synthesis was replaced with 34a, and the rest operations were the same as those of A-337. M+1: 302.15.
A-345: 33a in A-337 synthesis was replaced with 35a, and the rest operations were the same as those of A-337. M+1: 316.17.

Examples of synthesis of A-350, A-357 and A-364

A-350: 33a in A-337 synthesis was replaced with 33b, and the rest operations were the same as those of A-337. M+1: 288.13.
A-357: 33a in A-337 synthesis was replaced with 33c, and the rest operations were the same as those of A-337. M+1: 302.15.
A-364: 33a in A-337 synthesis was replaced with 33d, and the rest operations were the same as those of A-337. M+1: 302.1.

Examples of synthesis of A-351, A-352, A-358, A-359, A-365 and A-366

A-351: 33a in A-337 synthesis was replaced with 34b, and the rest operations were the same as those of A-337. M+1: 316.17.
A-352: 33a in A-337 synthesis was replaced with 35b, and the rest operations were the same as those of A-337. M+1: 330.18.
A-358: 33a in A-337 synthesis was replaced with 34c, and the rest operations were the same as those of A-337. M+1: 330.18.
A-359: 33a in A-337 synthesis was replaced with 35c, and the rest operations were the same as those of A-337. M+1: 344.20.
A-365: 33a in the A-337 synthesis was replaced with 34d, and the rest operations were the same as those of A-337. M+1: 330.16.
A-366: 33a in A-337 synthesis was replaced with 35d, and the rest operations were the same as those of A-337. M+1: 344.15.

Example of synthesis of A-346

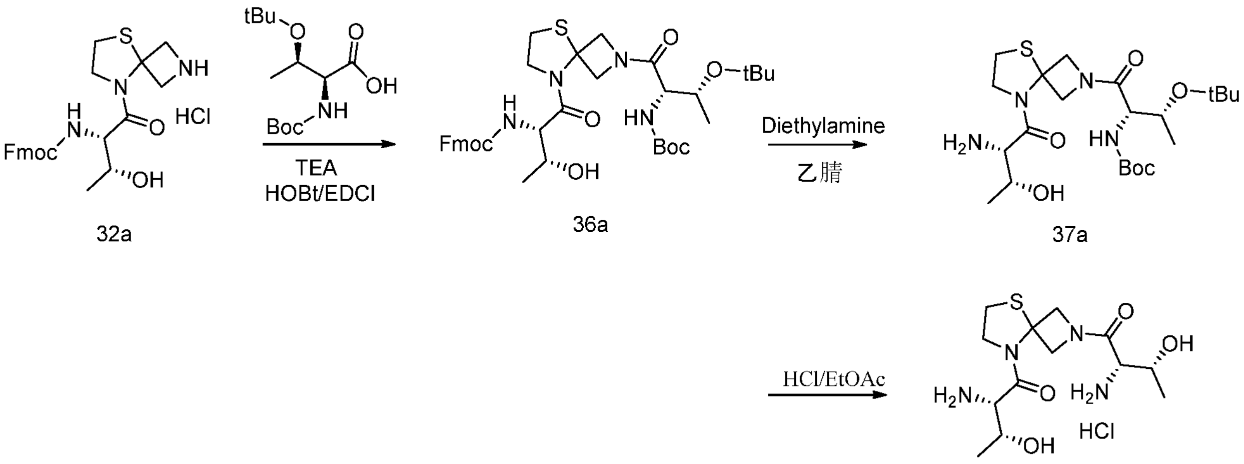


32a 36a 37a A-346

Synthesis of intermediate 36a:

The synthesis method was similar to that of 1a, and a total of 0.22 g of yellow powder was obtained with a yield of 37%. $^1$H-NMR (400MHz, DMSO-$d_6$), δppm: 1.13-1.42 (m,24H), 2.7-3.57 (m,4H), 4.26-4.70 (m,11H), 5.37 (s,1H), 7.28-7.90 (m,10H). M+1: 711.34.

Synthesis of intermediate 37a:

The synthesis method was similar to that of the intermediate 16a, and 0.13 g of a solid was obtained with a yield of 41%. $^1$H-NMR (400MHz, DMSO-$d_6$), δppm: 1.13-1.42(m,24H), 2.7 (t,2H), 3.47-4.64(m,10H), 5.37(s,1H), 7.28-7.90(m,3H). M+1: 489.27.

Synthesis of A-346:

The synthesis method was similar to that of A-331, 59 mg of a substance was obtained with a yield of 22.3%. $^1$H-NMR (400MHz, DMSO-$d_6$), δppm: 1.16(s,6H), 2.7 (t,2H), 3.47-4.64(m,10H), 5.37(s,2H), 8.95(s,4H). M+1: 333.16.

In the synthesis of an intermediate 36a, the intermediate 32a was replaced with 32b, 32c and 32d respectively, and the other operations were the same as those of the synthesis of the intermediate 36a, and an intermediate 36b-1, an intermediate 36c-1 and an intermediate 36d-1 were prepared respectively;

similarly, in the synthesis of the intermediate 36a, the raw material intermediate was respectively replaced with 32b, 32c and 32d,

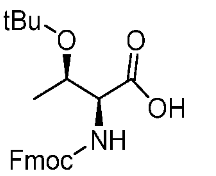

was respectively replaced with

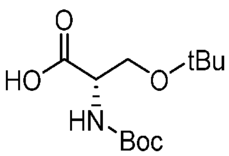
and
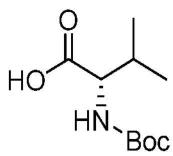
,

so that 36b-2, 36b-3, 36c-2, 36c-3, 36d-2 and 36d-3 were obtained respectively.

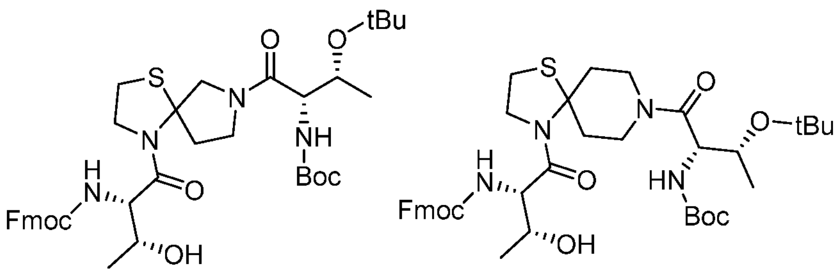

36b-1

36c-1

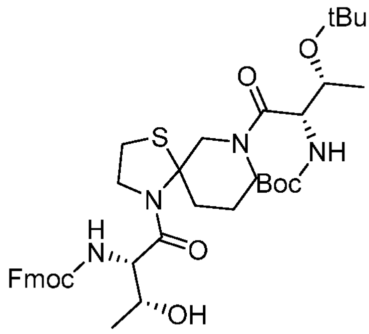

36d-1

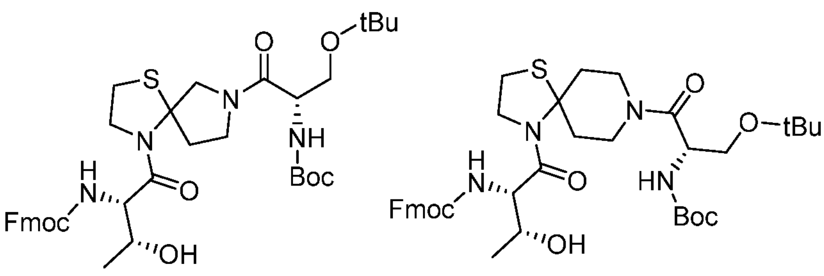

36b-2

36c-2

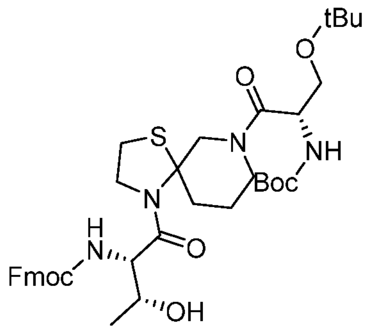

36d-2

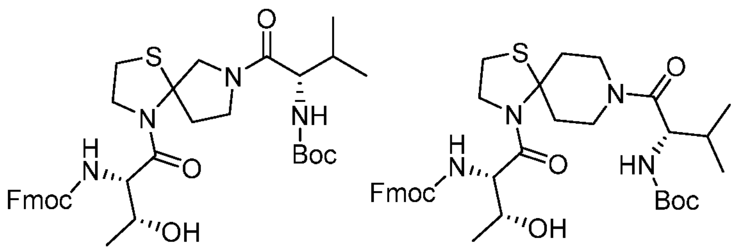

36b-3

36c-3

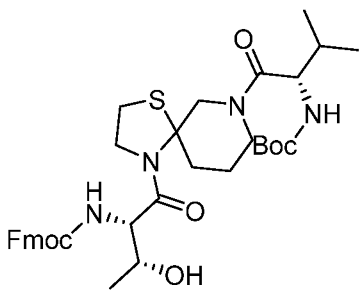

36d-3

Examples of synthesis of A-353, A-360 and A-367

A-353: 36a in A-346 synthesis was replaced with 36b-1, and the rest operations were the same as those of A-346. M+1: 347.17.

A-360: 36a in A-346 synthesis was replaced with 36c-1, and the rest operations were the same as those of A-346. M+1: 361.19.

A-367: 36a in A-346 synthesis was replaced with 36d-1, and the rest operations were the same as those of A-346. M+1: 361.2.

Examples of synthesis of A-354, A-355, A-361, A-362, A-368 and A-369

A-354: 36a in A-346 synthesis was replaced with 36b-2, and the rest operations were the same as those of A-346. M+1: 333.16.

A-355: 36a in A-346 synthesis was replaced with 36b-3, and the rest operations were the same as those of A-346. M+1: 345.19.

A-361: 36a in A-346 synthesis was replaced with 36c-2, and the rest operations were the same as those of A-346. M+1: 347.17.

A-362: 36a in A-346 synthesis was replaced with 36c-3, and the rest operations were the same as those of A-346. M+1: 359.21.

A-368: 36a in the synthesis of A-346 was replaced with 36d-2, and the rest operations were the same as those of A-346. M+1: 347.3.

A-369: 36a in A-346 synthesis was replaced with 36d-3, and the rest operations were the same as those of A-346. M+1: 359.25.

Example of synthesis of A-374

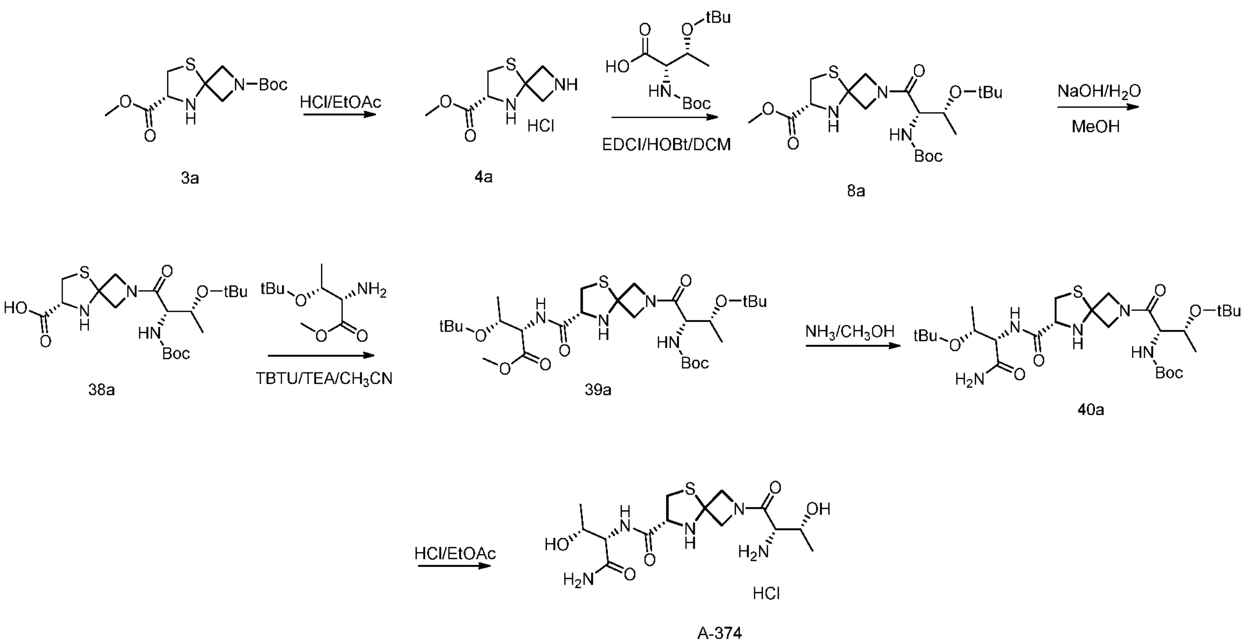

3a 4a 8a 38a 39a 40a A-374

Synthesis of intermediate 38a:

8.0g (18 mmol) of the intermediate 8a was dissolved in 30 mL of methanol, 3.6g (90 mmol) of NaOH was taken and dissolved in 10 mL of water to obtain an aqueous NaOH solution, the aqueous NaOH solution was slowly added to the reaction system of the intermediate 8a under ice bath conditions, the color of the reaction solution became darker, and the raw materials were gradually returned to room temperature and reacted for 3 hours; when the completion of the raw materials reaction was monitored by TLC, the reaction was terminated; the mixture was concentrated under reduced pressure to remove methanol in the system, pH value of the system was adjusted to slightly acidic by dilute hydrochloric acid, a white solid was precipitated from the system, filtered, and dried by infrared lamp, and 4.3 g of the white solid was obtained with a yield of 58.1%. $^1$H-NMR (400MHz, DMSO-$d_6$), δppm: 1.13(s,12H), 1.37(s,9H), 2.67-3.77(m,3H), 4.12-4.64(m,6H), 7.39-7.06(m,2H), 12.01(s,1H). M-1: 432.1.

Synthesis of intermediate 39a:

Under ice bath conditions, a total of 4.3 g (10 mmol) of the intermediate 38a was dissolved in 20 mL of acetonitrile, 2.7 mL (20 mmol) of TEA and 3.78 g (12 mmol) of TBTU were added, then 1.83 g (10.7 mmol) of O-tert-butyl-L-serine methyl ester hydrochloride was added, and the raw materials were gradually returned to room temperature and reacted for 4 hours; when the completion of the raw materials reaction was monitored by TLC, the reaction was terminated. The mixture was washed with saturated citric acid and then washed with saturated sodium chloride, dried with anhydrous sodium sulfate, filtered, concentrated, and separated by a silica gel column to obtain 4.58 g of an oily substance with a yield of 76.1%. $^1$H-NMR (400MHz, DMSO-$d_6$), δppm:1.13(s,24H), 1.37(s,9H), 2.67-3.84(m,3H), 3.67(s,3H), 4.12-4.64(m,8H), 7.39-8.06(m,3H). M+1: 603.34.

Synthesis of intermediate 40a:

A total of 4.58 g (7.60 mmol) of the intermediate 39a was dissolved in a small amount of methanol, and 10 mL of 7 mol/L $NH_3/CH_3OH$ solution was added under ice bath, and the raw materials were reacted overnight; after the completion of the raw materials reaction was monitored by TLC, a reaction solution was subjected to rotary evaporation to obtain a total of 4.04 g of an off-white solid intermediate 40a with a yield of 90.4%. $^1$H-NMR (400MHz, DMSO-$d_6$), δppm: 1.13(s,24H), 1.37(s,9H), 2.67-3.84(m,3H), 4.12-4.64(m,8H), 7.39-8.06(m,5H). M+1: 588.34.

Synthesis of A-374:

1 g of the intermediate 40a was dissolved in 4 mL of ethyl acetate, 3 mL of 4 mol/L HCl/EtOAc was added, and the raw materials were stirred at room temperature for 3 h; when the completion of the raw materials reaction was monitored by TLC, a white solid was generated in the system, then filtered, and dried to obtain 0.51 g of a white solid with a yield of 79%. $^1$H-NMR (400MHz, DMSO-d6), δppm: 1.16(m,6H), 2.67-4.62(m,11H), 5.32(s,2H), 7.3(s,2H), 8.96(s,4H). M+1: 376.16.

In the synthesis of the intermediate 38a, the raw material

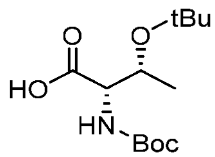

was replaced with

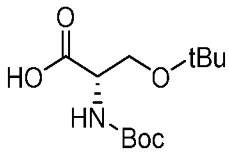

and

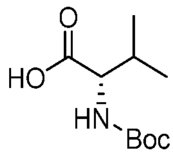

respectively, and the other operations were the same as those of the synthesis of the intermediate 38a, so that an intermediate 38b and an intermediate 38c were prepared respectively;

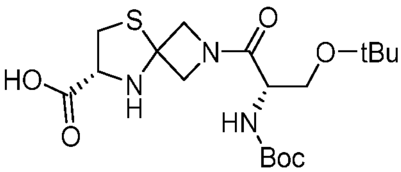

38b

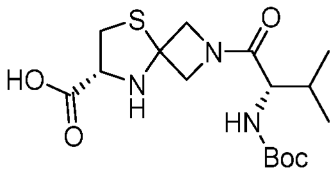

38c

similarly, in the synthesis of the intermediate 39a, the raw material

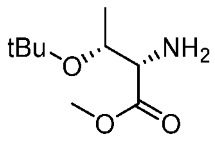

was respectively replaced with

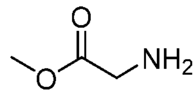, 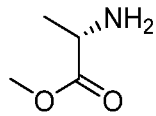 and 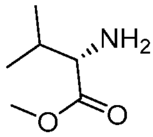,

and 39b, 39c and 39d were obtained respectively;

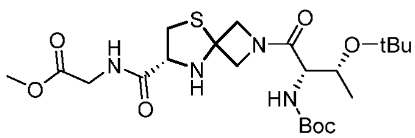

39b

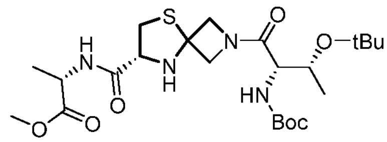

39c

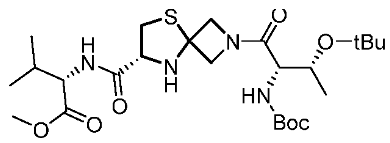

39d

Examples of synthesis of A-375 and A-376

A-375: the intermediate 38a in A-374 synthesis was replaced with 38b, and the rest operations were the same as those of A-374. M+1: 362.15.
A-376: the intermediate 38a in the A-374 synthesis was replaced with 38c, and the rest operations were the same as those of A-374. M+1: 374.18.

Examples of synthesis of A-377, A-378, A-379, A-380, A-381 and A-382

A-377: the intermediate 39a in A-374 synthesis was replaced with 39b, and the rest operations were the same as those of A-374. M+1: 332.13.

A-378: 39a in the synthesis step of the intermediate 39a was replaced with 39c, and the rest operations were the same as those of A-374. M+1: 346.15.

A-379: the intermediate 39a in A-374 synthesis was replaced with 39d, and the rest operations were the same as those of A-374. M+1: 388.20.

A-380: the intermediate 38a in A-374 synthesis was replaced with 38c,

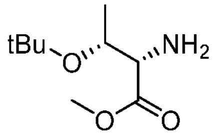

in the synthesis step of the intermediate 39a was replaced with

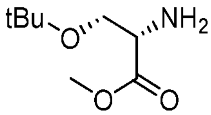

, and the rest operations were the same as those of A-374. M+1: 422.18.

A-381: the intermediate 38a in A-374 synthesis was replaced with 38b,

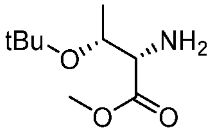

in the synthesis step of the intermediate 39a was replaced with

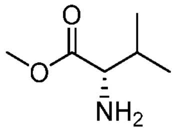

,

and the rest operations were the same as those of A-374. M+1: 389.16.

A-382: The intermediate 38a in A-374 synthesis was replaced with 38c,

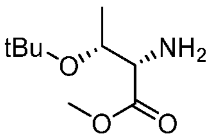

in the synthesis step of the intermediate 39a was replaced with

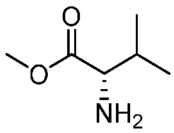

,

and the rest operations were the same as those of A-374. M+1: 438.18.

Examples of synthesis of A-387, A-388 and A-389

A-387: the intermediate 3a in A-374 synthesis was replaced with 3b, and the rest operations were the same as those of A-374. M+1: 390.18.

A-388: the intermediate 3a in A-374 synthesis was replaced with 3b,

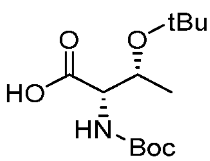

in the synthesis step of the intermediate 38a was replaced with

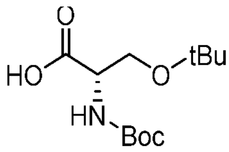

,

and the rest operations were the same as those of A-374. M+1: 376.16.

A-389: the intermediate 3a in A-374 synthesis was replaced with 3b,

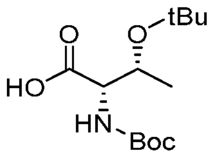

in the synthesis step of the intermediate 38a was replaced with

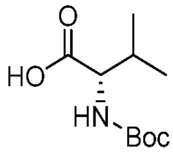

,

and the rest operations were the same as those of A-374. M+1: 388.2.

Examples of synthesis of A-394, A-395 and A-396

A-394: the intermediate 3a in A-374 synthesis was replaced with 3c, and the rest operations were the same as those of A-374. M+1: 404.19.

A-395: the intermediate 3a in A-374 synthesis was replaced with 3c,

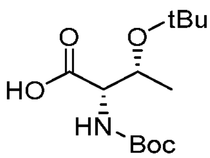

in the synthesis step of the intermediate 38a was replaced with

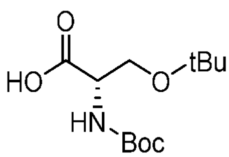

,

and the rest operations were the same as those of A-374. M+1: 390.18.

A-396: the intermediate 3a in A-374 synthesis was replaced with 3c,

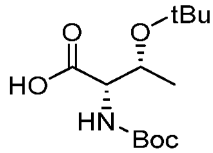

in the synthesis step of the intermediate 38a was replaced with

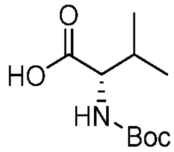,

and the rest operations were the same as those of A-374. M+1: 402.21.

Examples of synthesis of A-401, A-402 and A-403

A-401: the intermediate 3a in A-374 synthesis was replaced with 3d, and the rest operations were the same as those of A-374. M+1: 404.19.

A-402: the intermediate 3a in A-374 synthesis was replaced with 3d,

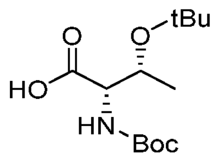

in the synthesis step of the intermediate 38a was replaced with

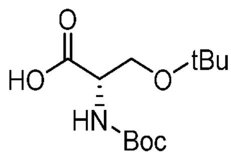,

and the rest operations were the same as those of A-374. M+1: 390.18.

A-403: the intermediate 3a in A-374 synthesis was replaced with 3d,

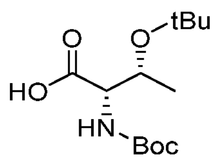

in the synthesis step of the intermediate 38a was replaced with

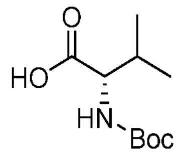
,

and the rest operations were the same as those of A-374. M+1: 402.21.

Example of synthesis of A-405

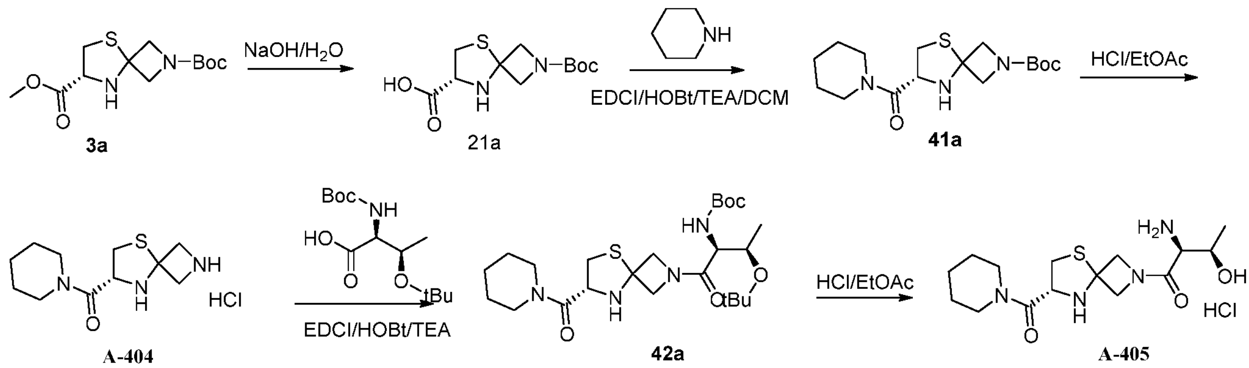

3a 21a 41a A-404 42a A-405

Synthesis of intermediate 41a:

21a (4.32 g, 15.7 mmol) was added to 100 ml of DCM (insoluble) under ice bath conditions, a solid in the solution was gradually dissolved after adding 2.5 eq of TEA, 1.1 eq of piperidine, 1.2 eq of HOBt and 1.2 eq of EDCI were added, and the raw materials were subjected to a thermal reaction for 10 min and then reacted overnight at room temperature; after the completion of the raw materials reaction was monitored by TLC (PE: EA = 1:1), the mixture was washed sequentially with water, saturated citric acid solution and saturated saline, dried with anhydrous sodium sulfate and filtered, and a filtrate was concentrated and separated by column chromatography to obtain 4.9g of a substance with a yield of 90%. $^1$H-NMR (400MHz, DMSO-d6), δppm: 1.42(s,9H), 1.55-2.92(m,8H), 3.54-4.38(m,9H), 7.28 (s,1H). M+1: 342.18.

Synthesis of A-404:

The intermediate 41a (4.9 g, 14.4 mmol) was added to ethyl acetate for complete dissolution, then 28 ml of HCl-EtOAc solution was added, the raw materials were stirred at room temperature for 6 h; when the completion of the raw materials reaction was monitored by TLC (PE: EA = 1:2), the mixture was filtered, and a filter cake was washed with ethyl acetate, and dried to obtain 4.69 g of a white solid with a yield of 93%. $^1$H-NMR (400MHz, DMSO-d6), δppm: 1.55-2.92(m,8H), 3.54-4.38(m,9H), 7.28 (s,2H). M+1: 242.13.

Synthesis of intermediate 42a:

Under ice bath conditions, A-404 (0.5 g, 1.8 mmol) was added to 20 ml of DCM, a solid was insoluble, and the solid was gradually dissolved after adding 2.5 eq of TEA, 1.1 eq of Boc-tBu-Thr, 1.2 eq of HOBt and 1.2 eq of EDCI were added, and the raw materials were subjected to a thermal reaction for 10 min and reacted at room temperature for 5 h; after the completion of the raw materials reaction was monitored by TLC (DCM: MeOH = 10:1), the mixture was sequentially washed with water, saturated citric acid solution and saturated saline, then dried with anhydrous sodium sulfate and filtered, and a filtrate was concentrated and separated by column chromatography to obtain 0.85 g substance with a yield of 94%. $^1$H-NMR (400MHz, DMSO-d6), δppm: 1.16(s,21H), 1.55-2.92(m,8H), 3.54-4.64(m,11H), 7.28 (s,2H). M+1: 499.29.

Synthesis of A-405:

The intermediate 42a was added to 8 ml ethyl acetate for complete dissolution, 8 ml of HCl-EtOAc solution was added, the raw materials were stirred at room temperature for 6 h; after the completion of the raw materials reaction was monitored by TLC (PE: EA = 1:2), the mixture was filtered, and a filter cake was washed with ethyl acetate, and then dried to obtain 0.45 g of a white solid with a yield of 69.6%. $^1$H-NMR (400MHz, DMSO-d6), δppm: 1.16(s,3H), 1.55-2.92(m,8H), 3.54-4.38(m,11H), 5.37 (s,1H),7.28 (s,1H),8.93 (s,2H). M+1: 343.18.

Examples of synthesis of A-408, A-412 and A-416

A-408:

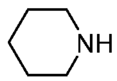

in A-404 synthesis was replaced with

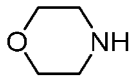,

and the rest operations were the same as those in A-404 synthesis. M+1: 244.11.
A-412:

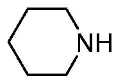

in A-404 synthesis was replaced with

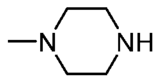,

and the rest operations were the same as those in A-404 synthesis. M+1: 257.14.
A-416:

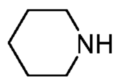

in A-404 synthesis was replaced with

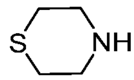,

and the rest operations were the same as those in A-404 synthesis. M+1: 260.08.

Examples of synthesis of A-406 and A-407

A-406:

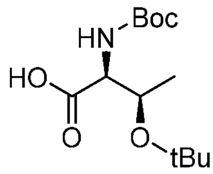

in A-405 synthesis was replaced with

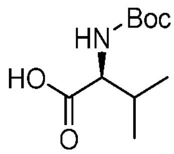,

and the rest operations were the same as those in A-405 synthesis. M+1: 341.20.

A-407:

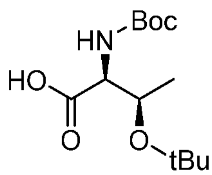

in A-405 synthesis was replaced with

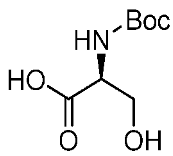,

and the rest operations were the same as those in A-405 synthesis. M+1: 329.16.

Examples of synthesis of A-409, A-410 and A-411

A-409: A-404 in A-405 synthesis was replaced with A-408, and the rest operations were the same as those in A-405 synthesis. M+1: 345.16.

A-410: A-404 in A-405 synthesis was replaced with A-408,

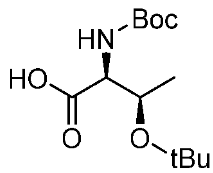

was replaced with

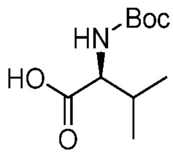

,

and the rest operations were the same as those of A-405. M+1: 343.18.

A-411: A-404 in A-405 synthesis was replaced with A-408,

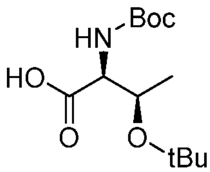

was replaced with

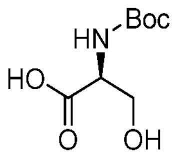

,

and the rest operations were the same as those of A-405. M+1: 331.14.

Examples of synthesis of A-413, A-414 and A-415

A-413: A-404 in A-405 synthesis was replaced with A-412, and the rest operations were the same as those in A-405 synthesis. M+1: 358.19.

A-414: A-404 in A-405 synthesis was replaced with A-412,

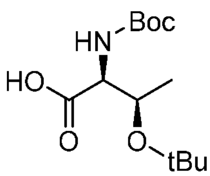

was replaced with

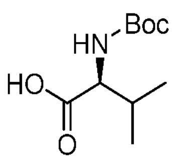

,

and the rest operations were the same as those in A-405 synthesis. M+1: 356.21.

A-415: A-404 in A-405 synthesis was replaced with A-412,

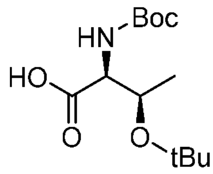

was replaced with

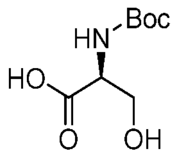
,

and the rest operations were the same as those of A-405. M+1: 344.17.

Examples of synthesis of A-417, A-418 and A-419

A-417: A-404 in A-405 synthesis was replaced with A-416, and the rest operations were the same as those in A-405 synthesis. M+1: 361.13.

A-418: A-404 in A-405 synthesis was replaced with A-416,

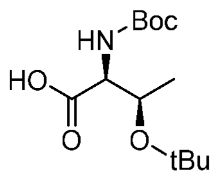

was replaced with

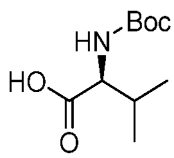
,

and the rest operations were the same as those of A-405. M+1: 359.15.

A-419: A-404 in A-405 synthesis was replaced with A-416,

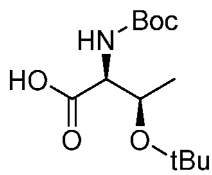

was replaced with

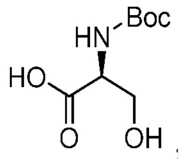

and the rest operations were the same as those of A-405. M+1: 347.12.

Examples of synthesis of A-420, A-436 and A-452

A-420: the intermediate 3a in A-404 synthesis was replaced with 3b, and the rest operations were the same as those of A-404. M+1: 256.14.

A-436: the intermediate 3a in A-404 synthesis was replaced with 3c, and the rest operations were the same as those of A-404. M+1: 270.16.

A-452: the intermediate 3a in A-404 synthesis was replaced with 3d, and the rest operations were the same as those of A-404. M+1: 270.16.

Examples of synthesis of A-421, A-422 and A-423

A-421: A-404 in A-405 synthesis was replaced with A-420, and the rest operations were the same as those in A-405 synthesis. M+1: 357.19.

A-422: A-404 in A-405 synthesis was replaced with A-420,

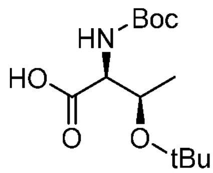

was replaced with

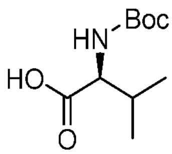

,

and the rest operations were the same as those in A-405 synthesis. M+1: 355.21.

A-423: A-404 in A-405 synthesis was replaced with A-420,

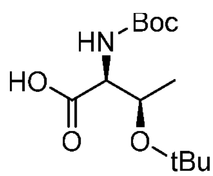

was replaced with

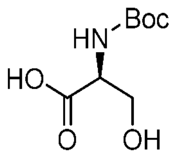,

and the rest operations were the same as those in A-405 synthesis. M+1: 343.18.

Examples of synthesis of A-424, A-428 and A-432

A-424: 3a in A-404 synthesis was replaced with 3b,

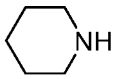

was replaced with

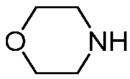,

and the rest operations were the same as those of A-404. M+1: 258.12.
A-428: 3a in A-404 synthesis was replaced with 3b,

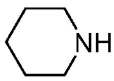

was replaced with

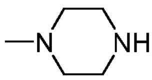,

and the rest operations were the same as those of A-404. M+1: 271.15.
A-432: 3a in A-404 synthesis was replaced with 3b,

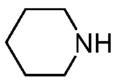

was replaced with

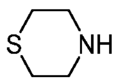,

and the rest operations were the same as those of A-404. M+1: 274.10.

Examples of synthesis of A-425, A-426 and A-427

A-425: A-404 in A-405 synthesis was replaced with A-424, and the rest operations were the same as those in A-405 synthesis. M+1: 359.17.

A-426: A-404 in A-405 synthesis was replaced with A-424,

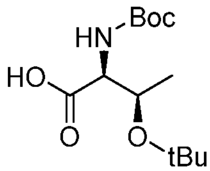

was replaced with

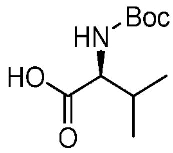,

, and the rest operations were the same as those of A-405. M+1: 357.19.

A-427: A-404 in A-405 synthesis was replaced with A-424,

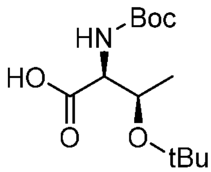

was replaced with

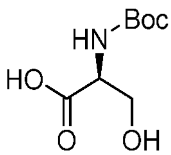,

and the rest operations were the same as those in A-405 synthesis. M+1: 345.16.

Examples of synthesis of A-429, A-430 and A-431

A-429: A-404 in A-405 synthesis was replaced with A-428, and the rest operations were the same as those in A-405 synthesis. M+1: 372.20.

A-430: A-404 in A-405 synthesis was replaced with A-428,

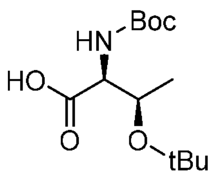

was replaced with

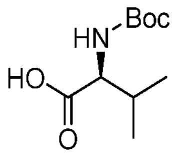

,

and the rest operations were the same as those of A-405. M+1: 370.22.

A-431: A-404 in A-405 synthesis was replaced with A-428,

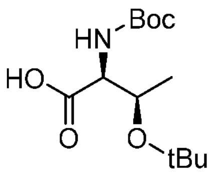

was replaced with

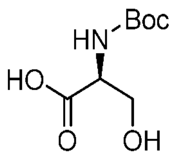

,

and the rest operations were the same as those in A-405 synthesis. M+1: 358.19.

Examples of synthesis of A-433, A-434 and A-435

A-433: A-404 in A-405 synthesis was replaced with A-432, and the rest operations were the same as those in A-405 synthesis. M+1: 375.15.

A-434: A-404 in A-405 synthesis was replaced with A-432,

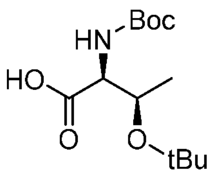

was replaced with

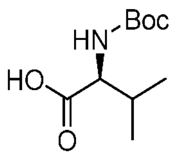

,

and the rest operations were the same as those in A-405 synthesis. M+1: 373.17.

A-435: A-404 in A-405 synthesis was replaced with A-432,

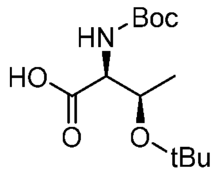

was replaced with

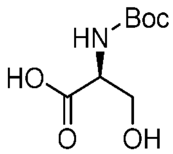,

and the rest operations were the same as those in A-405 synthesis. M+1: 361.13.

Examples of synthesis of A-437, A-438 and A-439

A-437: A-404 in A-405 synthesis was replaced with A-436, and the rest operations were the same as those in A-405 synthesis. M+1: 371.21.

A-438: A-404 in A-405 synthesis was replaced with A-436,

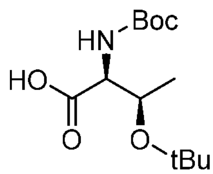

was replaced with

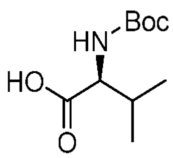,

and the rest operations were the same as those in A-405 synthesis. M+1: 369.23.

A-439: A-404 in A-405 synthesis was replaced with A-436,

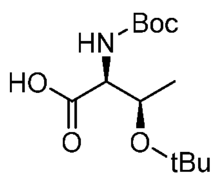

was replaced with

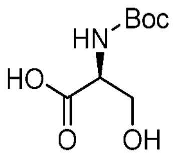,

and the rest operations were the same as those in A-405 synthesis. M+1: 357.19.

Examples of synthesis of A-440, A-444 and A-448

A-440: 3a in A-404 synthesis was replaced with 3c,

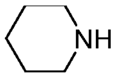

was replaced with

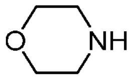,

and the rest operations were the same as those of A-404. M+1: 272.14.
A-444: 3a in A-404 synthesis was replaced with 3c,

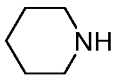

was replaced with

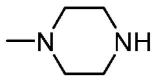,

and the rest operations were the same as those of A-404. M+1: 285.17.
A-448: 3a in A-404 synthesis was replaced with 3c,

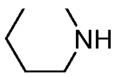

was replaced with

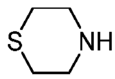,

and the rest operations were the same as those of A-404. M+1: 288.12.

Examples of synthesis of A-441, A-442 and A-443

A-441: A-404 in A-405 synthesis was replaced with A-440, and the rest operations were the same as those in A-405 synthesis. M+1: 373.19.

A-442: A-404 in A-405 synthesis was replaced with A-440,

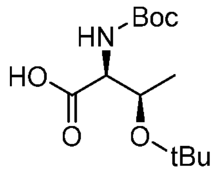

was replaced with

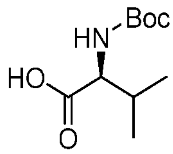,

and the rest operations were the same as those in A-405 synthesis. M+1: 371.21.

A-443: A-404 in A-405 synthesis was replaced with A-440,

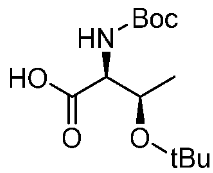

was replaced with

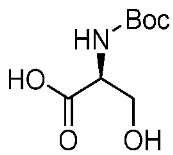,

and the rest operations were the same as those in A-405 synthesis. M+1: 359.17.

Examples of synthesis of A-445, A-446 and A-447

A-445: A-404 in A-405 synthesis was replaced with A-444, and the rest operations were the same as those in A-405 synthesis. M+1: 386.22.

A-446: A-404 in A-405 synthesis was replaced with A-444,

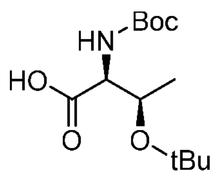

was replaced with

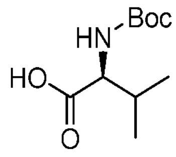,

and the rest operations were the same as those in A-405 synthesis. M+1: 384.24.

A-447: A-404 in A-405 synthesis was replaced with A-444,

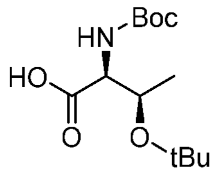

was replaced with

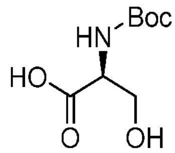,

and the rest operations were the same as those in A-405 synthesis. M+1: 372.20.

Examples of synthesis of A-449, A-450 and A-451

A-449: A-404 in A-405 synthesis was replaced with A-448, and the rest operations were the same as those in A-405 synthesis. M+1: 389.16.

A-450: A-404 in A-405 synthesis was replaced with A-448,

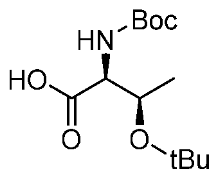

was replaced with

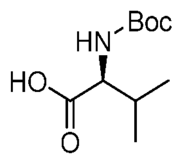,

and the rest operations were the same as those in A-405 synthesis. M+1: 387.18.

A-451: A-404 in A-405 synthesis was replaced with A-448,

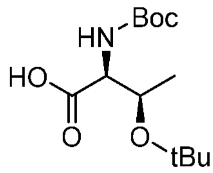

was replaced with

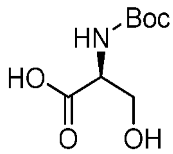,

and the rest operations were the same as those in A-405 synthesis. M+1: 375.15.

Examples of synthesis of A-456, A-460 and A-464

A-456: 3a in A-404 synthesis was replaced with 3d,

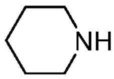

was replaced with

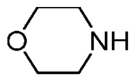,

, and the rest operations were the same as those of A-404. M+1: 272.14.
A-460: 3a in A-404 synthesis was replaced with 3d,

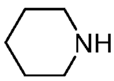

was replaced with

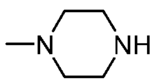,

and the rest operations were the same as those of A-404. M+1: 285.17.
A-464: 3a in A-404 synthesis was replaced with 3d,

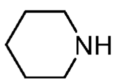

was replaced with

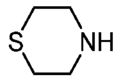,

and the rest operations were the same as those of A-404. M+1: 288.12.

Examples of synthesis of A-453, A-454 and A-455

A-453: A-404 in A-405 synthesis was replaced with A-452, and the rest operations were the same as those in A-405 synthesis. M+1: 371.21.

A-454: A-404 in A-405 synthesis was replaced with A-452,

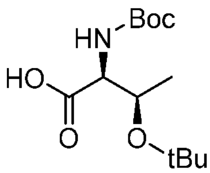

was replaced with

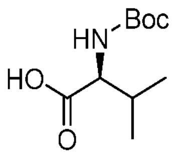,

and the rest operations were the same as those in A-405 synthesis. M+1: 369.23.

A-455: A-404 in A-405 synthesis was replaced with A-452,

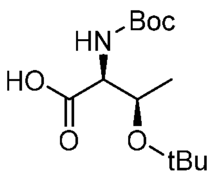

was replaced with

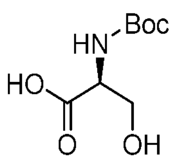,

and the rest operations were the same as those in A-405 synthesis. M+1: 357.19.

Examples of synthesis of A-457, A-458 and A-459

A-457: A-404 in A-405 synthesis was replaced with A-456, and the rest operations were the same as those in A-405 synthesis. M+1: 373.19.

A-458: A-404 in A-405 synthesis was replaced with A-456,

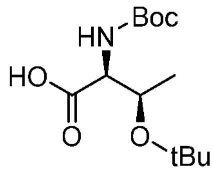

was replaced with

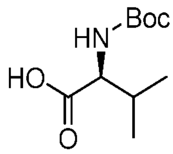,

and the rest operations were the same as those in A-405 synthesis. M+1: 371.21.

A-459: A-404 in A-405 synthesis was replaced with A-456,

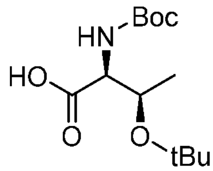

was replaced with

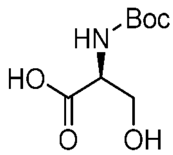,

and the rest operations were the same as those in A-405 synthesis. M+1: 359.17.

Examples of synthesis of A-461, A-462 and A-463

A-461: A-404 in A-405 synthesis was replaced with A-460, and the rest operations were the same as those in A-405 synthesis. M+1: 386.22.

A-462: A-404 in A-405 synthesis was replaced with A-460,

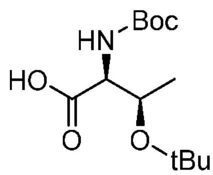

was replaced with

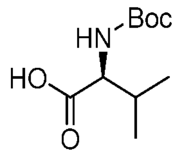

,

and the rest operations were the same as those in A-405 synthesis. M+1: 384.24.

A-463: A-404 in A-405 synthesis was replaced with A-460,

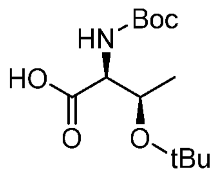

was replaced with

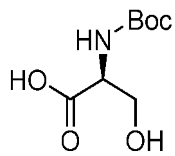

,

and the rest operations were the same as those in A-405 synthesis. M+1: 372.20.

Examples of synthesis of A-465, A-466 and A-467

A-465: A-404 in A-405 synthesis was replaced with A-464, and the rest operations were the same as those in A-405 synthesis. M+1: 389.16.

A-466: A-404 in A-405 synthesis was replaced with A-464,

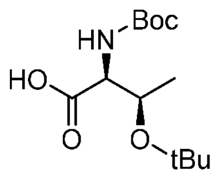

was replaced with

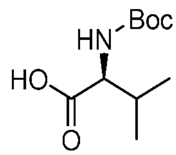

,

and the rest operations were the same as those in A-405 synthesis. M+1: 387.18.

A-467: A-404 in A-405 synthesis was replaced with A-464,

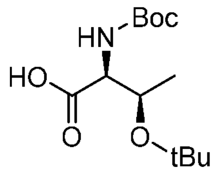

was replaced with

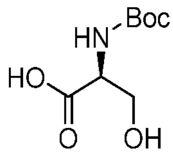,

and the rest operations were the same as those in A-405 synthesis. M+1: 375.15.

Example of synthesis of 468

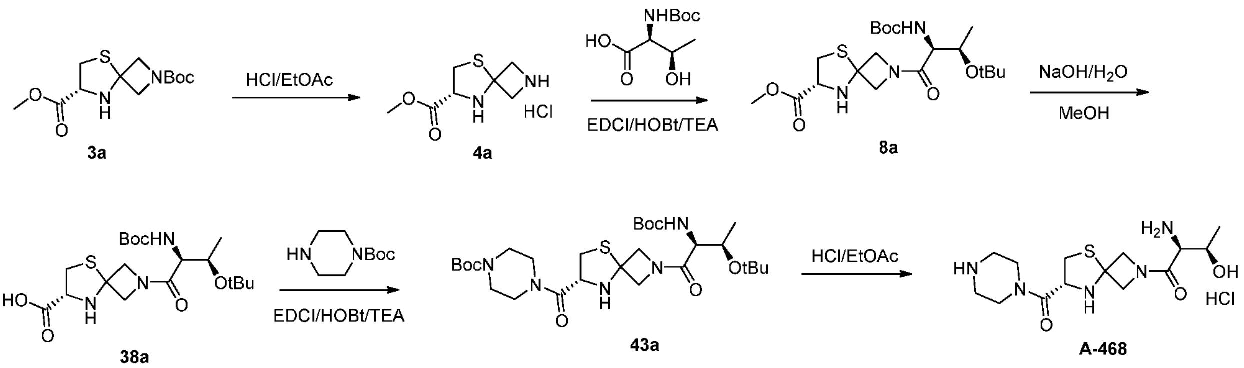


3a 4a 8a 38a 43a A-468

Synthesis of intermediate 43a:

Under ice bath conditions, the intermediate 38a (1.1g, 2.55 mmol) was added into 50ml of DCM, a solid was insoluble, and the solid was gradually dissolved after 2.5eq of TEA was added, then 1.1eq of N-Boc piperazine, 1.2eq of HOBt and 1.2eq of EDCI were added, and the raw materials were subjected to thermal reaction for 10 min and then reacted at room temperature overnight; after the completion of the raw materials reaction was monitored by TLC (DCM: MeOH = 5:1), a mixture was washed sequentially with water, saturated citric acid solution and saturated saline, dried with anhydrous sodium sulfate, and filtered, and then a filtrate was concentrated and separated by column chromatography to obtain 0.87 g of substance with a yield of 57.2%. $^1$H-NMR (400MHz, DMSO-$d_6$), δppm: 1.16(s,12H), 1.42(s,18H), 2.67-4.64(m,17H), 7.38 (s,2H). M+1: 600.34.

Synthesis of A-468:

The intermediate 43a (0.87 g, 1.45 mmol) was added to 10 ml of ethyl acetate, 10 ml of HCl-EtOAc solution was added, and the raw materials were stirred at room temperature for 6 h; when the completion of the raw materials reaction was monitored by TLC (PE: EA=1:2), a mixture was filtered, and a filter cake was washed with ethyl acetate, and dried to obtain 0.74 mg of a white solid with a yield of 95%. $^1$H-NMR (400MHz, DMSO-$d_6$), δppm: 1.16(s,3H), 2.81-4.38(m,17H), 5.37 (s,1H), 7.28 (s,4H). M+1: 344.17.

Examples of synthesis of A-469 and A-470

A-469:

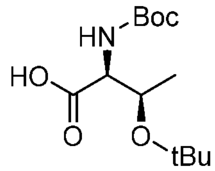

in A-468 synthesis was replaced with

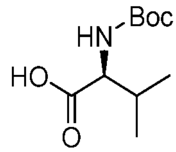,

and the rest operations were the same as those of A-468. M+1: 342.19.

A-470:

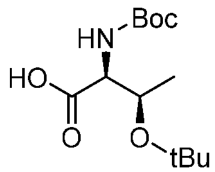

in A-468 synthesis was replaced with

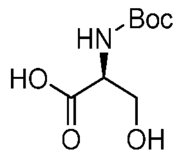,

and the rest operations were the same as those in A-468 synthesis. M+1: 330.16.

Examples of synthesis of A-471, A-472 and A-473

A-471: the intermediate 3a in A-468 synthesis was replaced with 3b, and the rest operations were the same as those of A-468. M+1: 358.19.

A-472: the intermediate 3a in A-468 synthesis was replaced with 3b,

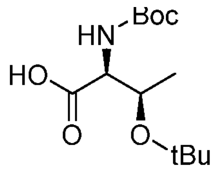

was replaced with

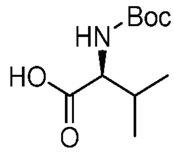
,

and the rest operations were the same as those of A-468. M+1: 356.21.

A-473: the intermediate 3a in A-468 synthesis was replaced with 3b,

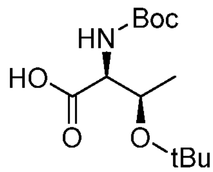

was replaced with

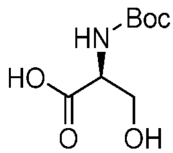
,

and the rest operations were the same as those of A-468. M+1: 344.17.

Examples of synthesis of A-474, A-475 and A-476

A-474: the intermediate 3a in A-468 synthesis was replaced with 3c, and the rest operations were the same as those of A-468. M+1: 372.20.

A-475: the intermediate 3a in A-468 synthesis was replaced with 3c,

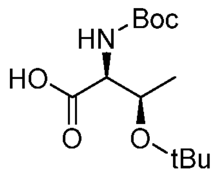

was replaced with

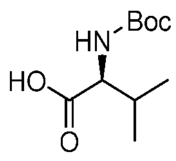

and the rest operations were the same as those of A-468. M+1: 370.22.

A-476: the intermediate 3a in A-468 synthesis was replaced with 3c,

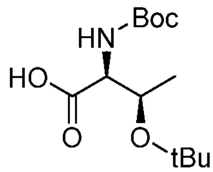

was replaced with

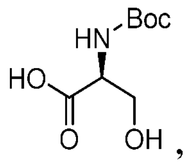

and the rest operations were the same as those of A-468. M+1: 358.19.

Examples of synthesis of A-477, A-478 and A-479

A-477: the intermediate 3a in A-468 synthesis was replaced with 3c, and the rest operations were the same as those of A-468. M+1: 372.1.

A-478: the intermediate 3a in A-468 synthesis was replaced with 3c,

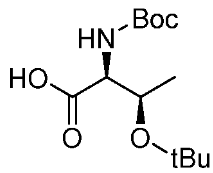

was replaced with

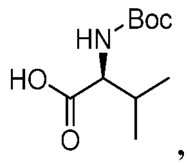

and the rest operations were the same as those of A-468. M+1: 370.2.

A-479: the intermediate 3a in A-468 synthesis was replaced with 3c,

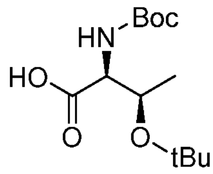

was replaced with

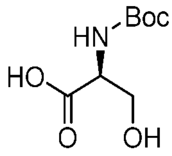,

and the rest operations were the same as those of A-468. M+1: 358.19.

Test example 1: In-vitro receptor binding assay

1. Experimental purpose

The affinity of the tested compounds on NMDA receptor was researched by receptor ligand binding assays.

2. Experimental method

Ten SD male rats of SPF grade were ordered and acclimated for 7 days for future use.

(1) Preparation of crude synaptosomes in prefrontal cortex and hippocampus

After the rats were decapitated, the prefrontal cortex and hippocampus were rapidly separated on ice and weighed, 10 volumes of 50 mM Tris-HCl buffer (50 mM Tris-HCl, 5 mM $MgCl_2 \cdot 6H_2O$, 1 mM EDTA, 0.5% (W/V) BSA, 1 mM PMSF, 0.32 M sucrose, pH 7.4) was added, and the mixture was homogenized for five times at 1500 revolutions/min with 30s each time. A homogenate was centrifuged for 10 min at 1000×g, and a supernatant was taken and centrifuged for 10 min at 40,000×g, the precipitates were collected, re-suspended with 10 volumes of Tris-HCl buffer, incubated at 37°C for 10 min, and centrifuged for 10 min at 40,000×g, finally the precipitates were re-suspended with the above buffer, aliquoted and stored at -80°C for later use.

(2) Detection of inhibitory function of tested medicines on binding of crude synaptosomes in rats to [$^3$H] -MK-801

50 μg of rat crude synaptosomal proteins was added to all tubes respectively. A volume of 50 μl of MK-801 (dizocilpine) was added to a non-specific binding tube to achieve a final concentration of 100 μM, and the materials were reacted for 15 min in advance. 20 μL of control drug at corresponding concentration was added to the test tube for reaction for 15 min. A volume of 30 μl of labeled ligand [$^3$H]-MK-801 was successively added to all tubes to achieve a final concentration of 10 nM. All reaction tubes were supplemented to a volume of 200 μl with 50 mM Tris-HCl buffer (50 mM Tris-HCl, 5 mM $MgCl_2 \cdot 6H_2O$, 1 mM EDTA, 0.5% (W/V) BSA, 0.1% NaNa, pH 7.4). A reaction was carried out at 37°C for 10 min. A type-49 glass fiber filter membrane was prepared and a sample was applied at the same time. The filter membrane was placed in a multi-head cell collector, and a reaction system was subjected to suction filtration under negative pressure and washed with an ice-cold 50 mM Tris-HCl buffer, 10 ml each time, for a total of 5 times. After suction drying, 1 ml of scintillation liquid was added to the filter membrane, the filter membrane was placed on a shaker for shaking for 1.5 h and then placed in a liquid scintillation counter on the next day to determine the radioactive intensity.

(3) Detection of influence of tested medicines on NMDA receptor agonistic properties in rat crude synaptosomal proteins

100 μg of rat crude synaptosomal proteins was added to all tubes respectively. 50 μl of 5,7-dichlorokynurenic acid was added to a non-specific binding tube to achieve a final concentration of 10 μM. All tubes were preloaded with 50 μM of glutamic acid, and the materials were reacted for 15 min in advance. 20 μL of control drug at corresponding

concentration was added to the test tube, and then reacted for 15 min. 1 mM glycine was added to the maximum reaction tube. A volume of 30 μl of labeled ligand [$^3$H]-MK-801 was successively added to all tubes to achieve a final concentration of 10 nM, and reacted for 15 min. All reaction tubes were supplemented to a volume of 500 μl with 50 mM Tris-HCl buffer (50 mM Tris-HCl, 5 mM $MgCl_2 \cdot 6H_2O$, 1 mM EDTA, 0.5% (W/V) BSA, 0.1% $NaN_3$, pH 7.4). A reaction was carried out at 37°C for 15 min. A type-49 glass fiber filter membrane was prepared and a sample was applied at the same time. The filter membrane was placed in a multi-head cell collector, and the reaction system was subjected to suction filtration under negative pressure and washed with an ice-cold 50 mM Tris-HCl buffer, 10 ml each time, for a total of 5 times. After suction drying, 1 ml of scintillation liquid was added to the filter membrane, the filter membrane was placed on the shaker for shaking for 1.5 h and then placed in the liquid scintillation counter on the next day to determine the radioactive intensity.

(4) Data processing and statistical analysis

GraphPad5.0 software was used to analyze the data, and the percentage of competitive inhibition was calculated through nonlinear fitting. wherein:

**Percentage of competitive inhibition% = ((total binding tube cpm - compound cpm)/(total binding tube cpm - non-specific binding tube cpm))×100%;**

Specific binding amount=total binding amount - non-specific binding amount, wherein each binding site was determined by double compound tubes.

**In an agonistic experiment, maximum [$^3$H] -MK-801 binding%= ((tested compounds cpm − 5,7 dichlorokynurenic acid cpm)/(1 mM glycine cpm − 5,7 dichlorokynurenic acid cpm))×100%.**

Table 2 Affinity and maximum agonistic efficacy of compounds of examples on NMDA receptor

| Number | $IC_{50}$ (nM) | Maximum agonistic efficacy (%) | Number | $IC_{50}$ (nM) | Maximum agonistic efficacy (%) |
|---|---|---|---|---|---|
| A-002 | 0.33 | 45 | A-006 | 0.44 | 32 |
| A-007 | 0.89 | 56 | A-008 | 0.89 | 35 |
| A-016 | 0.35 | 56 | A-020 | 0.25 | 42 |
| A-030 | 0.35 | 39 | A-031 | 0.35 | 52 |
| A-034 | 0.89 | 25 | A-035 | 0.88 | 19 |
| A-036 | 0.89 | 28 | A-044 | 0.66 | 28 |
| A-048 | 0.55 | 38 | A-059 | 0.75 | 33 |
| A-066 | 0.35 | 29 | A-068 | 1.30 | 48 |
| A-074 | 0.89 | 38 | A-078 | 1.59 | 56 |
| A-079 | 1.25 | 41 | A-082 | 0.89 | 45 |
| A-090 | 0.45 | 36 | A-093 | 0.48 | 65 |
| A-098 | 0.98 | 28 | A-111 | 1.58 | 38 |
| A-114 | 0.89 | 35 | A-119 | 1.89 | 35 |
| A-122 | 1.65 | 38 | A-130 | 0.48 | 43 |
| A-162 | 0.45 | 38 | A-195 | 0.43 | 46 |
| A-226 | 0.33 | 45 | A-234 | 1.58 | 75 |
| A-256 | 0.89 | 28 | A-258 | 0.33 | 21 |
| A-260 | 0.26 | 26 | A-266 | 3.2 | 45 |
| A-272 | 0.54 | 19 | A-274 | 0.28 | 55 |
| A-275 | 0.35 | 12 | A-276 | 0.25 | 28 |

(continued)

| Number | $IC_{50}$ (nM) | Maximum agonistic efficacy (%) | Number | $IC_{50}$ (nM) | Maximum agonistic efficacy (%) |
|---|---|---|---|---|---|
| A-282 | 0.32 | 35 | A-291 | 0.88 | 13 |
| A-303 | 0.24 | 35 | A-329 | 0.73 | 26 |
| A-331 | 0.59 | 31 | A-333 | 0.41 | 19 |
| A-334 | 0.38 | 34 | A-335 | 0.48 | 42 |
| A-342 | 0.56 | 32 | A-353 | 0.48 | 53 |
| A-358 | 0.86 | 78 | A-367 | 0.29 | 39 |
| A-370 | 0.36 | 46 | A-378 | 1.66 | 49 |
| A-384 | 0.42 | 39 | A-390 | 0.39 | 55 |
| A-394 | 0.79 | 63 | A-401 | 0.44 | 75 |
| A-409 | 0.56 | 36 | A-421 | 0.38 | 45 |
| A-429 | 0.66 | 57 | A-437 | 0.33 | 42 |
| A-449 | 0.47 | 38 | A-463 | 0.52 | 59 |
| A-470 | 0.35 | 33 | A-476 | 0.96 | 56 |

The experimental results show that all the compounds of examples have NMDA receptor agonistic activity, and the maximum agonistic efficacy is between 12% and 78%, and the compounds belong to NMDA receptor partial agonists.

Test examaple 2: In-vivo metabolism study in rats

The pharmacokinetic parameters such as exposure of plasma concentrations of the compounds of examples were evaluated by oral/tail intravenous injection model tests in rats in order to evaluate the in-vivo metabolic stability in rats.

The male SD rats weighing 200 ± 20 g and being SPF grade were used in this experiment and purchased from SPF.

Preparation of intravenous solution: 1 mg of each tested medicine was weighed accurately, and a volume was metered to 5 mL with 0.9% sodium chloride solution to prepare 0.2 mg/mL. A dose volume was 1 mL for per 200 g of weight, and the dose was 1 mg for per 1kg of weight based on the concentration conversion.

Preparation of intragastric solution: 10 mg of each tested medicine was accurately weighed, and a volume was metered to 10 mL with 0.9% sodium chloride solution to prepare 1 mg/mL of the solution. A dose volume was 2 mL for per 200 g of weight, and the dose was 10 mg for per 1kg of weight based on the concentration conversion.

Rats were randomly divided into 8 groups, 6 rats were in each group, oral gavage administration and tail intravenous administration were performed according to the above doses. About 0.2 mL of orbital blood was collected before administration and at 2 min, 5 min, 15 min, 30 min, 1 h, 2 h, 3 h, 4 h and 6 h after administration, respectively, and centrifuged at 3000 rpm for 10 min at 4°C, and a supernatant was taken for LC/MS/MS determination. A DAS pharmacokinetics program was used to analyze the measured data and calculate the main pharmacokinetic parameters. The experimental results are shown in the following table.

Table 3 Pharmacokinetic parameters of compounds of examples

| Example | Mode of administration | $C_{max}$ (ng/mL) | $AUC_{(0 \rightarrow t)}$ (ng/mL*h) | F (%) |
|---|---|---|---|---|
| **A-006** | Oral | 18360 | 34957 | 21.2 |
| | Intravenous injection | 11633 | 16470 | - |
| **A-066** | Oral | 2083 | 7556 | 54.5 |
| | Intravenous injection | 1692 | 1385 | - |
| **A-082** | Oral | 5580 | 13431 | 19.6 |
| | Intravenous injection | 18267 | 6852 | - |
| **A-098** | Oral | 767 | 1822 | 19.1 |
| | Intravenous injection | 1094 | 952 | - |
| **A-258** | Oral | 4907 | 11400 | 27.3 |
| | Intravenous injection | 3630 | 4178 | - |

(continued)

| Example | Mode of administration | $C_{max}$ (ng/mL) | $AUC_{(0 \rightarrow t)}$ (ng/mL*h) | F (%) |
|---|---|---|---|---|
| **A-260** | Oral | 6212 | 10849 | 74.6 |
| | Intravenous injection | 2240 | 1454 | - |
| **A-274** | Oral | 1883 | 3354 | 62.7 |
| | Intravenous injection | 2477 | 1628 | - |

The results shows that the compounds of examples have good pharmacokinetic properties after oral/injection in rats.

Test examaple 3: Animal pharmacodynamic test

Pharmacodynamic evaluation was performed by the forced swimming tests in rats. The experimental animals were male SD rats weighing 150 g-180 g and being SPF grade, and acclimated for one week after purchase, and then subjected to forced swimming tests, the rats need be fasted for 12 hours before the tests. The compounds of examples were dissolved with normal saline, and blank control group (normal saline) and example compound administration group were established. 8-10 rats were provided for each group.

On the day before the tests, the rats were placed in a glass cylinder with a height of 40 cm, an inner diameter of 18 cm, and a water depth of 23 cm for pre-swimming for 15 min, and a water temperature was 28°C. After the pre-swimming the rats were removed, wiped with a dry cloth, and dried with an electrical heater, then returned to a rearing cage, and administered. A formal forced swimming test was performed on the next day for a total of 5 min. The accumulated immobility time within 5 min was recorded, and a second forced swimming test was performed on the seventh day; the criteria for determining immobility were that the rats stopped struggling in the water, were in a floating state, and had only slight limb movements to keep the head floating on the water surface. The results are shown in Table 4, and the experimental data were statistically analyzed by GraphPad Prism 5.0 software.

Table 4 Forced swimming test results of compounds of examples in rats

| Group | Mode of administration | Dose (mg/kg) | Reduction percentage (%) of floating time | |
|---|---|---|---|---|
| | | | Dav 2 | Dav 7 |
| **A-006** | Po | 0.1 | 36.4%* | 22.2% |
| **A-007** | Po | 0.1 | 35.1%* | 32.4% |
| **A-008** | Po | 0.1 | 54.9%** | 57.3%* |
| **A-020** | Po | 0.1 | 51.8%* | 59.3%* |
| **A-034** | Po | 0.1 | 71.9%** | 60.1* |
| **A-066** | Po | 0.1 | 68%** | 55.3%* |
| **A-098** | Po | 0.1 | 64.5%** | 13.9% |
| **A-258** | Po | 0.1 | 74.8%** | 56.4%* |
| **A-260** | Po | 0.1 | 31.6%* | 15.5% |
| Note: n=8-10, *P < 0.05**P < 0.01***P < 0.001 compared with the control group | | | | |

The experimental results show that intragastric administration with the compounds of examples is effective and the efficacy can be maintained after 2 to 7 days.

Although the embodiments of the invention have been described in detail, the technicians in the field will understand that various modifications and replacements may be made to those details according to all the instructions already disclosed, and these changes are within the scope of protection of the present invention. The full scope of the invention is given by the appended claims and any equivalents thereto.

## Claims

**1.** A compound represented by a formula I:

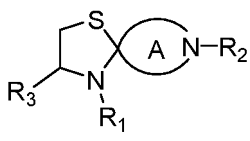

I

or a pharmaceutically acceptable salt or ester, stereoisomer, or solvate thereof, wherein, Ring A is 3- to 8-membered aliphatic heterocycle;
$R_1$ is selected from H, C1-C6 alkyl, aryl-C1-C4 alkyl, C2-C6 acyl, -CONRR' and natural amino acid fragments;
$R_2$ is selected from H, C1-C6 alkyl, C1-C6 alkoxycarbonyl, C2-C6 acyl, -CONRR' and natural amino acid fragments;
$R_3$ is selected from H, cyano, 3- to 8-membered nitrogen-containing aliphatic heterocycle- C1-C4 acyl, C1-C6 alkoxycarbonyl and $-CONHR_4$;
R and R' are each independently selected from H and C1-C6 alkyl;
$R_4$ is selected from H, C1-C6 alkyl, natural amino acid fragments and carboxylic acid derivatives of the said natural amino acid fragments;
optionally, the C1-C6 alkyl, aryl-C1-C4 alkyl, C2-C6 acyl, 3- to 8-membered nitrogen-containing aliphatic heterocycle- C1-C4 acyl, C1-C6 alkoxycarbonyl, -CONRR', $-CONHR_4$ and natural amino acid fragments are each independently substituted by one or more substituents selected from: halogen, amino, hydroxyl, cyano, carboxyl, nitro, C1-C6 alkyl and C1-C6 alkoxy;
and, $R_1$, $R_2$ and $R_3$ satisfy:

(1) $R_1$, $R_2$ and $R_3$ are not H at the same time;
(2) when $R_1$ and $R_3$ are H, $R_2$ is not Boc;
(3) when the formula I is

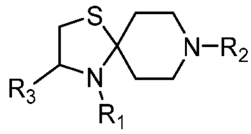,

$R_1$ is

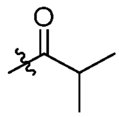,

and $R_3$ is H; $R_2$ is not H,

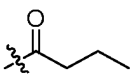, 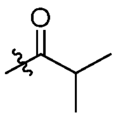, 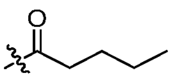, 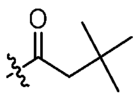, 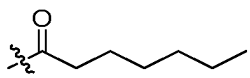, 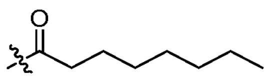,

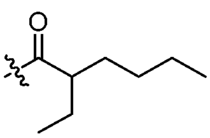, 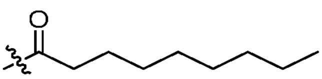, 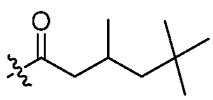, 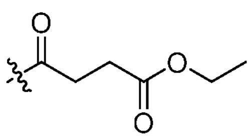, 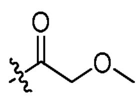,

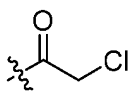, 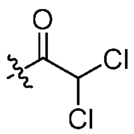, 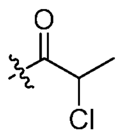, 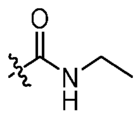, 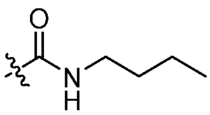, 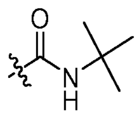 or 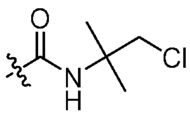;

(4) when the formula I is

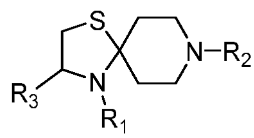,

$R_1$ is

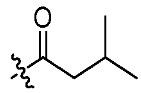,

and $R_3$ is H; $R_2$ is not C2-C6 acyl or -CONRR';
(5) when the formula I is

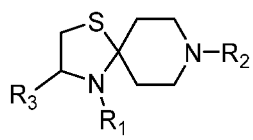,

$R_1$ and $R_3$ are H; $R_2$ is not C1-C4 alkyl, acetyl,

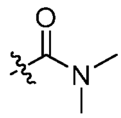

or

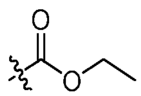;

(6) the compound of the formula I is not

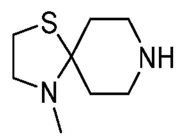, 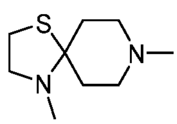, 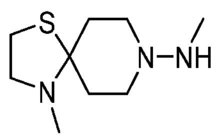,

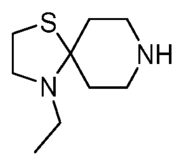, 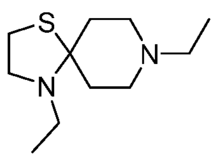, 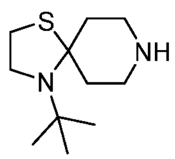, 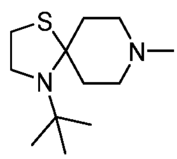, 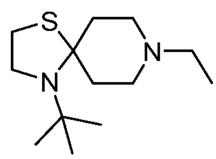,

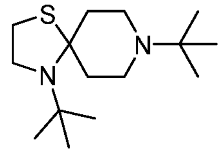

or

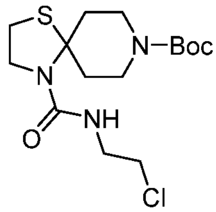

.

2. The compound according to claim 1, or a pharmaceutically acceptable salt or ester, stereoisomer, or solvate thereof, wherein Ring A is selected from 4- to 7-membered aliphatic heterocycle, preferably 4- to 6-membered aliphatic heterocycle;

preferably, Ring A is selected from 4- to 7-membered nitrogen-containing aliphatic heterocycle, more preferably 4- to 6-membered nitrogen-containing aliphatic heterocycle;
preferably, Ring A is selected from

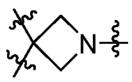

,

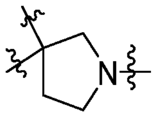

,

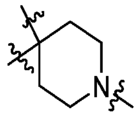

,

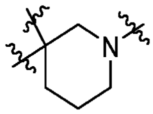

,

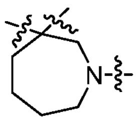

and

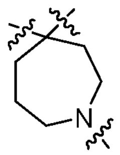

.

3. The compound according to claim 1 or 2, or a pharmaceutically acceptable salt or ester, stereoisomer, or solvate thereof, wherein, $R_1$ is selected from H, C1-C6 alkyl, phenyl-C1-C4 alkyl, C2-C6 acyl and natural amino acid fragments;

preferably, $R_1$ is selected from H, C1-C6 alkyl and C2-C6 acyl;
preferably, $R_1$ is H;
preferably, $R_1$ is selected from carboxyl residues of natural amino acids;
preferably, $R_1$ is selected from H, acetyl, methyl,

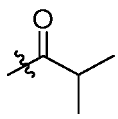

,

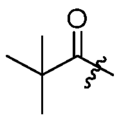

,

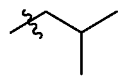

,

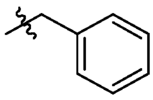

,

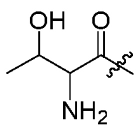

,

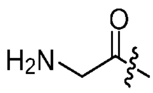

,

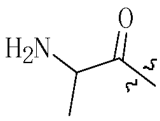

,

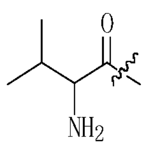

,

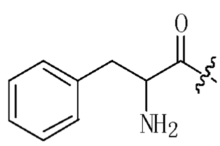

,

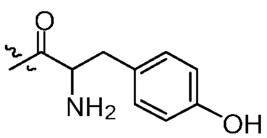

,

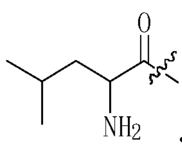

,

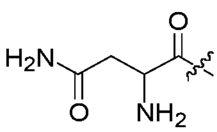

and

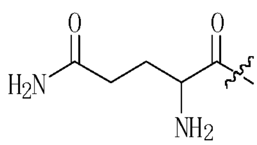

.

4. The compound according to any one of claims 1 to 3, or a pharmaceutically acceptable salt or ester, stereoisomer, or solvate thereof, wherein, $R_2$ is selected from H, C1-C6 alkyl, C1-C6 alkoxycarbonyl, C2-C6 acyl and natural amino acid fragments; optionally, the C1-C6 alkyl, phenyl-C1-C4 alkyl, C2-C6 acyl, and natural amino acid fragments are

each independently substituted by one or more substituents selected from: halogen, amino, hydroxyl, cyano, carboxyl, nitro, C1-C6 alkyl and C1-C6 alkoxy;

preferably, $R_2$ is selected from H, C1-C4 alkoxycarbonyl, C2-C6 acyl and carboxyl residues of the natural amino acids;
preferably, $R_2$ is selected from H, C1-C6 alkyl, C2-C6 acyl and carboxyl residues of natural amino acids;
preferably, $R_2$ is selected from H, C1-C6 alkyl and C2-C6 acyl;
preferably, $R_2$ is selected from carboxyl residues of natural amino acids;
preferably, $R_2$ is selected from H, methyl, ethyl, propyl, butyl, tert-butoxycarbonyl, acetyl,

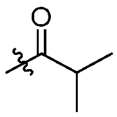 , 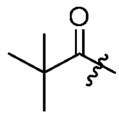 , 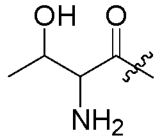 , 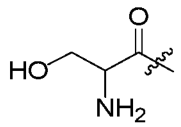 , 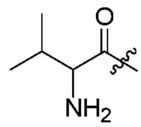 , 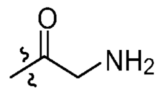 ,

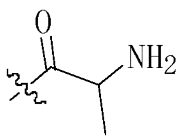 , 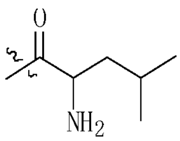 , 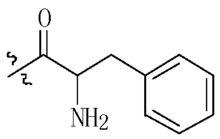 , 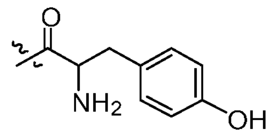 , 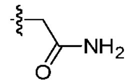 ,

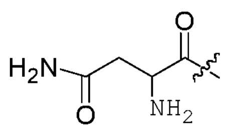 and 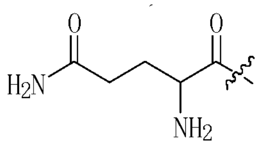 .

**5.** The compound according to any one of claims 1 to 4, or a pharmaceutically acceptable salt or ester, stereoisomer, or solvate thereof, wherein, $R_3$ is selected from H, cyano, 5- to 7-membered nitrogen-containing aliphatic heterocycle-C1-C3 acyl, C1-C4 alkoxycarbonyl and -$CONHR_4$; $R_4$ is selected from H, natural amino acid fragments, amide derivatives of the said natural amino acid fragments and cyano derivatives of the said natural amino acid fragments;

preferably, $R_3$ is selected from H, cyano, 5- to 7-membered nitrogen-containing aliphatic heterocycle- C1-C3 acyl, C1-C4 alkoxycarbonyl and -$CONHR_4$; $R_4$ is selected from H, natural amino acid fragments and amide derivatives of the said natural amino acid fragments;
preferably, $R_3$ is H;
preferably, $R_3$ is -$CONHR_4$; $R_4$ is selected from amino residues of natural amino acids, and amide derivatives of the said amino residue of natural amino acids;
preferably, $R_3$ is selected from H, cyano,

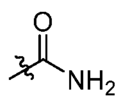 , 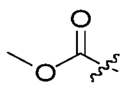 , 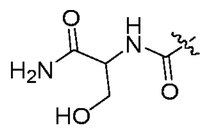 ,

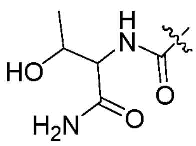 , 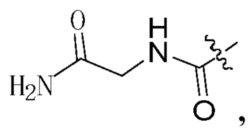 , 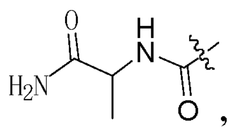 , 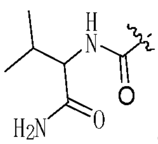 , 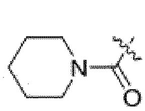 , 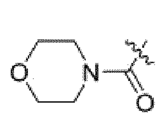 ,

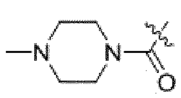 , 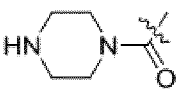 and 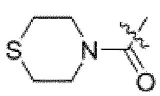 .

6. The compound according to any one of claims 1 to 5, or a pharmaceutically acceptable salt or ester, stereoisomer, or solvate thereof, wherein the said natural amino acid fragments are selected from the carboxyl or amino residues of the following amino acids: Thr, Ser, Val, Gly, Ala, Ile, Phe, Gln and Tyr;

preferably, the said natural amino acid fragments are selected from the carboxyl residues of the following amino acids: Thr, Ser, Val, Gly, Ala, Ile, Phe, Gln and Tyr;
preferably, the said natural amino acid fragments are selected from the amino residues of the following amino acids: Thr, Ser, Val, Gly, Ala, Ile, Phe, Gln and Tyr.

7. The compound according to any one of claims 1 to 6, or a pharmaceutically acceptable salt or ester, stereoisomer, or solvate thereof, wherein the compound is selected from:

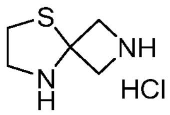
**A-001**

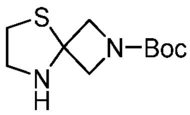
**A-002**

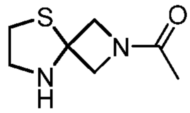
**A-003**

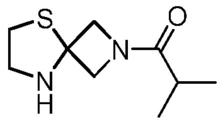
**A-004**

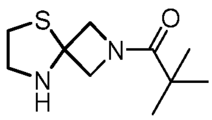
**A-005**

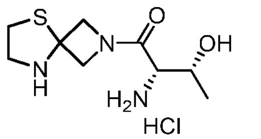
**A-006**

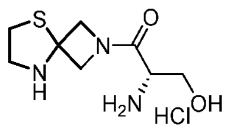
**A-007**

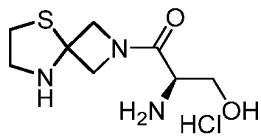
**A-007-1**

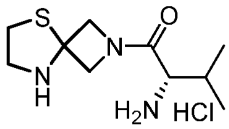
**A-008**

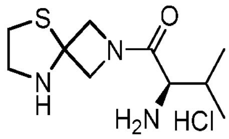
**A-008-1**

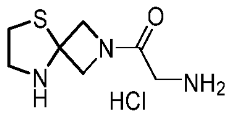
**A-009**

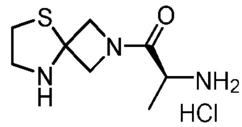
**A-010**

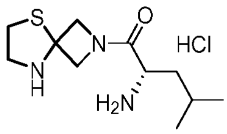
**A-011**

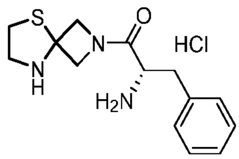
**A-012**

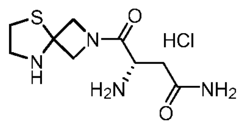
**A-013**

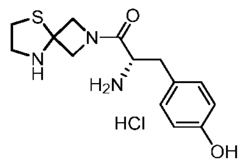
**A-014**

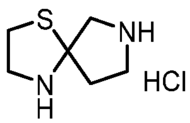
**A-015**

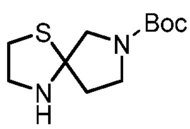
**A-016**

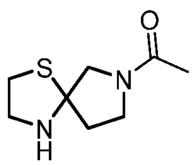
**A-017**

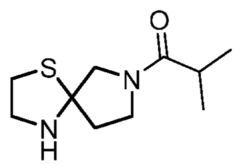
**A-018**

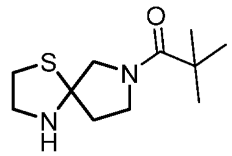

**A-019**

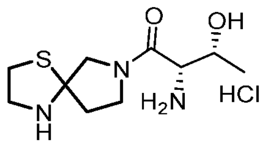

**A-020**

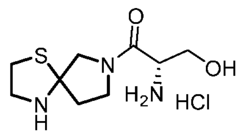

**A-021**

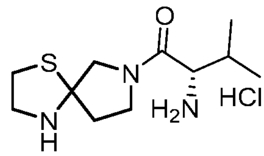

**A-022**

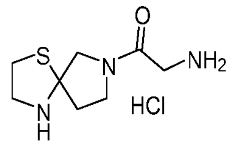

**A-023**

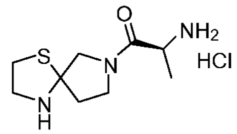

**A-024**

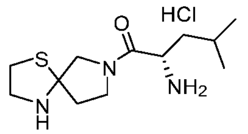

**A-025**

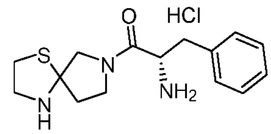

**A-026**

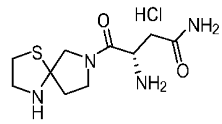

**A-027**

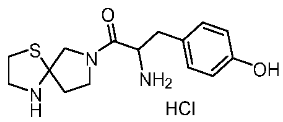

**A-028**

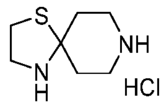

**A-029**

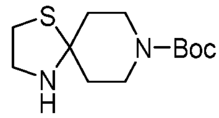

**A-030**

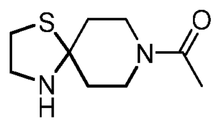

**A-031**

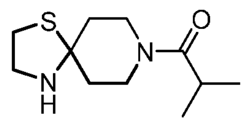

**A-032**

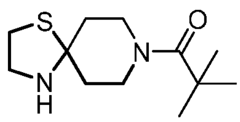

**A-033**

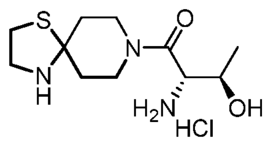

**A-034**

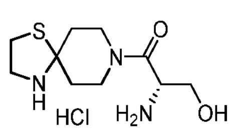

**A-035**

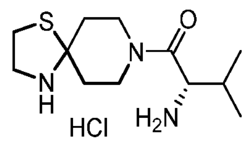

**A-036**

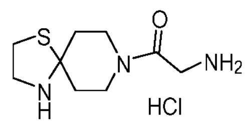

**A-037**

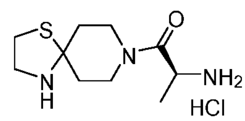

**A-038**

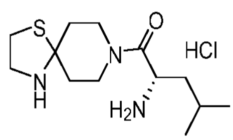

**A-039**

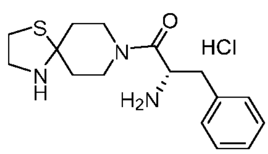

**A-040**

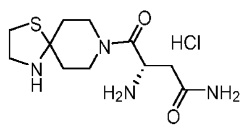

**A-041**

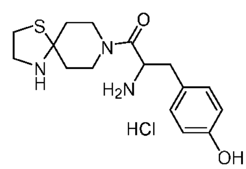

**A-042**

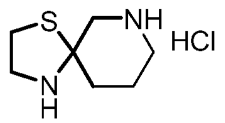

**A-043**

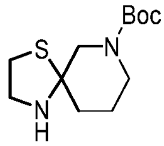

**A-044**

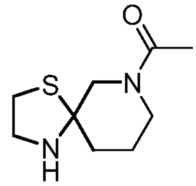

**A-045**

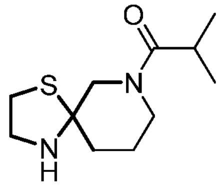

**A-046**

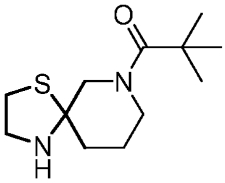

**A-047**

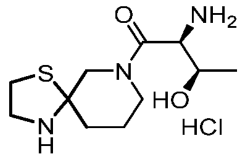

**A-048**

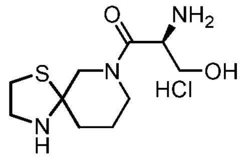

**A-049**

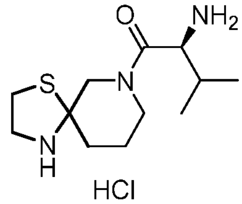

**A-050**

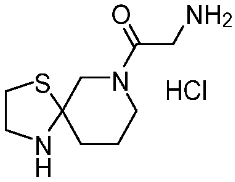

**A-051**

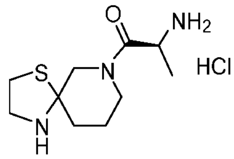

**A-052**

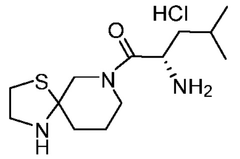

**A-053**

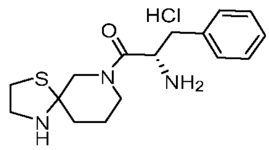

**A-054**

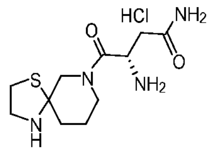

**A-055**

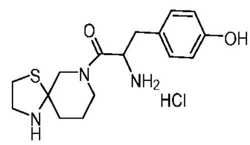

**A-056**

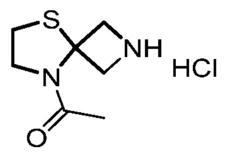

**A-057**

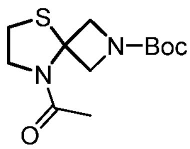

**A-058**

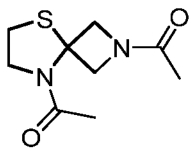

**A-059**

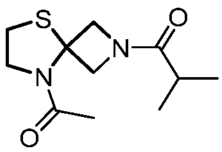

**A-060**

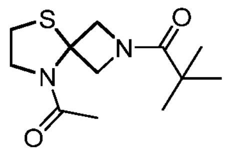

**A-061**

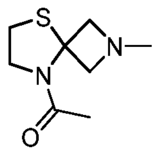

**A-062**

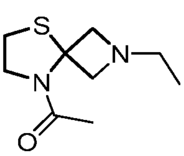

**A-063**

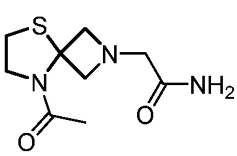

**A-064**

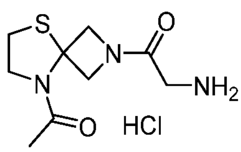

**A-065**

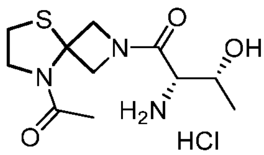

**A-066**

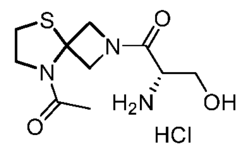

**A-067**

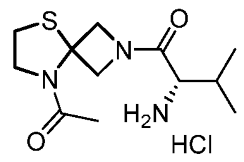

**A-068**

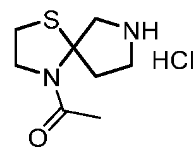

**A-069**

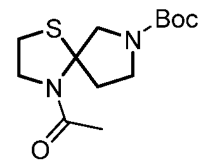

**A-070**

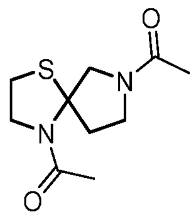

**A-071**

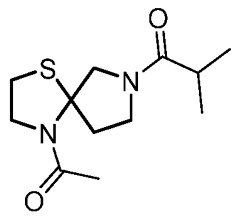

**A-072**

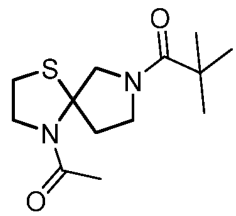

**A-073**

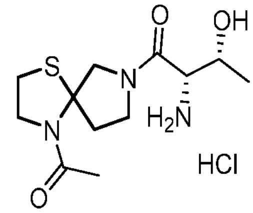

**A-074**

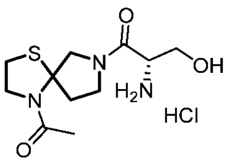

**A-075**

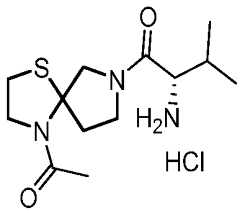

**A-076**

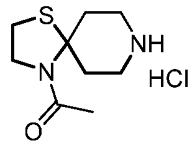

**A-077**

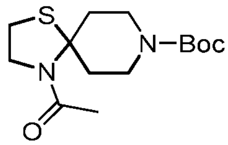

**A-078**

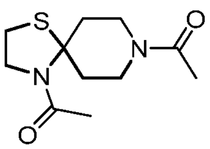

**A-079**

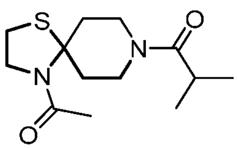

**A-080**

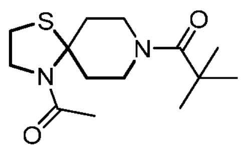

**A-081**

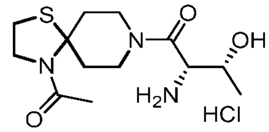

**A-082**

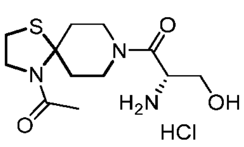

**A-083**

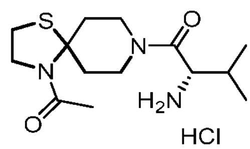

**A-084**

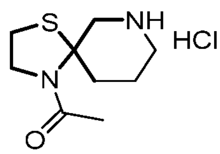

**A-085**

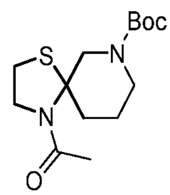

**A-086**

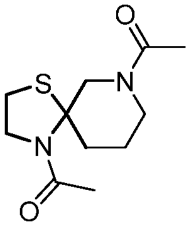

**A-087**

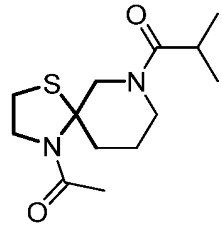

**A-088**

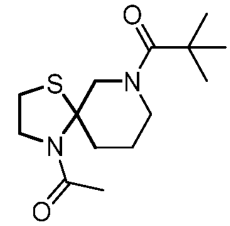

**A-089**

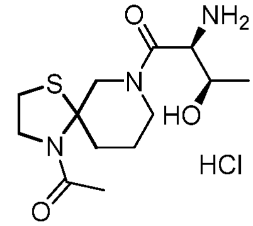

**A-090**

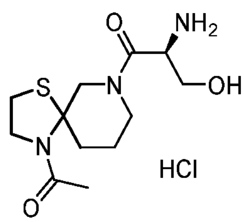

**A-091**

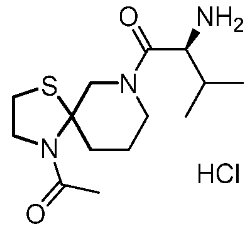

**A-092**

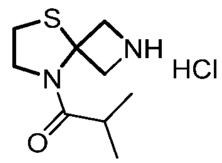

**A-093**

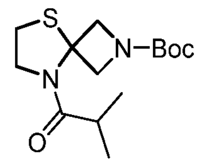

**A-094**

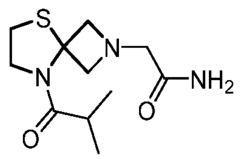

**A-095**

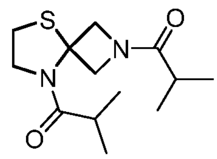

**A-096**

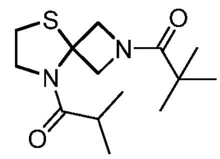

**A-097**

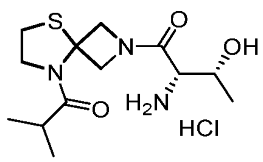

**A-098**

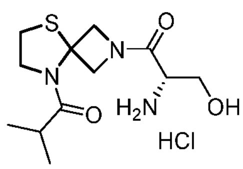

**A-099**

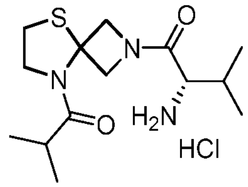

**A-100**

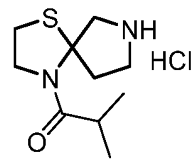

**A-101**

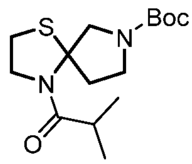

**A-102**

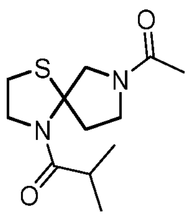

**A-103**

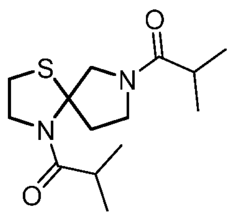

**A-104**

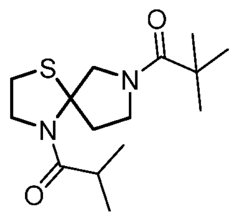

**A-105**

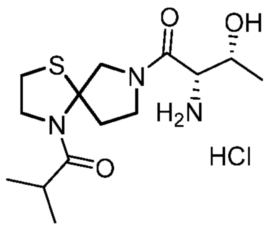

**A-106**

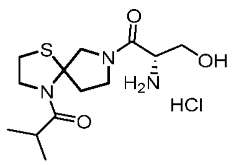

**A-107**

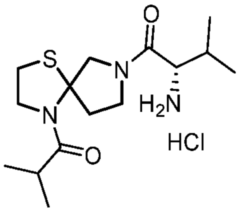

**A-108**

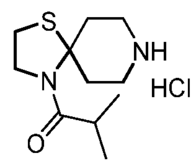

**A-109**

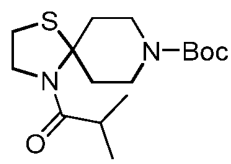

**A-110**

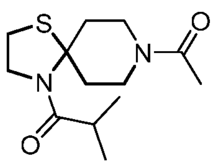

**A-111**

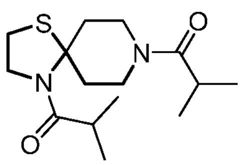

**A-112**

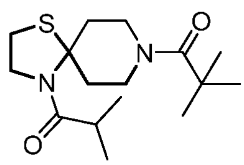

**A-113**

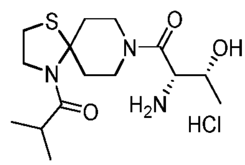

**A-114**

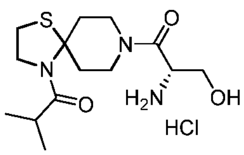

**A-115**

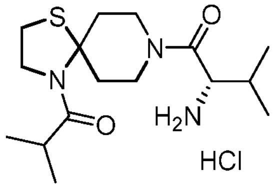

**A-116**

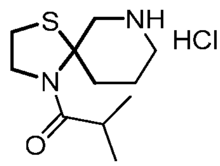

**A-117**

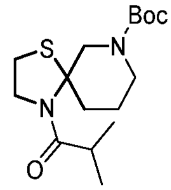

**A-118**

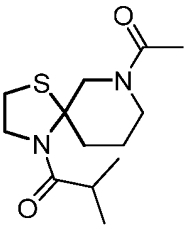

**A-119**

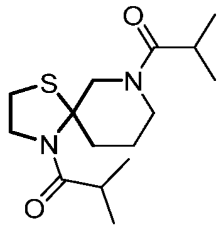

**A-120**

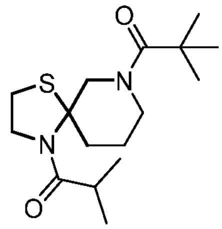

**A-121**

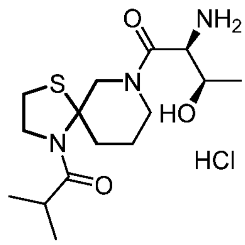

**A-122**

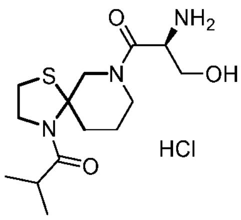

**A-123**

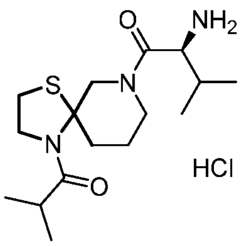

**A-124**

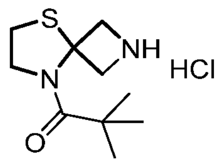

**A-125**

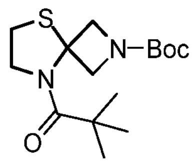

**A-126**

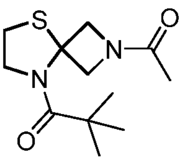

**A-127**

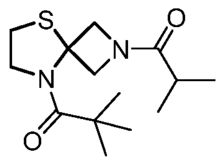

**A-128**

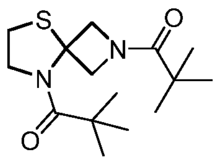

**A-129**

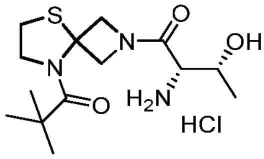

**A-130**

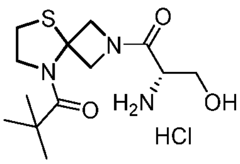

**A-131**

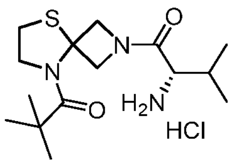

**A-132**

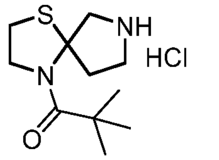

**A-133**

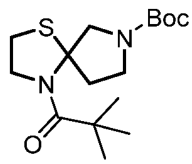

**A-134**

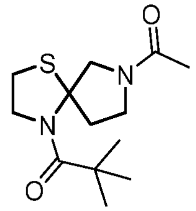

**A-135**

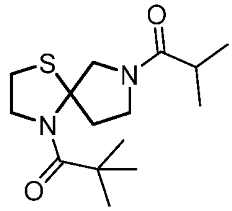

**A-136**

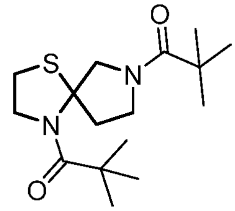

**A-137**

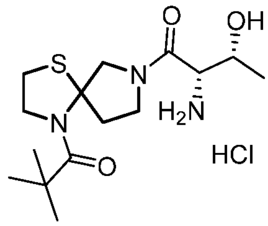

**A-138**

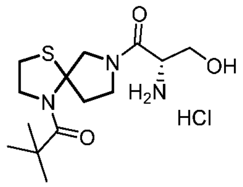

**A-139**

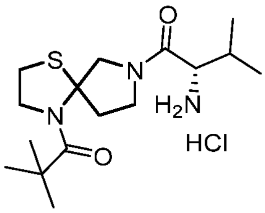

**A-140**

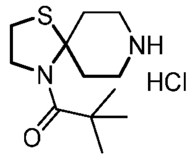

**A-141**

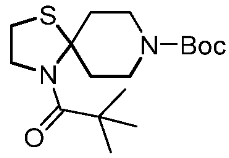

**A-142**

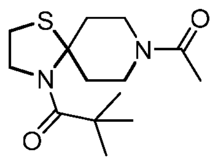

**A-143**

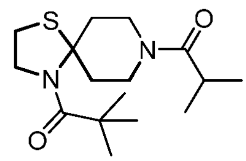

**A-144**

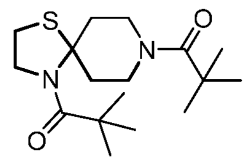

**A-145**

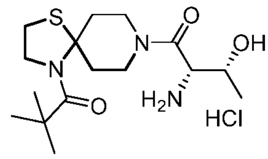

**A-146**

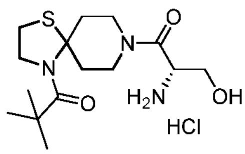

**A-147**

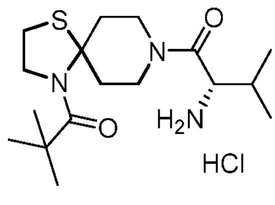

**A-148**

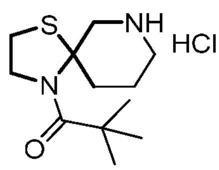

**A-149**

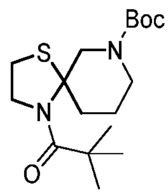

**A-150**

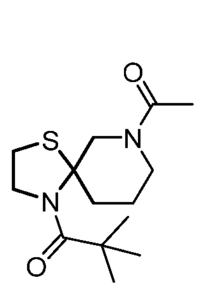

**A-151**

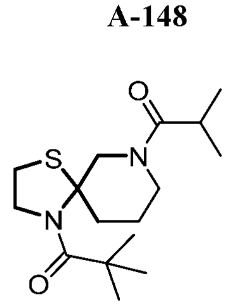

**A-152**

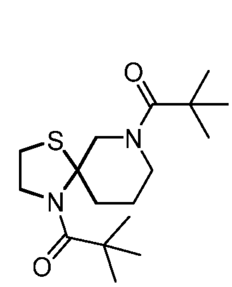

**A-153**

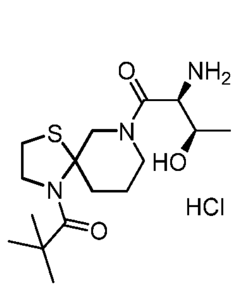

**A-154**

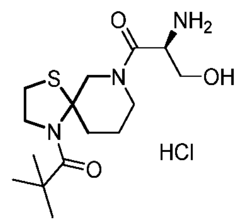
**A-155**

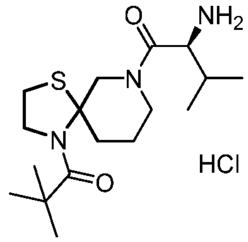
**A-156**

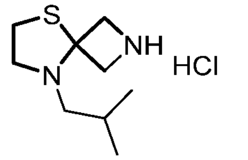
**A-157**

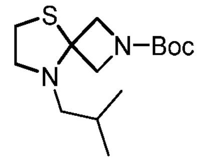
**A-158**

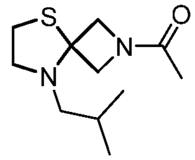
**A-159**

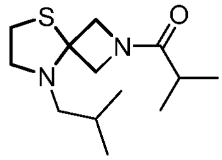
**A-160**

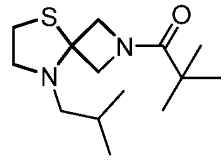
**A-161**

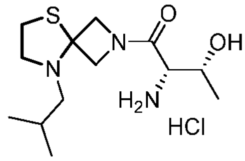
**A-162**

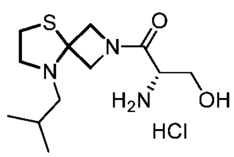
**A-163**

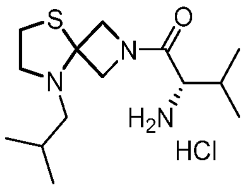
**A-164**

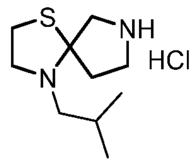
**A-165**

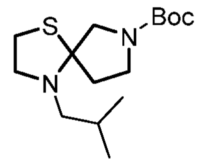
**A-166**

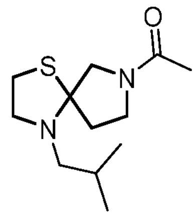
**A-167**

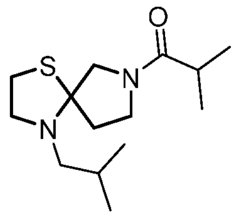
**A-168**

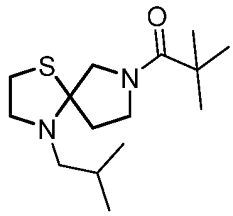
**A-169**

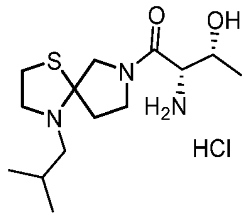
**A-170**

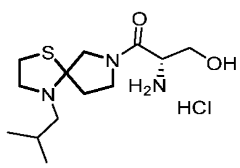
**A-171**

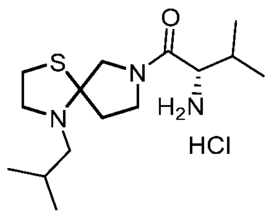
**A-172**

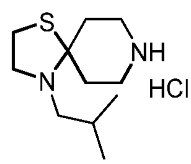
**A-173**

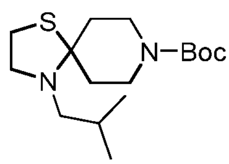
**A-174**

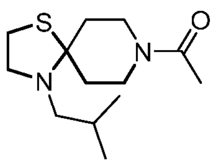
**A-175**

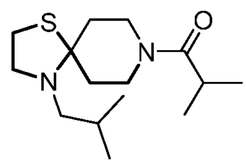
**A-176**

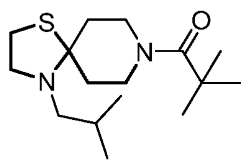
**A-177**

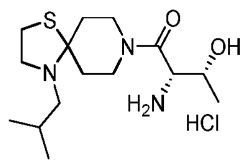
**A-178**

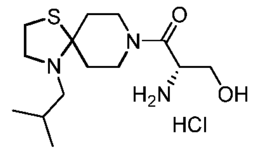

**A-179**

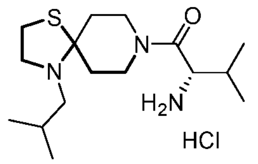

**A-180**

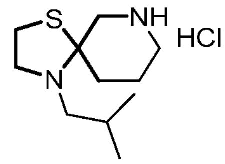

**A-181**

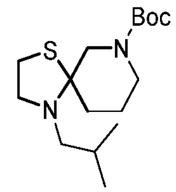

**A-182**

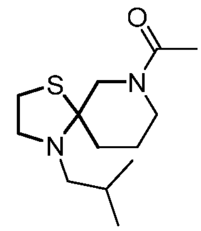

**A-183**

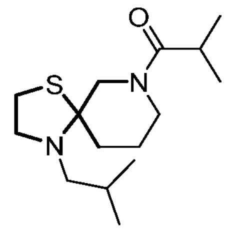

**A-184**

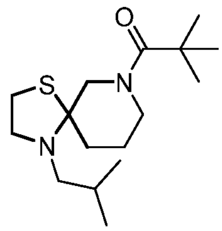

**A-185**

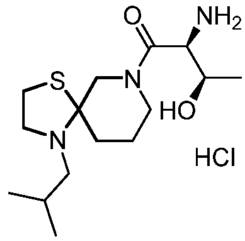

**A-186**

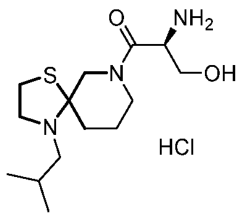

**A-187**

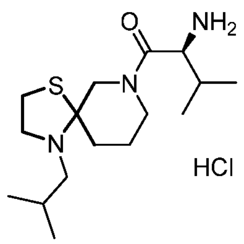

**A-188**

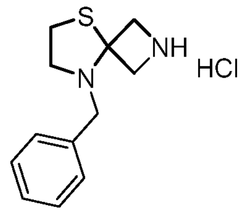

**A-189**

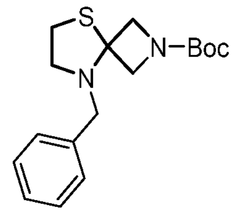

**A-190**

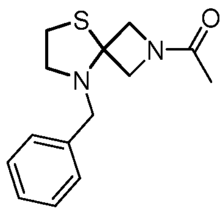

**A-191**

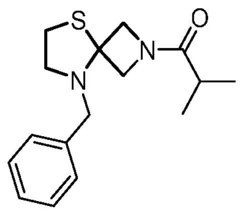

**A-192**

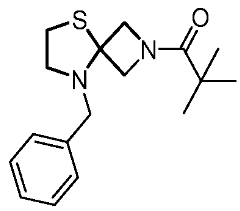

**A-193**

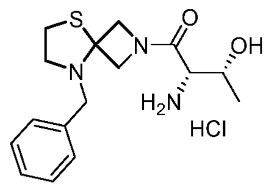

**A-194**

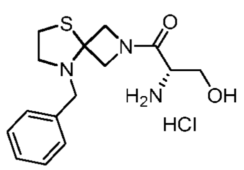

**A-195**

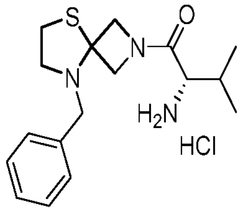

**A-196**

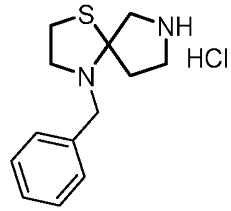

**A-197**

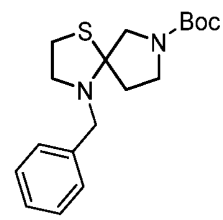

**A-198**

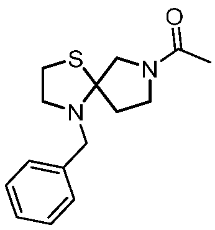

**A-199**

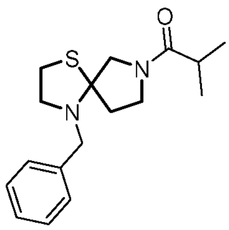

**A-200**

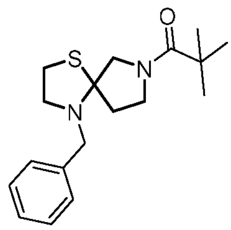

**A-201**

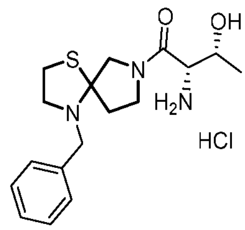

**A-202**

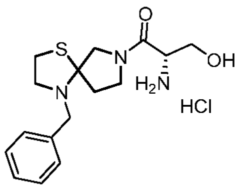

**A-203**

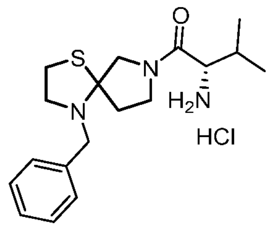

**A-204**

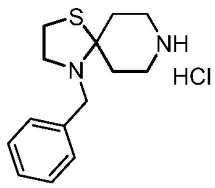

**A-205**

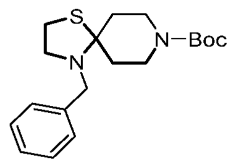

**A-206**

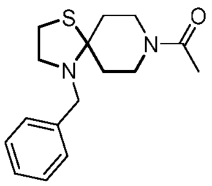

**A-207**

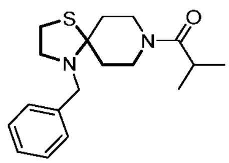

**A-208**

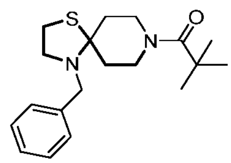

**A-209**

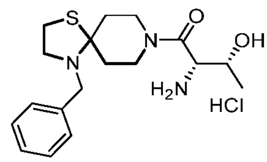

**A-210**

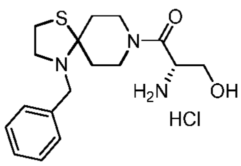

**A-211**

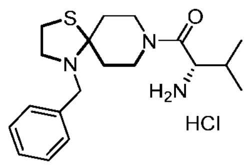

**A-212**

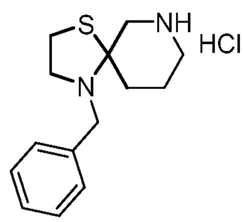

**A-213**

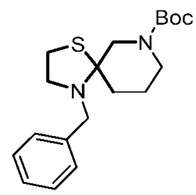

**A-214**

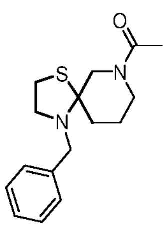

**A-215**

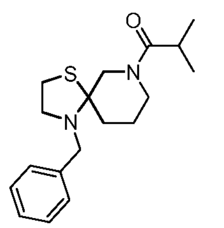

**A-216**

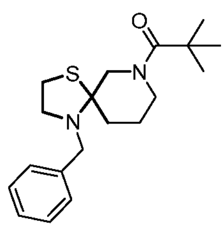

**A-217**

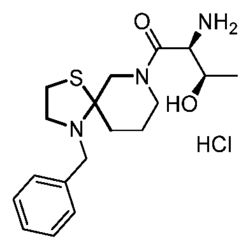

**A-218**

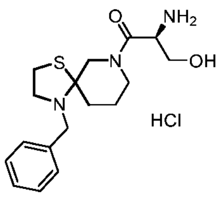

**A-219**

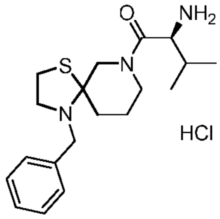

**A-220**

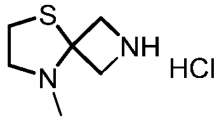

**A-221**

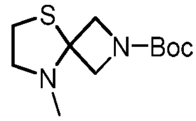

**A-222**

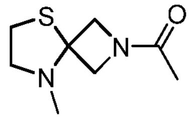

**A-223**

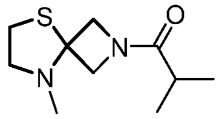

**A-224**

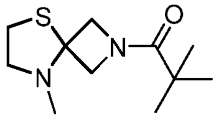

**A-225**

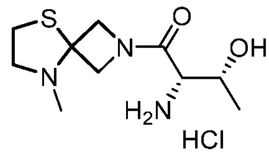

**A-226**

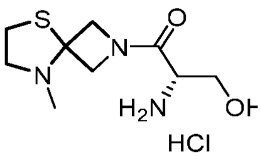

**A-227**

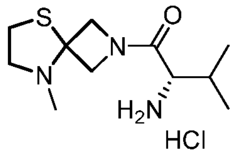

**A-228**

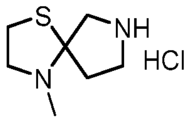

**A-229**

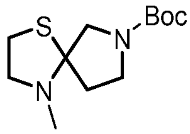

**A-230**

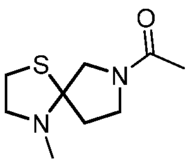

**A-231**

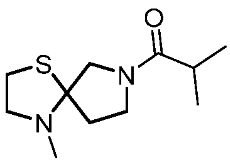

**A-232**

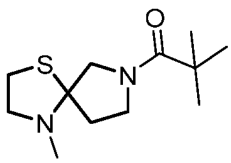

**A-233**

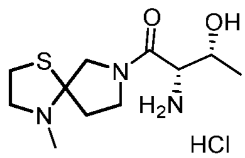

**A-234**

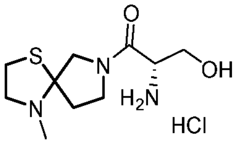

**A-235**

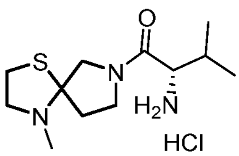

**A-236**

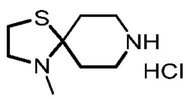

**A-237**

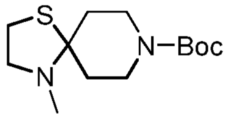

**A-238**

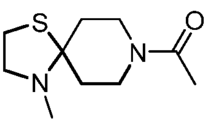

**A-239**

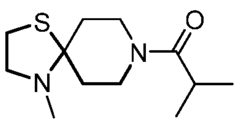

**A-240**

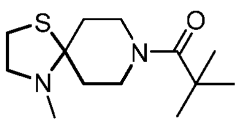

**A-241**

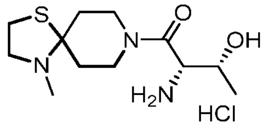

**A-242**

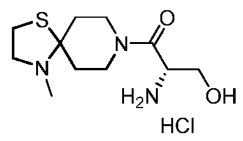

**A-243**

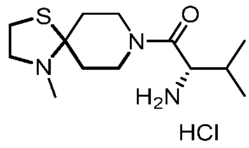

**A-244**

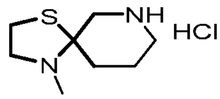

**A-245**

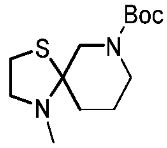

**A-246**

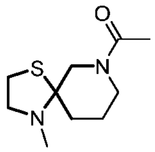

**A-247**

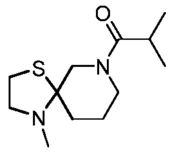

**A-248**

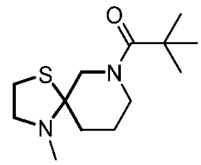

**A-249**

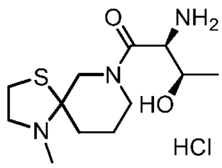

**A-250**

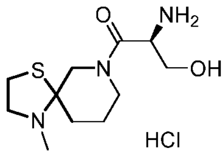

**A-251**

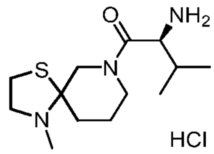

**A-252**

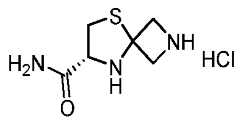

**A-253**

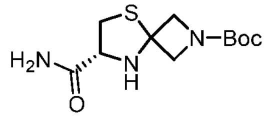

**A-254**

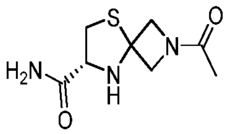

**A-255**

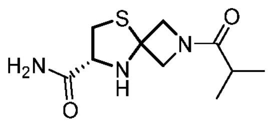

**A-256**

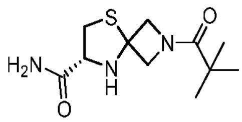

**A-257**

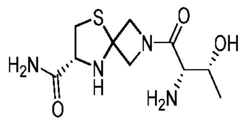

**A-258**

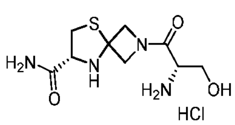

**A-259**

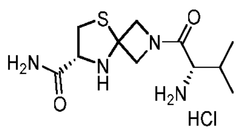

**A-260**

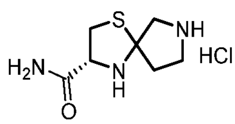

**A-261**

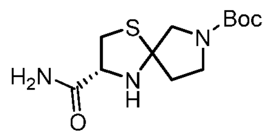

**A-262**

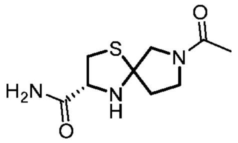

**A-263**

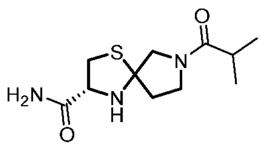

**A-264**

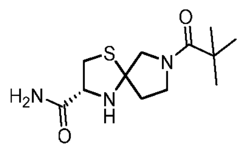

**A-265**

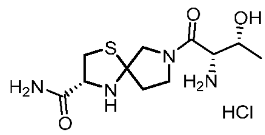

**A-266**

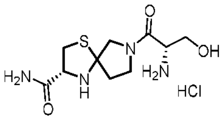

**A-267**

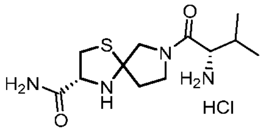

**A-268**

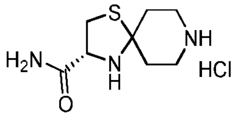

**A-269**

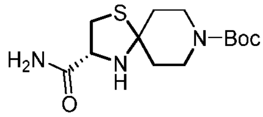

**A-270**

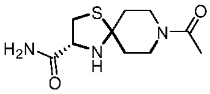

**A-271**

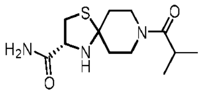

**A-272**

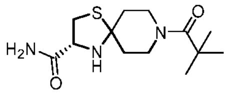

**A-273**

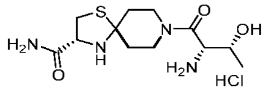

**A-274**

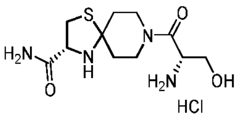

**A-275**

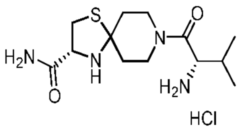

**A-276**

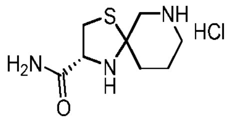

**A-277**

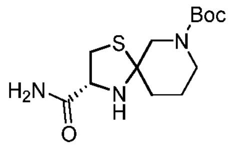

**A-278**

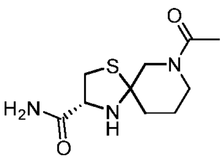

**A-279**

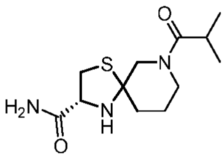

**A-280**

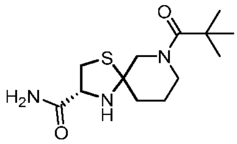

**A-281**

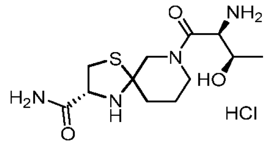

**A-282**

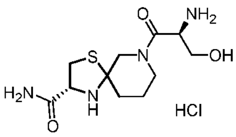

**A-283**

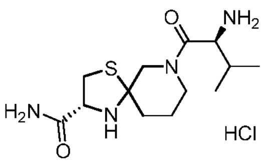

**A-284**

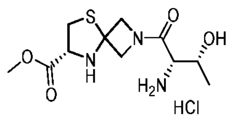

**A-285**

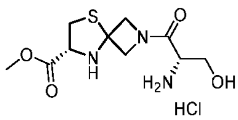

**A-286**

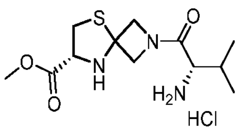

**A-287**

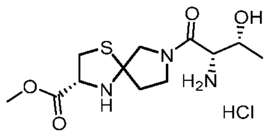

**A-288**

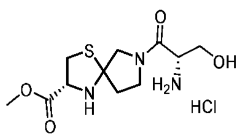

**A-289**

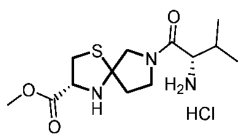

**A-290**

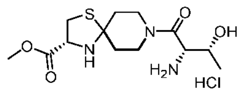
A-291

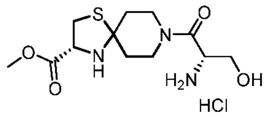
A-292

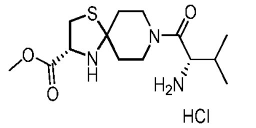
A-293

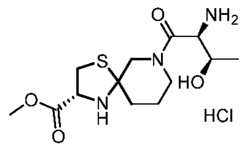
A-294

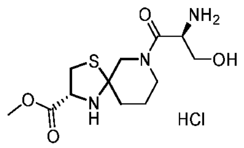
A-295

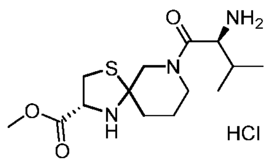
A-296

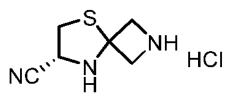
A-297

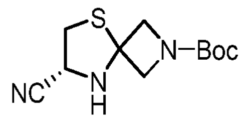
A-298

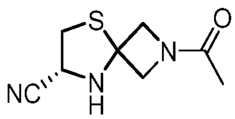
A-299

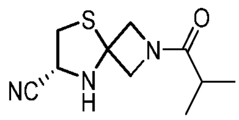
A-300

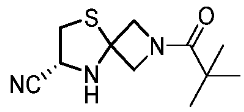
A-301

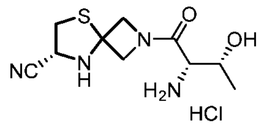
A-302

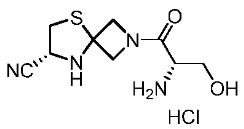
A-303

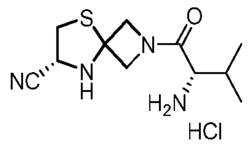
A-304

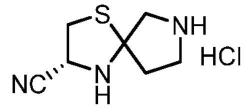
A-305

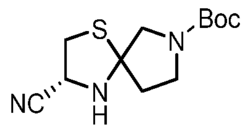
A-306

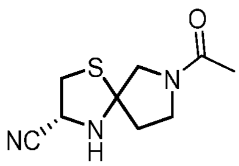
A-307

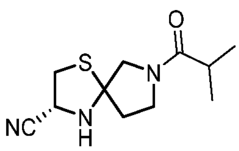
A-308

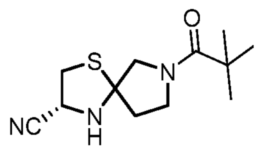
A-309

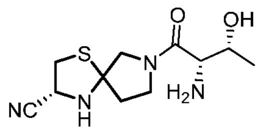
A-310

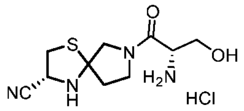
A-311

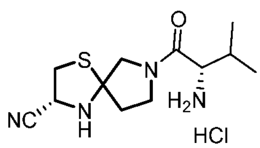
A-312

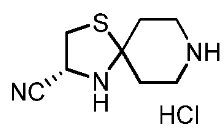
A-313

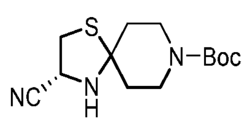
A-314

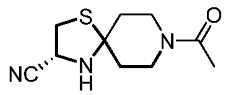

**A-315**

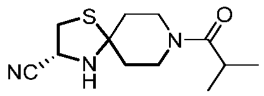

**A-316**

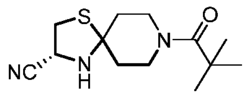

**A-317**

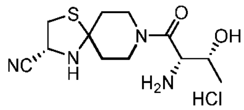

**A-318**

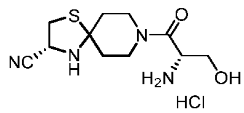

**A-319**

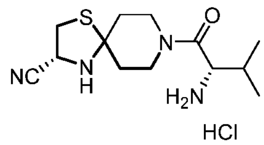

**A-320**

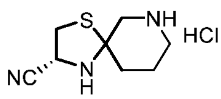

**A-321**

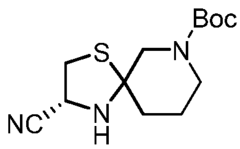

**A-322**

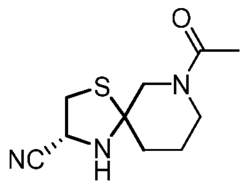

**A-323**

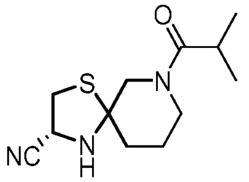

**A-324**

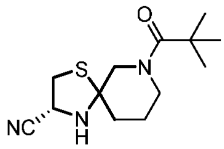

**A-325**

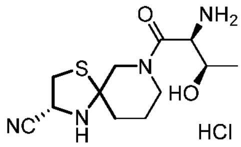

**A-326**

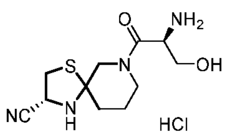

**A-327**

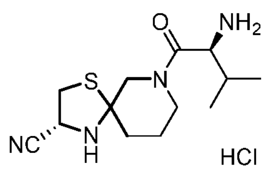

**A-328**

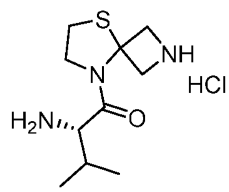

**A-329**

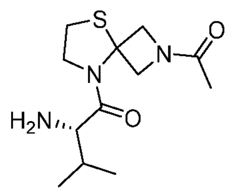

**A-330**

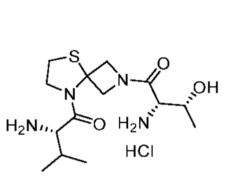

**A-331**

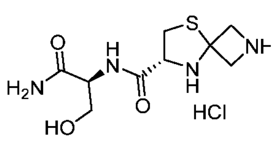

**A-332**

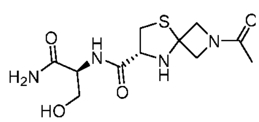

**A-333**

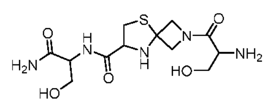

**A-334**

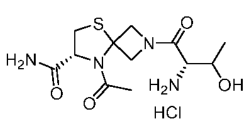

**A-335**

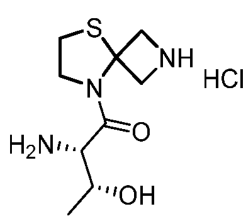

**A-336**

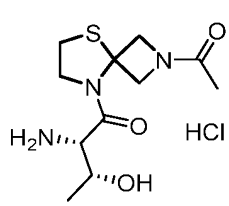

**A-337**

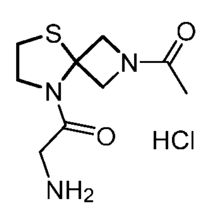

**A-338**

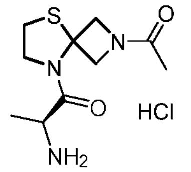

**A-339**

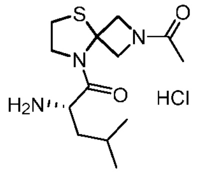

**A-340**

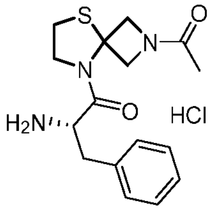

**A-341**

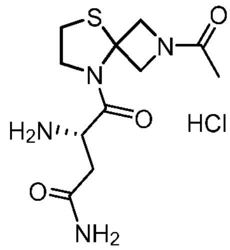

**A-342**

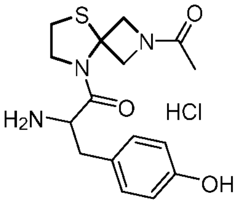

**A-343**

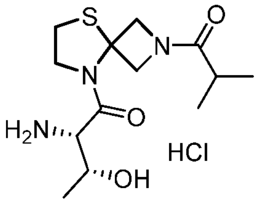

**A-344**

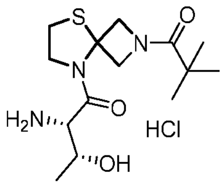

**A-345**

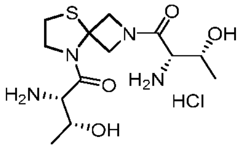

**A-346**

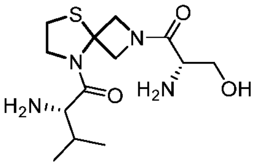

**A-347**

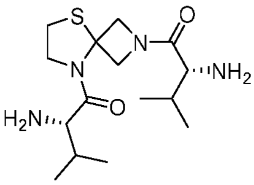

**A-348**

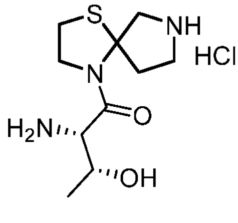

**A-349**

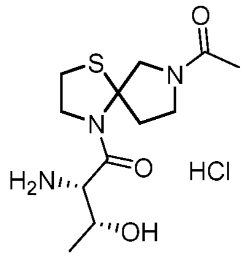

**A-350**

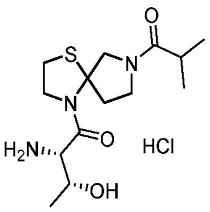

**A-351**

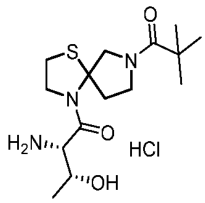

**A-352**

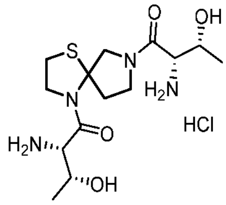

**A-353**

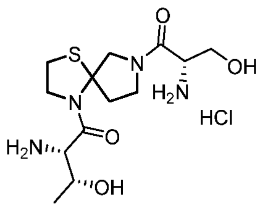

**A-354**

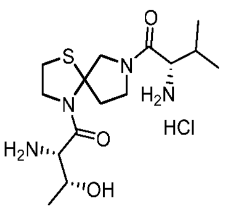

**A-355**

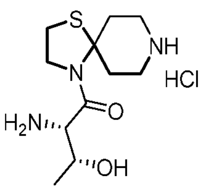

**A-356**

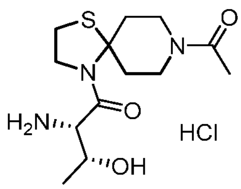

**A-357**

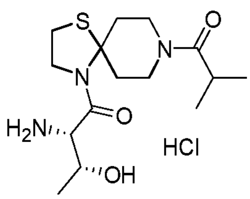

**A-358**

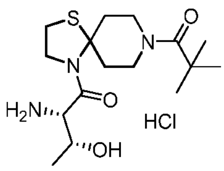

**A-359**

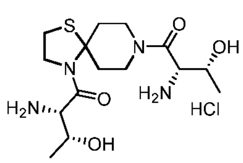

**A-360**

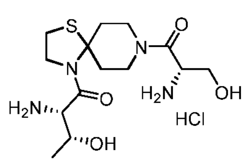

**A-361**

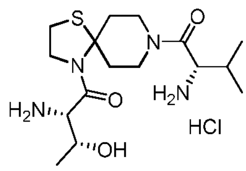

**A-362**

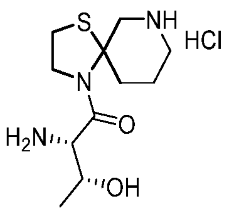

**A-363**

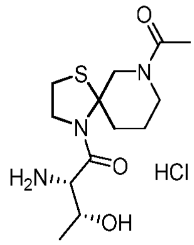

**A-364**

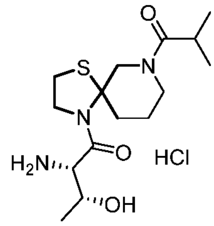

**A-365**

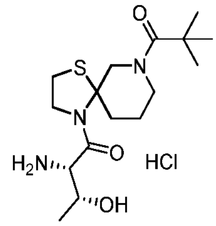

**A-366**

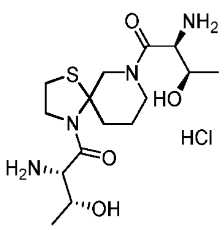

**A-367**

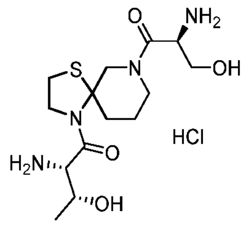

**A-368**

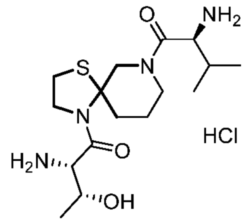

**A-369**

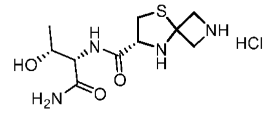

**A-370**

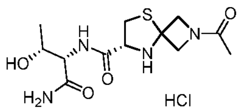

**A-371**

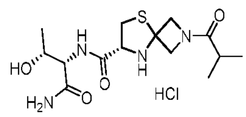

**A-372**

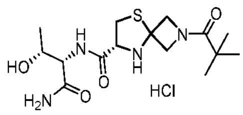

**A-373**

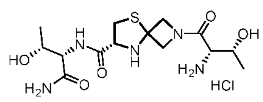

**A-374**

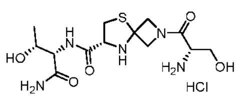

**A-375**

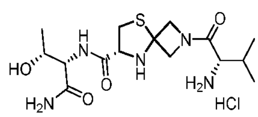

**A-376**

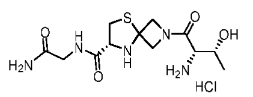

**A-377**

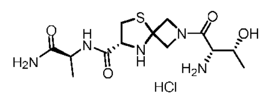

**A-378**

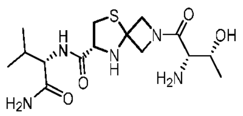

**A-379**

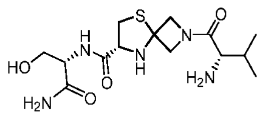

**A-380**

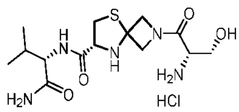

**A-381**

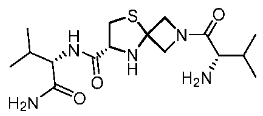

**A-382**

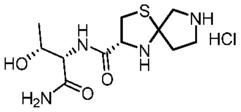

**A-383**

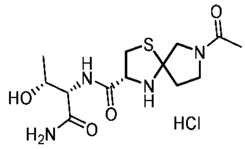

**A-384**

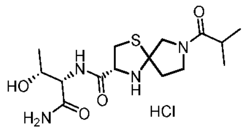

**A-385**

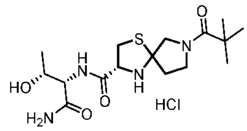

**A-386**

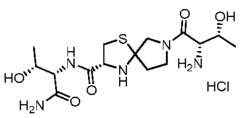

**A-387**

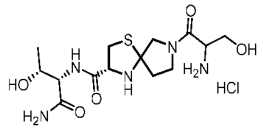

**A-388**

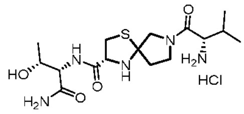

**A-389**

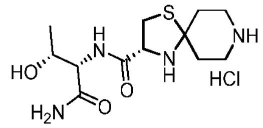

**A-390**

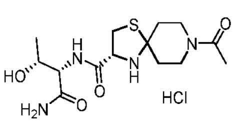

**A-391**

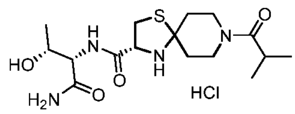

**A-392**

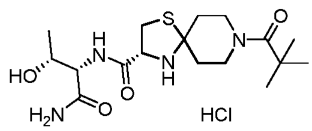

**A-393**

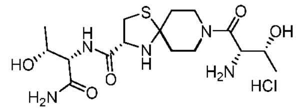

**A-394**

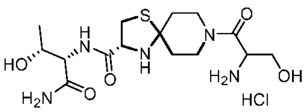

**A-395**

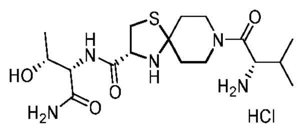

**A-396**

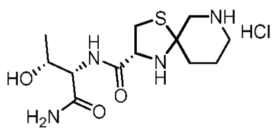

**A-397**

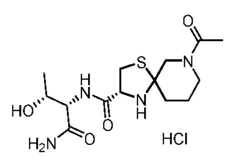

**A-398**

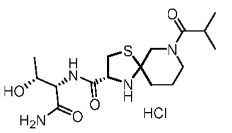

**A-399**

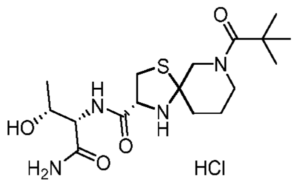

**A-400**

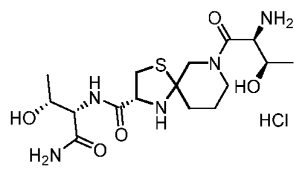

**A-401**

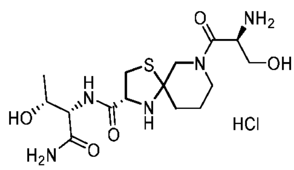

**A-402**

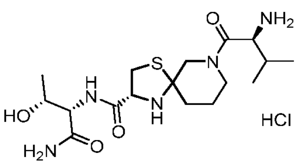

**A-403**

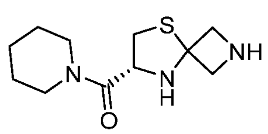

**A-404**

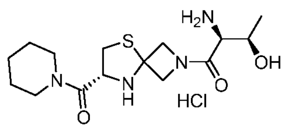

**A-405**

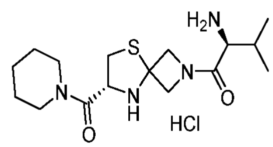

**A-406**

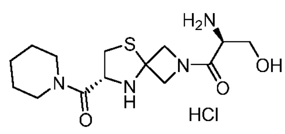

A-407

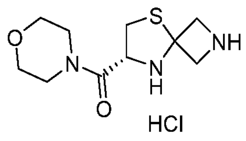

A-408

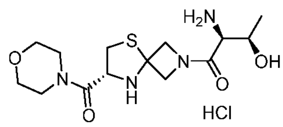

A-409

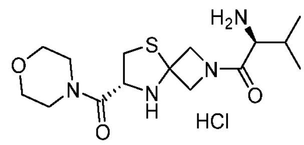

A-410

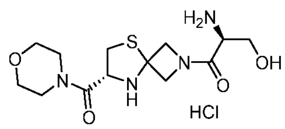

A-411

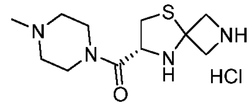

A-412

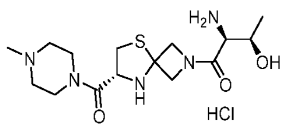

A-413

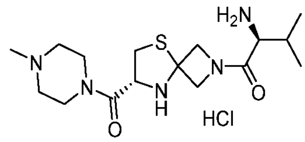

A-414

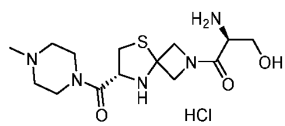

A-415

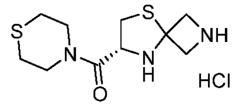

A-416

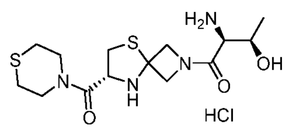

A-417

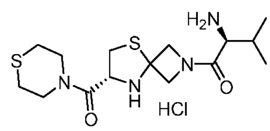

A-418

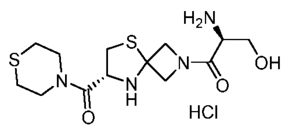

A-419

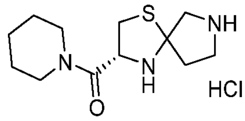

A-420

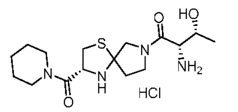

A-421

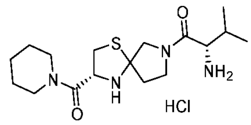

A-422

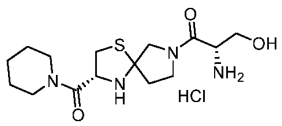

A-423

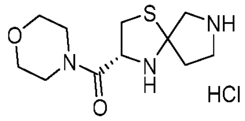

A-424

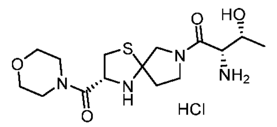

A-425

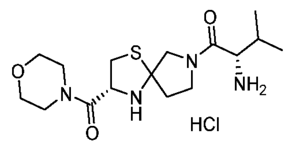

A-426

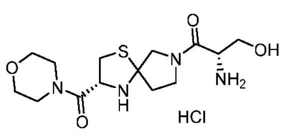

A-427

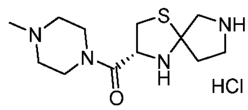

A-428

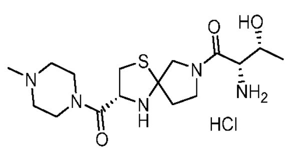

A-429

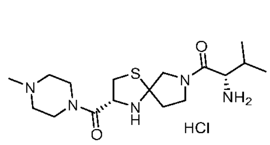

A-430

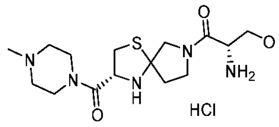

**A-431**

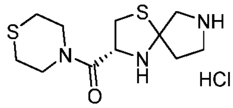

**A-432**

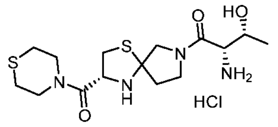

**A-433**

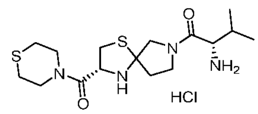

**A-434**

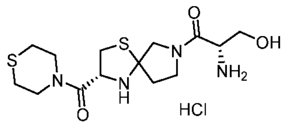

**A-435**

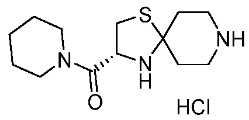

**A-436**

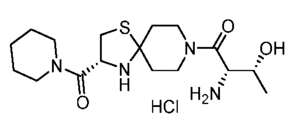

**A-437**

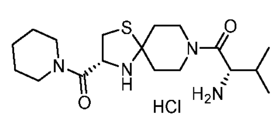

**A-438**

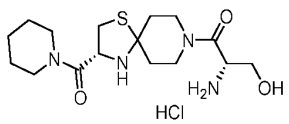

**A-439**

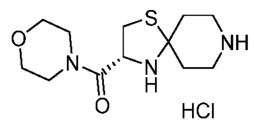

**A-440**

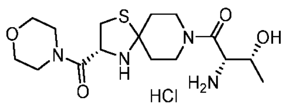

**A-441**

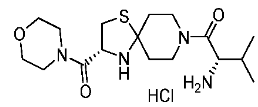

**A-442**

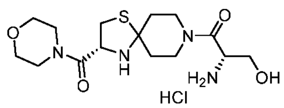

**A-443**

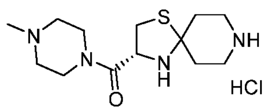

**A-444**

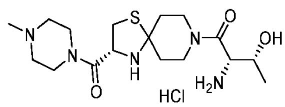

**A-445**

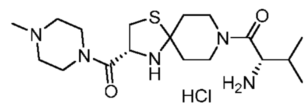

**A-446**

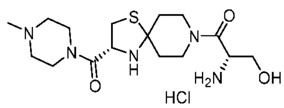

**A-447**

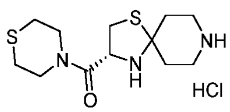

**A-448**

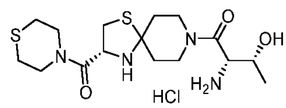

**A-449**

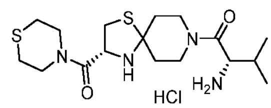

**A-450**

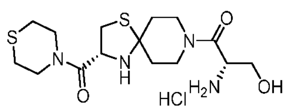

**A-451**

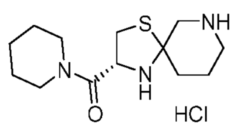

**A-452**

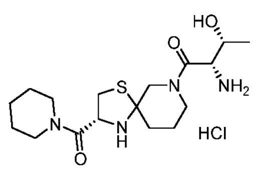

**A-453**

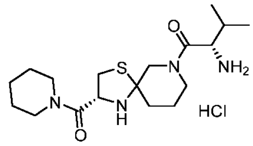

**A-454**

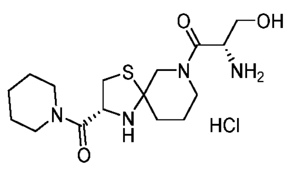

**A-455**

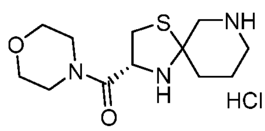

**A-456**

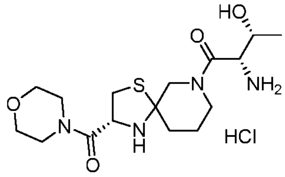

**A-457**

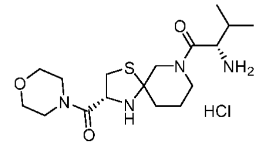

**A-458**

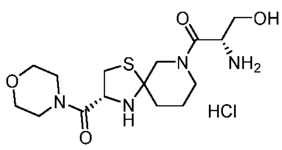

**A-459**

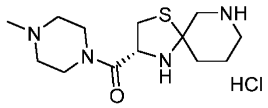

**A-460**

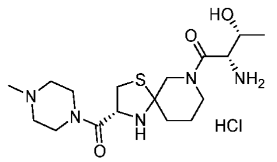

**A-461**

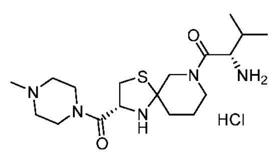

**A-462**

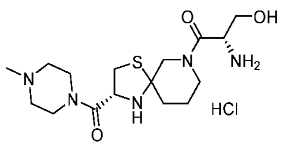

**A-463**

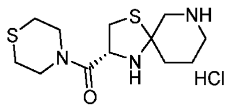

**A-464**

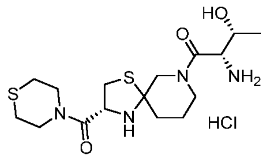

**A-465**

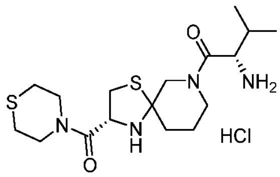

**A-466**

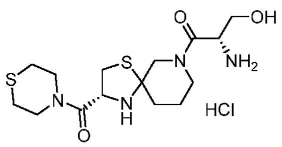

**A-467**

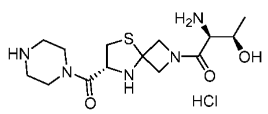

**A-468**

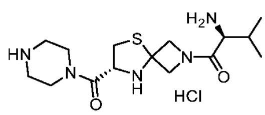

**A-469**

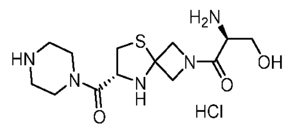

**A-470**

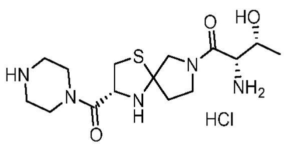

**A-471**

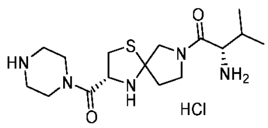

**A-472**

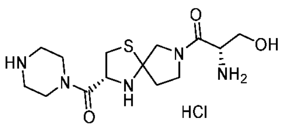

**A-473**

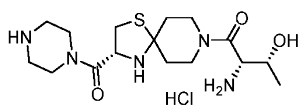

**A-474**

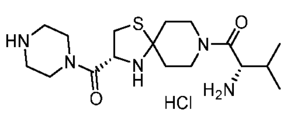

**A-475**

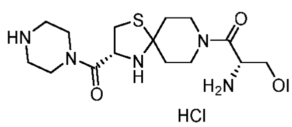

**A-476**

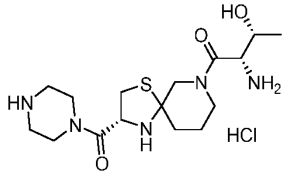

**A-477**

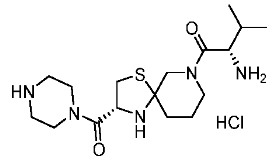

**A-478**

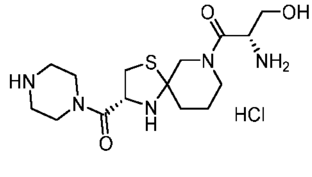

**A-479**

8. The compound of the formula I according to any one of claims 1 to 7, a pharmaceutically acceptable salt or ester, stereoisomers or solvate thereof, wherein the said solvate is a hydrate.

9. A pharmaceutical composition comprising an effective amount of the compound of the formula I according to any one of claims 1 to 8, a pharmaceutically acceptable salt or ester, stereoisomer or solvate thereof;

optionally, the pharmaceutical composition also comprises one or more pharmaceutically acceptable excipients.

**10.** Use of the compound of the formula I according to any one of claims 1 to 8, pharmaceutically acceptable salt or ester, stereoisomer or solvate thereof in preparation of medicaments for treatment and/or prevention of depression, anxiety, stroke, Huntington's disease, Alzheimer's disease, neuralgia or schizophrenia.

**11.** Use of the compound of the formula I according to any one of claims 1 to 8, pharmaceutically acceptable salt or ester, stereoisomer or solvate thereof in preparation of medicaments for in-vivo or in-vitro regulation (e.g., up- or down-regulation) of the activity of an NMDA receptor (e.g., human NMDA receptor).

**12.** The compound of the formula I according to any one of claims 1 to 8, pharmaceutically acceptable salt or ester, stereoisomer or solvate thereof for use in treatment and/or prevention of depression, anxiety, stroke, Huntington's disease, Alzheimer's disease, neuralgia or schizophrenia.

**13.** The compound of the formula I according to any one of claims 1 to 8, pharmaceutically acceptable salt or ester, stereoisomer or solvate thereof for use in in-vivo or in-vitro regulation (e.g., up- or down-regulation) of the activity of an NMDA receptor (e.g., human NMDA receptor).

**14.** A method for preventing and/or treating depression, anxiety, stroke, Huntington's disease, Alzheimer's disease, neuralgia or schizophrenia in subjects, including administering an effective amount of the compound of the formula I according to any one of claims 1 to 8, pharmaceutically acceptable salt or ester, stereoisomer or solvate thereof, or an effective amount of the pharmaceutical composition according to claim 9 to the subjects in need.

**15.** A method for regulating activity of NMDA receptor in vivo or in vitro, including providing a subject, a mammalian cell or an NMDA receptor (preferably a human NMDA receptor) with an effective amount of the compound of the formula I according to any one of claims 1 to 8, pharmaceutically acceptable salt or ester, stereoisomer or solvate thereof; preferably, the regulation is up-regulation or down-regulation.

**INTERNATIONAL SEARCH REPORT**

International application No.
**PCT/CN2021/093264**

**A. CLASSIFICATION OF SUBJECT MATTER**

C07D 513/10(2006.01)i; A61K 31/425(2006.01)i; A61P 25/24(2006.01)i; A61P 25/22(2006.01)i; A61P 25/28(2006.01)i; A61P 25/00(2006.01)i

According to International Patent Classification (IPC) or to both national classification and IPC

**B. FIELDS SEARCHED**

Minimum documentation searched (classification system followed by classification symbols)

C07D513/-, A61K31/-, A61P25/-

Documentation searched other than minimum documentation to the extent that such documents are included in the fields searched

Electronic data base consulted during the international search (name of data base and, where practicable, search terms used)

CNPAT, CNKI, WPI, EPODOC, ISI Web of Knowledge, STN: 北京广为医药科技有限公司, 螺环, 噻唑烷, 哌啶, 吡咯, NMDA受体, 抑郁, 焦虑, 紧张, 神经, spirocyclic, thiazolidine, piperidine, pyrrole, NMDA receptor, depression, anxiety, nervousness。

**C. DOCUMENTS CONSIDERED TO BE RELEVANT**

| Category* | Citation of document, with indication, where appropriate, of the relevant passages | Relevant to claim No. |
|---|---|---|
| X | "STN REGISTRY"<br>*CAS RN 1214842-69-9 et al.,* 25 November 2019 (2019-11-25), | 1-5, 7, 12, 13 |
| A | CN 105408336 A (APTINYX INC.) 16 March 2016 (2016-03-16)<br>the abstract, claim 1, and description, table 1 | 1-15 |
| A | CN 109937204 A (APTINYX INC.) 25 June 2019 (2019-06-25)<br>the abstract, and claims 1 and 4 | 1-15 |
| A | CN 1662506 A (ISRAEL INSTITUTE FOR BIOLOGICAL RESEARCH) 31 August 2005 (2005-08-31)<br>the abstract, claim 1, and description, page 20, paragraph 1 | 1-15 |
| A | CN 1244528 A (F. HOFFMANN-LA ROCHE & CO., AG) 16 February 2000 (2000-02-16)<br>the abstract, claim 1, and description, page 9, paragraph 3 | 1-15 |
| A | CN 1129942 A (ISRAEL INSTITUTE FOR BIOLOGICAL RESEARCH) 28 August 1996 (1996-08-28)<br>the abstract, claim 1, and description, page 24, paragraph 1 | 1-15 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **19 July 2021** | **12 August 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/093264**

**C. DOCUMENTS CONSIDERED TO BE RELEVANT**

| Category* | Citation of document, with indication, where appropriate, of the relevant passages | Relevant to claim No. |
|---|---|---|
| A | CN 110198944 A (PFIZER INC.) 03 September 2019 (2019-09-03)<br>the abstract, and claim 1 | 1-15 |

Form PCT/ISA/210 (second sheet) (January 2015)

**INTERNATIONAL SEARCH REPORT**

International application No.
**PCT/CN2021/093264**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **14-15**
because they relate to subject matter not required to be searched by this Authority, namely:

[1] This written opinion is made on the basis of the subject matter of claims 14 and 15 being "the application of a compound or composition in the preparation of a pharmaceutical product".

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2021/093264**

| Patent document cited in search report | | | Publication date (day/month/year) | Patent family member(s) | | | Publication date (day/month/year) |
|---|---|---|---|---|---|---|---|
| CN | 105408336 | A | 16 March 2016 | EP | 2951182 | B1 | 18 March 2020 |
| | | | | US | 9828384 | B2 | 28 November 2017 |
| | | | | US | 2015368252 | A1 | 24 December 2015 |
| | | | | NZ | 709872 | A | 25 September 2020 |
| | | | | CL | 2015002122 | A1 | 18 December 2015 |
| | | | | AU | 2014212485 | C1 | 25 October 2018 |
| | | | | US | 2018215767 | A1 | 02 August 2018 |
| | | | | JP | 6531042 | B2 | 12 June 2019 |
| | | | | HK | 1218418 | A1 | 17 February 2017 |
| | | | | WO | 2014120784 | A1 | 07 August 2014 |
| | | | | AU | 2014212485 | A1 | 30 July 2015 |
| | | | | PH | 12015501596 | A1 | 19 October 2015 |
| | | | | IL | 240162 | A | 31 August 2020 |
| | | | | JP | 2016506959 | A | 07 March 2016 |
| | | | | CN | 105408336 | B | 26 June 2018 |
| | | | | EP | 2951182 | A1 | 09 December 2015 |
| | | | | MX | 2015009772 | A | 31 May 2016 |
| | | | | PE | 20151427 | A1 | 10 October 2015 |
| | | | | KR | 20150110788 | A | 02 October 2015 |
| | | | | US | 2018179218 | A1 | 28 June 2018 |
| | | | | EA | 201591402 | A1 | 30 December 2015 |
| | | | | SG | 11201505942Y | A | 28 August 2015 |
| | | | | AU | 2014212485 | B2 | 19 April 2018 |
| | | | | EA | 032153 | B1 | 30 April 2019 |
| | | | | IL | 240162 | D0 | 24 September 2015 |
| | | | | US | 2018244680 | A1 | 30 August 2018 |
| | | | | BR | 112015018089 | A2 | 18 July 2017 |
| | | | | CA | 2898774 | A1 | 07 August 2014 |
| CN | 109937204 | A | 25 June 2019 | SG | 11201900443V | A | 27 February 2019 |
| | | | | CA | 3031537 | A1 | 08 February 2018 |
| | | | | BR | 112019001768 | A2 | 11 June 2019 |
| | | | | AU | 2017306152 | A1 | 31 January 2019 |
| | | | | US | 2019177334 | A1 | 13 June 2019 |
| | | | | CO | 2019000945 | A2 | 08 February 2019 |
| | | | | JP | 2019527233 | A | 26 September 2019 |
| | | | | KR | 20190033595 | A | 29 March 2019 |
| | | | | PE | 20190503 | A1 | 10 April 2019 |
| | | | | SG | 10202101055V | A | 30 March 2021 |
| | | | | WO | 2018026779 | A1 | 08 February 2018 |
| | | | | PH | 12019500202 | A1 | 29 July 2019 |
| | | | | MX | 2019001319 | A | 04 July 2019 |
| | | | | EP | 3490992 | A1 | 05 June 2019 |
| | | | | IL | 264514 | D0 | 28 February 2019 |
| | | | | EA | 201990424 | A1 | 30 August 2019 |
| | | | | CL | 2019000248 | A1 | 21 June 2019 |
| CN | 1662506 | A | 31 August 2005 | AU | 2003223093 | A1 | 17 November 2003 |
| | | | | MX | PA04010921 | A | 27 May 2005 |
| | | | | IL | 164857 | A | 30 December 2010 |
| | | | | US | 2006052406 | A1 | 09 March 2006 |
| | | | | EP | 1507765 | A4 | 02 November 2006 |

Form PCT/ISA/210 (patent family annex) (January 2015)

**INTERNATIONAL SEARCH REPORT**
**Information on patent family members**

International application No.
**PCT/CN2021/093264**

| Patent document cited in search report | Publication date (day/month/year) | Patent family member(s) | Publication date (day/month/year) |
|---|---|---|---|
| | | AR 039965 A1 | 09 March 2005 |
| | | US 2008306103 A1 | 11 December 2008 |
| | | BR 0309875 A | 10 April 2007 |
| | | TW I330640 B | 21 September 2010 |
| | | IL 164857 D0 | 18 December 2005 |
| | | JP 4759649 B2 | 31 August 2011 |
| | | JP 4584706 B2 | 24 November 2010 |
| | | WO 03092580 B1 | 24 June 2004 |
| | | KR 100998804 B1 | 06 December 2010 |
| | | NZ 560368 A | 28 November 2008 |
| | | US 7439251 B2 | 21 October 2008 |
| | | KR 20050025168 A | 11 March 2005 |
| | | EP 1507765 A2 | 23 February 2005 |
| | | NZ 536610 A | 28 February 2009 |
| | | BR PI0309875 A | 10 April 2007 |
| | | US 7049321 B2 | 23 May 2006 |
| | | TW 200404808 A | 01 April 2004 |
| | | KR 101027570 B1 | 06 April 2011 |
| | | CA 2484599 A1 | 13 November 2003 |
| | | WO 03092580 A3 | 06 May 2004 |
| | | JP 2010184946 A | 26 August 2010 |
| | | WO 03092580 A2 | 13 November 2003 |
| | | KR 20100075700 A | 02 July 2010 |
| | | ZA 200408861 B | 26 July 2006 |
| | | AU 2003223093 B2 | 04 February 2010 |
| | | ZA 200408861 A | 26 July 2006 |
| | | JP 2005530742 A | 13 October 2005 |
| | | US 2004044018 A1 | 04 March 2004 |
| CN 1244528 A | 16 February 2000 | CZ 9902039 A3 | 15 March 2000 |
| | | TR 9901380 A2 | 22 January 2001 |
| | | PE 20000540 A1 | 15 July 2000 |
| | | HU 9901910 A3 | 28 November 2001 |
| | | PT 963985 T | 30 June 2003 |
| | | NO 312591 B1 | 03 June 2002 |
| | | TW 434241 B | 16 May 2001 |
| | | US 2003176701 A1 | 18 September 2003 |
| | | CN 1229377 C | 30 November 2005 |
| | | HR P990187 A2 | 29 February 2000 |
| | | EP 0963985 A3 | 24 January 2001 |
| | | BR 9902653 A | 02 May 2000 |
| | | DE 69905798 D1 | 17 April 2003 |
| | | MA 26645 A1 | 20 December 2004 |
| | | NO 992876 L | 13 December 1999 |
| | | EP 0963985 A2 | 15 December 1999 |
| | | YU 26799 A | 18 March 2002 |
| | | KR 100393311 B1 | 02 August 2003 |
| | | PT 963985 E | 30 June 2003 |
| | | PL 333693 A1 | 20 December 1999 |
| | | NZ 336226 A | 27 October 2000 |
| | | AT 234302 T | 15 March 2003 |

Form PCT/ISA/210 (patent family annex) (January 2015)

**INTERNATIONAL SEARCH REPORT**
**Information on patent family members**

International application No.
**PCT/CN2021/093264**

| Patent document cited in search report | | | Publication date (day/month/year) | Patent family member(s) | | | Publication date (day/month/year) |
|---|---|---|---|---|---|---|---|
| | | | | US | 6642247 | B2 | 04 November 2003 |
| | | | | CZ | 203999 | A3 | 15 March 2000 |
| | | | | NO | 992876 | A | 13 December 1999 |
| | | | | ZA | 993901 | B | 13 December 1999 |
| | | | | ZA | 993853 | B | 13 December 1999 |
| | | | | TR | 199901380 | A2 | 22 January 2001 |
| | | | | ES | 2194402 | T3 | 16 November 2003 |
| | | | | CA | 2274201 | C | 09 February 2010 |
| | | | | JP | 3369123 | B2 | 20 January 2003 |
| | | | | HU | 9901910 | D0 | 30 August 1999 |
| | | | | TR | 199901380 | A3 | 22 January 2001 |
| | | | | NO | 992876 | D0 | 11 June 1999 |
| | | | | DE | 69905798 | T2 | 12 February 2004 |
| | | | | AU | 752835 | B2 | 03 October 2002 |
| | | | | IL | 130375 | D0 | 01 June 2000 |
| | | | | JP | 2000026466 | A | 25 January 2000 |
| | | | | CN | 1110497 | C | 04 June 2003 |
| | | | | ZA | 9903901 | A | 13 December 1999 |
| | | | | ZA | 9903853 | A | 13 December 1999 |
| | | | | ID | 23306 | A | 05 April 2000 |
| | | | | CA | 2274201 | A1 | 12 December 1999 |
| | | | | DK | 0963985 | T3 | 23 June 2003 |
| | | | | CO | 5040061 | A1 | 29 May 2001 |
| | | | | EP | 0963985 | B1 | 12 March 2003 |
| | | | | SG | 83135 | A1 | 18 September 2001 |
| | | | | CN | 1242367 | A | 26 January 2000 |
| | | | | CZ | 291323 | B6 | 12 February 2003 |
| CN | 1129942 | A | 28 August 1996 | DK | 0711292 | T3 | 06 May 2002 |
| | | | | DE | 69429724 | T2 | 29 August 2002 |
| | | | | EP | 0711292 | B1 | 23 January 2002 |
| | | | | PL | 178931 | B1 | 30 June 2000 |
| | | | | WO | 9503303 | A2 | 02 February 1995 |
| | | | | ES | 2171458 | T3 | 16 September 2002 |
| | | | | PT | 711292 | E | 31 July 2002 |
| | | | | BG | 100370 | A | 29 November 1996 |
| | | | | CZ | 295929 | B6 | 14 December 2005 |
| | | | | BR | 9407088 | A | 13 August 1996 |
| | | | | AT | 212347 | T | 15 February 2002 |
| | | | | BG | 62774 | B1 | 31 July 2000 |
| | | | | IL | 110340 | D0 | 21 October 1994 |
| | | | | JP | 3802050 | B2 | 26 July 2006 |
| | | | | ZA | 9405284 | A | 12 October 1995 |
| | | | | US | 5534520 | A | 09 July 1996 |
| | | | | PT | 711292 | T | 31 July 2002 |
| | | | | NO | 960225 | D0 | 18 January 1996 |
| | | | | IL | 110340 | A | 20 June 1999 |
| | | | | DE | 69429724 | D1 | 14 March 2002 |
| | | | | RO | 117851 | B1 | 30 August 2002 |
| | | | | CN | 1129942 | B | 10 August 2011 |
| | | | | HU | 224638 | B1 | 28 December 2005 |

Form PCT/ISA/210 (patent family annex) (January 2015)

**INTERNATIONAL SEARCH REPORT**
**Information on patent family members**

International application No.
**PCT/CN2021/093264**

| Patent document cited in search report | | | Publication date (day/month/year) | Patent family member(s) | | | Publication date (day/month/year) |
|---|---|---|---|---|---|---|---|
| | | | | EP | 0711292 | A1 | 15 May 1996 |
| | | | | FI | 113777 | B | 15 June 2004 |
| | | | | FI | 960238 | A0 | 17 January 1996 |
| | | | | KR | 100341683 | B1 | 30 November 2002 |
| | | | | PL | 312684 | A1 | 13 May 1996 |
| | | | | JP | H09503491 | A | 08 April 1997 |
| | | | | AU | 7191594 | A | 20 February 1995 |
| | | | | NO | 310075 | B1 | 14 May 2001 |
| | | | | HU | T74588 | A | 28 January 1997 |
| | | | | CA | 2167584 | C | 24 October 2006 |
| | | | | CZ | 18296 | A3 | 17 July 1996 |
| | | | | NO | 960225 | L | 12 March 1996 |
| | | | | KR | 960703914 | A | 31 August 1996 |
| | | | | ZA | 945284 | B | 12 October 1995 |
| | | | | CZ | 9600182 | A3 | 17 July 1996 |
| | | | | FI | 960238 | A | 18 March 1996 |
| | | | | RU | 2129555 | C1 | 27 April 1999 |
| | | | | NO | 960225 | A | 12 March 1996 |
| | | | | AU | 696530 | B2 | 10 September 1998 |
| | | | | HU | 9600126 | D0 | 28 March 1996 |
| | | | | NZ | 268637 | A | 26 January 1998 |
| | | | | WO | 9503303 | A3 | 02 March 1995 |
| | | | | CA | 2167584 | A1 | 02 February 1995 |
| CN | 110198944 | A | 03 September 2019 | WO | 2018134698 | A1 | 26 July 2018 |
| | | | | BR | 112019014099 | A2 | 11 February 2020 |
| | | | | TW | I665199 | B | 11 July 2019 |
| | | | | AU | 2018208848 | A1 | 18 July 2019 |
| | | | | EP | 3571202 | A1 | 27 November 2019 |
| | | | | US | 2018208608 | A1 | 26 July 2018 |
| | | | | JP | 2020506903 | A | 05 March 2020 |
| | | | | US | 2019292203 | A1 | 26 September 2019 |
| | | | | SG | 11201906427Q | A | 27 August 2019 |
| | | | | US | 10858373 | B2 | 08 December 2020 |
| | | | | MX | 2019008690 | A | 18 September 2019 |
| | | | | IL | 267781 | D0 | 26 September 2019 |
| | | | | CA | 3050853 | A1 | 26 July 2018 |
| | | | | KR | 20190097242 | A | 20 August 2019 |
| | | | | TW | 201838995 | A | 01 November 2018 |
| | | | | RU | 2726631 | C1 | 15 July 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **STAHL ; WERMUTH'S.** Handbook of Pharmaceutical Salts: Properties, Selection, and Use. Wiley-VCH, 2002
- **S. M. BERGE et al.** Pharmaceutical Salts. *J. Pharm. Sci.,* 1977, vol. 66, 1-19